# EUROPEAN PATENT SPECIFICATION

(11) **EP 2 646 436 B1**
(45) Date of publication and mention of the grant of the patent: **27.05.2015**
(21) Application number: 11794237.5
(22) Date of filing: 15.11.2011
(51) Int. Cl.: C07D 417/14, A61K 31/427, A61K 31/4439, A61P 31/04

(54) **MONOBACTAMS**
MONOBACTAME
MONOBACTAMES

(30) Priority: 29.11.2010 US 417575 P
(43) Date of publication of application: 09.10.2013
(73) Proprietor: Pfizer Inc., New York, NY 10017 (US)
(72) Inventor: BROWN, Matthew, Frank, Connecticut 06340 (US); MITTON-FRY, Mark. J., Granville Ohio 43023 (US); HAN, Seungil, Connecticut 06340 (US); LALL, Manjinder, Connecticut 06340 (US); PLUMMER, Mark, Stephen, Westbrook Connecticut 06498 (US); RISLEY, Hud, Lawrence, Connecticut 06340 (US); SHANMUGASUNDARAM, Veerabahu, Connecticut 06340 (US); STARR, Jeremy, T., Connecticut 06340 (US)
(74) Representative: Pfizer
(86) International application number: PCT/IB2011/055104
(87) International publication number: WO 2012/073138

(56) References cited:
- WO-A1-2010/070523

## Description

### Field of the Invention

This invention relates to novel monobactam derivatives that are useful for the treatment of bacterial infections, especially Gram-negative infections. There are also disclosed methods of using such compounds in the treatment of bacterial infections and to pharmaceutical compositions and pharmaceutical combinations containing such compounds.

### Background of the Invention

Monobactams are a class of antibacterial agents which contain a monocyclic beta-lactam ring as opposed to a beta-lactam fused to an additional ring which is found in other beta-lactam classes, such as cephalosporins, carbapenems and penicillins. The drug Aztreonam is an example of a marketed monobactam; Carumonam is another example. The early studies in this area were conducted by workers at the Squibb Institute for Medical Research, Cimarusti, C. M. & R.B. Sykes: Monocyclic β-lactam antibiotics. Med. Res. Rev. 1984, 4, 1-24. Despite the fact that selected monobacatams were discovered over 25 years ago, there remains a continuing need for new antibiotics to counter the growing number of resistant organisms.

Although not limiting to the present invention, it is believed that monobactams of the present invention exploit the iron uptake mechanism in bacteria through the use of siderophore-monobactam conjugates. For background information, see: M. J. Miller, et al. BioMetals (2009), 22(1), 61-75.

The mechanism of action of beta-lactam antibiotics, including monobactams, is generally known to those skilled in the art and involves inhibition of one or more penicillin binding proteins (PBPs), although the present invention is not bound or limited by any theory. PBPs are involved in the synthesis of peptidoglycan, which is a major component of bacterial cell walls.

### Summary of the Invention

A new class of monobactams has been discovered. These compounds or their pharmaceutically acceptable salts are represented by Formula (I) below: 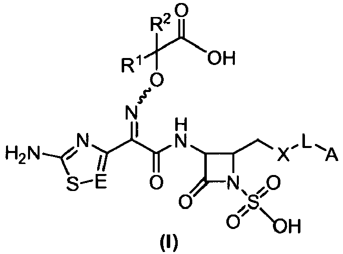 wherein
R¹ and R² are each independently hydrogen, optionally substituted (C₁-C₆)alkyl, or phenyl(C₁-C₆)alkyl wherein the phenyl and the (C₁-C₆)alkyl moieties of the phenyl(C₁-C₆)alkyl are optionally substituted; or
R¹ and R² together, with the carbon atom to which they are attached, form an optionally substituted (C₃-C₆)cycloalkyl or an optionally substituted 4-6-membered heterocycle;
E is C(H), C(F), C(CI), or N;
X is -O-C(=O)-, -NH-C(=O)-, -NH-SO₂-, -NH-C(=N-CN)-, -NH-T-, or triazole;
L is absent, -(CH₂)ₚ-, -(CH₂)ₚ-NH-(CH₂)_{q}-, -(CH₂)ₚ-O-(CH₂)_{q}-, -(CH₂)ₚ-C(=O)-NH-(CH₂)_{q}-, -(CH₂)ₚ-NH-C(=O)-(CH₂)_{q}-, -CH(CH₃)-NH-C(=O)-(CH₂)_{q}-, -(CH₂)ₚ-NH-C(=O)-NH-(CH₂)_{q}-, -CH(CH₃)-NH-C(=O)-NH-(CH₂)_{q}-, -(CH₂)ₚ-T-SO₂-NH-(CH₂)_{q}-, -(CH₂)ₚ-T-(CH₂)_{q}, -(CH₂)ₚ-T-C(=O)-NH-(CH₂)_{q}-, -(CH₂)ₚ-T-(CH₂)_{q}-NH-C(=O)-, -NH-(CH₂)ₚ-T-, -O-(CH₂)ₚ-T-, -(CH₂)ₚ-Y-C(=O)-(CH₂)_{q}-, or -(CH₂)ₚ-Y-(CH₂)_{q}-;
T is an optionally substituted phenyl or an optionally substituted 5- or 6-membered heteroaryl;
Y is an optionally substituted 4-6 membered heterocycle;
p and q are each independently 0, 1, 2, or 3;
A is 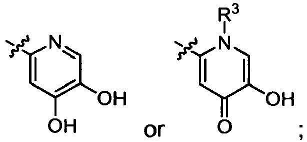 and
R³ is hydrogen, (C₁-C₃)alkyl, or OH;
provided that Formula (I) does not include 2-(((1-(2-aminothiazol-4-yl)-2-(((2S,3R)-2-((3-((1,5-dihydroxy-4-oxo-1,4-dihydropyridin-2-yl)methyl)ureido)methyl)-4-oxo-1-sulfoazetidin-3-yl)amino)-2-oxoethylidene)amino)oxy)-2-methylpropanoic acid.

In another aspect, the present invention provides pharmaceutical compositions comprising a compound of Formula (I), or a pharmaceutically acceptable salt thereof, and at least one pharmaceutically acceptable carrier.

There are also disclosed methods of treating bacterial infections in a patient comprising administering to the patient in need of such treatment a therapeutically effective amount of a compound of Formula (I) or a pharmaceutically acceptable salt thereof.

There are also disclosed methods of treating Gram-negative bacterial infections (as well as conditions arising from such infections) that include nosocomial pneumonia, urinary tract infections, systemic infections (bacteremia and sepsis), skin and soft tissue infections, surgical infections, intraabdominal infections, lung infections (including those in patients with cystic fibrosis) in a patient comprising administering to the patient in need of such treatment a therapeutically effective amount of a compound of Formula (I), or a pharmaceutically acceptable salt thereof. Examples of Gram-negative organisms include *Pseudomonas aeruginosa, Klebsiella pneumoniae, Escherichia coli,* and *Acinetobacter baumanii.*

There are also disclosed methods of treating Gram-negative bacterial infections (as well as conditions arising from such infections) including *Helicobacter pylori* (and relief of associated gastric complications such as peptic ulcer disease, gastric carcinogenesis, etc.), endocarditis, diabetic foot infections, osteomyelitis, infections associated with burns or wounds, infections from devices such as catheters, ocular infections, otic infections, and central nervous system infections in a patient comprising administering to the patient in need of such treatment a therapeutically effective amount of a compound of Formula (I), or a pharmaceutically acceptable salt thereof. Examples of Gram-negative organisms include *Pseudomonas aeruginosa, Klebsiella pneumoniae, Escherichia coli, and Acinetobacter baumanii.*

In another aspect, the present invention provides the use of a compound of Formula (I) for the manufacture of a medicament for treating bacterial infections.

In another aspect, the present invention provides the use of a compound of Formula (I) for the manufacture of a medicament for treating Gram-negative bacterial infections (as well as conditions arising from such infections) that include nosocomial pneumonia, urinary tract infections, systemic infections (bacteremia and sepsis), skin and soft tissue infections, surgical infections, intraabdominal infections, lung infections (including those in patients with cystic fibrosis).

In another aspect, the present invention provides the use of a compound of Formula (I) for the manufacture of a medicament for treating Gram-negative bacterial infections (as well as conditions arising from such infections) that include *Helicobacter pylori* (and relief of associated gastric complications such as peptic ulcer disease, gastric carcinogenesis, etc.), endocarditis, diabetic foot infections, osteomyelitis, infections associated with burns or wounds, infections from devices such as catheters, ocular infections, otic infections, and central nervous system infections

### Detailed Description of the Invention

In one aspect, the present invention provides compounds of Formula (IA) 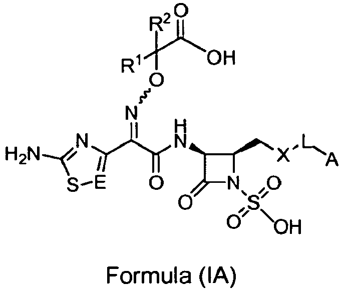 wherein
R¹ and R² are each independently hydrogen, optionally substituted (C₁-C₆)alkyl, or phenyl(C₁-C₆)alkyl wherein the phenyl and the (C₁-C₆)alkyl moieties of the phenyl(C₁-C₆)alkyl are optionally substituted; or
R¹ and R² together, with the carbon atom to which they are attached, form an optionally substituted (C₃-C₆)cycloalkyl or an optionally substituted 4-6-membered heterocycle;
E is C(H), C(F), C(CI), or N;
X is -O-C(=O)-, -NH-C(=O)-, -NH-SO₂-, -NH-C(=N-CN)-, -NH-T-, or triazole;
L is absent, -(CH₂)ₚ-, -(CH₂)ₚ-NH-(CH₂)_{q}-, -(CH₂)ₚ-O-(CH₂)_{q}-, -(CH₂)ₚ-C(=O)-NH-(CH₂)_{q}-, -(CH₂)ₚ-NH-C(=O)-(CH₂)_{q}-, -CH(CH₃)-NH-C(=O)-(CH₂)_{q}-, -(CH₂)ₚ-NH-C(=O)-NH-(CH₂)_{q}-, -CH(CH₃)-NH-C(=O)-NH-(CH₂)_{q}-, -(CH₂)ₚ-T-SO₂-NH-(CH₂)_{q}-, -(CH₂)ₚ-T-(CH₂)_{q}, -(CH₂)ₚ-T-C(=O)-NH-(CH₂)_{q}--(CH₂)ₚ-T-(CH₂)_{q}-NH-C(=O)-, -NH-(CH₂)ₚ-T-, -O-(CH₂)ₚ-T-, -(CH₂)ₚ-Y-C(=O)-(CH₂)_{q}-, or -(CH₂)ₚ-Y-(CH₂)_{q}-;
T is an optionally substituted phenyl or an optionally substituted 5- or 6-membered heteroaryl;
Y is an optionally substituted 4-6 membered heterocycle;
p and q are each independently 0, 1, 2, or 3; 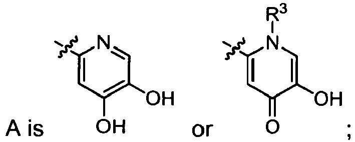 and
R³ is hydrogen, (C₁-C₃)alkyl, or OH.

In another aspect, the present invention provides compounds of Formula (IA) wherein E is C(H); R¹ and R² are independently (C₁-C₆)alkyl; X is -O-C(=O)-; L is -(CH₂)ₚ-NH-C(=O)-(CH₂)_{q}-, -CH(CH₃)-NH-C(=O)-(CH₂)_{q}-, or -(CH₂)ₚ-T-(CH₂)_{q} where T is isoxazole, thiazole, or pyrimidine; p, q, and A are as defined in Formula (IA); and R³ is hydrogen or OH.

In another aspect, the present invention provides compounds of Formula (IA) wherein E is C(H); R¹ and R² are each methyl; X is -O-C(=O)-; L is -(CH₂)ₚ-NH-C(=O)-(CH₂)_{q}-; p, q, and A are as defined in Formula (IA); and R³ is hydrogen or OH.

In another aspect, the present invention provides compounds of Formula (IA) wherein E is C(H); R¹ and R² are each methyl; X is -O-C(=O)-; L is -CH(CH₃)-NH-C(=O)-(CH₂)_{q}-; q and A are as defined in Formula (IA); and R³ is hydrogen or OH.

In another aspect, the present invention provides compounds of Formula (IA) wherein E is C(H); R¹ and R² are each methyl; X is -O-C(=O)-; L is -(CH₂)ₚ-T-(CH₂)_{q} where T is isoxazole, thiazole, or pyrimidine; p, q, and A are as defined in Formula (IA); and R³ is hydrogen or OH.

In another aspect, the present invention provides compounds of Formula (IA) wherein E is C(H); R¹ and R² are independently (C₁-C₆)alkyl; X is triazole; L is absent or -(CH₂)ₚ-O-(CH₂)_{q}-; p, q, and A are as defined in Formula (IA); and R³ is hydrogen or OH.

In another aspect, the present invention provides compounds of Formula (IA) wherein E is C(H); R¹ and R² are each methyl; X is triazole; L is absent; A is as defined in Formula (IA); and R³ is hydrogen or OH.

In another aspect, the present invention provides compounds of Formula (IA) wherein E is C(H); R¹ and R² are each methyl; X is triazole; L is -(CH₂)ₚ-O-(CH₂)_{q}-; p, q, and A are as defined in Formula (IA); and R³ is hydrogen or OH.

In another aspect, the present invention provides compounds of Formula (IA) wherein E is C(H); R¹ and R² are independently (C₁-C₆)alkyl; X is -NH-SO₂-; L is - (CH₂)ₚ-NH-(CH₂)_{q}- or -(CH₂)ₚ-T-C(=O)-NH-(CH₂)_{q}- where T is phenyl; p, q, and A are as defined in Formula (IA); and R³ is hydrogen or OH.

In another aspect, the present invention provides compounds of Formula (IA) wherein E is C(H); R¹ and R² are each methyl; X is -NH-SO₂-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(H); R¹ and R² are each methyl; X is -NH-SO₂-; L is -(CH₂)ₚ-T-C(=O)-NH-(CH₂)_{q}- where T is phenyl; p, q, and A are as defined in Formula (IA); and R³ is hydrogen or OH.

In another aspect, the present invention provides compounds of Formula (IA) wherein E is C(H); R¹ and R² are independently (C₁-C₆)alkyl; X is -NH-T-; L is -(CH₂)ₚ-C(=O)-NH-(CH₂)_{q}-; T is pyridine; p, q, and A are as defined in Formula (IA); and R³ is hydrogen or OH.

In another aspect, the present invention provides compounds of Formula (IA) wherein E is C(H); R¹ and R² are each methyl; X is -NH-T-; L is -(CH₂)ₚ-C(=O)-NH-(CH₂)_{q}-; T is pyridine; p, q, and A are as defined in Formula (IA); and R³ is hydrogen or OH.

In another aspect, the present invention provides compounds of Formula (IA) wherein E is C(H); R¹ and R² are independently (C₁-C₆)alkyl; X is -NH-C(=N-CN)-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(H); R¹ and R² are each methyl; X is -NH-C(=N-CN)-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(H); R¹ and R² are independently (C₁-C₆)alkyl; X is -NH-C(=O)-; L is absent, -(CH₂)ₚ-, -(CH₂)ₚ-NH-(CH₂)_{q}-, -(CH₂)ₚ-O-(CH₂)_{q}, -(CH₂)ₚ-C(=O)-NH-(CH₂)_{q}-, -(CH₂)ₚ-NH-C(=O)-(CH₂)_{q}-, -CH(CH₃)-NH-C(=O)-(CH₂)_{q}-, -CH(CH₃)-NH-C(=O)-NH-(CH₂)_{q}, -(CH₂)ₚ-T-(CH₂)_{q}, -(CH₂)ₚ-T-C(=O)-NH-(CH₂)_{q}-, -(CH₂)ₚ-T-(CH₂)_{q}-NH-C(=O)-, -(CH₂)ₚ-T-SO₂-NH-(CH₂)_{q}-, -NH-(CH₂)ₚ-T-, -O-(CH₂)ₚ-T-, -(CH₂)ₚ-Y-C(=O)-(CH₂)_{q}-, or -(CH₂)ₚ-Y-(CH₂)_{q}-; T is isoxazole, oxazole, pyrimidine, thiazole, or phenyl; p, q, Y, and A are as defined in Formula (IA); and R³ is hydrogen or OH.

In another aspect, the present invention provides compounds of Formula (IA) wherein E is C(H); R¹ and R² are each methyl; X is -NH-C(=O)-; L is absent; A is as defined in Formula (IA); and R³ is hydrogen or OH.

In another aspect, the present invention provides compounds of Formula (IA) wherein E is C(H); R¹ and R² are each methyl; X is -NH-C(=O)-; L is -(CH₂)ₚ-; A is as defined in Formula (IA); and R³ is hydrogen or OH.

In another aspect, the present invention provides compounds of Formula (IA) wherein E is C(H); R¹ and R² are each methyl; X is -NH-C(=O)-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(H); R¹ is hydrogen; R² is isobutyl; X is -NH-C(=O)-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(H); R¹ and R² are each methyl; X is -NH-C(=O)-; L is -(CH₂)ₚ-O-(CH₂)_{q}; p, q, and A are as defined in Formula (IA); and R³ is hydrogen or OH.

In another aspect, the present invention provides compounds of Formula (IA) wherein E is C(H); R¹ and R² are each methyl; X is -NH-C(=O)-; L is -(CH₂)ₚ-C(=O)-NH-(CH₂)_{q}-; p, q, and A are as defined in Formula (IA); and R³ is hydrogen or OH.

In another aspect, the present invention provides compounds of Formula (IA) wherein E is C(H); R¹ and R² are each methyl; X is -NH-C(=O)-; L is -(CH₂)ₚ-NH-C(=O)-(CH₂)_{q}-; p, q, and A are as defined in Formula (IA); and R³ is hydrogen or OH.

In another aspect, the present invention provides compounds of Formula (IA) wherein E is C(H); R¹ and R² are each methyl; X is -NH-C(=O)-; L is -CH(CH₃)-NH-C(=O)-(CH₂)_{q}-; q and A are as defined in Formula (IA); and R³ is hydrogen or OH.

In another aspect, the present invention provides compounds of Formula (IA) wherein E is C(H); R¹ and R² are each methyl; X is -NH-C(=O)-; L is -CH(CH₃)-NH-C(=O)-NH-(CH₂)q-; q and A are as defined in Formula (IA); and R³ is hydrogen or OH.

In another aspect, the present invention provides compounds of Formula (IA) wherein E is C(H); R¹ and R² are each methyl; X is -NH-C(=O)-; L is -(CH₂)ₚ-T-(CH₂)_{q}; T is isoxazole, oxazole, pyrimidine, or thiazole; p, q, and A are as defined in Formula (IA); and R³ is hydrogen or OH.

In another aspect, the present invention provides compounds of Formula (IA) wherein E is C(H); R¹ and R² are each methyl; X is -NH-C(=O)-; L is -(CH₂)ₚ-T-C(=O)-NH-(CH₂)_{q}-; T is phenyl; p, q, and A are as defined in Formula (IA); and R³ is hydrogen or OH.

In another aspect, the present invention provides compounds of Formula (IA) wherein E is C(H); R¹ and R² are each methyl; X is -NH-C(=O)-; L is -(CH₂)ₚ-T-(CH₂)_{q}-NH-C(=O)-; T is phenyl; p, q, and A are as defined in Formula (IA); and R³ is hydrogen or OH.

In another aspect, the present invention provides compounds of Formula (IA) wherein E is C(H); R¹ and R² are each methyl; X is -NH-C(=O)-; L is -(CH₂)ₚ-T-SO₂-NH-(CH₂)_{q}-; T is phenyl; p, q, and A are as defined in Formula (IA); and R³ is hydrogen or OH.

In another aspect, the present invention provides compounds of Formula (IA) wherein E is C(H); R¹ and R² are each methyl; X is -NH-C(=O)-; L is -NH-(CH₂)ₚ-T-; T is isoxazole, oxazole, pyrimidine, or thiazole; p and A are as defined in Formula (IA); and R³ is hydrogen or OH.

In another aspect, the present invention provides compounds of Formula (IA) wherein E is C(H); R¹ and R² are each methyl; X is -NH-C(=O)-; L is -O-(CH₂)ₚ-T-; T is isoxazole, oxazole, pyrimidine, or thiazole; p and A are as defined in Formula (IA); and R³ is hydrogen or OH.

In another aspect, the present invention provides compounds of Formula (IA) wherein E is C(H); R¹ and R² are each methyl; X is -NH-C(=O)-; L is -(CH₂)ₚ-Y-C(=O)-(CH₂)_{q}-; Y is azetidine; p, q, and A are as defined in Formula (IA); and R³ is hydrogen or OH.

In another aspect, the present invention provides compounds of Formula (IA) wherein E is C(H); R¹ and R² are each methyl; X is -NH-C(=O)-; L is -(CH₂)ₚ-Y-(CH₂)_{q}-; Y is azetidine; p, q, and A are as defined in Formula (IA); and R³ is hydrogen or OH.

In another aspect, the present invention provides compounds of Formula (IA) wherein E is C(CI) or N; R¹ and R² are independently (C₁-C₆)alkyl; X is -NH-C(=O)-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(CI) or N; R¹ is hydrogen or methyl; R² is methyl; X is -NH-C(=O)-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(H); R¹ is hydrogen; R² is phenyl(C₁-C₆)alkyl; X is -NH-C(=O)-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(H); R¹ is hydrogen; R² is benzyl; X is -NH-C(=O)-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form an optionally substituted (C₃-C₆)cycloalkyl; X is -O-C(=O)-; L is -(CH₂)ₚ-NH-C(=O)-(CH₂)_{q}-, -CH(CH₃)-NH-C(=O)-(CH₂)_{q}-, or -(CH₂)ₚ-T-(CH₂)_{q} where T is isoxazole, thiazole, or pyrimidine; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -O-C(=O)-; L is -(CH₂)ₚ-NH-C(=O)-(CH₂)_{q}-; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -O-C(=O)-; L is -CH(CH₃)-NH-C(=O)-(CH₂)_{q}-; q and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -O-C(=O)-; L is -(CH₂)ₚ-T-(CH₂)_{q} where T is isoxazole, thiazole, or pyrimidine; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form an optionally substituted (C₃-C₆)cycloalkyl; X is triazole; L is absent or -(CH₂)ₚ-O-(CH₂)_{q}-; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is triazole; L is absent; A is as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is triazole; L is -(CH₂)ₚ-O-(CH₂)_{q}-; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form an optionally substituted (C₃-C₆)cycloalkyl; X is -NH-SO₂-; L is -(CH₂)ₚ-NH-(CH₂)_{q}-or -(CH₂)ₚ-T-C(=O)-NH-(CH₂)_{q}- where T is phenyl; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-SO₂-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-SO₂-; L is -(CH₂)ₚ-T-C(=O)-NH-(CH₂)_{q}- where T is phenyl; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form an optionally substituted (C₃-C₆)cycloalkyl; X is -NH-T-; L is -(CH₂)ₚ-C(=O)-NH-(CH₂)_{q}-; T is pyridine; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-T-; L is -(CH₂)ₚ-C(=O)-NH-(CH₂)_{q}-; T is pyridine; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form an optionally substituted (C₃-C₆)cycloalkyl; X is -NH-C(=N-CN)-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=N-CN)-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form an optionally substituted (C₃-C₆)cycloalkyl; X is -NH-C(=O)-; L is absent, -(CH₂)ₚ-, -(CH₂)ₚ-NH-(CH₂)_{q}-, -(CH₂)ₚ-O-(CH₂)_{q}, -(CH₂)ₚ-C(=O)-NH-(CH₂)_{q}-, -(CH₂)ₚ-NH-C(=O)-(CH₂)_{q}-, -CH(CH₃)-NH-C(=O)-(CH₂)_{q}-, -CH(CH₃)-NH-C(=O)-NH-(CH₂)_{q}-, --(CH₂)ₚ-T-(CH₂)_{q}, -(CH₂)ₚ-T-C(=O)-NH-(CH₂)_{q}-, -(CH₂)ₚ-T-(CH₂)_{q}-NH-C(=O)-, -(CH₂)ₚ-T-SO₂-NH-(CH₂)_{q}-, -NH-(CH₂)ₚ-T-, -O-(CH₂)ₚ-T-, -(CH₂)ₚ-Y-C(=O)-(CH₂)_{q}-, or -(CH₂)ₚ-Y-(CH₂)_{q}-; T is isoxazole, oxazole, pyrimidine, thiazole, or phenyl; p, q, Y, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is absent; A is as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -(CH₂)ₚ-; A is as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -(CH₂)ₚ-O-(CH₂)_{q}; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -(CH₂)ₚ-C(=O)-NH-(CH₂)_{q}-; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -(CH₂)ₚ-NH-C(=O)-(CH₂)_{q}-; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -CH(CH₃)-NH-C(=O)-(CH₂)_{q}-; q and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -CH(CH₃)-NH-C(=O)-NH-(CH₂)_{q}-; q and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -(CH₂)ₚ-T-(CH₂)_{q}; T is isoxazole, oxazole, pyrimidine, or thiazole; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -(CH₂)ₚ-T-C(=O)-NH-(CH₂)_{q}-; T is phenyl; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -(CH₂)ₚ-T-(CH₂)_{q}-NH-C(=O)-; T is phenyl; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -(CH₂)ₚ-T-SO₂-NH-(CH₂)_{q}-; T is phenyl; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -NH-(CH₂)ₚ-T-; T is isoxazole, oxazole, pyrimidine, or thiazole; p and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -O-(CH₂)ₚ-T-; T is isoxazole, oxazole, pyrimidine, or thiazole; p and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -(CH₂)ₚ-Y-C(=O)-(CH₂)_{q}-; Y is azetidine; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form cyclobutyl or cyclopentyl; X is -NH-C(=O)-; L is -(CH₂)ₚ-Y-(CH₂)_{q}-; Y is azetidine; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form an optionally substituted 4-6 membered heterocycle; X is -NH-C(=O)-; L is -(CH₂)ₚ-NH-(CH₂)_{q}-; p, q, and A are as defined in Formula (IA); and R³ is hydrogen or OH.

In another aspect, the present invention provides compounds of Formula (IA) wherein E is C(H); R¹ and R² together, with the carbon atom to which they are attached, form tetrahydropyran; X is -NH-C(=O)-; L is -(CH₂)ₚ-NH-(CH₂)_{q}-; p, q, and A are as defined in Formula (IA); and R³ is hydrogen or OH.

In another aspect, the present invention provides the compound 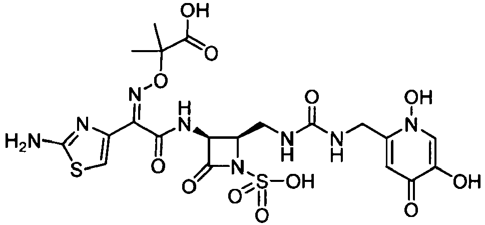 or a pharmaceutically acceptable salt thereof.

In another aspect, the present invention provides the compound 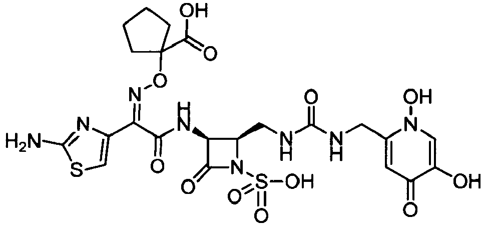 or a pharmaceutically acceptable salt thereof.

In another aspect, the present invention provides the compound 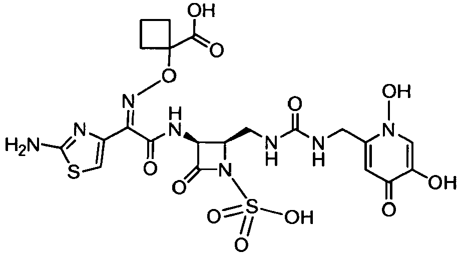 or a pharmaceutically acceptable salt thereof.

The compounds of Formula (I) and Formula (IA) exhibit antibacterial activity, especially against Gram-negative organisms. They may be used to treat bacterial infections in mammals, especially humans. The compounds may also be used for veterinary applications, such as treating infections in livestock and companion animals.

The compounds of Formula (I) and Formula (IA) are useful for treating a variety of infections; especially Gram-negative infections (as well as conditions arising from such infections), including nosocomial pneumonia, urinary tract infections, systemic infections (bacteremia and sepsis), skin and soft tissue infections, surgical infections, intraabdominal infections, lung infections (including those in patients with cystic fibrosis), *Helicobacter pylori* (and relief of associated gastric complications such as peptic ulcer disease, gastric carcinogenesis, etc.), endocarditis, diabetic foot infections, osteomyelitis, infections associated with burns or wounds, infections from devices such as catheters, ocular infections, otic infections, and central nervous system infections. Examples of Gram-negative organisms include *Pseudomonas aeruginosa, Klebsiella pneumoniae, Escherichia coli,* and *Acinetobacter baumanii.* Preferred compounds of Formula (IA) useful in the methods of the present invention are Examples 4, 26, and 30.

In order to simplify administration, the compounds will typically be admixed with at least one excipient and formulated into a pharmaceutical dosage form. Examples of such dosage forms include tablets, capsules, solutions/suspensions for injection, aerosols for inhalation and solutions/suspensions for oral ingestion.

In another aspect, the present invention provides pharmaceutical compositions comprising a compound of Formula (IA), or a pharmaceutically acceptable salt thereof, and at least one pharmaceutically acceptable carrier.

There are also disclosed methods of treating bacterial infections in a patient comprising administering to the patient in need of such treatment a therapeutically effective amount of a compound of Formula (IA) or a pharmaceutically acceptable salt thereof.

There are also disclosed methods of treating Gram-negative bacterial infections (as well as conditions arising from such infections), that include nosocomial pneumonia, urinary tract infections, systemic infections (bacteremia and sepsis), skin and soft tissue infections, surgical infections, intraabdominal infections, lung infections (including those in patients with cystic fibrosis) in a patient comprising administering to the patient in need of such treatment a therapeutically effective amount of a compound of Formula (IA), or a pharmaceutically acceptable salt thereof. Examples of Gram-negative organisms include *Pseudomonas aeruginosa, Klebsiella pneumoniae, Escherichia coli,* and *Acinetobacter baumanni.* Preferred compounds of Formula (IA) useful in the methods of the present invention are Examples 4, 26, and 30.

There are also disclosed methods of treating Gram-negative bacterial infections (as well as conditions arising from such infections) including *Helicobacter pylori* (and relief of associated gastric complications such as peptic ulcer disease, gastric carcinogenesis, etc.), endocarditis, diabetic foot infections, osteomyelitis, infections associated with burns or wounds, infections from devices such as catheters, ocular infections, otic infections, and central nervous system infections in a patient comprising administering to the patient in need of such treatment a therapeutically effective amount of a compound of Formula (IA), or a pharmaceutically acceptable salt thereof. Examples of Gram-negative organisms include *Pseudomonas aeruginosa, Klebsiella pneumoniae, Escherichia coli, and Acinetobacter baumanii.* Preferred compounds of Formula (IA) useful in the methods of the present invention are Examples 4, 26, and 30.

In another aspect, the present invention provides the use of a compound of Formula (IA) for the manufacture of a medicament for treating bacterial infections. Preferred compounds of Formula (IA) useful in the manufacture of medicaments for treating bacterial infections are Examples 4, 26, and 30.

In another aspect, the present invention provides the use of a compound of Formula (IA) for the manufacture of a medicament for treating Gram-negative bacterial infections (as well as conditions arising from such infections) that include nosocomial pneumonia, urinary tract infections, systemic infections (bacteremia and sepsis), skin and soft tissue infections, surgical infections, intraabdominal infections, lung infections (including those in patients with cystic fibrosis). Preferred compounds of Formula (IA) useful in the manufacture of medicaments for treating Gram-negative bacterial infections are Examples 4, 26, and 30.

In another aspect, the present invention provides the use of a compound of Formula (IA) for the manufacture of a medicament for treating Gram-negative bacterial infections (as well as conditions arising from such infections) that include *Helicobacter pylori* (and relief of associated gastric complications such as peptic ulcer disease, gastric carcinogenesis, etc.), endocarditis, diabetic foot infections, osteomyelitis, infections associated with burns or wounds, infections from devices such as catheters, ocular infections, otic infections, and central nervous system infections. Preferred compounds of Formula (IA) useful in the manufacture of medicaments for treating Gram-negative bacterial infections are Examples 4, 26, and 30.

In another aspect, the present invention contemplates pharmaceutical combinations comprising a compound of Formula (I) or Formula (IA), or a pharmaceutically acceptable salt thereof, and one or more additional anti-bacterial agents. Such use of compounds of the invention in combination with one or more additional anti-bacterial agents may be for simultaneous, separate or sequential use. The additional antibacterial agent is selected from beta-lactams, quinolones, fluoroquinolones, aminoglycosides, glycopeptides, lipopeptides, macrolides, ketolides, streptogramins, anasamycins oxazolidinones, polymyxins, penicillins, folate pathway inhibitors, phenicols, tetracyclines, and lincosamides.

In another aspect, the present invention provides a pharmaceutical combination comprising a compound of Formula (I) or Formula (IA), or a pharmaceutically acceptable salt thereof, and an additional antibacterial that is a beta-lactam anti-bacterial. The beta-lactam anti-bacterial is selected from penicillins, cephamycins, cephalosporins, carbapenems, monobactams, and beta-lactamase inhibitors or beta-lactam/beta-lactamase inhibitor combinations. Preferred beta-lactamase inhibitors include, but are not limited to, tazobactam, clavulanic acid, sulbactam, NXL-104, NXL-105, and MK-7655. A preferred beta lactam/beta-lactamase inhibitor is CXA-201. A preferred compound of Formula (IA) is Example 4, 26, or 30.

In another aspect, the present invention provides a pharmaceutical combination comprising a compound of Formula (I) or Formula (IA), or a pharmaceutically acceptable salt thereof, and an additional antibacterial that is selected from clindamycin, metronidazole, ampicillin, piperacillin, tetracycline, doxycycline, tigecycline, TP-434, PTK-0796, gentamicin, amikacin, ACHN-490, azithromycin, ciprofloxacin, levofloxacin, trimethoprim/sulfamethoxazole, colistin, polylmyxin B, imipenem, meropenem, doripenem, ertapenem, ceftazidime, cefazolin, cefepime, cefpodoxime, and a third generation cephalosporin. A preferred compound of Formula (IA) is Example 4, 26, or 30.

In another aspect, the present invention provides a pharmaceutical combination comprising a compound of Formula (I) or Formula (IA), or a pharmaceutically acceptable salt thereof, and an additional antibacterial that is cefepime. A preferred pharmaceutical combination is Example 4, 26, or 30 and cefepime.

In another aspect, the present invention provides a pharmaceutical combination comprising a compound of Formula (I) or Formula (IA), or a pharmaceutically acceptable salt thereof, and an additional antibacterial that is meropenem. A preferred pharmaceutical combination is Example 4, 26, or 30 and meropenem.

In another aspect, the present invention provides pharmaceutical compositions comprising a pharmaceutical combination, as described herein, and at least one pharmaceutically acceptable carrier.

There are also disclosed methods of treating Gram-negative bacterial infections (as well as conditions arising from such infections), that include nosocomial pneumonia, urinary tract infections, systemic infections (bacteremia and sepsis), skin and soft tissue infections, surgical infections, intraabdominal infections, lung infections (including those in patients with cystic fibrosis) in a patient comprising administering to the patient in need of such treatment a therapeutically effective amount of a pharmaceutical combination, as described herein. Examples of Gram-negative organisms include *Pseudomonas aeruginosa, Klebsiella pneumoniae, Escherichia coli,* and *Acinetobacter baumanni.*

There are also disclosed methods of treating Gram-negative bacterial infections (as well as conditions arising from such infections) including *Helicobacter pylori* (and relief of associated gastric complications such as peptic ulcer disease, gastric carcinogenesis, etc.), endocarditis, diabetic foot infections, osteomyelitis, infections associated with burns or wounds, infections from devices such as catheters, ocular infections, otic infections, and central nervous system infections in a patient comprising administering to the patient in need of such treatment a therapeutically effective amount of a pharmaceutical combination, as described herein. Examples of Gram-negative organisms include *Pseudomonas aeruginosa, Klebsiella pneumoniae, Escherichia coli, and Acinetobacter baumanii.*

In another aspect, the present invention provides the use of a pharmaceutical combination, as described herein, for the manufacture of a medicament for treating Gram-negative bacterial infections (as well as conditions arising from such infections) that include nosocomial pneumonia, urinary tract infections, systemic infections (bacteremia and sepsis), skin and soft tissue infections, surgical infections, intraabdominal infections, lung infections (including those in patients with cystic fibrosis).

In another aspect, the present invention provides the use of a pharmaceutical combination, as described herein, for the manufacture of a medicament for treating Gram-negative bacterial infections (as well as conditions arising from such infections) that include *Helicobacter pylori* (and relief of associated gastric complications such as peptic ulcer disease, gastric carcinogenesis, etc.), endocarditis, diabetic foot infections, osteomyelitis, infections associated with burns or wounds, infections from devices such as catheters, ocular infections, otic infections, and central nervous system infections.

### Definitions

As used throughout this application, including the claims, the following terms have the meanings defined below, unless specifically indicated otherwise. The plural and singular should be treated as interchangeable, other than the indication of number.

The term "(C₁-C₆)alkoxy" as used herein, means a (C₁-C₆)alkyl group, as defined herein, appended to the parent molecular moiety through an oxygen atom. Representative examples of (C₁-C₆)alkoxy include, but are not limited to, methoxy, ethoxy, propoxy, 2-propoxy, butoxy, tert-butoxy, pentyloxy, and hexyloxy.

The term "(C₁-C₆)alkyl" as used herein, means a branched or straight chained alkyl group containing from 1 to 6 carbon atoms. Representative examples of (C₁-C₆)alkyl include, but are not limited to methyl, ethyl, n-propyl, isopropyl, n-butyl, isobutyl, pentyl, and hexyl. The (C₁-C₆)alkyl group may be optionally substituted with up to 3 substituents selected from halogen, cyano, -OR^{a}, -SR^{a}, and -NR^{a}R^{b} where R^{a} and R^{b} are each independently represented by hydrogen or (C₁-C₆)alkyl.

The term "(C₁-C₃)alkyl" as used herein, means a branched or straight chained alkyl group containing from 1 to 3 carbon atoms that include methyl, ethyl, propyl, and isopropyl. The (C₁-C₃)alkyl group may be optionally substituted with one substituent selected from halogen, cyano, -OR^{a}, -SR^{a}, and -NR^{a}R^{b} where R^{a} and R^{b} are each independently hydrogen or (C₁-C₆)alkyl.

The term "cyano" as used herein, means a CN group.

The term "halo" or "halogen" as used herein, means -F, -Cl, -Br, and -I.

The term "phenyl(C₁-C₆) alkyl" as used herein, means a phenyl group is attached to the parent molecule via a (C₁-C₆)alkyl group, as defined herein. Representative examples of phenyl(C₁-C₆) alkyl include, but are not limited to, benzyl, 2-phenylethyl, 3-phenylpropyl, and 4-phenylbutyl. The phenyl(C₁-C₆) alkyl group may be optionally substituted with 1 to 5 substituents on the phenyl group selected from halogen, cyano, nitro, hydroxy, (C₁-C₆)alkyl optionally substituted, (C₁-C₆)alkoxy optionally substituted, trifluoromethyl, trifluromethoxy, phosphate, oxo, -SO₂NR⁴, -(CH₂)ₘ-N-C(O)-R⁴, -(CH₂)ₘ-C(O)-N-R⁴, -C(O)-R⁴, -C(O)-O-R⁴, -SR⁴, -SO₂R⁴ and -NR⁴R⁵, where R⁴ and R⁵ are each independently selected from hydrogen or (C₁-C₆)alkyl optionally substituted as defined above, and m is 0-4. In addition, the phenyl(C₁-C₆) alkyl group may also be optionally substituted with 1 to 3 substituents on the (C₁-C₆)alkyl group where the substituents are selected from halogen, cyano, -OR^{a}, -SR^{a}, and -NR^{a}R^{b} where R^{a} and R^{b} are each independently hydrogen or (C₁-C₆)alkyl.

The term "4-6 membered heterocyclic ring", "4- to 6-membered heterocyclic ring" or" 4-6-membered heterocycle" refers to any 4-membered ring containing a heteroatom selected from oxygen, nitrogen or sulfur; or a 5- or 6-membered ring containing 1, 2, or 3 nitrogen atoms; 1 oxygen atom; 1 sulfur atom; 1 nitrogen and 1 sulfur atom; 1 nitrogen and 1 oxygen atom; 2 oxygen atoms in non-adjacent positions; 1 oxygen and 1 sulfur atom in non-adjacent positions; or 2 sulfur atoms in non-adjacent positions. The 5-membered ring has 0 to 1 double bonds and the 6-membered rings have 0 to 2 double bonds. Heterocycles of the present invention include, but are not limited to, azetidine, oxetane, thietane, piperidine, pyrrolidine, tetrahydrofuran, tetrahydropyran, tetrahydrothiophen, piperazine, morpholine, tetrahydrotriazine, tetrahydropyrazole, tetrahydro-oxazole, tetrahydro-oxazine, thiomorpholine, and tetrahydropyrimidine. The heterocyclic rings of the present invention are optionally substituted with one, two, or three substituents independently selected from halogen, cyano, nitro, hydroxy, (C₁-C₆)alkyl optionally substituted, (C₁-C₆)alkoxy optionally substituted, trifluoromethyl, trifluromethoxy, phosphate, oxo, SO₂NR⁴, -(CH₂)ₘ-N-C(O)-R⁴, -(CH₂)ₘ-C(O)-N-R⁴, -C(O)-R⁴, -C(O)-O-R⁴, -SR⁴, -SO₂R⁴ and -NR⁴R⁵, where R⁴ and R⁵ are each independently selected from hydrogen or (C₁-C₆)alkyl optionally substituted as defined above, and m is 0-4. These substituents may be the same or different and may be located at any position of the ring that is chemically permissible. Any nitrogen atom within such a heterocyclic ring may optionally be substituted with (C₁-C₆)alkyl, if such substitution is chemically permissible. In addition, the present invention includes substitution of any nitrogen atom contained within a heterocycle with two independent (C₁-C₆)alkyl groups, as defined here, to form a quaternary amine or ammonium cation.

The term "hydroxyl" or "hydroxy" means an OH group.

The term "nitro" as used herein means a NO₂ group.

The term "oxo" as used herein means =0.

The term "optionally substituted phenyl" refers to a phenyl ring that may be optionally substituted with 1-5 substituents independently selected from halogen, cyano, nitro, hydroxy, (C₁-C₆)alkyl optionally substituted, (C₁-C₆)alkoxy optionally substituted, trifluoromethyl, trifluromethoxy, phosphate, -SO₂NR⁴, -(CH₂)ₘ-N-C(O)-R⁴, -(CH₂)ₘ-C(O)-N-R⁴, -C(O)-R⁴,-C(O)-O-R⁴, -SR⁴, -SO₂R⁴ and -NR⁴R⁵, in which in which R⁴, R⁵ and m are as defined above.

The term "5- to 6-membered heteroaryl," "5-6 membered heteroaryl ring," or "5-6 membered heteroaryl" as used herein means a 5- or 6-membered aromatic ring containing one, or more, heteroatoms. These aromatic rings may contain 1, 2, or 3 nitrogen atoms; 1 oxygen atom; 1 sulfur atom; 1 nitrogen and 1 sulfur atom; or 1 nitrogen and 1 oxygen atom. Examples of such 5- to 6-membered heteroaryls include, but are not limited to, pyridinyl, pyridazinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, triazinyl, oxadiazolyl, thiadiazolyl, and furazanyl. The heteroaryl rings of the present invention are optionally substituted with 1 to 3 substituents independently selected from halogen, nitro, cyano, hydroxy, (C₁-C₆)alkyl optionally substituted, (C₁-C₆)alkoxy optionally substituted, trifluoromethyl, trifluoromethoxy, phosphate, -SO₂NR⁴, -(CH₂)ₘ-N-C(O)-R⁴, -(CH₂)ₘ-C(O)-N-R⁴, -C(O)-R⁴,-C(O)-O-R⁴, -SR⁴, -SO₂R⁴ and -NR⁴R⁵, in which in which R⁴, R⁵ and m are as defined above.

The term "therapeutically effective amount" refers to an amount of a compound of Formula (I) that, when administered to a patient, provides the desired effect. Examples include, but are not limited to: lessening in the severity of the symptoms associated with a bacterial infection, decreasing the number of bacteria in the affected tissue, and/or preventing bacteria in the affected tissue from increasing in number, eliminating the bacteria, and preventing infection-related patient mortality.

The term "patient" refers to warm blooded animals such as, guinea pigs, mice, rats, gerbils, cats, rabbits, dogs, monkeys, chimpanzees, pigs, cows, humans, etc.

The term "treat" refers to the ability of the compounds to relieve, alleviate or slow the progression of the patient's bacterial infection (or condition) or any tissue damage associated with the disease. It should also be construed to include prophylactic use prior to surgery, dental procedures, etc., in which health care professionals routinely administer antibiotics to decrease the likelihood of infection.

The term "pharmaceutically acceptable" indicates that the substance or composition must be compatible chemically and/or toxicologically, with the other ingredients comprising a formulation, and/or the mammal being treated therewith.

The term "pharmaceutically acceptable carrier" or "carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; cocoa butter and suppository waxes; oils such as peanut oil, cottonseed oil, safflower oil, sesame oil, olive oil, corn oil and soybean oil; glycols such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; and phosphate buffer solutions; as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of one skilled in the art of formulations.

The term "isomer" means "stereoisomer" and/or "geometric isomer" as defined below.

The term "stereoisomer" means compounds that possess one or more chiral centers and each center may exist in the R or S configuration. Stereoisomers include all diastereomeric, enantiomeric and epimeric forms as well as racemates and mixtures thereof.

The term "geometric isomer" means that a compound may exist in cis, trans, syn, anti, *entgegen* (E), and *zusammen* (Z) forms as well as mixtures thereof.

Compounds of Formula (I), compounds of Formula (IA), and compounds of the present invention, etc. are being used interchangeably throughout the application and should be treated as synonyms.

The phrase "pharmaceutically acceptable salt(s)", as used herein, unless otherwise indicated, includes salts of acidic or basic groups which may be present in the compounds of the present invention. The compounds of the present invention that are basic in nature are capable of forming a wide variety of salts with various inorganic and organic acids. The acids that may be used to prepare pharmaceutically acceptable acid addition salts of such basic compounds are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts. The compounds of the present invention that include a basic moiety, such as an amino group, may form pharmaceutically acceptable salts with various amino acids, in addition to the acids mentioned above.

The invention also relates to base addition salts of the compounds of the present invention and include, but are not limited to, those derived from such pharmacologically acceptable cations such as alkali metal cations (e.g., potassium and sodium) and alkaline earth metal cations (e.g., calcium and magnesium), ammonium or water-soluble amine addition salts such as N-methylglucamine-(meglumine), and the lower alkanolammonium and other base salts of pharmaceutically acceptable organic amines. Non-limiting examples of such suitable base salts include aluminum, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulphate, hemicalcium, and hemisodium salts.

For a review on suitable salts, see Handbook of Pharmaceutical Salts: Properties, Selection, and Use by Stahl and Wermuth (Wiley-VCH, 2002). Methods for making pharmaceutically acceptable salts of compounds of the invention are known to one of skill in the art. They are also illustrated in Examples 1-69 below.

Certain of the compounds of the Formula (I) may exist as geometric isomers. The compounds of the Formula (I) may possess one or more asymmetric centers, thus existing as two, or more, stereoisomeric forms. The present invention includes all the individual stereoisomers and geometric isomers of the compounds of Formula (I) and mixtures thereof. Individual enantiomers can be obtained by chiral separation or using the relevant enantiomer in the synthesis.

In addition, the compounds of the present invention can exist in unsolvated as well as solvated forms with pharmaceutically acceptable solvents such as water (hydrates), ethanol, and the like. In general, the solvated forms are considered equivalent to the unsolvated forms for the purposes of the present invention. The compounds may also exist in one or more crystalline states, i.e. polymorphs, or they may exist as amorphous solids. All such forms are encompassed by the claims.

Also disclosed are prodrugs of the compounds of the invention. Thus certain derivatives of compounds of the invention which may have little or no pharmacological activity themselves can, when administered into or onto the body, be converted into compounds of the invention having the desired activity, for example, by hydrolytic cleavage. Such derivatives are referred to as "prodrugs". Further information on the use of prodrugs may be found in Pro-drugs as Novel Delivery Systems, Vol. 14, ACS Symposium Series (T. Higuchi and W. Stella) and Bioreversible Carriers in Drug Design, Pergamon Press, 1987 (Ed. E. B. Roche, American Pharmaceutical Association).

This invention also encompasses compounds of the invention containing protective groups. One skilled in the art will also appreciate that compounds of the invention can also be prepared with certain protecting groups that are useful for purification or storage and can be removed before administration to a patient. The protection and deprotection of functional groups is described in "Protective Groups in Organic Chemistry", edited by J.W.F. McOmie, Plenum Press (1973) and "Protective Groups in Organic Synthesis", 4th edition, T.W. Greene and P.G.M. Wuts, Wiley-Interscience (2007).

The present invention also includes isotopically-labeled compounds, which are identical to those recited in Formula (I), but for the fact that one or more atoms are replaced by an atom having an atomic mass or mass number different from the atomic mass or mass number predominantly found in nature. Examples of isotopes that can be incorporated into compounds of the invention include isotopes of hydrogen, carbon, nitrogen, oxygen, phosphorous, fluorine and chlorine, such as, but not limited to, ²H, ³H, ¹³C, ¹⁴C, ¹⁵N, ¹⁸O, ¹⁷O, ³¹P, ³²P, ³⁵S, ¹⁸F, and ³⁶Cl, respectively. Compounds of the present invention and pharmaceutically acceptable salts of said compounds which contain the aforementioned isotopes and/or other isotopes of other atoms are within the scope of this invention. Certain isotopically-labeled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased in vivo half-life or reduced dosage requirements and, hence, may be preferred in some circumstances. Further information may be found in: M. B. Fisher, et al. Curr. Op. Drug Disc. Dev. 2006, 9(1), 101-109. Isotopically-labeled compounds of this invention and prodrugs thereof can generally be prepared by carrying out the procedures disclosed in the Schemes and/or in the Examples below, by substituting a readily available isotopically-labeled reagent for a non-isotopically-labeled reagent.

The compounds of Formula (I) contain an azetidinone moiety as depicted below

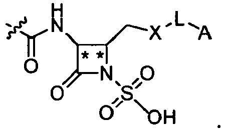

In addition, two of the carbon atoms of the azetidinone moiety contain chiral centers, marked with *. Thus the compounds of the present invention may exist as a racemate, a mixture of cis and trans diastereomers, a mixture of cis enantiomers, or a mixture of trans enantiomers. The present invention also contemplates a single cis enantiomer or a single trans enantiomer where the enantiomeric excess ("ee") is ≥90%. The preferred ee is 95%-98% and the most preferred ee is ≥99%. The four enantiomers are designated (R,R), (S,S), (R,S), or (S,R). Unless depicted otherwise, the claims encompass all four enantiomers and any combination thereof.

The compounds of Formula (I) contain a pyridinone or pyridinium ring as depicted by the Formula (I) term "A." When "A" is a pyridinone and "R³" is hydrogen or H, shown below, the pyridinone may exist as tautomers.

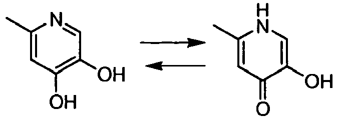

The claims of the present invention encompass each of these tautomeric forms and mixtures thereof. As is readily apparent to one skilled in the art, an equilibrium may exist between these two forms and depending upon pH, pKa, physical form (i.e. solid or solution), crystalline form, etc., one tautomeric form may predominate over the other.

When "A" is a pyridinone and "R³" is hydroxy, the pyridinone moiety, again, may exist as tautomers, shown below for clarity purposes.

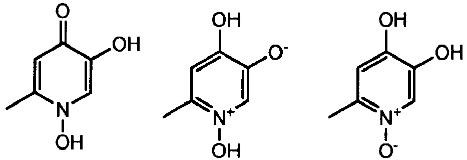

The claims of the present invention encompass each of these tautomeric forms and mixtures thereof. It is to be understood that one of the tautomers shown above may predominate over the other depending on reaction conditions, the method of isolation, the physical state, and/or storage conditions.

When "A" is a pyridinone and "R³" is alkyl, the pyridinone moiety, again, may exist as tautomers, shown below for clarity purposes.

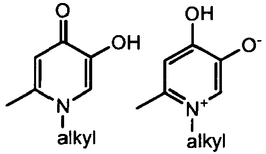

The claims of the present invention encompass each of these tautomeric forms and mixtures thereof. It is to be understood that one of the tautomers shown above may predominate over the other depending on reaction conditions, the method of isolation, the physical state, and/or storage conditions.

The compounds of Formula (I) contain an oxime-ether moiety. This group may exist in either the cis or trans geometric configuration, as depicted below, or as a mixture of both cis and trans.

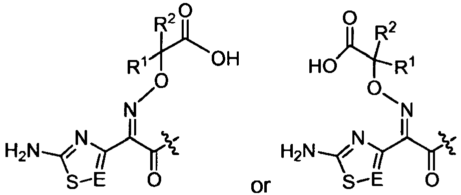

Unless otherwise indicated, the claims of the present invention encompass mixtures of the cis and trans geometric isomers, the isolated cis isomer, or the isolated trans isomer.

The compounds of Formula (I) contain acidic (e.g. carboxylic acid, sulfate) and basic moieties (e.g. aminothiazole). As readily apparent to one skilled in the art, the presence or positioning of acidic protons may vary depending on factors such as pKa, pH, physical state, etc. This is illustrated below in two examples, however, other possibilities exist and the claims of the present invention encompass all possibilities. One skilled in the art would appreciate that the structures depicted throughout this application are not meant to be definitive with regard to the positioning of acidic protons.

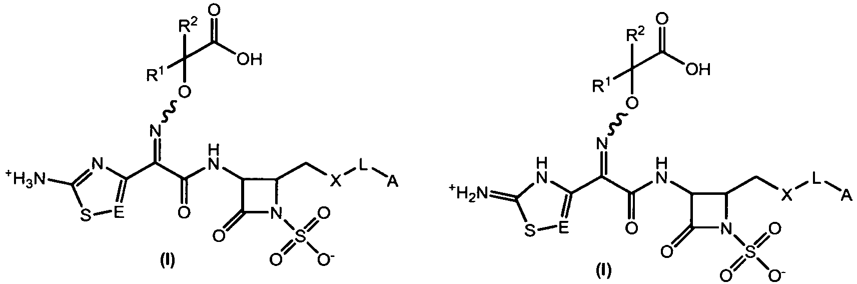

### Synthesis

The compounds of Formula (I) and Formula (IA) can be prepared by a variety of methods that are analogously known in the art. The synthetic schemes presented below illustrate methods for preparing these compounds. Others, including modifications thereof, will be readily apparent to one skilled in the art.

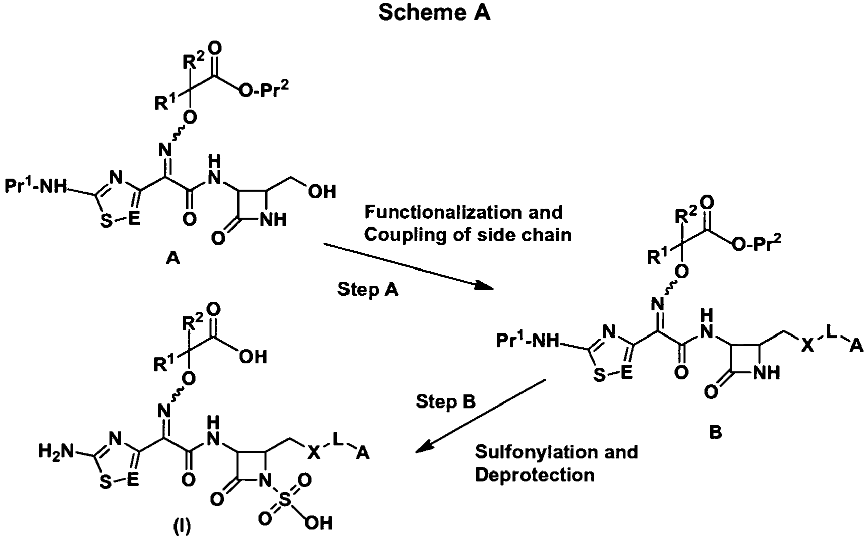

The initial step in the synthesis is to produce intermediate A (see Scheme B for its synthesis). In compound A, R¹, R² and E will be represented by the same moiety as is desired in the final product. Pr¹ and Pr² will both be appropriate protecting groups. This intermediate is then subjected to the appropriate functionalization reaction to place the desired X-L-A side chain on the methylene bonded to the 4-position of the azetidinone as shown in Step A below (while depicted as a single step, it will often encompass multiple reactions). This can be accomplished using techniques well known in the art and discussed in detail infra. The final step is the generation of the N-1 sulfonic acid and deprotection as depicted in Step B (will also often encompass multiple reactions). This can also be accomplished using techniques well known in the art and is discussed in detail infra. In some instances, the order in which the reactions are carried out is not critical. For example, if desired, the sulfonyl moiety may be attached to the azetidinone moiety first, followed by attachment of the side chain to the methylene moiety. This reaction scheme depicted above for producing the compound of Formula (I), is merely illustrative. As is readily apparent to one skilled in the art, alternative strategies may be employed to assemble the compounds of Formula (I) depending upon the specific compound, availability of reagents, preference of the chemist, etc.

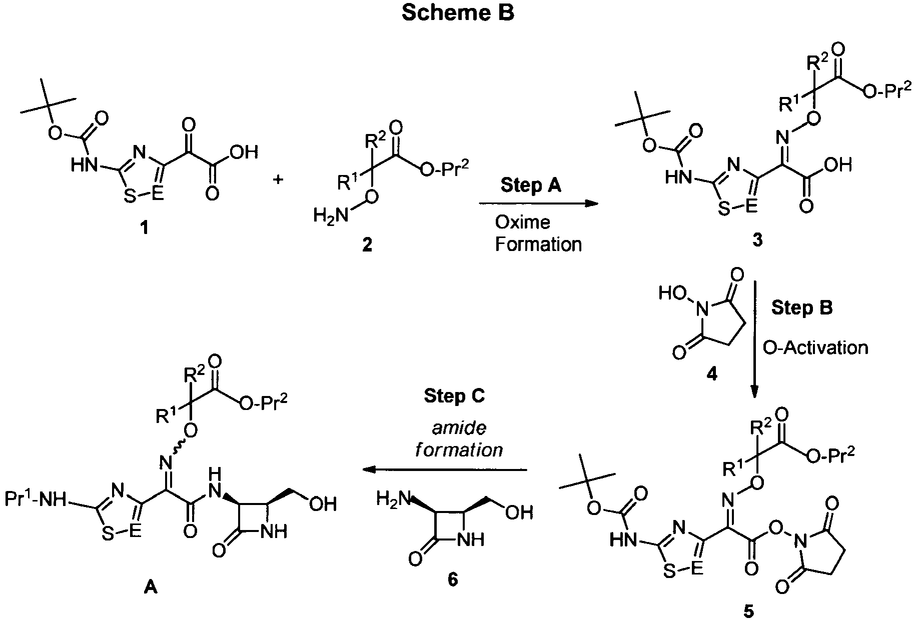

Scheme B above describes the synthesis of intermediate A, which may be used to produce compounds of Formula (I) wherein E, R¹ and R² are as defined in Formula (I) of the Summary section herein. Schemes C-I, below, describes alternative methods for placing the various X-L-A side chains.

One of the starting materials is the 5-membered heteroaryl moiety depicted by structure 1. E will be represented by the same function as is desired in the final product. The amine will be protected as depicted. Scheme B shows a Boc group, but other appropriate protecting groups may be utilized. Methods for producing this compound are described in Yamawaki, K., et al. Bioorganic & Medicinal Chem., (2007), 15, 6716 and Yamamoto, H., et al., Bioorganic and Medicinal Chem., (2002), 10, 1535. The other starting material is the compound of structure 2, which may be prepared as described in WO 2007/065288. In this compound, R¹ and R² will be represented by the same moiety as desired in the final product and Pr² will be represented by a protecting group appropriate for carboxy functions, as is known in the art.

In Step A, the oxime (structure 3) is formed using techniques well known in the art (see Yamawaki et al supra, WO 2007/065288 or WO 2010/070523). Typically, equivalent amounts of the compounds of structure 1 and 2 are contacted in methanol at room temperature. The reaction is allowed to proceed to completion. The desired product of structure 3 is recovered and isolated as is known in the art (i.e. rotary evaporation, precipitation followed by filtration, etc). It may optionally be purified by chromatography or used as the crude in Step B.

In Step B, the activated ester 5 can be prepared by the reaction of equivalent amounts of structure 3 with N-hydroxysuccinimide (structure 4) in the presence of a coupling reagent such as dicyclohexylcarbodiimide or diisopropylcarbodiimide in a polar aprotic solvent such as dichloromethane at ambient, or reduced, temperatures. The activated ester 5 can be isolated and/or purified using techniques known in the art such as by chromatography.

The co-reactant of Step C, structure 6, can be prepared as described in Kishimoto et al in Chemical and Pharmaceutical Bulletin Vol. 23, 2646 (1984) and Takahashi et al in Chemical and Pharmaceutical Bulletin Vol. 34, 2732 (1986). The amidation of Step C can be carried out as is known in the art. Typically equivalent amounts of the reactants are contacted with a base such as triethylamine, in a polar protic solvent such as methanol or ethanol, at ambient temperature and the reaction is allowed to proceed to completion. Intermediate A may then be recovered, isolated and purified using techniques known in the art.

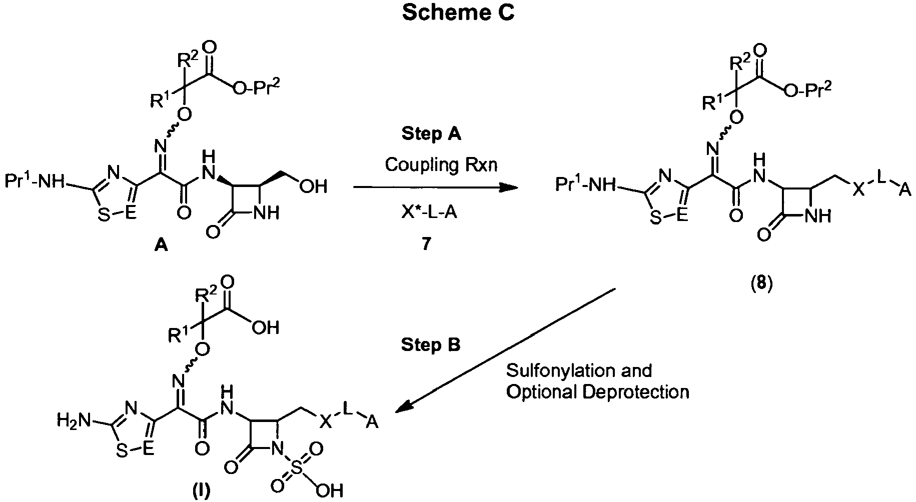

Reaction Scheme C, above, describes methods for preparing compounds of Formula (I) where X is represented by -O-C(O)- and E, R¹, R², L, and A are as defined in Formula (I) of the Summary section herein. Intermediate A is treated with structure 7in which X* represents a carbonyl moiety and a suitable group that is or can be activated to react with the alcohol found in structure A and thus produce the desired X moiety found in the final product. L and A will each be represented by the moiety desired in the final product, or a protected version of it. For example, A typically represents 3,4-dihydroxy-pyridinones or 3,4-dihydroxy-pyridyls. These hydroxyl functions may be protected with benzyl groups during the reaction. The compounds represented by structure 7 are known in the art.

The coupling reaction of Step A can be carried out as is known in the art. Equivalent amounts of structure 7 and intermediate A are contacted in a polar aprotic solvent such as dichloromethane, dimethylformamide, etc. The reaction is carried out in the presence of a coupling agent, such as dicyclohexylcarbodiimide, and a base, such as 4-dimethylaminopyridine, at ambient temperature. The desired product, structure 8, is recovered, isolated, and purifed using techniques known in the art.

The sulfonylation in Step B can be carried out using techniques known in the art. The compound of structure 8 is contacted with a molar excess of a sulfur trioxide N,N-dimethylformamide complex, sulfur trioxide pyridine complex, etc. in an aprotic solvent such as dimethylformamide. The reaction is allowed to proceed at ambient temperature until completion. The resulting product can be recovered and isolated using techniques known in the art.

If the recovered product still contains protecting groups, these can be removed using techniques known in the art. For example, the protected molecule may be contacted with trifluoroacetic acid in an aprotic solvent such as dichloromethane to remove the protecting groups. Alternatively, the protected molecule may be contacted with boron trichloride in an aprotic solvent such as para-xylene or dichloromethane. A more complete discussion of synthetic procedures for removing various protecting groups can be found in Greene et al supra.

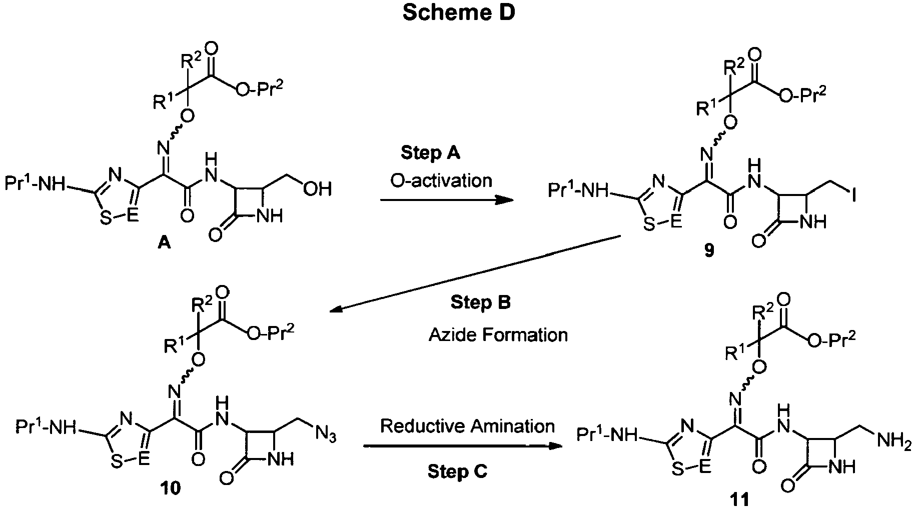

For those compounds in which X contains a nitrogen atom (i.e. NH-C(O)-, NH-SO₂-, -NH-C(N-CN)-, NH-T, or triazole), Scheme D describes synthetic methods for preparing such compounds of Formula (I). For intermediate A, where E, R¹ and R² are the same moieties as is desired in the final product, the amine and carboxy moieties are typically protected using synthetic procedures known in the art. In step A, the hydroxyl moiety appended to the 4-position methylene of the azetidinone ring is converted to a reactive iodo moiety. Typically intermediate A is contacted with an excess of triphenyl phosphine and imidazole, in an aprotic solvent such as dichloromethane. An excess of iodine is then added and the reaction is allowed to proceed to completion at ambient temperature. The product, structure 9, is recovered, isolated, and purified using techniques known in the art such as by chromatography.

Alternatively the iodine function can be introduced into the molecule by contacting intermediate A with an excess of p-toluenesulfonyl chloride and pyridine in an aprotic solvent, followed by sodium iodide. The reaction is allowed to proceed to completion at ambient temperature and the desired product may be recovered, isolated and optionally purified using techniques known in the art.

In Step B, the iodo moiety is converted into an azide. This can be accomplished by contacting the product of Step A, structure 9, with an excess of a base, such as triethyl amine, in an aprotic solvent such as 2-methyltetrahydrofuran, at ambient temperature. An equivalent amount of tetrabutylammonium azide will be added slowly to the reaction and the reaction will be allowed to proceed to completion. The reaction may be heated to speed the reaction. Structure 10 may be isolated and optionally purified by techniques known in the art.

The reduction in Step C can be carried out as is known in the art. The azide (structure 10) is placed in a protic solvent such as ethanol, in the presence of a hydrogenation catalyst, such as 10% palladium on carbon. The reduction is carried out in the presence of hydrogen under pressure and allowed to proceed to completion at ambient temperature. The amine of structure 11 is recovered, isolated, and optionally purified using techniques known in the art.

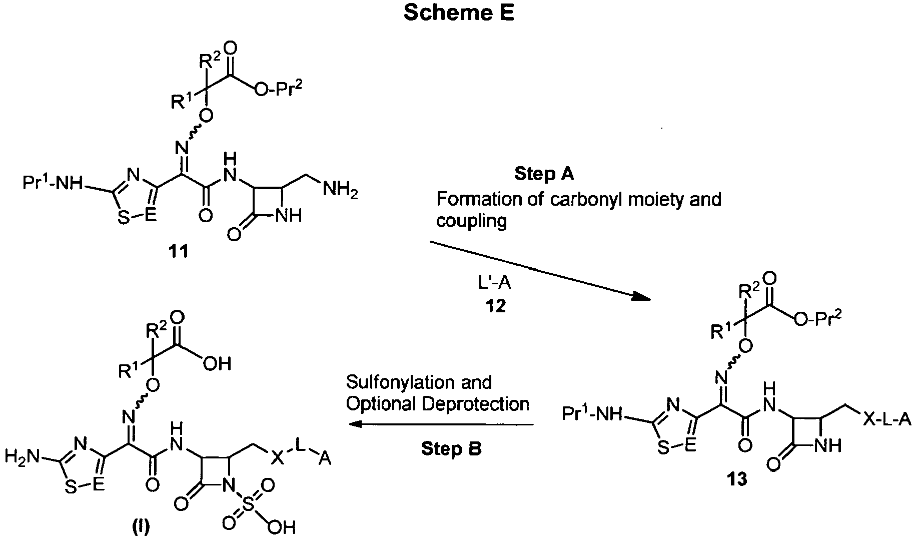

For compounds of Formula (I) where X and L form a carbamate (i.e. -NH-C(O)-O) or a urea (i.e. -NH-C(O)-NH), then structure 11 may be subjected to the reactions described in Scheme E to produce the desired product of Formula (I) as shown above.

In Step A, one of the co-reactants is the compound of structure 12, L'-A. In structure 12, A should be represented by the same moiety as desired in the final product, or a protected embodiment of it. L' will be represented by the same moiety as desired in the final product, except that it will be further substituted with a leaving group, which can be displaced by the primary amine in structure 11 to produce structure 13. Methods for producing such compounds are known in the art.

Initially, a carbonyl function is attached to the L moiety of structure 12. This may be accomplished by contacting the compound of structure 12, with an excess of 1,1-carbonyldiimidazole, in an aprotic solvent such as tetrahydrofuran. The reaction is allowed to proceed to completion at ambient temperature. An equivalent amount of the amine of structure 11 is then added to the reaction and the reaction is continued at ambient temperature for a sufficient period of time to allow generation of the compound of structure 13. The compound of structure 13 may be recovered, isolated and purified using techniques known in the art.

The desired compound of Formula (I) may be obtained by subjecting structure 13 to a sulfonylation and optional deprotection reaction in a similar manner as described in Step B of Scheme B above.

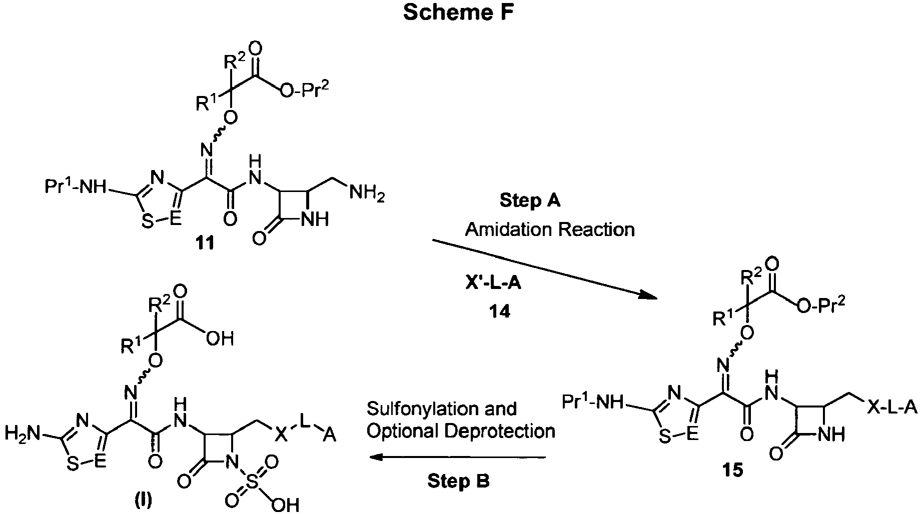

For compounds in which X is an amide, the coupling reaction may be carried out as described in Scheme F above. One of the reactants will be the compound of structure 14, X'-L-A where L and A are as is desired in the final product, or a protected variant. X' is a carbonyl function bearing an appropriate group that is or can be activated so that an amide bond may be formed with the amine of structure 11.

The coupling reaction can be carried out using amidation techniques known in the art. For example, an admixture of equivalent amounts of the compound of structure 14 and the amine of structure 11 in an aprotic solvent such as N,N-dimethylformamide are treated with an excess of a base, such as sodium bicarbonate, and a coupling agent, such as *O*-(7-azabenzotriazol-1-yl)-*N,N,N',N*'-tetramethyluronium hexafluorophosphate at ambient temperature to provide a compound of structure 15. The compound of structure 15 may be recovered, isolated and purified using techniques known in the art.

The desired compound of Formula (I) is obtained by subjecting structure 15 to a sulfonylation and optional deprotection reaction similar to the procedure described above in Step B of Scheme B.

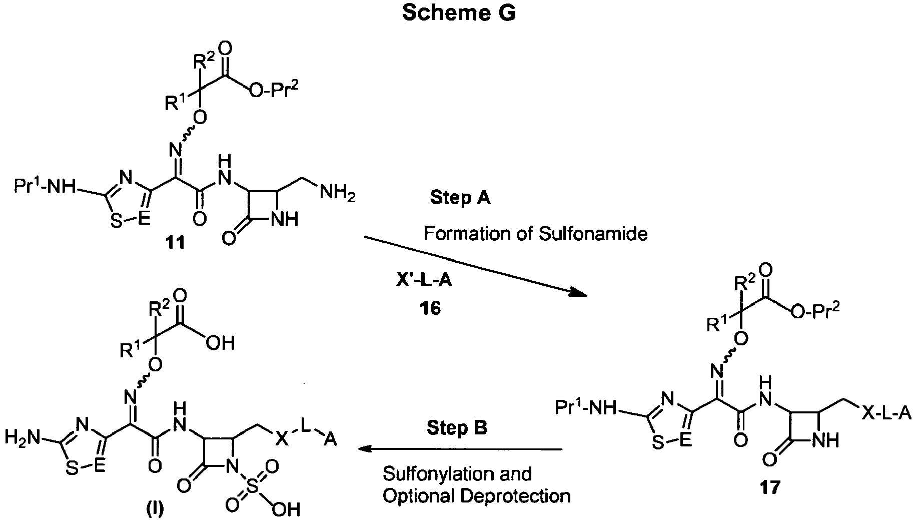

For compounds of Formula (I) where X is a sulfonamide, the sulfonamidation reaction may be carried out as described in Scheme G, above. One of the reactants is the compound of structure 16, X'-L-A, where L and A are the same moieties as is desired in the final product, or a protected variant. X' is a sulfonyl group bearing an appropriate leaving group, such as chloride, enabling formation of a sulfonamide bond with the amine function of structure 11. The compound of structure 16 is reacted with structure 11 in the presence of an equivalent amount of base, such as triethylamine, in an aprotic solvent such as dichloromethane or acetonitrile, at reduced (i.e. ice bath) to ambient temperatures for a sufficient period of time to allow the reaction to proceed to completion to provide structure 17, which is recovered, isolated and purified using techniques known in the art. The desired compound of Formula (I) is obtained following sulfonylation and optional deprotection of the compound of structure 17 in a similar manner as described in Step B of Reaction Scheme B.

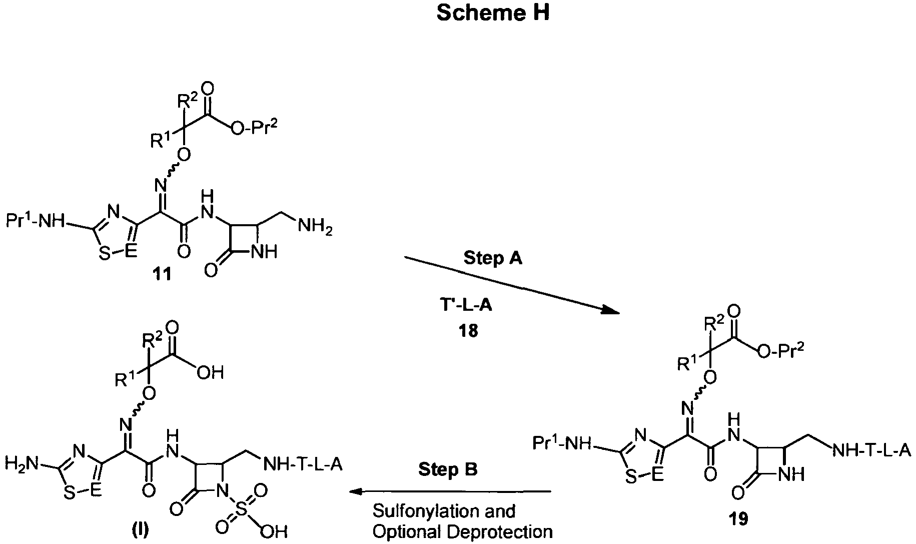

Compounds of Formula (I), where X is NH-T and T is an optionally substituted 5-or 6-membered heteroaryl, are prepared using the synthetic methods described in Scheme H above. A compound of structure 18 where T' is the same entity as is desired in the final product, except that the carbon atom of T that serves as the point of attachment (to the depicted nitrogen atom) is substituted with a leaving group such as fluorine, etc. Methods for preparing the compounds of structure 18 are known in the art and are further illustrated in the Examples below. The desired compound of Formula (I) may be obtained by subjecting a compound of structure 18 to a sulfonylation and optional deprotection reaction in a manner similar to that described in Step B of Reaction Scheme B.

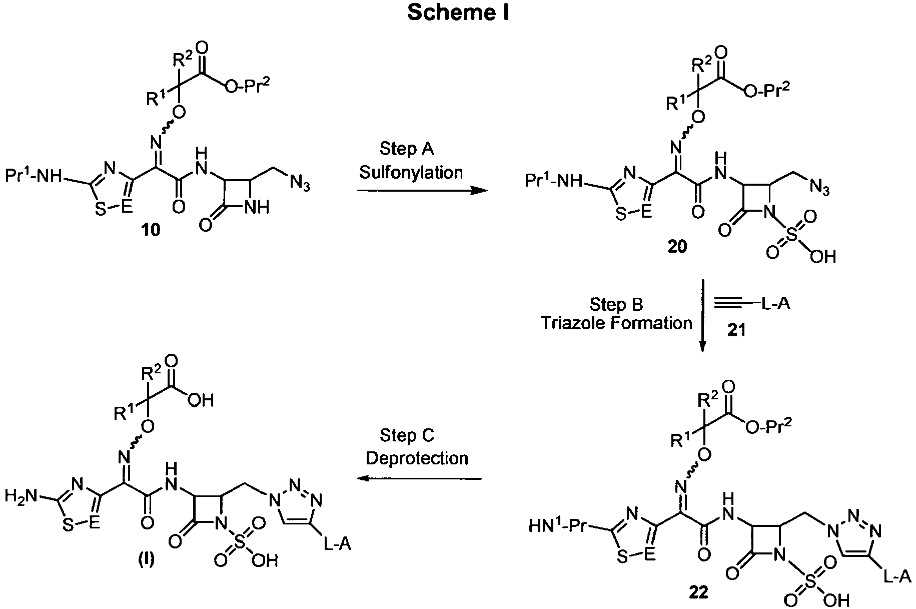

Scheme I describes synthetic methods for preparing compounds of Formula (I) where X is a triazole heteroaryl group. A compound of structure 10, prepared as described in Scheme D, is sulfonylated in a manner similar to the procedure described in Step B of Scheme C to provide structure 20. The compound of structure 20 and an alkyne of structure 21 are combined in dimethylsulfoxide, water, and tert-butanol and treated with a catalytic amount of copper and optionally a catalytic amount of an antioxidant such as sodium I-ascorbate at ambient temperature to provide a compound of structure 22. The compound of structure 22 is isolated, recovered and purified using techniques known in the art. In Step C, the protecting groups are removed from structure 22 using synthetic methods known in the art. For example, the protected molecule may be contacted with trifluoroacetic acid in an aprotic solvent such as dichloromethane to remove the protecting groups. Alternatively the protected molecule may be contacted with boron trichloride in an aprotic solvent such as para-xylene or dichloromethane. Additional conditions for removing protecting groups can be found in Greene et al or McOmie supra.

### Medical and Veterinary Uses

The compounds may be used for the treatment or prevention of infectious disorders, especially those caused by susceptible and multi-drug resistant (MDR) Gram-negative bacteria. Examples of such Gram-negative bacteria include *Acinetobacter baumannii, Acinetobacter spp., Achromobacter spp., Aeromonas* spp., *Bacteroides fragilis, Bordetella* spp., *Borrelia* spp., *Brucella* spp., *Campylobacter* spp., *Citrobacter diversus (koseri), Citrobacter freundii, Enterobacter aerogenes, Enterobacter cloacae, Escherichia coli, Francisella tularensis, Fusobacterium* spp., *Haemophilus influenzae* (β-lactamase positive and negative), *Helicobacter pylori, Klebsiella oxytoca, Klebsiella pneumoniae* (including those encoding extended-spectrum β-lactamases (ESBLs), *Legionella pneumophila, Moraxella catarrhalis* (β-lactamase positive and negative), *Morganella morganii, Neisseria gonorrhoeae, Neisseria meningitidis, Proteus vulgaris, Porphyromonas* spp., *Prevotella* spp., members of the *Enterobacteriaceae* that express ESBLs, KPCs, CTX-M, metallo-β-lactamases, and AmpC-type beta-lactamases that confer resistance to currently available cephalosporins, cephamycins, carbapenems, and beta-lactam/beta-lactamase inhibitor combinations, *Mannheimia haemolyticus, Pasteurella* spp., *Proteus mirabilis, Providencia* spp., *Pseudomonas aeruginosa, Pseudomonas* spp., *Salmonella* spp., *Shigella* spp., *Serratia marcescens, Treponema* spp., *Burkholderia cepacia, Vibrio* spp., *Yersinia* spp., and *Stenotrophomonas maltophilia.*

In a more specific embodiment, the Gram-negative bacteria are selected from the group consisting of *Acinetobacter baumannii, Acinetobacter spp., Enterobacter aerogenes, Enterobacter cloacae, Escherichia coli, Klebsiella oxytoca, Klebsiella pneumoniae, Serratia marcescens, Pseudomonas aeruginosa* and members of the *Enterobacteriaceae and Pseudomonas* that express ESBLs, KPCs, CTX-M, metallo-β-lactamases, and AmpC-type beta-lactamases that confer resistance to currently available cephalosporins, cephamycins, carbapenems, and beta-lactam/beta-lactamase inhibitor combinations.

Examples of infections (and conditions arising from infections) that may be treated with the compounds of Formula (I) include nosocomial pneumonia, urinary tract infections, systemic infections (bacteremia and sepsis), skin and soft tissue infections, surgical infections, intraabdominal infections, lung infections in patients with cystic fibrosis, patients suffering from lung infections, endocarditis, diabetic foot infections, osteomyelitis, and central nervous system infections.

In addition, the compounds can be used to treat *Helicobacterpylori* infections in the GI tract of humans (and other mammals). Elimination of these bacteria is associated with improved health outcomes including fewer dyspeptic symptoms, reduced peptic ulcer recurrence and rebleeding, reduced risk of gastric cancer, etc. A more detailed discussion of eradicating H. *pylori* and its impact on gastrointestinal illness may be found at: www.informahealthcare.com, Expert Opin. Drug Saf. (2008) 7(3).

In order to exhibit this anti-infective activity, the compounds need to be administered in a therapeutically effective amount. A "therapeutically effective amount" is meant to describe a sufficient quantity of the compound to treat the infection, at a reasonable benefit/risk ratio applicable to any such medical treatment. It will be understood, however, that the attending physician, within the scope of sound medical judgement, will decide the total daily dosage of the compound. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. As a general guideline however, the total daily dose will typically range from about 0.1 mg/kg/day to about 5000mg/kg/day in single or in divided doses. Typically, dosages for humans will range from about 100 mg to about 10,000 mg per day, in a single or multiple doses.

Any route typically used to treat infectious illnesses, including oral, parenteral, topical, rectal, transmucosal, and intestinal, can be used to administer the compounds. Parenteral administrations include injections to generate a systemic effect or injections directly into to the afflicted area. Examples of parenteral administrations are subcutaneous, intravenous, intramuscular, intradermal, intrathecal, and intraocular, intranasal, intraventricular injections or infusion techniques (including extended or continual infusions). Topical administrations include the treatment of areas readily accessible by local application, such as, for example, eyes, ears (including external and middle ear infections), vagina, open wound, skin (including the surface skin and the underneath dermal structures), orlower intestinal tract. Transmucosal administration includes nasal aerosol or inhalation applications.

### Formulations

Compounds of the invention can be formulated for administration in any way for use in human or veterinary medicine, by analogy with other bioactive agents such as antibiotics. Such methods are known in the art and are summarized below.

The composition can be formulated for administration by any route known in the art, such as subdermal, by-inhalation, oral, topical or parenteral. The compositions may be in any form known in the art, including but not limited to tablets, capsules, powders, granules, lozenges, creams or liquid preparations, such as oral or sterile parenteral solutions or suspensions.

The topical formulations of the present invention can be presented as, for instance, ointments, creams or lotions, ophthalmic ointments/drops and otic drops, impregnated dressings and aerosols, and may contain appropriate conventional additives such as preservatives, solvents to assist drug penetration and emollients, etc. Such topical formulations may also contain conventional carriers, such as cream or ointment bases and ethanol or oleyl alcohol for lotions. Such carriers may be present, for example, from about 1% up to about 98% of the formulation.

Tablets and capsules for oral administration may be in unit dose presentation form, and may contain conventional excipients such as binding agents, for example acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods will known in normal pharmaceutical practice.

Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerin, propylene glycol, or ethyl alcohol; preservatives, for example methyl or propyl p-hydroxybenzoate or sorbic acid, and, if desired, conventional flavoring or coloring agents.

If desired, and for more effective distribution, the compounds of the present invention can be incorporated into slow-release or targeted-delivery systems such as polymer matrices, liposomes, and microspheres. They may be sterilized, for example, by filtration through a bacteria-retaining filter or by incorporation of sterilizing agents in the form of sterile solid compositions, which may be dissolved in sterile water or some other sterile injectable medium immediately before use.

Injectable depot forms are made by forming microencapsulated matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

For parenteral administration, fluid unit dosage forms are prepared utilizing the compound and a sterile vehicle, water being typical. The compound, depending on the vehicle and concentration used, can be either suspended or dissolved in the vehicle or other suitable solvent. In preparing solutions, the compound can be dissolved in water for injection and filter sterilized before filling into a suitable vial or ampoule and sealing. Advantageously, agents such as a local anesthetic preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain, for example, from about 0.1% by weight, to about 60% by weight, of the active material, depending on the method of administration. Where the compositions comprise dosage units, each unit will contain, for example, from about 5-500 mg of the active ingredient. The dosage as employed for adult human treatment will range, for example, from about 100 to 10000 mg per day, depending on the route and frequency of administration.

The examples and preparations provided below further illustrate and exemplify the compounds of the present invention and methods of preparing such compounds. It is to be understood that the scope of the present invention is not limited in any way by the scope of the following examples and preparations.

### EXAMPLES

### Experimental Procedures

Experiments were generally carried out under an inert atmosphere (nitrogen or argon), particularly in cases where oxygen- or moisture-sensitive reagents or intermediates were employed. Commercial solvents and reagents were generally used without further purification, including anhydrous solvents where appropriate (generally Sure-Seal™ products from the Aldrich Chemical Company, Milwaukee, Wisconsin). Mass spectrometry data is reported from either liquid chromatography-mass spectrometry (LCMS) or atmospheric pressure chemical ionization (APCI). Chemical shifts for nuclear magnetic resonance (NMR) data are expressed in parts per million (ppm, δ) referenced to residual peaks from the deuterated solvents employed. Melting points are uncorrected. Low Resolution Mass Spectra (LRMS) were recorded on either a Hewlett Packard 5989®, utilizing chemical ionization (ammonium), or a Fisons (or Micro Mass) Atmospheric Pressure Chemical Ionization (APCI) platform which uses a 50/50 mixture of acetonitrile/water with 0.1% formic acid as the ionizing agent. Room or ambient temperature refers to 20-25 °C.

For syntheses referencing procedures in other Examples, reaction conditions (length of reaction and temperature) may vary. In general, reactions were followed by thin layer chromatography or mass spectrometry, and subjected to work-up when appropriate. Purifications may vary between experiments: in general, solvents and the solvent ratios used for eluents/gradients were chosen to provide appropriate R_{f}s or retention times.

In the discussion above and in the examples below, the following abbreviations have the following meanings. If an abbreviation is not defined, it has a generally accepted meaning known to those skilled in the art.
- Aq.: = aqueous
- bm: = broad multiplet
- BOC: = *tert*-butoxycarbonyl
- bd or bd d: = broad doublet
- bs or bd s: = broad singlet
- br dd: = broad doublet of doublets
- CDI: = 1,1'-carbonyldiimidazole
- d: = doublet
- dd: = doublet of doublets
- dq: = doublet of quartets
- dt: = doublet of triplets
- DMF: = dimethylformamide
- DMA: = dimethylacetamide
- DMAP: = dimethylaminopyridine
- DMSO: = dimethyl sulfoxide
- eq.: = equivalents
- g: = grams
- h: = hours
- HPLC: = high pressure liquid chromatography
- KF: = Karl Fischer (test for moisture content)
- LG: = leaving group
- m: = multiplet
- M: = molar
- M%: = mole percent
- max: = maximum
- meq: = milliequivalent
- mg: = milligram
- mL: = milliliter
- mm: = millimeter
- mmol: = millimol
- q: = quartet
- s: = singlet
- t or tr: = triplet
- TBS: = tert-butyldimethylsilyl
- TFA: = trifluoroacetic acid
- THF: = tetrahydrofuran
- TLC: = thin layer chromatography
- p-TLC: = preparative thin layer chromatography
- µL: = microliter
- N: = normality
- MeOH: = methanol
- DCM: = dichloromethane
- HCl: = hydrochloric acid
- ACN: = acetonitrile
- MS: = mass spectrometry
- rt: = room or ambient temperature
- EtOAc: = ethyl acetate
- EtO: = ethoxy
- Ac: = acetate
- NMP: = 1-methyl-2-pyrrolidinone
- µL: = microliter
- J: = coupling constant
- NMR: = nuclear magnetic resonance
- MHz: = megahertz
- Hz: = hertz
- m/z: = mass to charge ratio
- min: = minutes
- ppt: = precipitate
- CBZ: = benzyloxycarbonyl
- DCC: = 1,3-dicyclohexylcarbodiimide
- PyBop: = benzotriazole-1-yl-oxy-trispyrrolidinophosphonium hexafluorophosphate
- Pd(dppf)Cl₂: = bis(diphenylphosphino)ferrocenepalladium(II) chloride Pd(dppf)Cl₂ DCM complex
- Pd tetrakis: = Tetrakis(triphenylphosphine)palladium(0)
- Pd (II) EnCat=: Pd (II) EnCat™BINAP 30
- LDA: = lithium diisopropylamide
- mCPBA: = meta-chloroperbenzoic acid
- TMS: = trimethyl silyl
- TPP: = triphenyl phosphine
- TPPO: = triphenyl phosphine oxide
- DME: = dimethyl ether
- IPA: = isopropanol
- Et₂O: = diethyl ether
- LiHMDS: = lithium hexamethyldisilazide/ lithium bis(trimethylsilyl)amide
- 9-BBN: = 9-Borabicyclo[3.3.1]nonane
- sat.: = saturated
- UV: = ultraviolet
- v br s: = very broad singlet

### PREPARATION OF STARTING MATERIALS

### Preparation 1

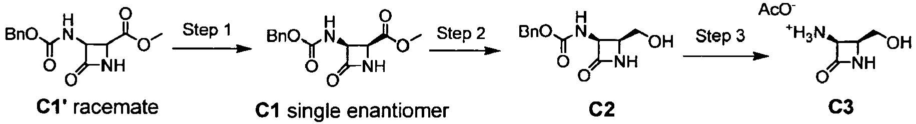

**Step 1: Preparation of C1.** For the synthesis of **C1'** racemate, see Kishimoto, S., et al., Chemical & Pharmaceutical Bulletin 1984, 32, 2646-2659. Chiral resolution of **C1'** racemate was achieved by supercritical fluid chromatography (Chiralcel OJ-H: CO₂ / propanol) to afford **C1** as a white solid. For literature characterization of **C1,** see Takahashi, Y., et al., Chemical & Pharmaceutic al Bulletin 1986, 34, 2732-2742. Yield: 32.84 g, 0.12 mmol, 25%, 99.7% ee. LCMS *m*/*z* 279.2 (M+1). [α]²⁰ +81.93° (c 0.035, CHCl₃). ¹H NMR (400 MHz, DMSO-*d*₆) δ 8.60 (br s, 1 H), 8.14 (d, *J*=8.8 Hz, 1 H), 7.34-7.29 (m, 5H), 5.05-5.01 (m, 3H), 4.35 (d, *J*=5.5 Hz, 1 H), 3.53 (s, 3H).

**Step 2: Preparation of C2.** A solution of **C1** (72.5 g, 260 mmol) in methanol (725 mL) at 20 °C was treated with a suspension of sodium borohydride (18.7 g, 495 mmol) in isopropyl alcohol (145 mL) added portionwise over 30 minutes, the temperature was maintained between 26-33 °C. The reaction mixture was stirred for 20 minutes. The methanol was removed using the rotary evaporator and the mixture was treated with brine solution (200 mL) and water (200 mL). The white slurry was extracted with ethyl acetate (700 mL) and washed with brine solution (3 x 200 mL). The aqueous layer was back extracted with ethyl acetate / isopropyl alcohol (10:1, 2 x 220 mL) and the combined organic layers were dried over magnesium sulfate. The suspension was filtered under vacuum and the filtrate concentrated using the rotary evaporator to give crude material (81.2 g) as a solid. The crude material was treated with ethyl acetate (400 mL) followed by Darco KB (2 g) and Celite (5 g) and the mixture was stirred at room temperature for 30 minutes. The mixture was filtered and the solids washed with ethyl acetate (100 mL). The filtrate was treated with heptane (750 mL) over 30 minutes. The white slurry was filtered and the solid washed with ethyl acetate / heptane (2 : 3, 150 mL) to afford **C2** as a white solid. Yield: 59.3 g, 237 mmol, 91%. LCMS *m*/*z* 251.6 (M+1). [α]²⁰ +9.03° (*c* 0.064, CHCl₃). mp 125-127 °C. ¹H NMR (400 MHz, CDCl₃) δ 7.35-7.28 (m, 5H), 5.67 (br s, 1 H), 6.18 (d, *J*=9.9 Hz, 1 H), 5.15 (dd, *J*=9.8, 4.8 Hz, 1 H), 5.08 (s, 2H), 3.85-3.79 (m, 2H), 3.65 (m, 1 H), 3.36 (br s, 1 H). For alternative **C2** synthesis and literature characterization, see R. Thomas, Tetrahedron Letters 1989, 30, 5239-5242.

**Step 3: Preparation of C3.** To a solution of **C2** (29.4 g, 117.6 mol) in ethanol (589 mL) was added 9.0 g of Darco (Norit KB). The resulting slurry was stirred for 1 hour and the Darco was removed by vacuum filtration, then the Darco cake was rinsed with ethanol (294 mL). The ethanolic filtrate was treated with glacial acetic acid (13.5 mL, 236 mmol) and the resulting mixture was charged with 5.9 g of 20% palladium hydroxide. The mixture was purged with nitrogen followed by hydrogen and pressurized to 50 psi hydrogen. The reaction mixture was agitated at 25 °C for 16 hours. The mixture was filtered and the filter cake was rinsed with ethanol (150 mL). The ethanolic filtrate was concentrated to afford **C3** as an oil. Yield: 26.82 g. ¹H NMR (400 MHz, CD₃OD) δ 4.29 (d, J=4.8 Hz, 1 H), 3.89 (dd, J=13.0, 4.8 Hz, 1 H), 3.76-3.81 (m, 2H), 1.98 (s, 3H).

### Preparation 2

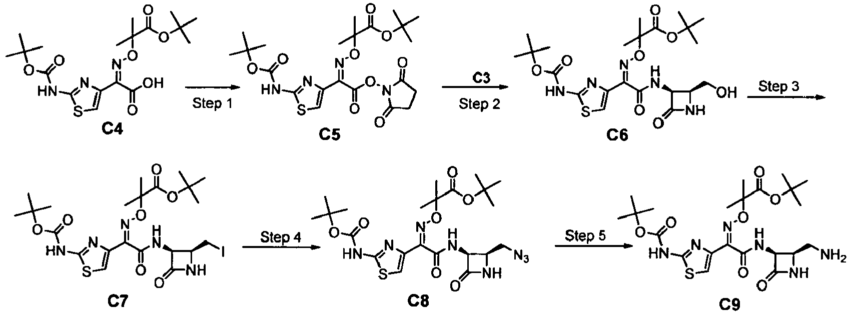

**Step 1: Preparation of C5.** 1-Hydroxypyrrolidine-2,5-dione (8.84 g, 76.8 mmol) was added to a suspension of **C4** (30 g, 70 mmol) in dichloromethane (400 mL). For synthesis of **C4.** see Yamawaki. K.. et al., Bioorganic and Medicinal Chemistry 2007, 15, 6716-6732. The mixture was cooled to 0 °C, *N,N*'-dicyclohexylcarbodiimide (97%, 15.6 g, 73.3 mmol) was added, and the reaction was stirred at 0 °C for 30 minutes and then at room temperature for 3 hours. The mixture was filtered through Celite and concentrated in vacuo to afford **C5** as a colorless solid. Yield: 36.17 g, 68.7 mmol, 98%. LCMS m/z 527.2 (M+1). ¹H NMR (400 MHz, CDCl₃) δ 8.31 (br s, 1 H), 7.50 (s, 1 H), 2.91 (br s, 4H), 1.61 (s, 6H), 1.54 (s, 9H), 1.43 (s, 9H).

**Step 2: Preparation of C6**. A flask charged with **C3** (26.82 g) was treated with a solution of C5 (41.5 g, 78.81 mmol) in acetonitrile (200 mL), followed by triethylamine (55.0 mL, 394.6 mmol) added over 5 minutes. The reaction mixture was stirred overnight for 16 hours. The solution was concentrated by a rotary evaporator to yield a yellow glass. The glass was dissolved in methyl *tert*-butyl ether (500 mL) and washed with water (1 x 250 mL), saturated aqueous sodium bicarbonate solution (1 x 250 mL), saturated brine solution (1 x 250 mL), 1% aqueous potassium carbonate solution (1 x 500 mL) and saturated brine solution (1 x 500 mL). The methyl *tert*-butyl ether organic layer was concentrated using the rotary evaporator to give crude material (38.0 g) as an off-white solid. The crude material (38.0 g) was treated with acetone (95 mL) and heptane (285 mL). The mixture was heated to 45 °C and was held at this temperature for 30 minutes. The thin slurry was cooled to 20 °C and stirred for 16 hours. The off-white solids were collected by vacuum filtration and the isolated filter cake was washed with 25% acetone - heptane (100 mL) to afford a white solid. The solid was dried in a vacuum oven at 30 °C to afford **C6.** Yield: 23.13 g, 43.8 mmol, 56%. LCMS *m*/*z* 528.1 (M+1). ¹H NMR (400 MHz, CDCl₃) δ 8.80 (br s, 1 H), 8.02 (d, *J*=7.7 Hz, 1 H), 7.31 (s, 1H), 6.48 (br s, 1H), 5.44 (br dd, *J*=7.7, 4.8 Hz, 1H), 4.30-4.36 (m, 1H), 4.02-4.06 (m, 1 H), 3.84-3.89 (m, 2H), 1.57 (s, 3H), 1.55 (s, 3H), 1.53 (s, 9H), 1.45 (s, 9H).

**Step 3: Preparation of C7.** To a solution of **C6** (10.0 g, 18.9 mmol) in anhydrous dichloromethane (100 mL) at 20 °C under nitrogen was added triphenylphosphine (10.0 g, 38 mmol), followed by imidazole (2.58 g, 38 mmol). The reaction mixture was treated with iodine (9.62 g, 38 mmol) in portions over 10 minutes. The solution was allowed to warm to 20 °C and stirring was continued for 15 hours. The reaction mixture was washed with saturated aqueous sodium thiosulfate (100 mL) and the aqueous layer was extracted with dichloromethane (2 x 100 mL). The combined organic layers were washed with brine solution (100 mL), dried over sodium sulfate, filtered under vacuum and the filtrate was concentrated using the rotary evaporator to give crude material (24 g) as an orange foam. The orange foam was purified by chromatography on silica gel (heptane /ethyl acetate 30 to 80%) to afford **C7** as a white foam. Yield: 9.41 g, 14.8 mmol, 78%. LCMS *m*/*z* 638.0 (M+1). ¹H NMR (400 MHz, DMSO-*d*₆) δ 11.83 (br s, 1H), 9.24 (d, *J*=9.0 Hz, 1H), 8.71 (br s, 1H), 7.29 (d, *J*=0.8 Hz, 1H), 5.17 (ddd, *J*=9.0, 4.9, 1.7 Hz, 1H), 4.11 (ddd, *J*=10.6, 4.8, 3.7 Hz, 1 H), 3.28 (dd, *J*=10.4, 3.6 Hz, 1 H), 3.17 (dd, *J*=10.5, 10.4 Hz, 1 H), 1.46 (s, 9H), 1.42 (s, 3H), 1.40 (s, 12H).

**Step 4: Preparation** of **C8.** A solution of **C7** (6.3 g, 9.88 mmol) in 2-methyltetrahydrofuran (60 mL) under nitrogen at 20 °C was treated with triethylamine (2.75 mL, 19.76 mmol), followed by dropwise addition of a 15% solution of tetrabutylammonium azide in tetrahydrofuran (22.49 g, 11.86 mmol). The reaction mixture was stirred at 20 °C for 2 hours, and then heated at 35 °C and stirred for 15 hours. The solution was cooled to room temperature, filtered under vacuum, the white solid was washed with methyl *tert*-butyl ether (2 x 100 mL), the filtrate was collected and the solvent was removed using the rotary evaporator to give a foam. The foam was dissolved in methyl *tert*-butyl ether (200 mL), washed with water (2 x 100 mL), brine solution (100 mL), dried over sodium sulfate, and filtered under vacuum. The filtrate was collected and concentrated using the rotary evaporator to afford a foam. The foam was dissolved in acetonitrile and concentrated (3 x 15 mL) and the residue was held under high vacuum to give **C8** as a yellow foam. Yield: 5.24 g, 9.48 mmol, 96%. LCMS *m*/*z* 553.1 (M+1). ¹H NMR (400 MHz, DMSO-*d*₆) δ 11.82 (br s, 1 H), 9.19 (d, *J*=8.9 Hz, 1 H), 8.67 (br s, 1 H), 7.28 (s, 1 H), 5.24 (ddd, *J*=8.7, 5.1, 1.4 Hz, 1 H), 3.89-3.95 (m, 1 H), 3.64 (dd, *J*=12.9, 3.9 Hz, 1H), 3.39 (dd, *J*=12.9, 8.9 Hz, 1H), 1.46 (s, 9H), 1.44 (s, 3H), 1.42 (s, 3H), 1.40 (s, 9H).

**Step 5: Preparation of C9.** A Parr shaker vessel was charged with **C8** (14.37 g, 26.0 mmol) and ethanol (140 mL). The mixture was purged with nitrogen and then treated with 10% palladium on carbon (5.7 g) and pressurized to 30 psi hydrogen. The reaction mixture was agitated at room temperature for 4 hours. The solution was filtered through a micro filter and the solvent was removed using the rotary evaporator to afford **C9** as a brown solid. Yield: 13.22 g, 25.1 mmol, 97%. LCMS *m*/*z* 527.1 (M+1). ¹H NMR (400 MHz, DMSO-*d*₆) δ 9.1-9.3 (v br s, 1 H), 8.25 (br s, 1H), 7.26 (s, 1H), 5.17 (d, *J*=4.9 Hz, 1H), 3.65 (ddd, *J*=6, 6, 5 Hz, 1H), 2.78 (dd, *J*=13.4, 5.8 Hz, 1H), 2.62 (dd, *J*=13.4, 6.3 Hz, 1 H), 1.46 (s, 9H), 1.43 (s, 3H), 1.41 (s, 3H), 1.39 (s, 9H).

### Preparation 3

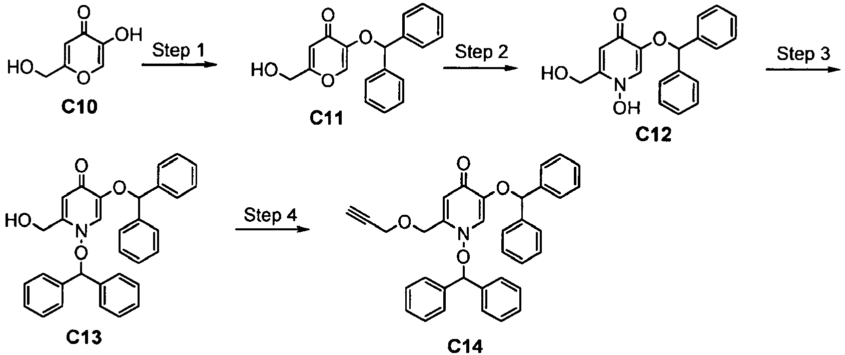

**Step 1: Preparation of C11.** A solution of **C10** (7.00 g, 49.3 mmol) in *N,N-*dimethylformamide (141 mL) was treated with potassium carbonate (14.33 g, 103.7 mmol) and 1,1'-(bromomethylene)dibenzene (14.60 g, 59.1 mmol). The resulting mixture was heated to 80 °C overnight, then cooled to room temperature and concentrated in vacuo to remove solvents. The resulting residue was taken up in ethyl acetate / water and the layers were separated. The aqueous layer was back extracted two times with ethyl acetate. The combined organic layers were washed three times with water, once with brine solution, dried over magnesium sulfate, filtered and concentrated in vacuo to give a sticky oil. The residue was purified by chromatography on silica gel (dichloromethane / methanol 0 to 10%) to afford **C11** as a sticky brown oil. Yield: 5.48 g, 17.7 mmol, 36%. ¹H NMR (400 MHz, CDCl₃) δ 7.46 (s, 1H), 7.25-7.45 (m, 10H), 6.48-6.49 (m, 1 H), 6.37 (s, 1 H), 4.40 (br s, 2H), 2.65 (br s, 1 H).

**Step 2: Preparation of C12.** A suspension of **C11** (4.97 g, 16.1 mmol) in a mixture of ethanol (20 mL) and water (20 mL) was treated with hydroxylamine hydrochloride (11.40 g, 164.1 mmol) and sodium acetate trihydrate (22.40 g, 164.6 mmol). The resulting mixture was heated to 60 °C overnight, then cooled to room temperature and filtered to remove white solids. The solid was washed sequentially with water, ethanol and ethyl acetate and dried in vacuo providing **C12** as a white solid. Yield: 1.91 g, 5.91 mmol, 37%. ¹H NMR (400 MHz, DMSO-*d*₆) δ 10.59-10.77 (br s, 1H), 7.87-8.01 (br s, 1H), 7.50 (br d, *J*=7.4 Hz, 4H), 7.36 (br dd, *J*=7.6, 7.4 Hz, 4H), 7.25-7.30 (m, 2H), 6.86-7.00 (br s, 1 H), 6.64 (s, 1 H), 5.40-5.54 (br s, 1 H), 4.38 (br s, 2H).

**Step 3: Preparation of C13.** A suspension of **C12** (1.91 g, 5.91 mmol) in dimethyl sulfoxide (30 mL) was heated at 100 °C to dissolve the compound. The reaction mixture was cooled to room temperature and treated with potassium carbonate (1.22 g, 8.85 mmol), sodium iodide (1.33 g, 8.90 mmol) and chlorodiphenylmethane (1.60 mL, 9.00 mmol). The mixture was stirred at room temperature overnight and then treated with ice cold water. The yellow solid was filtered, dissolved in excess ethyl acetate and washed twice with water and once with brine solution. Theorganic layer was concentrated to dryness and the resulting solid was suspended in a minimal amount of ethyl acetate, collected by filtration and washed with ethyl acetate to afford **C13** as a light yellow solid. Yield: 2.66 g, 5.43 mmol, 92%. ¹H NMR (400 MHz, CD₃OD) δ 7.33-7.43 (m, 15H), 7.26-7.31 (m, 2H), 7.19-7.24 (m, 4H), 6.43-6.44 (m, 1H), 6.18 (s, 1H), 6.04 (s, 1H), 4.27 (s, 2H).

**Step 4: Preparation of C14.** A solution of **C13** (1.00 g, 2.04 mmol) in dimethyl sulfoxide (7.00 mL) was treated with cesium carbonate (1.41 g, 4.33 mmol) and a solution of propargyl bromide 80% in toluene (342.5 µL, 3.10 mmol). The resulting suspension was allowed to stir at room temperature overnight. Additional propargyl bromide 80% in toluene (225 µL, 2.02 mmol) and cesium carbonate (0.70 g, 2.15 mmol) were added and the reaction mixture was stirred overnight. Additional cesium carbonate (0.356 g, 1.09 mmol) was added to the reaction mixture and stirring was continued for four days at room temperature. The reaction mixture was diluted with water and ethyl acetate and the layers separated. The aqueous layer was back extracted three times with ethyl acetate. The combined organic layers were washed once with water and once with brine solution and dried over magnesium sulfate. The mixture was filtered, concentrated in vacuo and purified by chromatography on silica gel (heptane / ethyl acetate 15 to 100%) to afford **C14** as a solid. Yield: 0.353 g, 0.67 mmol, 33%. LCMS *m*/*z* 528.7 (M+1). ¹H NMR (400 MHz, CDCl₃) δ 7.20-7.46 (m, 20H), 6.77 (s, 1 H), 6.37 (s, 1 H), 6.10 (s, 1 H), 5.99 (s, 1 H), 4.23 (s, 2H), 4.01 (d, *J*=2.3 Hz, 2H), 2.36 (t, *J*=2.3 Hz, 1 H).

### Preparation 4

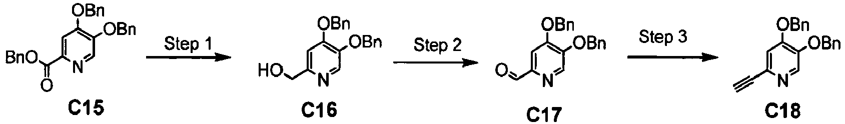

**Step 1: Reparation of C16.** A suspension of C15 (10.0 g, 23.5 mmol) in ethanol (100 mL) at 0 °C was treated with sodium borohydride (8.89 g, 235 mmol) added portionwise over a 20 minute period. For the synthesis of C15, see WO 2010070523. The reaction was warmed to room temperature as the ice bath expired and stirring continued overnight. The reaction was quenched by addition of 1 N aqueous sodium hydroxide (100 mL) and then concentrated in vacuo. The residue was partitioned between dichloromethane and water. The layers were separated and the aqueous layer was extracted with dichloromethane. The combined organic layers were dried over sodium sulfate and concentrated in vacuo. The resultant residue was purified by chromatography on silica gel (98:2 dichloromethane / methanol) to provide **C16** as a white solid. Yield: 7.0 g, 21.7 mmol, 92%. LCMS *m*/*z* 322.4 (M+1). ¹H NMR (400 MHz, DMSO-d₆) δ 8.11 (s, 1H), 7.45-7.49 (m, 2H), 7.29-7.44 (m, 8H), 7.19 (s, 1H), 5.30 (t, *J*=5.8 Hz, 1H), 5.23 (s, 2H), 5.17 (s, 2H), 4.43 (d, *J*=5.8 Hz, 2H).

**Step 2: Preparation of C17.** A solution of **C16** (1.0 g, 3.11 mmol) in dichloromethane (15 mL) at 0 °C under nitrogen was treated with dimethyl sulfoxide (2.43 g, 31.1 mmol), triethylamine (1.57 g, 15.6 mmol) and sulfur trioxide pyridine complex (1.98 g, 12.4 mmol). After 2 hours the reaction was quenched by addition of water and then concentrated in vacuo. The resulting suspension was partitioned between diethyl ether and water. The organic layer was washed with brine solution, dried over sodium sulfate, and concentrated in vacuo. The resultant residue was purified by chromatography on silica gel (70:30 hexanes / ethyl acetate) to provide **C17** as a colorless solid. Yield: 0.95 g, 2.95 mmol, 96%. LCMS *m*/*z* 320.4 (M+1). ¹H NMR (400 MHz, DMSO-*d*₆) δ 9.81 (s, 1 H), 8.51 (s, 1 H), 7.61 (s, 1 H), 7.44-7.49 (m, 4H), 7.38-7.43 (m, 4H), 7.32-7.37 (m, 2H), 5.38 (s, 2H), 5.35 (s, 2H).

**Step 3: Preparation of C18.** A solution of **C17** (800 mg, 2.50 mmol) in methanol (25 mL) was treated with potassium carbonate (692 mg, 5.01 mmol) and (1-diazo-2-oxopropyl)phosphonic acid dimethyl ester (530 mg, 2.76 mmol). The resulting suspension was stirred at room temperature for 2 hours. The reaction was filtered through Celite and concentrated in vacuo. The resultant residue was purified by chromatography on silica gel (70:30 hexanes / ethyl acetate) to provide **C18** as a white solid. Yield: 0.6 g, 1.87 mmol, 76%. LCMS *m*/*z* 316.4 (M+1). ¹H NMR (400 MHz, DMSO-*d*₆) δ 8.21 (s, 1 H), 7.32-7.46 (m, 10H), 7.30 (s, 1H), 5.26 (s, 2H), 5.23 (s, 2H), 4.15 (s, 1H).

### Preparation 5

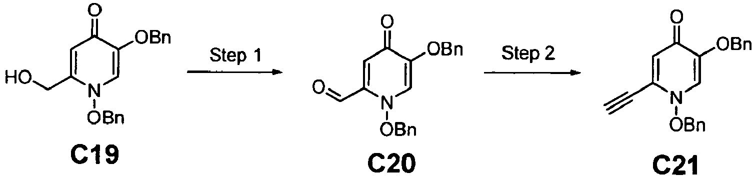

**Step 1: Preparation of C20.** Dimethyl sulfoxide (10 mL), triethylamine (6.70 mL, 48.1 mmol) and sulfur trioxide pyridine complex (5.90 g, 37.1 mmol) were added sequentially to a cooled (0 °C) solution of **C19** (2.50 g, 7.41 mmol) in anhydrous dichloromethane (27 mL). The reaction mixture was stirred at 0 °C for 3 hours and then diluted with ethyl acetate and water. The organic layer was washed four times with water, dried over sodium sulfate, filtered and concentrated. The residue was purified by chromatography on silica gel (ethyl acetate / 2-propanol) to yield **C20** as a bright yellow solid. Yield: 1.54 g, 4.59 mmol, 62%. ¹H NMR (400 MHz, CDCl₃) δ 9.65 (s, 1H), 7.31-7.49 (m, 8H), 7.17 (br d, *J*=8 Hz, 2H), 7.07 (s, 1H), 6.76 (s, 1H), 5.14 (s, 2H), 5.05 (s, 2H).

**Step 2: Preparation of C21.** Potassium carbonate (0.124 g, 0.897 mmol) and (1-diazo-2-oxopropyl)phosphonic acid dimethyl ester (0.100 g, 0.521 mmol) were added to a solution of **C20** (0.150 g, 0.447 mmol) in anhydrous methanol (4.0 mL). The reaction was allowed to stir at room temperature for 2 hours and filtered through Celite and the filter cake was washed with ethyl acetate. The filtrate was concentrated and the crude product purified by chromatography on silica gel (ethyl acetate / 2-propanol) to giving **C21** as glassy solid. Yield: 0.041 g, 0.124 mmol, 28%. LCMS *m*/*z* 332.1 (M+1). ¹H NMR (400 MHz, CDCl₃) δ 7.28-7.47 (m, 10H), 6.86 (s, 1 H), 6.61 (s, 1H), 5.11 (s, 2H), 4.93 (s, 2H), 3.58 (s, 1 H).

### Preparation 6

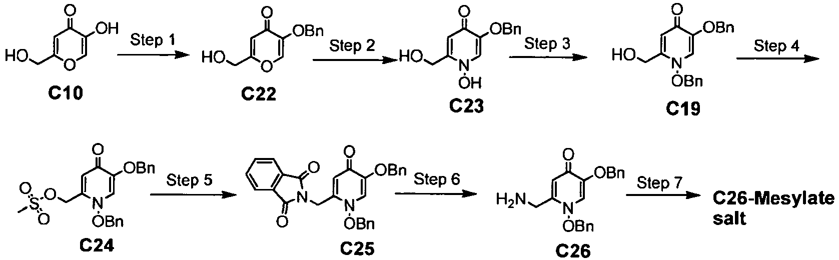

**Step 1: Preparation of C22.** A suspension of **C10** (48.92 g, 344.2 mmol) in methanol (313 mL) was treated with 1 *M* aqueous sodium hydroxide (32 mL, 379 mmol). To the mixture was added benzyl bromide (64.8 g, 379 mmol) dropwise over 10 minutes. The reaction mixture was refluxed overnight, then cooled to room temperature and treated with 1 N aqueous hydrochloric acid (600 mL) and stirred at room temperature for 20 minutes. The mixture was filtered and the solids washed with water (3 x 200 mL), then stirred in a mixture of heptanes (180 mL) and ethyl acetate (120 mL). The solid was collected by filtration to provide C22. Yield: 62.5 g, 269.0 mmol, 78%. LCMS *m*/*z* 233.3 (M+1). ¹H NMR (400 MHz, CDCl₃) δ 7.53 (s, 1H), 7.31-7.42 (m, 5H), 6.52 (s, 1H), 5.07 (s, 2H), 4.46 (d, *J*=6.4 Hz, 2H), 2.83-2.91 (m, 1 H).

**Step 2: Preparation of C23.** A suspension of **C22** (350 g, 1510 mmol) in ethanol (500 mL) and water (3500 mL) was treated with hydroxylamine hydrochloride (800 g, 11000 mmol), followed by sodium acetate trihydrate (930 g, 11300 mmol). The reaction mixture was heated at 60 °C overnight. The mixture was cooled to room temperature and ethanol was removed in vacuo. The resulting solid was collect by filtration, washed with water (200 mL) and dried under high vacuum to provide **C23.** Yield: 191.3 g, 773.7 mmol 51.3%. LCMS *mlz* 248.1 (M+1). ¹H NMR (400 MHz, CD₃OD) δ 7.89 (s, 1H), 7.45-7.49 (m, 2H), 7.30-7.41 (m, 3H), 7.00 (s, 1H), 5.19 (s, 2H), 4.67 (s, 2H).

**Step 3: Preparation of C19.** A solution of **C23** (53.0 g, 214.4 mmol) in dimethyl sulfoxide (268 mL) was treated with potassium carbonate (40.2 g, 257.0 mmol) and benzyl bromide (40.3 g, 236 mmol). The reaction mixture was stirred at room temperature for 1 hour. The mixture was diluted with water (1.5 L) and extracted with ethyl acetate (3 x 250 mL). The combined organic extracts were washed with water (2 x 100 mL), dried over magnesium sulfate, filtered, and concentrated to dryness to afford a solid. The solid was treated with ethyl acetate (200 mL) and the suspension was warmed to reflux, then allowed to cool to room temperature. The solid was collected by filtration to provide **C19.** Yield: 55.4 g, 164.2 mmol, 76.6%. LCMS *m*/*z* 338.4 (M+1). ¹H NMR (400 MHz, DMSO-*d*₆) δ 8.00 (s, 1 H), 7.32-7.53 (m, 10H), 6.13 (s, 1 H), 5.58 (br t, *J*=5 Hz, 1 H), 5.24 (s, 2H), 5.00 (s, 2H), 4.40 (d, *J*=5.2 Hz, 2H).

**Step 4: Preparation of C24.** A suspension of **C19** (43 g, 130 mmol) in dichloromethane (212 mL) was treated with triethylamine (21.2 mL, 153 mmol) and cooled to 0 °C. To the reaction mixture was added methanesulfonyl chloride (10.5 mL, 134 mmol) and the mixture was stirred for 1 hour. The reaction was treated with saturated aqueous ammonium chloride (100 mL) and the organic layer was separated, dried over sodium sulfate, filtered and concentrated to provide **C24** as a solid. Yield: 53 g, 130 mmol, quantitative. LCMS *m*/*z* 416.4 (M+1).

**Step 5: Preparation of C25.** A suspension of **C24** (53.0 g, 130 mmol) in *N,N-*dimethylformamide (255 mL) was treated with potassium phthalimide (25.3 g, 134 mmol) and potassium iodide (0.83 g, 4.96 mmol). The reaction was stirred at room temperature overnight. The reaction mixture was treated with water (1000 mL) and ethyl acetate (1000 mL). The aqueous layer was removed and the organic layer was washed with water (4 x 500 mL), dried over sodium sulfate, filtered and concentrated to provide **C25** as an oil. Yield: 53.0 g, 113.6 mmol, 89%. LCMS *m*/*z* 467.0 (M+1). ¹H NMR (400 MHz, DMSO-*d*₆) δ 8.07 (s, 1 H), 7.86-7.93 (m, 4H), 7.53-7.57 (m, 2H), 7.32-7.48 (m, 8H), 5.88 (s, 1 H), 5.36 (s, 2H), 5.00 (s, 2H), 4.72 (s, 2H).

**Step 6: Preparation of C26.** A solution of **C25** (5.0 g, 10.72 mmol) in ethanol (53 mL) was treated with hydrazine hydrate (64-65%, 5.3 mL, 107.2 mmol). The reaction was refluxed for 1 hour then cooled to room temperature. The mixture was diluted with ethanol (100 mL) and stirred for 5 minutes. The reaction mixture was filtered through Celite and the filter cake was washed with ethanol (100 mL). The filtrate was concentrated and ethanol (50 mL) was added. The resulting solids were removed by filtration and the filtrate was concentrated to provide **C26** as a solid. Yield: 3.00 g, 8.92 mmol, 83%. LCMS *m*/*z* 337.1 (M+1). ¹H NMR (400 MHz, DMSO-*d*₆) δ 7.97 (s, 1 H), 7.31-7.52 (m, 10H), 6.22 (s, 1 H), 5.24 (s, 2H), 5.01 (s, 2H), 3.64 (s, 2H), 2.0-2.8 (v br s, 2H).

**Step 7: Preparation of C26-mesylate salt.** A suspension of **C26** (0.5 g, 1.49 mmol) in a mixture of ethyl acetate (30 mL) and water (0.3 mL) was treated with methanesulfonic acid (0.107 mL, 1.64 mmol). The mixture was heated at 50 °C for 2 hours and then allowed to cool to room temperature. The reaction mixture was stirred for an additional 15 hours at room temperature. The suspension was filtered under vacuum and the white solid was washed with ethyl acetate(2 x 3 mL). The solid was dried under high vacuum for 4 hours to give C26-meslyate salt as a white solid. Yield: 0.52 g, 1.2 mmol, 81%. LCMS *m*/*z* 337.1 (M+1). ¹H NMR (400 MHz, DMSO-*d*₆) δ 8.13-8.38 (br s, 3H), 8.17 (s, 1H), 7.32-7.56 (m, 10H), 6.21 (s, 1H), 5.28 (s, 2H), 5.02 (s, 2H), 3.98 (s, 2H), 2.28 (s, 3H).

### Preparation 7

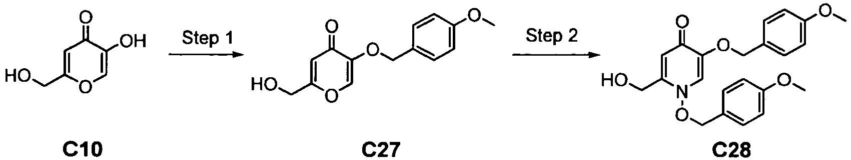

**Step1: Preparation of C27.** A solution of **C10** (10.0 g, 70.73 mmol) in *N*-methyl-2-pyrrolidone (100 mL) was treated with potassium carbonate (20.4 g, 148 mmol). p-Methoxybenzyl chloride (11.6 g, 73.9 mmol) was added and the suspension was heated at 75 °C for 2 hours. The reaction mixture was poured into ice water (ca. 400 mL) and the resulting suspension was stirred for 3 hours. The solids were collected by filtration and washed with water, followed by ethyl acetate to afford **C27.** Yield: 10.8 g, 41.1 mmol, 58%. LCMS *m*/*z* 263.1 (M+1). ¹H NMR (400 MHz, DMSO-*d*₆) δ 8.14 (s, 1H), 7.34 (br d, *J*=8.7 Hz, 2H), 6.95 (br d, *J*=8.8 Hz, 2H), 6.30-6.31 (m, 1 H), 5.67 (br t, J=5.6 Hz, 1 H), 4.86 (s, 2H), 4.29 (br d, *J*=5.1 Hz, 2H), 3.76 (s, 3H).

**Step 2: Preparation of C28.** A suspension of **C27** (10.0 g, 38.1 mmol) in N-methyl-2-pyrrolidone (50 mL) was treated with potassium carbonate (15.8 g, 114 mmol). Hydroxylamine hydrochloride (7.95 g, 114 mmol) was added and the resulting suspension was heated at 75 °C overnight. The reaction mixture was cooled to room temperature and *p*-methoxybenzyl chloride (8.96 g, 57.2 mmol) was added, then the mixture was stirred overnight at room temperature. The reaction was poured into ice water and extracted with dichloromethane (2 x). The organic layers were combined, washed with water (3 x), dried over sodium sulfate, filtered and concentrated in vacuo. The crude material was purified via chromatography on silica gel (dichloromethane / methanol) to yield **C28** as a solid. Yield: 4.0 g, 10.0 mmol, 26.4%. LCMS m/z 398.1 (M+1). ¹H NMR (400 MHz, DMSO-*d*₆) δ 7.94 (s, 1H), 7.41 (d, *J*=8.7 Hz, 2H), 7.36 (d, J=8.7 Hz, 2H), 6.94-7.00 (m, 4H), 6.11 (s, 1H), 5.53-5.59 (m, 1H), 5.16 (s, 2H), 4.92 (s, 2H), 4.36 (br d, *J*=4 Hz, 2H), 3.78 (s, 3H), 3.76 (s, 3H).

### Preparation 8

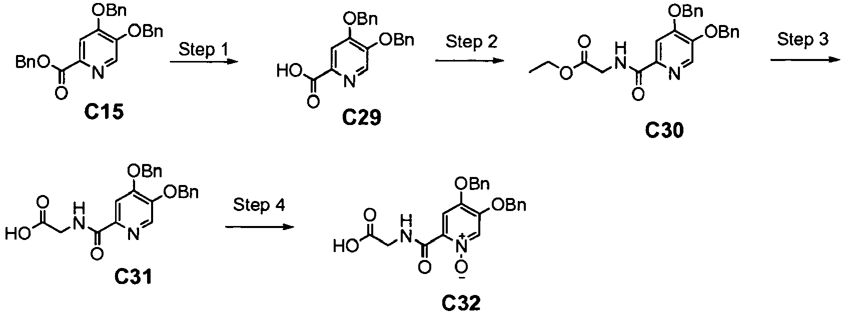

**Step 1: Preparation of C29.** A solution of 1 N aqueous potassium hydroxide (29.4 mL, 29.4 mmol) was added to a suspension of **C15** (10.0 g, 23.5 mmol) in tetrahydrofuran (100 mL). For the synthesis of **C15,** see WO 2010070523. The resulting mixture was stirred at room temperature for 7 hours. The reaction mixture was diluted with water (100 mL) and acidified to pH 2.5 with 1 N aqueous hydrochloric acid. The resulting solid was collected by filtration under vacuum, washed with water (3 x 25 mL), and dried to yield **C29** as a white solid. Yield: 7.79 g, 23.5 mmol, 99%. LCMS *m*/*z* 336.4 (M+1). ¹H NMR (400 MHz, DMSO-*d*₆) δ 8.35 (s, 1 H), 7.75 (s, 1 H), 7.31-7.48 (m, 10H), 5.33 (s, 4H).

**Step 2: Preparation of C30.** A suspension of ethyl glycinate hydrochloride (2.4 g, 17.3 mmol) in *N,N*-dimethylformamide (70 mL) was treated with triethylamine (1.75 g, 17.3 mmol), **C29** (5.8 g, 17.3 mmol), 1-hydroxybenzotriazole hydrate (2.79 g, 17.3 mmol), and dicyclohexylcarbodiimide (4.32 g, 20.8 mmol). The reaction mixture was stirred at room temperature for 65 hours, filtered, and the filter cake was rinsed with *N,N-*dimethylformamide (4 mL). The solvent was removed by vacuum distillation. The resulting crude product was recrystallized from methanol. The resulting crystals were collected by filtration and dried to yield **C30** as a clear gum. Yield: 4.92 g, 11.7 mmol, 67%. LCMS *m*/*z* 421.6 (M+1). ¹H NMR (400 MHz, DMSO-*d*₆) δ 8.89 (t, *J*=6.2 Hz, 1 H), 8.31 (s, 1H), 7.71 (s, 1H), 7.44-7.48 (m, 4H), 7.31-7.43 (m, 6H), 5.33 (s, 2H), 5.32 (s, 2H), 4.10 (q, *J*=7.1 Hz, 2H), 3.99 (d, *J*=6.2 Hz, 2H), 1.19 (t, *J*=7.1 Hz, 3H).

**Step 3: Preparation of C31.** A suspension of **C30** (4.9 g, 12 mmol) in ethanol (60 mL) was treated with water (28 mL) and sodium hydroxide (0.7 g, 18.6 mmol). The resulting mixture was heated to 50 °C for 1 hour. The ethanol was removed in vacuo and the resulting isolate was treated with water (250 mL). The solution was filtered and the filtrate was acidified in an ice bath to pH 3 with 1 N aqueous hydrochloric acid. The resulting solids were collected by filtration and dried to yield **C31** as a white solid. Yield: 4.5 g, 11.5 mmol, 98%. LCMS *m*/*z* 393.5 (M+1). ¹H NMR (400 MHz, DMSO-*d*₆) δ 8.78 (t, *J*=6.0 Hz, 1 H), 8.30 (s, 1H), 7.71 (s, 1 H), 7.31-7.48 (m, 10H), 5.33 (s, 2H), 5.32 (s, 2H), 3.93 (d, *J*=6.0 Hz, 2H).

**Step 4: Preparation of C32.** A solution of **C31** (3.46 g, 8.82 mmol) in acetonitrile (29 mL) was treated with urea hydrogen peroxide (3.25 g, 33.5 mmol). The resulting suspension was cooled in an ice bath and treated dropwise with trifluoroacetic anhydride (4.77 mL, 33.5 mmol). After the addition, the reaction was quenched with saturated sodium sulfite (30 mL). The reaction mixture was diluted with water (40 mL) and the resulting solid was isolated by filtration and dried to yield a mixture of **C31:C32** (1:4) as a white solid. Yield: 2.64 g. LCMS *m*/*z* 409.5 (M+1). ¹H NMR (400 MHz, DMSO-*d*₆), peaks attributed to product: δ 11.26 (t, *J*=4.5 Hz, 1 H), 8.24 (s, 1 H), 7.76 (s, 1 H), 7.32-7.48 (m, 10H), 5.29 (s, 2H), 5.27 (s, 2H), 3.57 (d, *J*=4.5 Hz, 2H).

### Preparation 9

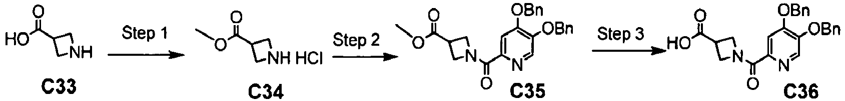

**Step 1: Preparation of C34.** A suspension of **C33** (1.0 g, 9.89 mmol) in methanol (50 mL) was cooled to 0 °C. Thionyl chloride (1.49 mL, 20.3 mmol) was slowly added dropwise over one minute. The reaction was stirred at 0 °C for 15 minutes. The cooling bath was removed and the reaction was stirred at room temperature for 2 hours. The reaction was concentrated to dryness. The residue was diluted with toluene, and then re-concentrated to dryness. The residue was dried under vacuum to afford the hydrochloride salt of **C34** as a sticky yellow solid. Yield: 1.52 g, 10.0 mmol, quantitative. ¹H NMR (400 MHz, DMSO-*d*₆) δ 9.56 (br s, 1 H), 9.24 (br s, 1 H), 3.98-4.13 (m, 4H), 3.68 (s, 3H), 3.66-3.76 (m, 1 H).

**Step 2: Preparation of C35.** *N,N*-Dimethylformamide (6.0 mL) was added to a mixture of **C34** (100 mg, 0.660 mmol), **C29** (221 mg, 0.660 mmol), *O*-(7-azabenzotriazol-1-yl)-*N,N,N',N*'-tetramethyluronium hexafluorophosphate (298 mg, 0.759 mmol), and sodium bicarbonate (195 mg, 2.31 mmol). The resulting mixture was sonicated for 2 to 3 minutes, and then stirred at room temperature overnight. The reaction mixture was diluted with water and extracted three times with ethyl acetate. The combined organic extracts were washed once with brine solution, dried over magnesium sulfate, filtered and concentrated to afford the crude product which was purified by chromatography on silica gel (dichloromethane / methanol 1 to 10%). The concentrated material was dissolved in ethyl acetate and washed three times with water. The organic layer was dried over magnesium sulfate, filtered and concentrated to afford a colorless gum. The residue was dissolved in dichloromethane and heptane and concentrated to dryness to afford **C35** as a sticky semi-solid. Yield: 221 mg, 0.511 mmol, 77%. LCMS *m*/*z* 433.1 (M+1). ¹H NMR (400 MHz, DMSO-*d*₆) δ 8.28 (s, 1H), 7.66 (s, 1H), 7.43-7.47 (m, 4H), 7.31-7.42 (m, 6H), 5.29 (br s, 4H), 4.74 (dd, *J*=9.8, 9.3 Hz, 1H), 4.60 (dd, *J*=10.0, 6.0 Hz, 1 H), 4.22 (dd, *J*=9.8, 9.5 Hz, 1 H), 4.08 (dd, *J*=10.0, 6.0 Hz, 1 H), 3.67 (s, 3H), 3.54 (dddd, *J*=9.1, 9.0, 5.8, 5.8 Hz, 1H).

**Step 3: Preparation of C36.** A solution of **C35** (221 mg, 0.511 mmol) in a mixture of tetrahydrofuran (4 mL) and methanol (4 mL) was treated with lithium hydroxide (107 mg, 2.56 mmol), followed by water (2 mL). The reaction was stirred overnight at room temperature. The reaction mixture was concentrated to remove tetrahydrofuran and methanol, diluted with water and the pH adjusted to between 4 and 5 by addition of 1 N aqueous hydrochloric acid. The aqueous layer was extracted three times with dichloromethane. The combined organic extracts were dried over magnesium sulfate, filtered and concentrated to dryness. The crude material was purified by chromatography on silica gel (dichloromethane / methanol 1 to 15%) to afford **C36** as an off-white solid. Yield: 120 mg, 0.287 mmol, 56%. LCMS *m*/*z 419.1* (M+1). ¹H NMR (400 MHz, DMSO-*d*₆) δ 12.68 (br s, 1 H), 8.29 (s, 1 H), 7.65 (s, 1 H), 7.31-7.47 (m, 10H), 5.29 (br s, 4H), 4.71 (dd, *J*=9.6, 9.6 Hz, 1 H), 4.57 (dd, *J*=10.2, 5.8 Hz, 1 H), 4.20 (dd, *J*=9.6, 9.6 Hz, 1 H), 4.05 (dd, *J*=10.0, 5.7 Hz, 1 H), 3.39-3.47 (m, 1 H).

### Preparation 10

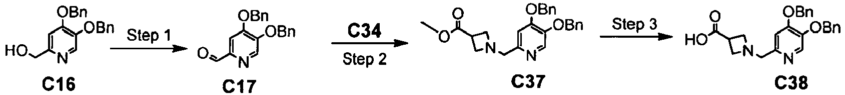

**Step 1: Preparation of C17.** A solution of **C16** (1.0 g, 3.11 mmol) in dichloromethane (15.6 mL) was treated with activated manganese dioxide (3.18 g, 31.1 mmol). The black reaction mixture was stirred overnight. Several small scoops of magnesium sulfate were added to the reaction mixture, which was then filtered through a pad of Celite washing with additional dichloromethane. The filtrate was concentrated to afford **C17** as a white solid. Yield: 820 mg, 2.57 mmol, 82%. LCMS *m*/*z* 320.1 (M+1). ¹H NMR (400 MHz, CDCl₃) δ 9.90 (s, 1 H), 8.32 (s, 1 H), 7.58 (s, 1 H), 7.32-7.48 (m, 10H), 5.33 (s, 2H), 5.28 (s, 2H).

**Step 2: Preparation of C37.** A solution of **C34** (100 mg, 0.660 mmol) in methanol (3 mL) was treated with triethylamine (0.092 mL, 0.660 mmol). The resulting solution was added to a separate solution of **C17** (211 mg, 0.660 mmol) in tetrahydrofuran (3.5 mL), followed by acetic acid (0.038 mL, 0.660 mmol) and the reaction mixture was stirred for 4 hours at room temperature. Sodium cyanoborohydride (83 mg, 1.32 mmol) was added and the reaction stirred overnight. The mixture was diluted with water and the pH adjusted to 6 - 7 with 1 N aqueous hydrochloric acid. The layers were separated and the aqueous layer was extracted three times with dichloromethane. The combined organics were dried over magnesium sulfate, filtered and concentrated in vacuo. The crude material was purified by chromatography on silica gel (dichloromethane / methanol 1 to 10%) to afford **C37** as a colorless gum. Yield: 204 mg, 0.488 mmol, 74%. LCMS *m*/*z* 419.2 (M+1). ¹H NMR (400 MHz, DMSO-*d*₆) δ 8.19 (s, 1 H), 7.30-7.49 (m, 10H), 7.16 (s, 1 H), 5.24 (s, 2H), 5.20 (s, 2H), 4.09 (br s, 2H), 3.90-3.98 (m, 2H), 3.79-3.88 (m, 2H), 3.67 (s, 3H), 3.51-3.61 (m, 1 H).

**Step 3: Preparation of C38.** A solution of **C37** (204 mg, 0.44 mmol) in tetrahydrofuran (4 mL) and methanol (4 mL) was treated with lithium hydroxide (92 mg, 2.20 mmol) followed by water (2 mL). The mixture was stirred for 4 hours at room temperature and then concentrated to remove the tetrahydrofuran and methanol. The residue was diluted with water, the pH adjusted to 7 with 1 N aqueous hydrochloric acid, and then concentrated to dryness. The crude material was purified by chromatography on silica gel (dichloromethane / methanol 5 to 75%) to afford **C38** as a colorless glass residue. Yield: 123 mg, 0.304 mmol, 69%. LCMS *m*/*z* 405.1 (M+1). ¹H NMR (400 MHz, DMSO-*d*₆), characteristic peaks: δ 8.10 (s, 1 H), 7.29-7.48 (m, 10H), 7.03 (s, 1 H), 5.23 (s, 2H), 5.15 (s, 2H), 3.51 (s, 2H).

### Preparation 11

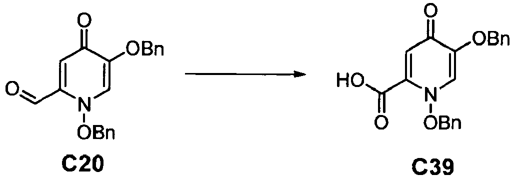

**Preparation of C39.** A solution of **C20** (8.29 g, 24.7 mmol) in acetonitrile (120 mL) at room temperature was treated with 2-methyl-2-butene (10.5 mL, 98.9 mmol) and potassium phosphate monobasic (53.8 g, 396 mmol). The resulting mixture was cooled to 0 °C and a solution of sodium chlorite (5.77 g, 68.5 mmol) in water (120 mL) was added over 15 minutes. The mixture was stirred at 0 °C for 45 minutes. To the reaction mixture was added acetonitrile (50 mL) and *tert*-butanol (30 mL) and the mixture was allowed to stir at room temperature for 2 hours. The solution was treated with potassium carbonate (68.3 g, 494 mmol) and the mixture was stirred at room temperature for 15 minutes. The mixture was concentrated to dryness in vacuo to afford a crude residue. The resulting solid was slurried in a mixture of dichloromethane and methanol (9:1, 600 mL) for 15 minutes at room temperature and the solids were collected by filtration. The solids were reslurried in dichloromethane and methanol (9:1, 500 mL) six additional times and the combined filtrates were concentrated in vacuo to afford **C39** as a tan solid. Yield: 8.47 g, 21.7 mmol, 88%. LCMS *m*/*z* 352.0 (M+1 ). ¹H NMR (400 MHz, DMSO-*d*₆) δ 7.67 (s, 1 H), 7.51-7.56 (m, 2H), 7.31-7.44 (m, 8H), 5.82 (s, 1 H), 5.37 (s, 2H), 4.95 (s, 2H).

### Preparation 12

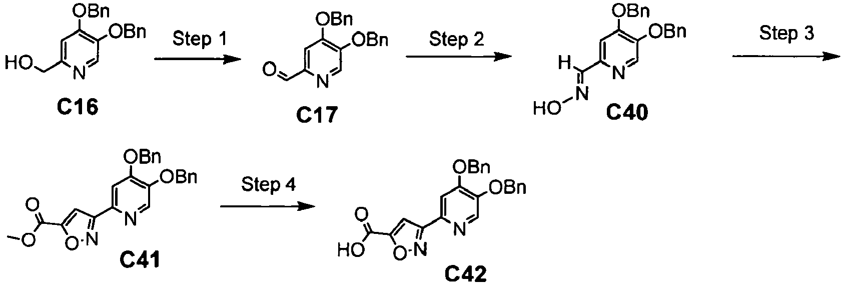

**Step 1: Preparation of C17.** A solution of **C16** (2.53 g, 7.87 mmol) in acetonitrile (25 mL) was treated with 1-hydroxy-1,2-benziodoxol-3(1*H*)-one-1-oxide (3.41 g, 11.8 mmol) and heated at 65 °C overnight. The reaction mixture was treated with brine solution (30 mL) and extracted with dichloromethane (3 x 30 mL). The combined organic layers were dried over sodium sulfate, filtered and concentrated in vacuo. The residue was purified by chromatography on silica gel (heptane / ethyl acetate) to afford **C17** as a white-yellow solid. Yield: 2.05 g, 6.42 mmol, 81%. APCI *m*/*z* 320.3 (M+1). ¹H NMR (400 MHz, DMSO-*d*₆) δ 9.81 (s, 1H), 8.51 (s, 1H), 7.61 (s, 1 H), 7.44-7.49 (m, 4H), 7.32-7.43 (m, 6H), 5.38 (s, 2H), 5.35 (s, 2H).

**Step 2: Preparation of C40.** A solution of **C17** (2.0 g, 6.27 mmol) in methanol (20 mL) was treated with sodium acetate (618 mg, 7.53 mmol) and hydroxylamine hydrochloride (524 mg, 7.53 mmol). The reaction mixture was heated at reflux for 3 hours. The solution was cooled to room temperature, diluted with water (10 mL) and extracted with ethyl acetate (3 x 15 mL). The combined organic layers were dried over sodium sulfate, filtered and concentrated in vacuo to afford **C40.** Yield: 1.99 g, 5.95 mmol, 94%. LCMS *m*/*z* 335.4 (M+1). ¹H NMR (400 MHz, DMSO-*d*₆) δ 11.41 (s, 1H), 8.23 (s, 1H), 7.93 (s, 1 H), 7.31-7.47 (m, 11 H), 5.28 (s, 2H), 5.24 (s, 2H).

**Step 3: Preparation of C41.** A solution of **C40** (30.68 g, 91.74 mmol) in tetrahydrofuran (400 mL) was treated with *N*-chlorosuccinimide (13.8 g, 102 mmol) and pyridine (3.71 mL, 45.9 mmol). The reaction mixture was heated for 30 minutes at 55 °C. The solution was cooled to 0 °C and treated with methyl propiolate (24.7 mL, 275 mmol) added dropwise over 10 minutes. A solution of triethylamine (12.8 mL, 91.7 mmol) in tetrahydrofuran (20 mL) was added dropwise over 45 minutes, maintaining the temperature at 5 °C. The reaction mixture was filtered and the solids were washed with tetrahydrofuran (200 mL). The filtrate was concentrated in vacuo and the residue partitioned between ethyl acetate (600 mL) and water (300 mL). The layers were separated and the organic layer was washed with water (3 x 300 mL), brine solution (300 mL), dried over magnesium sulfate, filtered and concentrated in vacuo to afford **C41** as a white solid. Yield: 31.25 g, 75.0 mmol, 82%. LCMS *m*/*z* 417.4 (M+1). ¹H NMR (400 MHz, DMSO-*d*₆) δ 8.39 (s, 1 H), 7.78 (s, 1 H), 7.59 (s, 1 H), 7.27 - 7.50 (m, 10H), 5.34 (s, 2H) 5.28 (s, 2H), 3.90 (s, 3H).

**Step 4: Preparation of C42.** A solution of **C41** (5.0 g, 12.0 mmol) in a mixture of tetrahydrofuran (30 mL) and methanol (30 mL) was treated with 1 N aqueous lithium hydroxide (0.86 g, 36.0 mmol, in water (30 mL)). The reaction mixture was stirred at room temperature for 2 hours. The solution was acidified to pH 3 with 1 N aqueous hydrogen chloride solution and the solids collected by vacuum filtration to afford **C42.** Yield: 4.43 g, 11.0 mmol, 92%. LCMS *m*/*z* 403.1 (M+1). ¹H NMR (400 MHz, DMSO-*d*₆) δ 8.39 (s, 1 H), 7.72 (s, 1 H), 7.27 - 7.60 (m, 11 H), 5.34 (s, 2H), 5.24 (s, 2H).

### Preparation 13

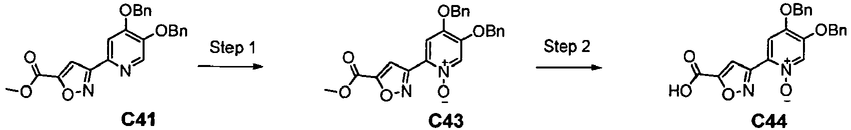

**Step 1: Preparation of C43.** A solution of **C41** (27.47 g, 65.96 mmol) in dichloromethane (625 mL) was cooled to 0 °C and treated with 3-chloroperoxybenzoic acid (37.0 g, 165 mmol). The reaction was held at 0 °C for 30 minutes, and then stirred overnight at room temperature. The reaction mixture was cooled to 0 °C and treated with saturated aqueous sodium carbonate solution (500 mL). The cooling bath was removed, and the mixture was allowed to warm to room temperature with vigorous stirring. The layers were separated and the aqueous layer was extracted with dichloromethane (1 x 500 mL). The combined organic layers were washed with saturated aqueous sodium carbonate solution (2 x 500 mL), dried over magnesium sulfate, filtered and concentrated to afford crude product. The crude material was slurried in a mixture of 12% heptane / 88% 2-methyltetrahydrofuran (220 mL). The mixture was sonicated and heated to 55-60 °C. The mixture was vigorously stirred and allowed to cool to room temperature, stirring was continued overnight. The slurry was filtered and the filter cake was washed with a mixture of 10% 2-methyl tetrahydrofuran / 90% heptane. The solids were dried under vacuum to afford **C43** as an off-white solid. Yield: 21.68 g, 50.14 mmol, 76%. LCMS *m*/*z* 433.0 (M+1). ¹H NMR (400 MHz, CDCl₃) δ 8.17 (s, 1 H), 8.00 (s, 1H), 7.66 (s, 1 H), 7.35-7.48 (m, 10H), 5.26 (s, 2H), 5.19 (s, 2H), 4.00 (s, 3H).

**Step 2: Preparation of C44.** A suspension of **C43** (2.0 g, 4.6 mmol) in a mixture of tetrahydrofuran (35 mL), methanol (35 mL), and water (23 mL) was treated with lithium hydroxide (332 mg, 13.9 mmol). The reaction mixture was stirred overnight at room temperature. The mixture was filtered and the solids washed with diethyl ether. The solids were dried under vacuum to afford **C44** as a white solid. Yield: 1.47 g, 3.46 mmol, 74.7%. LCMS *m*/*z* 419.0 (M+1). ¹H NMR (400 MHz, DMSO-*d*₆) δ 8.26 (s, 1 H), 7.60 (s, 1H), 7.32-7.48 (m, 10H), 7.29 (s, 1H), 5.30 (s, 2H), 5.26 (s, 2H).

### Preparation 14

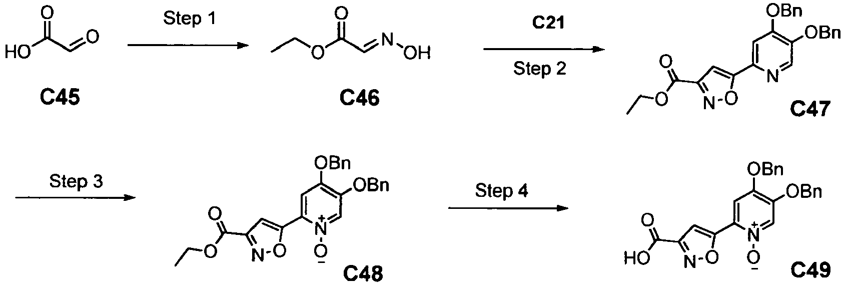

**Step 1: Preparation of C46.** A round bottom flask fitted with a Dean-Stark apparatus was charged with **C45** (2.00 g, 21.7 mmol), hydroxylamine hydrochloride (1.53 g, 22 mmol), p-toluene sulfonic acid monohydrate (0.310 g, 1.6 mmol) and ethanol (14 mL). The mixture was heated at 118 °C for 6 hours. The reaction mixture was cooled to room temperature, concentrated in vacuo and treated with diethyl ether and saturated sodium bicarbonate solution. The layers were separated and the organic layer was washed with saturated aqueous ammonium chloride solution, dried over magnesium sulfate, filtered and concentrated in vacuo to give **C46** as a clear oil. Yield: 2.04 g, 17.4 mmol, 80%. ¹H NMR (400 MHz, CDCl₃) δ 9.08 (br s, 1H), 7.57 (s, 1H), 4.34 (q, *J*=7.1 Hz, 2H), 1.36 (t, *J*=7.1 Hz, 3H).

**Step 2: Preparation of C47.** A reaction mixture containing **C21** (1.80 g, 5.71 mmol), trifluoroacetic acid (0.079 mL, 1.10 mmol), (diacetoxyiodo)benzene (7.51 g, 23 mmol) and anhydrous methanol (60 mL) was treated with a solution of **C46** (2.68 g, 22.9 mmol) in anhydrous methanol (20 mL), added over 4 hours using a syringe pump. The mixture was stirred at room temperature overnight, then concentrated directly onto 20 g of silica gel and purified by chromatography on silica gel (heptane / ethyl acetate) to yield crude **C47.** Yield: 0.660 g. LCMS *m*/z 431.1 (M+1). ¹H NMR (400 MHz, CDCl₃), product peaks only: δ 8.25 (s, 1 H), 7.53 (s, 1 H), 7.3-7.5 (m, 10H), 7.15 (s, 1 H), 5.31 (s, 2H), 5.28 (s, 2H), 4.47 (q, *J*=7.1 Hz, 2H), 1.44 (t, *J*=7.2 Hz, 3H).

**Step 3: Preparation of C48.** 3-Chloroperoxybenzoic acid (0.825 g, 3.70 mmol) was added to a cooled (0 °C) solution of **C47** (0.660 g, 1.53 mmol) in anhydrous dichloromethane (15 mL). The mixture was allowed to stir at room temperature overnight. The reaction mixture was cooled to 0 °C, diluted with brine solution and the layers separated. The aqueous layer was back extracted twice with dichloromethane. The combined organic layers were washed twice with saturated aqueous sodium carbonate solution, dried over magnesium sulfate, filtered and concentrated to a crude residue. The crude material was purified by chromatography on silica gel (dichloromethane / methanol) to yield **C48.** Yield: 0.410 g, 0.92 mmol, 60%. LCMS *m*/*z* 447.5 (M+1). ¹H NMR (400 MHz, DMSO-*d*₆), characteristic peaks: δ 8.41 (s, 1H), 7.73 (s, 1H), 5.38 (s, 2H), 5.29 (s, 2H), 4.41 (q, *J*=7.0 Hz, 2H), 1.35 (t, *J*=7.1 Hz, 3H).

**Step 4: Preparation of C49.** A solution of lithium hydroxide (0.067 g, 2.76 mmol) in water (8 mL) was added to a solution of **C48** (0.410 g, 0.92 mmol) in tetrahydrofuran (7 mL) and methanol (8 mL). The reaction mixture was stirred at room temperature overnight. The mixture was concentrated to near dryness and treated with water and the pH was adjusted to 2 with 1 N aqueous hydrochloric acid. The solids were collected by filtration, rinsed with water and dried under vacuum to afford crude **C49.** Yield: 0.310 g. LCMS *m*/*z* 419.0 (M+1). ¹H NMR (400 MHz, DMSO-*d*₆), characteristic peaks: δ 8.32 (s, 1 H), 7.62 (s, 1 H), 5.37 (s, 2H), 5.26 (s, 2H).

### Preparation 15

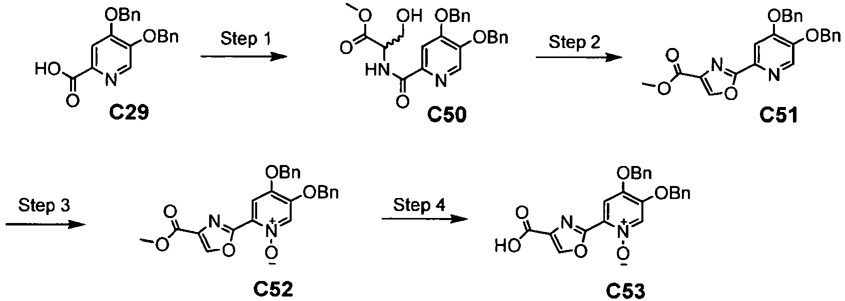

**Step 1: Preparation of C50.** To a suspension of **C29** (18.7 g, 55.7 mmol) in *N,N-*dimethylformamide (200 mL) was added 1,1'-carbonyldiimidazole (10.3 g, 61.3 mmol) and the reaction was stirred for 2 hours. In a separate flask, DL-serine methyl ester hydrochloride (8.68 g, 55.8 mmol) was dissolved in *N,N*-dimethylformamide (50 mL) and *N,N*-diisopropylethylamine (19.6 mL, 112 mmol) was added and stirred for 15 minutes. This solution was added to the above reaction and the mixture was stirred for 2 hours. The reaction mixture was concentrated and partitioned between water (200 mL) and ethyl acetate (200 mL). The layers were separated then the aqueous layer was extracted with ethyl acetate (3 x 100 mL). The combined organic layers were washed with saturated aqueous sodium bicarbonate, water, saturated aqueous ammonium chloride, and brine solution. The organic layer was dried over sodium sulfate, filtered, and concentrated to provide **C50** as a white solid. Yield: 18.0 g, 41.2 mmol, 74%. LCMS *m*/*z* 437.1 (M+1). ¹H NMR (400 MHz, DMSO-*d*₆) δ 8.64 (d, *J*=8.2 Hz, 1 H), 8.33 (s, 1 H), 7.73 (s, 1 H), 7.45-7.49 (m, 4H), 7.32-7.43 (m, 6H), 5.34 (s, 2H), 5.33 (s, 2H), 5.28 (dd, *J*=5.9, 5.8 Hz, 1 H), 4.55 (ddd, *J*=8.2, 4.1, 3.8 Hz, 1 H), 3.89 (ddd, *J*=11.2, 6.2, 4.1 Hz, 1 H), 3.75 (ddd, *J*=11.2, 5.4, 3.7 Hz, 1 H), 3.66 (s, 3H).

**Step 2: Preparation of C51.** A solution of **C50** (1.0 g, 2.29 mmol) in dichloromethane (11.5 mL) was cooled to 0 °C and treated with bis(2-methoxyethyl)aminosulfur trifluoride (0.489 mL, 2.52 mmol) added dropwise over 30 seconds. The reaction was stirred at 0 °C for 30 minutes. To the reaction was added anhydrous potassium carbonate (1.07 g, 6.87 mmol) at 0 °C and the reaction was stirred for 10 minutes. To the reaction was added 1,8-diazabicycloundec-7-ene (1.06 mL, 6.87 mmol) and the reaction was stirred for 2 minutes. Bromotrichloromethane (0.678 mL, 6.87 mmol) was added to the reaction and the mixture was stirred for 15 minutes. The reaction mixture was poured onto a silica plug and flushed with dichloromethane (150 mL). The filtrate was concentrated in vacuo and the resulting off-white solid was stirred in ethyl acetate / heptane (1:1) then collected by filtration to provide **C51** as a white solid. Yield: 752 mg, 1.8 mmol, 78%. LCMS *m*/*z* 417.1 (M+1). ¹H NMR (400 MHz, DMSO-*d*₆) δ 8.95 (s, 1H), 8.38 (s, 1 H), 7.78 (s, 1 H), 7.22 - 7.56 (m, 10 H), 5.35 (s, 2H), 5.30 (s, 2H), 3.82 (s, 3H).

**Step 3: Preparation of C52.** A solution of **C51** (0.19 g, 0.46 mmol) in anhydrous dichloromethane (4.6 mL) was cooled to 0 °C. To the reaction was added 3-chloroperoxybenzoic acid (77%, 0.12 g, 0.54 mmol) and the reaction was allowed to warm to room temperature and stirred for 14 hours. The reaction mixture was treated with additional 3-chloroperoxybenzoic acid (77%, 0.051 g, 0.23 mmol) and stirred for 2 hours. The reaction was quenched with water and extracted with dichloromethane. The organic layer was dried over sodium sulfate, filtered and concentrated in vacuo. The residue was purified by chromatography on silica gel (dichloromethane / 2-propanol) to provide **C52** as a white solid. Yield: 110 mg, 0.25 mmol, 56%. LCMS *m*/*z* 433.0 (M+1). ¹H NMR (400 MHz, DMSO-*d*₆) δ 9.08 (s, 1 H), 8.33 (s, 1 H), 7.60 (s, 1 H), 7.33-7.47 (m, 10H), 5.29 (s, 2H), 5.28 (s, 2H), 3.86 (s, 3H).

**Step 4: Preparation of C53.** To a solution of **C52** (2.14 g, 4.95 mmol) in tetrahydrofuran (20 mL) was added methanol (20 mL) and a solution of aqueous lithium hydroxide (358 mg, 14.8 mmol, in 20 mL water). The reaction was stirred at room temperature for 1 hour. The reaction was cooled to 0 °C and water (100 mL) was added then the reaction was brought to pH ∼3 using 1 N aqueous hydrochloric acid and stirred for 5 minutes. The solid was collected by filtration, washed with cold diethyl ether (3 x 5 mL), and dried under high vacuum to provide **C53** as a white solid. Yield: 1.29 g, 3.08 mmol, 62%. LCMS *m*/*z* 419.0 (M+1). ¹H NMR (400 MHz, DMSO-*d*₆) δ 8.95 (s, 1 H), 8.32 (s, 1 H), 7.59 (s, 1 H), 7.33-7.47 (m, 10H), 5.28 (br s, 4H).

### Preparation 16

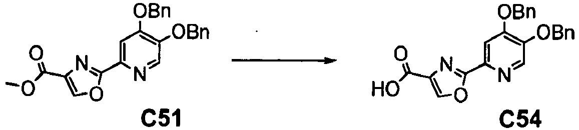

**Preparation of C54.** A solution of **C51** (150 mg, 0.360 mmol) in tetrahydrofuran (1 mL) was treated with methanol (1 mL), followed by a solution of aqueous lithium hydroxide (26 mg, 1.08 mmol, in 1 mL water). The reaction was stirred at room temperature for 1 hour. The mixture was cooled to 0 °C and water (5 mL) was added then the reaction was brought to pH ∼3 using 1 N aqueous hydrochloric acid and stirred for 5 minutes. The resulting solid was collected by filtration, washed with cold diethyl ether (3 x 1 mL), and dried under high vacuum to provide **C54** as a white solid. Yield: 0.11 g, 0.27 mmol, 76%. LCMS *m*/*z* 403.1 (M+1). ¹H NMR (400 MHz, DMSO-*d*₆) δ 13.0-13.2 (v br s, 1 H), 8.86 (s, 1 H), 8.40 (s, 1 H), 7.80 (s, 1 H), 7.32-7.51 (m, 10H), 5.38 (s, 2H), 5.33 (s, 2H).

### Preparation 17

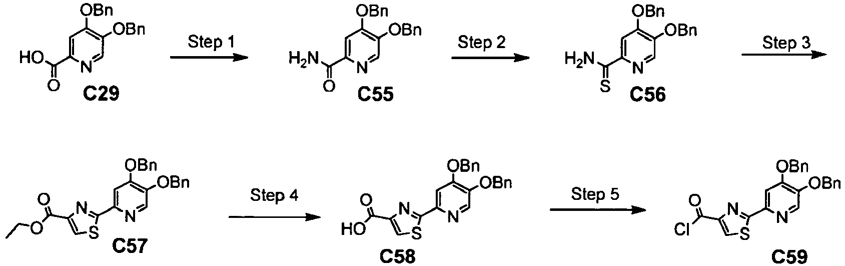

**Step 1: Preparation of C55.** A suspension of **C29** (2.09 g, 6.24 mmol) in anhydrous dichloromethane (20 mL) was treated with O-(7-azabenzotriazol-1-yl)-1,1,3,3-tetramethyluronium hexafluorophosphate (2.45 g, 6.24 mmol). The reaction mixture was refluxed for 4 hours. The solution was treated with ammonium hydroxide solution (8 mL) and stirred for 15 minutes, then concentrated under vacuum. The residue was treated with methanol (10 mL) and the solids were collected by filtration to afford **C55** as a white solid. Yield: 1.88 g, 5.55 mmol, 89%. APCI *m*/*z* 335.3 (M+1). ¹H NMR (400 MHz, DMSO-*d*₆) δ 8.25 (s, 1 H), 7.93 (br s, 1 H), 7.73 (s, 1 H), 7.31-7.50 (m, 11 H), 5.32 (s, 2H), 5.30 (s, 2H).

**Step 2: Preparation of C56.** A solution of **C55** (2.23 g, 6.64 mmol) in anhydrous tetrahydrofuran (50 mL) was treated with 2,4-bis(4-methoxyphenyl)-1,3,2,4-dithiadiphosphetane 2,4-disulfide (Lawesson reagent, 1.66 g, 3.98 mmol). The reaction mixture was stirred at 50 °C for 3 hours, then cooled to room temperature and the solvent was removed in vacuo. The residue was taken up in dichloromethane and washed with 1 N aqueous hydrochloric acid (3 x 30 mL), water (3 x 30 mL), sodium bicarbonate, brine solution (2 x 30 mL), dried over sodium sulfate, filtered and concentrated. The crude material was purified via chromatography on silica gel (heptane / ethyl acetate 20 to 70%) to afford **C56** as a yellow solid. Yield: 2.22 g, 6.34 mmol, 95%. LCMS *m*/*z* 351.1 (M+1). ¹H NMR (400 MHz, DMSO-*d*₆) δ 9.95 (br s, 1 H), 9.71 (br s, 1H), 8.27 (s, 1H), 8.22 (s, 1 H), 7.31-7.51 (m, 10H), 5.33 (s, 2H), 5.30 (s, 2H).

**Step 3: Preparation of C57.** A solution of **C56** (400 mg, 1.14 mmol) in ethanol (7 mL) was treated with ethyl bromopyruvate (222 mg, 1.14 mmol) and stirred for 3 hours. The reaction was heated to reflux for 2 hours. The solution was cooled to room temperature and concentrated in vacuo. The residue was taken up in ethyl acetate and washed with 1 N aqueous hydrochloric acid (3 x 10 mL), water (3 x 10 mL), aqueous sodium bicarbonate solution, brine solution (2 x 10 mL), dried over sodium sulfate, filtered and concentrated. The crude material was purified via chromatography on silica gel (heptane / ethyl acetate 0 to 100%) to afford **C57** as a yellow solid. Yield: 200 mg, 0.44 mmol, 39%. LCMS *m*/*z* 447.1 (M+1). ¹H NMR (400 MHz, DMSO-*d₆*) δ 8.56 (s, 1 H), 8.34 (s, 1H), 7.79 (s, 1H), 7.31-7.52 (m, 10H), 5.39 (s, 2H), 5.31 (s, 2H), 4.34 (q, *J*=7.1 Hz, 2H), 1.33 (t, *J*=7.0 Hz, 3H).

**Step 4: Preparation of C58.** A solution of **C57** (200 mg, 0.45 mmol) in 10 mL of ethanol was treated with 1 N aqueous sodium hydroxide (0.49 mL, 0.49 mmol) and stirred at 60 °C for 3 hours. The solution was cooled to room temperature, treated with 1 N aqueous hydrochloric acid (2 mL) and stirred for 30 minutes. The reaction mixture was concentrated and taken up in dichloromethane (10 mL) and washed with water (3 x 10 mL), brine solution (2 x 10 mL), dried over sodium sulfate, filtered and concentrated to afford **C58** as a light yellow solid. Yield: 188 mg, 0.45 mmol, quantitative. LCMS *m*/*z* 419.1 (M+1). ¹H NMR (400 MHz, DMSO-*d*₆) δ 8.50 (s, 1H), 8.34 (s, 1H), 7.81 (s, 1H), 7.31-7.52 (m, 10H), 5.38 (s, 2H), 5.31 (s, 2H).

**Step 5: Preparation of C59.** A suspension of **C58** (70 mg, 0.40 mmol) in anhydrous dichloromethane (10 mL) was treated with oxalyl chloride (0.044 mL, 0.44 mmol), followed by 1 drop of *N,N*-dimethylformamide. The reaction mixture was stirred for 3 hours and concentrated in vacuo to afford **C59** as an orange solid. Yield: 177 mg, 0.40 mmol, quantitative. LCMS, taken in methanol: *m*/*z* 433.0 (M+1 for methanolysis product).

### Preparation 18

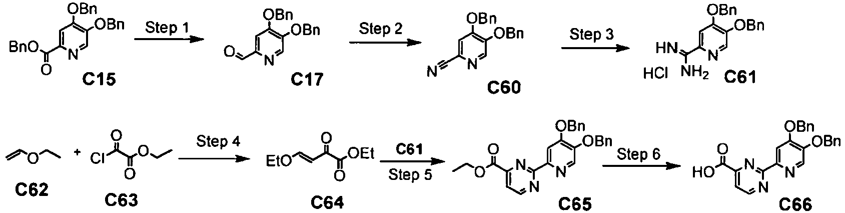

**Step 1: Preparation of C17.** A solution of **C15** (12.2 g, 29 mmol) in anhydrous dichloromethane (50 mL) under nitrogen at -78 °C was treated with diisobutylaluminum hydride (1.5 M in toluene, 28.8 mL, 43.1 mmol) added dropwise. The resulting reaction mixture was stirred at -78 °C for 1.5 hours. The reaction was quenched by slow addition of aqueous saturated potassium sodium tartrate (40 mL) at -78 °C. The solution was allowed to warm to room temperature and stirred overnight. The solvent was removed in vacuo and the residue was treated with ethyl acetate (300 mL) and additional aqueous saturated potassium sodium tartrate (100 mL) was added, and the mixture was stirred for an additional 5 hours. The organic layer was separated, concentrated in vacuo onto silica gel and purified via chromatography (heptane / ethyl acetate gradient) to afford **C17** as a white solid. Yield: 6.52 g, 20.0 mmol, 71%. LCMS *m*/*z* 320.1 (M+1). ¹H NMR (400 MHz, CDCl₃) δ 10.04 (s, 1H), 8.32 (s, 1H), 7.61 (s, 1H), 7.35-7.48 (m, 10H), 5.33 (s, 2H), 5.31 (s, 2H).

**Step 2: Preparation of C60.** A solution of **C17** (0.73 g, 2.3 mmol) in acetonitrile (3.5 mL) was treated with hydroxylamine hydrochloride (0.16 g, 2.3 mmol), sodium bicarbonate (0.2 g, 2.4 mmol), sodium sulfate (0.7 g, 4.9 mmol) and irradiated in a Biotage microwave oven at 150 °C for 10 minutes. The mixture was cooled to room temperature, acetic anhydride (0.44 mL, 4.6 mmol) was added and the reaction mixture was again irradiated in a Biotage microwave oven at 170 °C for 15 minutes. The sequence was repeated three more times on the same scale. The reaction mixtures were combined and treated with dichloromethane (65 mL) and water (10 mL), and the layers were separated. The aqueous layer was extracted with dichloromethane (25 mL). The combined organic layers were concentrated in vacuo and purified via chromatography on silica gel (heptane / dichloromethane: ethyl acetate (90:10)) to afford **C60** as a white solid. Yield: 2.37 g, 7.5 mmol, 82%. LCMS *m*/*z* 317.2 (M+1). ¹H NMR (400 MHz, DMSO-*d*₆) δ 8.43 (s, 1 H), 7.87 (s, 1 H), 7.32-7.48 (m, 10H), 5.33 (s, 2H), 5.30 (s, 2H).

**Step 3: Preparation of C61.** A solution of **C60** (1.45 g, 4.6 mmol) in anhydrous methanol (15 mL) was treated with sodium methoxide (25% wt. in methanol, 1.15 mL, 5.05 mmol). The resulting suspension was stirred at room temperature for 17 hours. The reaction mixture was concentrated in vacuo and the residue was treated with 7 M ammonia in methanol (20 mL, 100 mmol), followed by solid ammonium chloride (0.32 g, 5.9 mmol) and the mixture was stirred for 25.5 hours. The reaction mixture was concentrated in vacuo, methanol (25 mL) was added and the solids were removed by filtration. The filtrate was concentrated to afford crude product, which was triturated with methyl *tert*-butyl ether. The solid was collected by filtration to provide **C61** as a white solid. Yield: 1.32 g, 3.6 mmol, 78%. LCMS *m*/*z* 334.2 (M+1). ¹H NMR (400 MHz, DMSO-*d*₆) δ 8.35 (s, 1 H), 8.3-8.8 (v br s, 3H), 8.15 (s, 1 H), 7.29-7.54 (m, 11 H), 5.34 (s, 2H), 5.32 (s, 2H).

**Step 4: Preparation** of **C64.** A solution of **C62** (5.0 mL, 52 mmol) in 1,4-dioxane (3 mL) was treated with triethylamine (2.0 mL, 14 mmol) at 30 °C under nitrogen, followed by C63 (1.4 g, 10 mmol). The reaction mixture was stirred at 30 to 36 °C for 3.5 hours. The mixture was cooled to room temperature, diethyl ether (20 mL) was added and the mixture was filtered to remove the solids. The filtrate was washed with water (5 mL), dried over sodium sulfate and concentrated in vacuo to afford **C64** as a light yellow oil. Yield: 1.4 g (ca. 70% pure). ¹H NMR (400 MHz, CDCl₃), product peaks only: δ 7.88 (br d, *J*=12.6 Hz, 1 H), 6.19 (d, *J*=12.6 Hz, 1 H), 4.33 (q, *J*=7.1 Hz, 2H), 4.07 (qd, *J*=7.0, 0.4 Hz, 2H), 1.40 (t, *J*=7.1 Hz, 3H), 1.38 (t, *J*=7.1 Hz, 3H).

**Step 5: Preparation of C65.** A solution of **C61** (180 mg, 0.49 mmol) in 1,4-dioxane (1.5 mL) was refluxed for 1 hour. The reaction mixture was treated with **C64** (130 mg, 0.60 mmol) in 1,4-dioxane (0.5 mL) and the mixture was refluxed for 3 hours. A second quantity of **C64** (130 mg, 0.60 mmol) was added and the mixture was refluxed for an additional 6.5 hours. The reaction mixture was cooled to room temperature and purified via chromatography on silica gel (dichloromethane / 2-propanol) to afford a solid, which was dissolved in chloroform (3 mL) and washed with saturated aqueous sodium bicarbonate solution (1 mL). The organic layer was concentrated in vacuo to give **C65** as a solid. Yield: 40.7 mg, 0.092 mmol, 19%. LCMS *m*/*z* 442.3 (M+1). ¹H NMR (500 MHz, CDCl₃) δ 9.09 (d, *J*=4.9 Hz, 1H), 8.40 (s, 1H), 8.28 (s, 1H), 7.91 (d, *J*=4.9 Hz, 1H), 7.30-7.54 (m, 10H), 5.36 (s, 2H), 5.30 (s, 2H), 4.53 (q, *J*=7.2 Hz, 2H), 1.49 (t, *J*=7.1 Hz, 3H).

**Step 6: Preparation of C66.** A solution of **C65** (370 mg, 0.67 mmol) in tetrahydrofuran (1.7 mL) was treated with 1 N aqueous lithium hydroxide (1.7 mL, 1.7 mmol) at room temperature and stirred for 21 hours. The mixture was treated with 1 N aqueous hydrochloric acid (1 mL) and concentrated in vacuo to provide **C66** as a solid. The crude **C66** was used without further purification. LCMS *m*/*z* 414.3 (M+1).

### Preparation 19

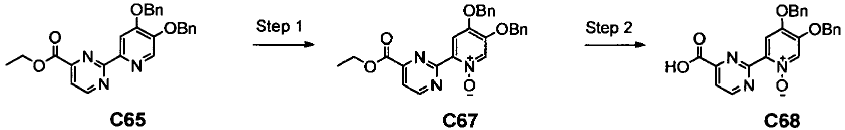

**Step 1: Preparation of C67**. Urea hydrogen peroxide (50.5 mg, 0.537 mmol) was added to **C65** (80 mg, 0.11 mmol) in acetonitrile (2 mL) followed by trifluoroacetic anhydride (0.078 mL, 0.55 mmol) at 0 °C. The reaction mixture was stirred at room temperature for 17 hours, then treated with ethyl acetate (3 mL) and washed with water (1.5 mL), 1 N aqueous sodium bicarbonate (1.5 mL) and water (1.5 mL). The organic layer was concentrated in vacuo and purified by chromatography on silica gel (ethyl acetate / 2-propanol) to yield **C67.** Yield: 20 mg, 0.044 mmol, 40%. LCMS *m*/*z* 458.2 (M+H). ¹H NMR (400 MHz, CD₃OD) δ 9.19 (br d, *J*=4.9 Hz, 1H), 8.18 (s, 1 H), 8.12 (d, *J*=5.0 Hz, 1H), 7.56 (s, 1H), 7.45-7.50 (m, 4H), 7.32-7.42 (m, 6H), 5.29 (s, 4H), 4.49 (q, *J*=7.1 Hz, 2H), 1.43 (t, *J*=7.1 Hz, 3H).

**Step 2: Preparation of C68.** A suspension of **C67** (129 mg, 0.28 mmol) in tetrahydrofuran (1.2 mL) was treated with 1 N aqueous lithium hydroxide (0.8 mL, 0.8 mmol). The reaction mixture was stirred at room temperature for 14.5 hours, then treated with 1 N aqueous hydrochloric acid (0.5 mL) and water (0.7 mL). The solids were collected by filtration and washed with hexanes (3 x 1 mL) to afford **C68** as a solid. Yield: 123 mg, 0.29 mmol, quantitative. LCMS *m*/*z* 430.2 (M+H).

### Preparation 20

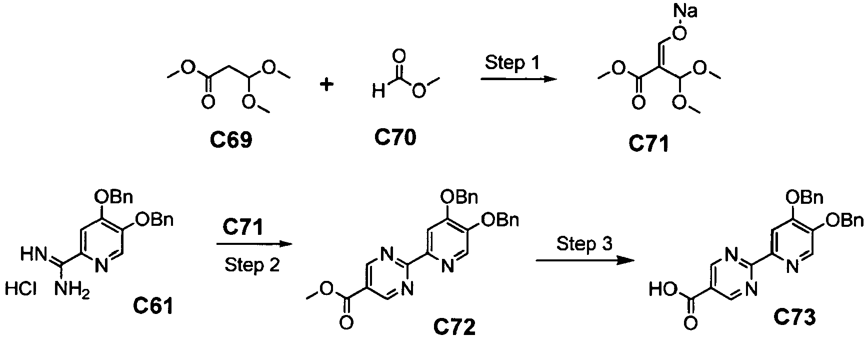

**Step 1: Preparation of C71.** To an oven-dried flask equipped with a refluxing condenser under nitrogen was added **C69** (2.5 mL, 17 mmol), 1,2-dimethoxyethane (12.5 mL), **C70** (2.7 mL, 43 mmol) and sodium hydride (60% in mineral oil, 0.86 g, 22 mmol). The resulting reaction mixture was heated to 43 °C, which took 10 minutes, and maintained at 43 to 45 °C for an additional 5 minutes before cooling in an ice-bath. The ice bath was then removed and the mixture was allowed to come to room temperature and stirred for 20.5 hours. Diethyl ether (20 mL) was added and the solid was collected by filtration to afford **C71** as an off-white solid. Yield: 2.8 g, 14 mmol, 83%. ¹H NMR (500 MHz, DMSO-*d*₆) δ 9.03 (s, 1 H), 5.10 (s, 1 H), 3.38 (s, 3H), 3.13 (s, 6H).

**Step 2: Preparation of C72.** A mixture of **C61** (230 mg, 0.62 mmol) and **C71** (123 mg, 0.62 mmol) in *N,N-*dimethylformamide (2 mL) was heated at 100 °C under nitrogen for 45 minutes. The reaction mixture was cooled to room temperature and treated with water (5 mL). The resulting solid was collected by filtration, washed with water (2 x 3 mL) and dried under high vacuum to afford **C72** as a solid. Yield: 200 mg, 0.46 mmol, 75%. LCMS *m*/*z* 428.3 (M+H). ¹H NMR (400 MHz, CDCl₃) δ 9.37 (s, 2H), 8.43 (br s, 1 H), 8.29 (s, 1 H), 7.30-7.53 (m, 10H), 5.35 (s, 2H), 5.31 (s, 2H), 4.01 (s, 3H).

**Step 3: Preparation of C73.** A suspension of **C72** (195 mg, 0.46 mmol) in tetrahydrofuran (1.7 mL) was treated with 1 N aqueous lithium hydroxide (1.2 mL, 1.2 mmol). The reaction mixture was stirred at room temperature for 15 hours, then treated with 1 N aqueous hydrochloric acid (1.5 mL), water (0.5 mL) and extracted with chloroform (4 mL, then 3 x 2 mL). The combined organic layers were concentrated to afford the first batch of **C73** (71 mg). The solid in the aqueous layer was filtered, collected by filtration and washed with hexanes to afford the second batch of **C73** (120 mg). Yield: 190 mg, 0.46 mmol, quantitative. LCMS *m*/*z* 414.2 (M+H). ¹H NMR (500 MHz, DMSO-*d*₆) δ 9.16 (s, 2H), 8.41 (s, 1 H), 8.18 (s, 1 H), 7.32-7.53 (m, 10H), 5.35 (s, 2H), 5.33 (s, 2H).

### Preparation 21

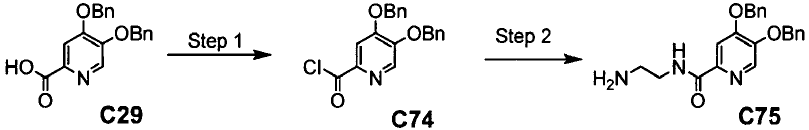

**Step 1: Preparation of C74.** A solution of **C29** (3.22 g, 9.6 mmol) in dichloromethane (20 mL) was treated with oxalyl chloride (0.86 mL, 9.60 mmol) dropwise. To the reaction was added *N,N*-dimethylformamide (1 drop) and stirring was continued at room temperature for 3 hours. The reaction was concentrated to provide **C74** as a light orange solid. Yield: 3.4 g, 9.60 mmol, quantitative. APCI, taken in methanol, m/z 350.4 (M+1 for methanolysis product). ¹H NMR (400 MHz, DMSO-*d*₆) δ 8.35 (s, 1H), 7.74 (s, 1 H), 7.31-7.48 (m, 10H), 5.33 (s, 4H).

**Step 2: Preparation of C75.** To a solution of 1,2-ethanediamine (0.082 mL, 1.20 mmol) and *N,N*-diisopropylethylamine (0.211 mL, 1.20 mmol) in dichloromethane (8 mL) at 0 °C was slowly added **C74** (142 mg, 0.401 mmol). The mixture was allowed to warm to room temperature and stirred for 18 hours. The reaction was diluted with ethyl acetate, washed with water, dried over sodium sulfate and the solvent removed in vacuo. The crude product was purified via chromatography on silica gel (50-100% ethyl acetate / heptane; 0-10% of a 10% triethylamine-methanol / ethyl acetate) to afford **C75** as a clear oil, which was approximately 50% pure by ¹H NMR. Yield: 53 mg, 0.14 mmol, 35%. LCMS *m*/*z* 378.2 (M+1). ¹H NMR (400 MHz, DMSO-*d*₆), characteristic peaks for product: δ 8.58 (br t, *J*=6 Hz, 1H), 8.26 (s, 1H), 7.71 (s, 1H), 5.32 (s, 2H), 5.30 (s, 2H), 3.22-3.28 (m, 2H), 2.67 (t, *J*=6.4 Hz, 2H).

### Preparation 22

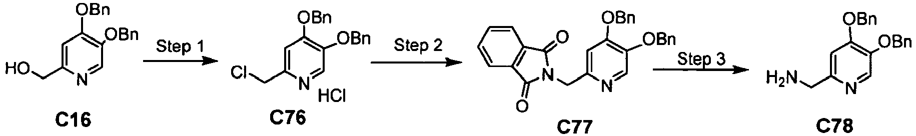

**Step 1: Preparation of C76.** A solution of C16 (2.53 g, 7.87 mmol) in dichloromethane (25 mL) was treated with thionyl chloride (1.73 mL, 23.8 mmol) dropwise. The reaction mixture was allowed to stir at room temperature for 3.5 hours. The solution was treated with heptane (50 mL) and the resulting suspension was stirred for 45 minutes, filtered and the solid was washed with heptane to give C76 as a solid. Yield: 2.80 g, 7.44 mmol, 95%. LCMS *m*/*z* 340.5 (M+1). ¹H NMR (400 MHz, CD₃OD) δ 8.42 (s, 1 H), 7.82 (s, 1H), 7.35-7.53 (m, 10H), 5.50 (s, 2H), 5.31 (s, 2H), 4.89 (s, 2H).

**Step 2: Preparation of C77.** A solution of C76 (7.07 g, 18.80 mmol) in anhydrous *N,N-*dimethylformamide (63 mL) was treated with potassium phthalimide (7.15 g, 38.0 mmol). The reaction mixture was heated at 110 °C and stirred for 1.5 hours. The mixture was cooled to room temperature and diluted with ethyl acetate and brine solution. Precipitates were taken back up in the organic layer by diluting with excess ethyl acetate. The aqueous layer was back extracted twice with ethyl acetate. The combined organic layers were dried over magnesium sulfate, filtered and concentrated in vacuo to give crude product. The crude material was recrystallized from 2-propanol to afford **C77** as an off-white solid. Yield: 6.49 g, 14.4 mmol, 77%. LCMS *m*/*z* 451.1 (M+1). ¹H NMR (400 MHz, DMSO-*d*₆) δ 8.04 (s, 1H), 7.86-7.92 (m, 4H), 7.27-7.46 (m, 10H), 7.18 (s, 1H), 5.21 (s, 2H), 5.13 (s, 2H), 4.79 (s, 2H).

**Step 3: Preparation of C78.** A solution of **C77** (0.68 g, 1.51 mmol) in ethanol (18 mL) was treated with hydrazine monohydrate (3.0 mL, 60 mmol) added dropwise. The reaction mixture was stirred at room temperature overnight. The solid was filtered under vacuum and the filtrate concentrated to dryness and the resulting residue was taken back up in ethyl acetate. The organic layer was washed with water and the aqueous layer back extracted with ethyl acetate. The combined organic layers were dried over magnesium sulfate, filtered and concentrated to give **C78** as solid. Yield: 0.43 g, 1.35 mmol, 90%. LCMS *m*/*z* 321.6 (M+1). ¹H NMR (400 MHz, CD₃OD) δ 8.04 (s, 1 H), 7.28-7.51 (m, 10H), 7.19 (s, 1 H), 5.25 (s, 2H), 5.16 (s, 2H), 3.79 (s, 2H).

### Preparation 23

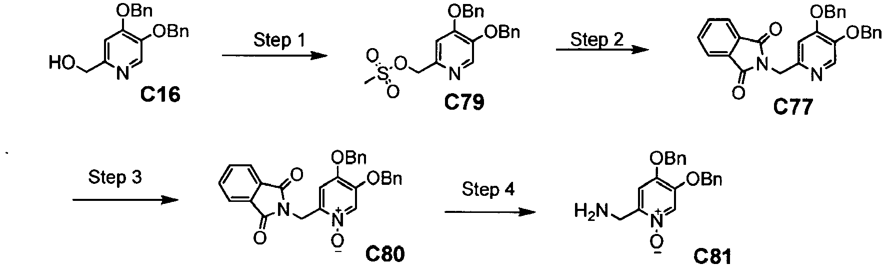

**Step 1: Preparation of C79.** A solution of **C16** (620 mg, 1.93 mmol) in 2-methyltetrahydrofuran (7 mL) was cooled to -30 °C and treated with methanesulfonyl chloride (0.21 mL, 2.70 mmol), followed by triethylamine (0.403 mL, 2.89 mmol). The solution was allowed to warm to 0 °C and stirred for 30 minutes. The reaction mixture was diluted with 2-methyl tetrahydrofuran (30 mL) and washed with water (2 x 30 mL), 1 N aqueous hydrochloric acid (1 x 30 mL), brine solution (1 x 30 mL), dried over sodium sulfate, filtered and concentrated in vacuo to afford **C79.** Yield: 0.77 g, quantitative. LCMS *m*/*z* 400.1 (M+1).

**Step 2: Preparation of C77.** A solution of **C79** (0.62 g, 1.55 mmol) in 2-methyltetrahydrofuran (10 mL) was treated with potassium phthalimide (575 mg, 3.10 mmol), followed by potassium fluoride (180 mg, 3.10 mmol) and heated at 65 °C overnight. The reaction mixture was cooled to 0 °C, diluted with ethyl acetate (50 mL) and extracted with water (3 x 30 mL), brine solution (1 x 30 mL), dried over sodium sulfate, filtered and concentrated in vacuo. The crude material was purified via chromatography on silica gel (heptane / ethyl acetate) to yield **C77.** Yield: 0.33 g, 47%. LCMS *m*/*z* 451.2 (M+1). ¹H NMR (400 MHz, DMSO-*d*₆) δ 8.04 (s, 1 H), 7.86-7.92 (m, 4H), 7.27-7.46 (m, 10H), 7.17 (s, 1H), 5.21 (s, 2H), 5.13 (s, 2H), 4.79 (s, 2H).

**Step 3: Preparation of C80.** A solution of **C77** (330 mg, 0.77 mmol) in dichloromethane (73 mL) was cooled to 0 °C and treated with 3-chloroperoxybenzoic acid (253 mg, 1.47 mmol). The reaction mixture was warmed to room temperature and stirred overnight. The solution was cooled to 0 °C, treated with brine solution (50 mL) and extracted with dichloromethane (3 x 50 mL). The combined organic layers were dried over sodium sulfate and concentrated in vacuo. The crude material was purified via chromatography on silica gel (heptane / ethyl acetate followed by ethyl acetate / 2-propanol) to afford **C80.** Yield: 0.368 g, quantitative. LCMS *m*/*z* 467.9 (M+1). ¹H NMR (400 MHz, DMSO-*d*₆) δ 8.18 (s, 1 H), 7.87-7.95 (m, 4H), 7.25-7.44 (m, 10H), 6.99 (s, 1 H), 5.18 (s, 2H), 5.13 (s, 2H), 4.75 (s, 2H).

**Step 4: Preparation of C81.** A solution of **C80** (368 mg, 0.79 mmol) in ethanol (100 mL) was treated with hydrazine monohydrate (0.31 mL, 6.30 mmol) and heated at 80 °C for 2 hours. The solids were collected by vacuum filtration and washed with ethanol (3 x 20 mL) to give **C81** as a white solid. Yield: 0.271 g, quantitative. LCMS *m*/*z* 337.1 (M+1). ¹H NMR (400 MHz, DMSO-*d*₆) δ 8.08 (s, 1 H), 7.33-7.48 (m, 10H), 7.32 (s, 1 H), 5.21 (s, 2H), 5.17 (s, 2H), 3.70 (s, 2H).

### General methods for purifying final compounds:

### Method A

Crude final compound was dissolved in DMSO:methanol (1:1) and loaded onto the column. The column used was a Phenomenex Max-RP 150 mm x 21.2 mm 5u using the following conditions: A gradient of 0.1% formic acid in water (MP-A) and 0.1% formic acid in methanol (MP-B) from 95% MP-A to 0% MP-B over 8.5 min with a flow rate of 27.0 ml/min. The sample was collected using either the UV detector at a wavelength of 215 nm or a mass spectrometer targeted for the appropriate molecular weight using APCI (+) mode. The isolated fraction had a purity of >85% and the total recovery by weight was as indicated.

### Method B

Using sonication, crude product was dissolved in a minimum amount of dimethyl sulfoxide. The solution of crude material was loaded onto a RediSepRf C-18 column and purified with 5% (acetonitrile with 0.1% formic acid)/(water with 0.1% formic acid) for 5 column volumes, 5-30% (acetonitrile with 0.1% formic acid)/(water with 0.1% formic acid) for 30 column volumes, 100% acetonitrile with 0.1% formic acid for 5 column volumes, and 75% (acetonitrile with 0.1 % formic acid)/(water with 0.1 % formic acid) for 4 column volumes. Fractions containing the desired product, detected using a UV detector at 210 nm and 254 nm, were concentrated in vacuo (17 torr, 32 °C) to yield the desired product along with a trace of formic acid. The formic acid was removed by azeotroping (5x) with acetonitrile to afford the desired product.

### Example 1

### 2-({[(1Z)-1-(2-amino-1,3-thiazol-4-yl)-2-({(2R,3S)-2-[(4-{[(1,5-dihydroxy-4-oxo-1,4-dihydropyridin-2-yl)methoxy]methyl}-1H-1,2,3-triazol-1-yl)methyl]-4-oxo-1-sulfoazetidin-3-yl}amino)-2-oxoethylidene] amino}oxy)-2-methylpropanoic acid (C84).

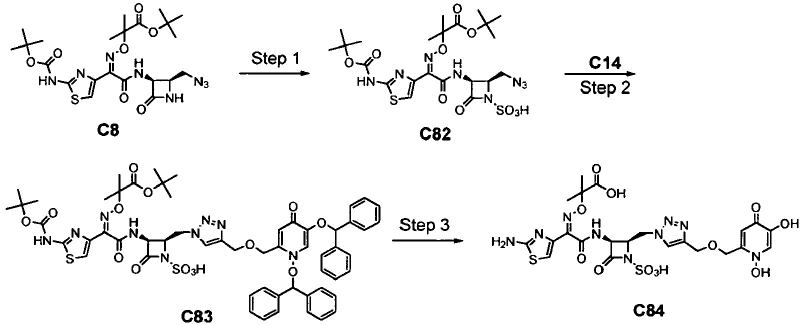

**Step 1: Preparation of C82.** A solution of **C8** (0.200 g, 0.362 mmol) in anhydrous *N,N-*dimethylformamide (3.60 mL) was treated with sulfur trioxide *N,N-*dimethylformamide complex (0.572 g, 3.74 mmol). The resulting solution was stirred at room temperature for 5 hours and then diluted with water and ethyl acetate. The layers were separated and the aqueous layer was back extracted twice with ethyl acetate. The combined organic layers were washed twice with water, once with brine solution, dried over magnesium sulfate, filtered and concentrated to afford **C82** as a foamy solid. Yield: 0.235 g, 0.37 mmol, quantitative. LCMS *m*/*z* 631.8 (M-1). ¹H NMR (400 MHz, DMSO-*d*₆) δ 11.82 (br s, 1 H), 9.07 (d, *J*=8.8 Hz, 1 H), 7.25 (s, 1 H), 5.24 (dd, *J*=8.8, 5.6 Hz, 1 H), 4.01-4.07 (m, 1 H), 3.72 (dd, half of ABX pattern, *J*=13, 6 Hz, 1 H), 3.66 (dd, half of ABX pattern, *J*=13, 4 Hz, 1H), 1.46 (s, 9H), 1.44 (s, 3H), 1.43 (s, 3H), 1.39 (s, 9H).

**Step 2: Preparation of C83.** A solution of **C14** (0.139 g, 0.264 mmol) and **C82** (0.179 g, 0.283 mmol) in a mixture of dimethyl sulfoxide (1.80 mL) / water (1.80 mL) / *tert-*butanol (1.80 mL) was treated with copper (II) sulfate (0.015 g, 0.094 mmol) and sodium L-ascorbate (0.025 g, 0.13 mmol). The reaction mixture was stirred at room temperature overnight and then diluted with water and ethyl acetate. The layers were separated and the aqueous layer was extracted three times with ethyl acetate. The combined organic layers were washed twice with water, once with brine solution, dried over magnesium sulfate, filtered and concentrated in vacuo to give a foamy solid. The crude material was purified by chromatography on silica gel (ethyl acetate / 2-propanol) to afford **C83** as light green solid. Yield: 0.212 g, 0.183 mmol, 69%. ¹H NMR (500 MHz, DMSO-*d*₆) δ 11.81 (br s, 1 H), 9.46 (d, *J*=8.3 Hz, 1 H), 8.08 (s, 1 H), 7.62 (s, 1 H), 7.34-7.40 (m, 14H), 7.26-7.31 (m, 2H), 7.18-7.22 (m, 5H), 6.38 (s, 1H), 6.30 (s, 1H), 6.02 (s, 1 H), 5.28 (dd, *J*=8.5, 5.6 Hz, 1 H), 4.84 (dd, *J*=14.8, 4.5 Hz, 1 H), 4.70 (dd, *J*=14.9, 6.1 Hz, 1 H), 4.36 (s, 2H), 4.24-4.28 (m, 1 H), 4.14 (s, 2H), 1.45 (s, 9H), 1.34 (s, 9H), 1.32 (s, 3H), 1.26 (s, 3H).

**Step 3: Preparation of C84.** A solution of **C83** (0.120 g, 0.104 mmol) in anhydrous dichloromethane (6.80 mL) was treated with triethylsilane (97%, 52 µL, 0.32 mmol). The reaction mixture was cooled to 0 °C and treated with trifluoroacetic acid (0.480 mL, 6.25 mmol), and allowed to warm to room temperature overnight. The solution was concentrated in vacuo and suspended in a mixture of methyl *tert*-butyl ether / heptane (1:1) causing precipitates to form. The solvent was removed in vacuo and the crude solid was purified by reverse phase flash chromatography (C18 column in acetonitrile : water solvent system with 0.1 % formic acid modifier) to afford **C84** as an off-white solid. Yield: 0.045 g, 0.067 mmol, 64%. LCMS *m*/*z* 672.7 (M+1). ¹H NMR (400 MHz, DMSO-*d*₆) δ 9.40 (d, *J*=8.6 Hz, 1 H), 8.20 (s, 1 H), 8.13 (br s, 1 H), 7.25-7.53 (br s, 2H), 7.10 (br s, 1 H), 6.71 (s, 1H), 5.26 (dd, *J*=8.6, 5.6 Hz, 1H), 4.86 (dd, *J*=14.8, 4.5 Hz, 1H), 4.67-4.75 (m, 5H), 4.25-4.30 (m, 1 H), 1.35 (s, 3H), 1.29 (s, 3H).

### Example 2

### 2-({[(1Z)-1-(2-amino-1,3-thiazol-4-yl)-2-{[(2R,3S)-2-{[4-(5-hydroxy-4-oxo-1,4-dihydropyridin-2-yl)-1H-1,2,3-triazol-1-yl]methyl}-4-oxo-1-sulfoazetidin-3-yl]amino}-2-oxoethylidene]amino}oxy)-2-methylpropanoic acid (C87).

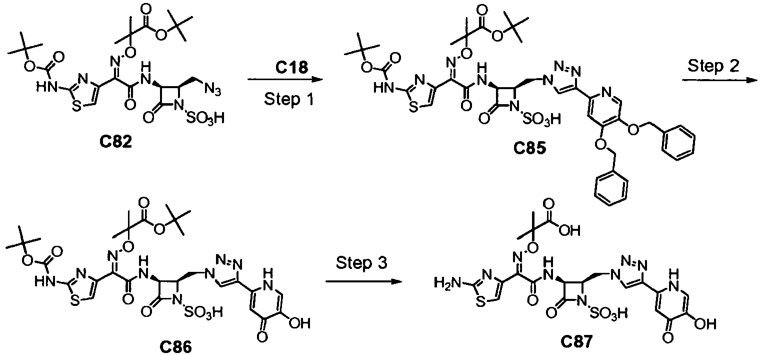

**Step 1: Preparation of C85.** A solution of **C82** (0.191 g, 0.30 mmol) and **C18** (0.091 g, 0.29 mmol) in a mixture of dimethyl sulfoxide / water / *tert*-butanol (1.50 mL each), was treated with copper (II) sulfate (0.005 g, 0.033 mmol) and sodium L-ascorbate (0.008 g, 0.041 mmol). The reaction mixture was stirred at room temperature overnight and then diluted with water and ethyl acetate. The layers were separated and the aqueous layer was back extracted three times with ethyl acetate. The combined organic layers were washed with water, and then brine solution. The combined aqueous layers were extracted again with ethyl acetate. All organic layers were combined, dried with magnesium sulfate, filtered, and concentrated in vacuo to give a light green foamy solid. Crude material was purified via chromatography on silica gel (dichloromethane / methanol) to give a mixture of **C85 / C82** (∼2:1). The mixture was used directly in the next step without further purification. Yield: 0.112 g total crude. LCMS *m*/*z* 948.8 (M+1). ¹H NMR (400 MHz, DMSO-*d*₆), peaks attributed to product: δ 11.89 (br s, 1 H), 9.43 (d, *J*=9 Hz, 1 H), 8.53 (s, 1 H), 8.24 (s, 1 H), 7.74 (s, 1 H), 7.30-7.52 (m, 10H), 7.22 (s, 1 H), 5.35 (s, 2H), 5.31 (dd, *J*=9, 6 Hz, 1 H), 5.23 (s, 2H), 4.94 (dd, *J*=15, 4 Hz, 1 H), 4.74 (dd, *J*=15, 6 Hz, 1 H), 4.30-4.35 (m, 1 H), 1.43 (s, 9H), 1.34 (s, 3H), 1.33 (s, 9H), 1.30 (s, 3H).

**Step 2: Preparation of C86.** Palladium black (0.044 g, 0.40 mmol) was added to a Parr bottle containing a (∼3:1) mixture of **C85 / C82** (0.101 g) dissolved in a mixture of anhydrous tetrahydrofuran (5.0 mL) and glacial acetic acid (50 µL). The resulting mixture was hydrogenated for 3 hours at 40 psi. The reaction mixture was filtered through a pad of Celite and washed multiple times with tetrahydrofuran. The filtrate was concentrated in vacuo to give **C86** as foamy yellow solid. The crude material was taken directly into the next reaction without further purification. Yield: 0.099 g. APCI *m*/*z* 766.4 (M-1).

**Step 3: Preparation of C87.** A solution of crude **C86** (0.082 g) in anhydrous dichloromethane (3 mL) was cooled to 0 °C and treated with trifluoroacetic acid (0.490 mL, 6.36 mmol). The mixture was stirred at room temperature for 12 hours. The reaction mixture was concentrated in vacuo, treated with a mixture of methyl *tert*-butyl ether / *n*-heptane (1:1) and concentrated in vacuo again. The crude material was purified via reverse phase chromatography (C-18 column; acetonitrile / water with 0.1% formic acid modifier) to afford **C87** as an off-white solid. Yield: 0.032 g, 0.052 mmol, 48%. LCMS *m*/*z* 612.1 (M+1). ¹H NMR (400 MHz, DMSO-*d*₆) δ 9.41 (d, *J*=8.4 Hz, 1H), 8.86 (s, 1H), 7.93 (s, 1H), 7.44 (s, 1 H), 7.2-7.5 (br s, 2H), 6.69 (s, 1H), 5.33 (dd, *J*=8.4, 5.7 Hz, 1 H), 4.91 (dd, half of ABX pattern, *J*=14.8, 5.8 Hz, 1 H), 4.79 (dd, half of ABX pattern, *J*=14.8, 5.0 Hz, 1 H), 4.39-4.45 (m, 1 H), 1.37 (s, 3H), 1.31 (s, 3H).

### Example 3

### 2-({[(1Z)-1-(2-amino-1,3-thiazol-4-yl)-2-{[(2R,3S)-2-{[4-(1,5-dihydroxy-4-oxo-1 ,4-dihydropyridin-2-yl)-1H-1,2,3-triazol-1-yl]methyl}-4-oxo-1-sulfoazetidin-3-yl]amino}-2-oxoethylidene]amino}oxy)-2-methylpropanoic acid (C89).

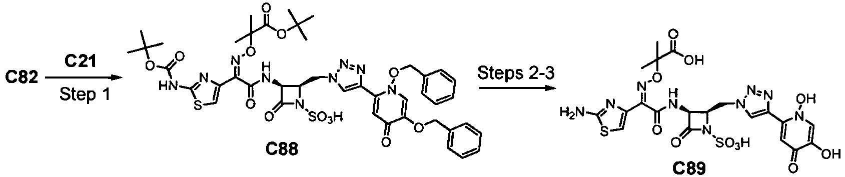

**Step 1: Preparation of C88.** Copper (II) sulfate (0.0076 g, 0.048 mmol) and sodium L-ascorbate (0.013 g, 0.064 mmol) were added sequentially to a reaction mixture containing **C82** (0.079 g, 0.12 mmol) and **C21** (0.041 g, 0.12 mmol) dissolved in a mixture of dimethyl sulfoxide, water and *tert*-butanol (1.40 mL each). The mixture was stirred at room temperature overnight and then diluted with water and ethyl acetate. The layers were separated and the aqueous layer was back extracted 3 times with ethyl acetate. The combined organic layers were washed with brine solution, dried over magnesium sulfate, filtered and concentrated in vacuo to give a light green glassy solid. The crude compound was purified by chromatography on silica gel (dichloromethane / methanol) to afford **C88** as a solid. Yield: 0.062 g, 0.064 mmol, 52%. LCMS *m*/*z* 964.57 (M+1).

**Step 2-3: Preparation of C89. C88** was converted to **C89** by methods analogous to those described in Example 2, Steps 2 and 3. The crude product was purified by reverse phase chromatography (C-18 column; acetonitrile / water with 0.1% formic acid modifier) to provide **C89** as an off-white solid. LCMS *m*/*z* 628.1 (M+1). ¹H NMR (400 MHz, DMSO-*d*₆) δ 9.35 (d, *J*=8.8 Hz, 1 H), 9.00 (s, 1 H), 8.01 (s, 1 H), 7.77 (s, 1 H), 6.66 (s, 1 H), 5.33 (dd, *J*=9.0, 5.5 Hz, 1 H), 4.93 (dd, half of ABX pattern, *J*=15, 5 Hz, 1 H), 4.78 (dd, half of ABX pattern, *J*=15, 6 Hz, 1H), 4.42-4.47 (m, 1 H), 1.36 (s, 3H), 1.32 (s, 3H).

### Example 4, Route 1

### 2-({[(1Z)-1-(2-amino-1,3-thiazol-4-yl)-2-({(2R,3S)-2-[({[(1,5-dihydroxy-4-oxo-1,4-dihydropyridin-2-yl)methyl]carbamoyl}amino)methyl]-4-oxo-1-sulfoazetidin-3-yl}amino)-2-oxoethylidene]amino}oxy)-2-methylpropanoic acid, bis sodium salt (C92-Bis Na Salt).

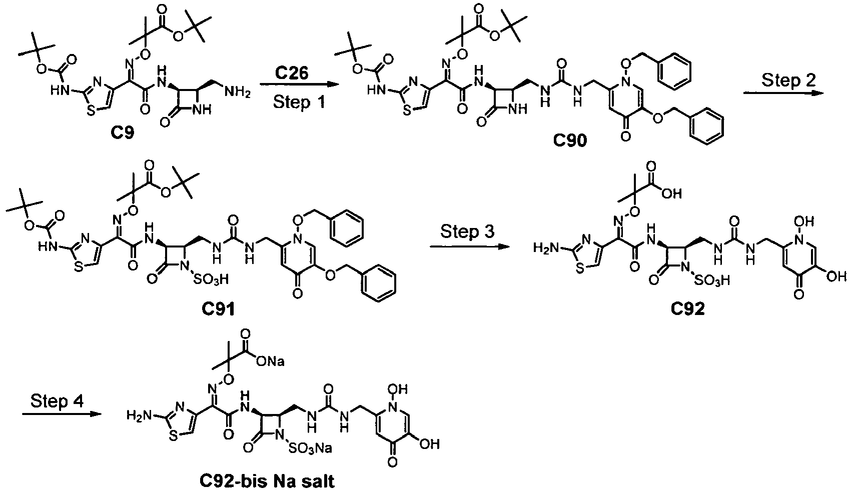

**Step 1: Preparation of C90.** A solution of **C26** (16.2 g, 43.0 mmol) in tetrahydrofuran (900 mL) was treated with 1,1'-carbonyldiimidazole (8.0 g, 47.7 mmol). After 5 minutes, the reaction mixture was treated with a solution of **C9** (15 g, 25.0 mmol) in anhydrous tetrahydrofuran (600 mL) at room temperature. After 15 hours, the solvent was removed and the residue was treated with ethyl acetate (500 mL) and water (500 mL). The layers were separated and the aqueous layer was back extracted with additional ethyl acetate (300 mL). The organic layers were combined, washed with brine solution (500 mL), dried over sodium sulfate, filtered and concentrated in vacuo. The crude product was purified via chromatography on silica gel (ethyl acetate / 2-propanol) to yield **C90** as a yellow foam. Yield: 17.44 g, 19.62 mmol, 78%. LCMS *m*/z 889.5 (M+1). ¹H NMR (400 MHz, DMSO-*d*₆) 11.90 (br s, 1 H), 9.25 (d, *J*=8.7 Hz, 1 H), 8.40 (br s, 1 H), 7.98 (s, 1 H), 7.50-7.54 (m, 2H), 7.32-7.47 (m, 8H), 7.28 (s, 1 H), 6.65 (br s, 1 H), 6.28 (br s, 1 H), 5.97 (s, 1 H), 5.25 (s, 2H), 5.18 (dd, *J*=8.8, 5 Hz, 1 H), 4.99 (s, 2H), 4.16-4.28 (m, 2H), 3.74-3.80 (m, 1H), 3.29-3.41 (m, 1H), 3.13-3.23 (m, 1H), 1.42 (s, 9H), 1.41 (s, 3H), 1.39 (br s, 12H).

**Step 2: Preparation of C91.** A solution of **C90** (8.5 g, 9.6 mmol) in anhydrous *N,N-*dimethylformamide (100 mL) was treated sulfur trioxide *N,N-*dimethylformamide complex (15.0 g, 98.0 mmol). The reaction was allowed to stir at room temperature for 20 minutes then quenched with water (300 mL). The resulting solid was collected by filtration and dried to yield **C91** as a white solid. Yield: 8.1 g, 8.3 mmol, 87%. LCMS m/z 967.6 (M-1). ¹H NMR (400 MHz, DMSO-*d*₆) δ 11.62 (br s, 1H), 9.29 (d, *J*=8.8 Hz, 1H), 9.02 (s, 1H), 7.58-7.61 (m, 2H), 7.38-7.53 (m, 9H), 7.27 (s, 1H), 7.07 (s, 1H), 6.40 (br d, *J*=8 Hz, 1 H), 5.55 (s, 2H), 5.25 (s, 2H), 5.20 (dd, *J*=8.8, 5.6 Hz, 1 H), 4.46 (br dd, half of ABX pattern, *J*=17, 5 Hz, 1 H), 4.38 (br dd, half of ABX pattern, *J*=17, 6 Hz, 1 H), 3.92-3.98 (m, 1 H), 3.79-3.87 (m, 1H), 3.07-3.17 (m, 1H), 1.40 (s, 9H), 1.39 (s, 3H), 1.38 (s, 12H).

**Step 3: Preparation of C92.** A solution of **C91** (8.1 g, 8.3 mmol) in anhydrous dichloromethane (200 mL) was treated with 1 M boron trichloride in *p*-xylenes (58.4 mL, 58.4 mmol) and allowed to stir at room temperature for 15 minutes. The reaction mixture was cooled in an ice bath, quenched with 2,2,2-trifluoroethanol (61 mL), and the solvent was removed in vacuo. A portion of the crude product (1 g) was purified via reverse phase chromatography (C-18 column; acetonitrile / water gradient with 0.1% formic acid modifier) to yield **C92** as a white solid. Yield: 486 mg, 0.77 mmol. LCMS m/z 633.3 (M+1). ¹H NMR (400 MHz, DMSO-*d*₆) δ 9.22 (d, *J*=8.7 Hz, 1 H), 8.15 (s, 1 H), 7.26-7.42 (br s, 2H), 7.18-7.25 (m, 1H), 6.99 (s, 1H), 6.74 (s, 1H), 6.32-6.37 (m, 1H), 5.18 (dd, *J*=8.7, 5.7 Hz, 1 H), 4.33 (br d, *J*=4.6 Hz, 2H), 3.94-4.00 (m, 1 H), 3.60-3.68 (m, 1H), 3.19-3.27 (m, 1 H), 1.40 (s, 3H), 1.39 (s, 3H).

**Step 4: Preparation of C92-Bis Na Salt.** A flask was charged with **C92** (388 mg, 0.61 mmol) and water (5.0 mL). The mixture was cooled in an ice bath and treated dropwise with a solution of sodium bicarbonate (103 mg, 1.52 mmol) in water (5.0 mL). The sample was lyophilized to yield **C92-Bis Na Salt** as a white solid. Yield: 415 mg, 0.61 mmol, quantitative. LCMS *m*/*z* 633.5 (M+1). ¹H NMR (400 MHz, D₂O) δ 7.80 (s, 1 H), 6.93 (s, 1 H), 6.76 (s, 1 H), 5.33 (d, *J*=5.7 Hz, 1 H), 4.44 (ddd, *J*=6.0, 6.0, 5.7 Hz, 1 H), 4.34 (AB quartet, *J*_{AB}=17.7 Hz, Δν_{AB}=10.9 Hz, 2H), 3.69 (dd, half of ABX pattern, *J*=14.7, 5.8 Hz, 1 H), 3.58 (dd, half of ABX pattern, *J*=14.7, 6.2 Hz, 1 H), 1.44 (s, 3H), 1.43 (s, 3H).

### Alternate preparation of C92

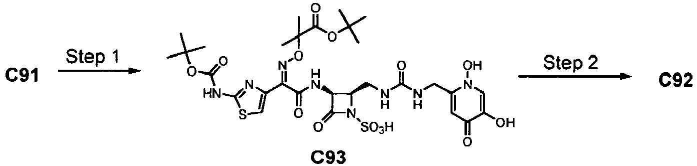

**Step 1: Preparation of C93.** An Atlantis pressure reactor was charged with 10% palladium hydroxide on carbon (0.375 g, John Matthey catalyst type A402028-10), **C91** (0.75 g, 0.77 mmol) and treated with ethanol (35 mL). The reactor was flushed with nitrogen and pressurized with hydrogen (20 psi) for 20 hours at 20 °C. The reaction mixture was filtered under vacuum and the filtrate was concentrated using the rotary evaporator to yield **C93** as a tan solid. Yield: 0.49 g, 0.62 mmol, 80%. LCMS *m*/*z* 787.6 (M-1). ¹H NMR (400 MHz, DMSO-*d*₆) δ 11.57 (br s, 1 H), 9.27 (d, *J*=8.5 Hz, 1 H), 8.16 (s, 1 H), 7.36 (br s, 1H), 7.26 (s, 1H), 7.00 (s, 1 H), 6.40 (br s, 1H), 5.18 (m, 1H), 4.35 (m, 2H), 3.83 (m, 1H), 3.41 (m, 1H), 3.10 (m, 1H), 1.41 (s, 6H), 1.36 (s, 18H).

**Step 2: Preparation of C92.** A solution of **C93** (6.0 g, 7.6 mmol) in anhydrous dichloromethane (45 mL) at 0 °C was treated with trifluoroacetic acid (35.0 mL, 456 mmol). The mixture was warmed to room temperature and stirred for 2 hours. The reaction mixture was cannulated into a solution of methyl *tert*-butyl ether (100 mL) and heptane (200 mL). The solid was collected by filtration and washed with a mixture of methyl *tert*-butyl ether (100 mL) and heptane (200 mL) then dried under vacuum. The crude product (∼5 g) was purified via reverse phase chromatography (C-18 column; acetonitrile / water gradient with 0.1 % formic acid modifier) and lyophilized to yield **C92** as a pink solid. Yield: 1.45 g, 2.29 mmol. LCMS *m*/*z* 631.0 (M-1). ¹H NMR (400 MHz, DMSO-*d*₆) δ 9.20 (d, J=8.7 Hz, 1 H), 8.13 (s, 1 H), 7.24-7.40 (br s, 2H), 7.16-7.23 (m, 1H), 6.97 (s, 1H), 6.71 (s, 1H), 6.31-6.35 (m, 1H), 5.15 (dd, J=8.7, 5.7 Hz, 1H), 4.31 (br d, J=4.6 Hz, 2H), 3.92-3.98 (m, 1H), 3.58-3.67 (m, 1 H), 3.17-3.25 (m, 1H), 1.37 (s, 3H), 1.36 (s, 3H).

### Example 4, route 2

### 2-({[(1Z)-1-(2-amino-1,3-thiazol-4-yl)-2-({(2R,3S)-2-[({[(1,5-dihydroxy-4-oxo-1,4-dihydropyridin-2-yl)methyl]carbamoyl}amino)methyl]-4-oxo-1-sulfoazetidin-3-yl}amino)-2-oxoethylidene]amino}oxy)-2-methylpropanoic acid (C92).

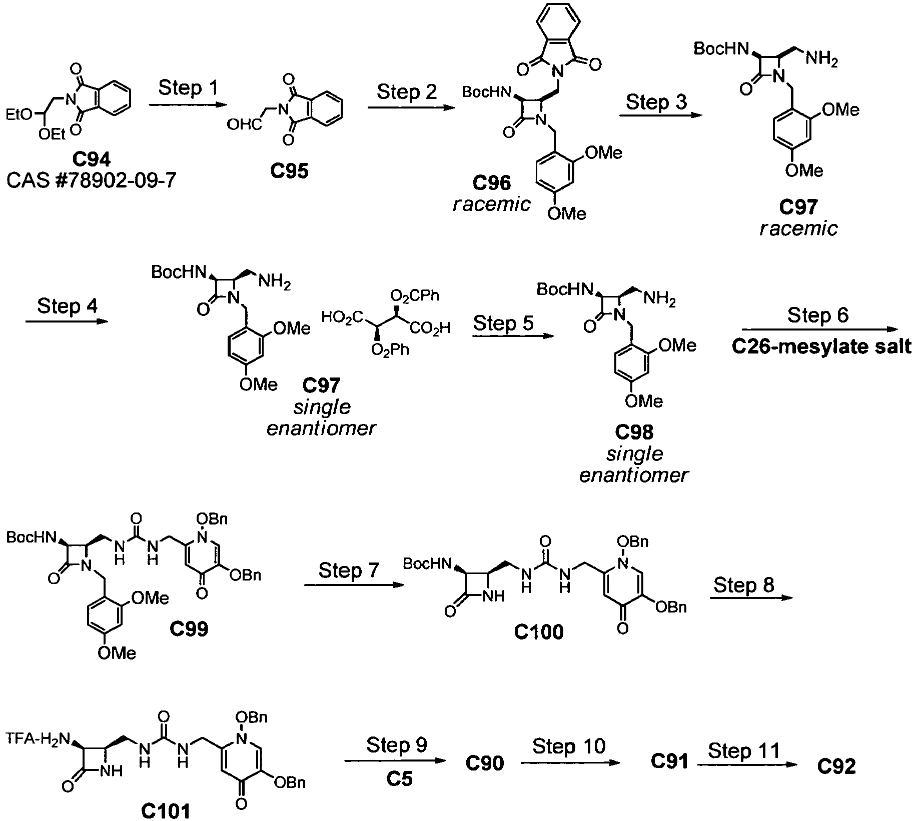

**Step 1. Preparation of C95.** A solution of **C94** (50.0 g, 189.9 mmol) in dichloromethane (100 mL) was treated with trifluoroacetic acid (50.0 mL, 661.3 mmol). The reaction mixture was stirred at room temperature for 24 hours. The dichloromethane and trifluoroacetic acid was displaced with toluene (4 x 150 mL) using vacuum, to a final volume of 120 mL. The solution was added to heptane (250 mL) and the solid was collected by filtration. The solid was washed with a mixture of toluene and heptane (1 : 3, 60 mL), followed by heptane (2 x 80 mL) and dried under vacuum at 50 °C for 19 hours to afford **C95** as a solid. Yield: 30.0 g, 158 mmol, 84%. ¹H NMR (400 MHz, CDCl₃) δ 9.66 (s, 1H), 7.86 - 7.93 (m, 2H), 7.73 - 7.80 (m, 2H), 4.57 (s, 2H). HPLC retention time 5.1 minutes; column: Agilent Extended C-18 column (75 mm x 3 mm, 3.5 µm); column temperature 45 °C; flow rate 1.0 mL / minute; detection UV 230 nm; mobile phase: solvent A = acetonitrile (100%), solvent B = acetonitrile (5%) in 10 mM ammonium acetate; gradient elusion: 0-1.5 minutes solvent B (100%), 1.5-10.0 minutes solvent B (5%), 10.0-13.0 minutes solvent B (100%); total run time 13.0 minutes.

**Step 2: Preparation of C96-racemic.** A solution of **C95** (32.75 g; 173.1 mmol) in dichloromethane (550 mL) under nitrogen was cooled to 2 °C. The solution was treated with 2,4-dimethoxybenzylamine (28.94 g, 173.1 mmol) added dropwise over 25 minutes, maintaining the temperature below 10 °C. The solution was stirred for 10 minutes at 2 °C and then treated with molecular sieves (58.36 g, UOP Type 3A). The cold bath was removed and the reaction slurry was stirred for 3 hours at room temperature. The slurry was filtered through a pad of Celite (34.5 g) and the filter cake was rinsed with dichloromethane (135 mL). The dichloromethane filtrate (imine solution) was used directly in the following procedure.

A solution of *N*-(*tert*-butoxycarbonyl)glycine (60.6 g, 346.1 mmol) in tetrahydrofuran (622 mL) under nitrogen was cooled to -45 °C and treated with triethylamine (38.5 g, 380.8 mmol). The mixture was stirred for 15 minutes at -45 °C and then treated with ethyl chloroformate (48.8 g, 450 mmol) over 15 minutes. The reaction mixture was stirred at -50 °C for 7 hours. The previously prepared imine solution was added via an addition funnel over 25 minutes while maintaining the reaction mixture temperature below -40 °C. The slurry was treated with triethylamine (17.5 g, 173 mmol) and the reaction mixture was slowly warmed to room temperature over 5 hours and stirred for an additional 12 hours. The reaction slurry was charged with water (150 mL) and the volatiles removed using a rotary evaporator. The reaction mixture was charged with additional water (393 mL) and the volatiles removed using a rotary evaporator. The mixture was treated with methyl *tert*-butyl ether (393 mL) and vigorously stirred for 1 hour. The solid was collected by vacuum filtration and the filter cake was rinsed with a mixture of methyl *tert*-butyl ether and water (1:1, 400 mL). The solid was collected and dried in a vacuum oven at 50 °C for 16 hours to afford **C96-racemic.** Yield: 55.8 g, 113 mmol, 65%. ¹H-NMR (400 MHz, DMSO-d₆) δ 7.85 (s, NH), 7.80 (s, 4H), 6.78 (d, *J*=7.8 Hz, 1H), 6.25 (m, 1H), 6.10 (m, 1H), 4.83 (m, 1H), 4.38 (d, *J*=9.5 Hz, 1H), 3.77-3.95 (m, 3H), 3.62 (s, 3H), 3.45 (m, 1H), 3.40 (s, 3H), 1.38 (s, 9H). HPLC retention time 6.05 minutes; XBridge C8 column (4.6 x 75 mm, 3.5 µm); column temperature 45 °C; flow rate 2.0 mL/minute; detection UV 210 nm, 230 nm, and 254 nm; mobile phase: solvent A = methanesulfonic acid (5%) in 10 mmol sodium octylsulfonate, solvent B = acetonitrile (100%); gradient elusion: 0-1.5 minutes solvent A (95%) and solvent B (5%), 1.5-8.5 minutes solvent A (5%) and solvent B (95%), 8.5-10.0 minutes solvent A (5%) and solvent B (95%), 10.01-12.0 minutes solvent A (95%) and solvent B (5%); total run time 12.0 minutes.

**Step 3: Preparation of C97-racemic.** A solution **of C96-racemic** (15.0 g, 30.3 mmol) in ethyl acetate (150 mL) under nitrogen was treated with ethanolamine (27.3 mL, 454.1 mmol). The reaction mixture was heated at 90 °C for 3 hours and then cooled to room temperature. The mixture was charged with water (150 mL) and the layers separated. The aqueous layer was extracted with ethyl acetate (75 mL) and the combined organic layers washed with water (2 x 150 mL) followed by saturated aqueous sodium chloride (75 mL). The organic layer was dried over magnesium sulfate, filtered and the filtrate concentrated to a volume of 38 mL. The filtrate was treated with heptane (152 mL) and the solid was collected by filtration. The solid was washed with heptane and dried at 50 °C in a vacuum oven overnight to yield **C97-racemic** as a solid. Yield: 9.68 g, 26.5 mmol, 88%. LCMS *m*/*z* 967.6 (M-1). ¹H NMR (400 MHz, DMSO-*d*₆) δ 7.64 (d, *J*=9.4 Hz, 1H), 7.14 (d, *J*=8.2 Hz, 1H), 6.56 (s, 1 H), 6.49 (dd, *J*=8.20, 2.3 Hz, 1 H), 4.78 (dd, *J*=9.37, 5.1 Hz, 1H), 4.30 (d, *J*=14.8 Hz, 1H), 4.14 (d, *J*=14.8 Hz, 1H), 3.77 (s, 3H), 3.75 (s, 3H), 3.45 - 3.53 (m, 1 H), 2.65 - 2.75 (m, 1 H), 2.56 - 2.64 (m, 1 H), 1.38 (s, 9H), 1.30-1.35 (m, 2H). HPLC retention time 5.1 minutes; column: Agilent Extended C-18 column (75 mm x 3 mm, 3.5 µm); column temperature 45 °C; flow rate 1.0 mL / minute; detection UV 230 nm; mobile phase: solvent A = acetonitrile (100%), solvent B = acetonitrile (5%) in 10 mM ammonium acetate; gradient elusion: 0-1.5 minutes solvent B (100%), 1.5-10.0 minutes solvent B (5%), 10.0-13.0 minutes solvent B (100%); total run time 13.0 minutes.

**Step 4: Preparation of C97-(2R,3S1 enantiomer**. A solution of **C97-racemic** (20.0 g, 54.7 mmol) in ethyl acetate (450 mL) was treated with diatomaceous earth (5.0 g) and filtered through a funnel charged with diatomaceous earth. The filter cake was washed with ethyl acetate (150 mL). The filtrate was charged with diatomaceous earth (20.0 g) and treated with (-)-L-dibenzoyltartaric acid (19.6 g, 54.7 mmol). The slurry was heated at 60 °C for 1.5 hours and then cooled to room temperature. The slurry was filtered and the solid washed with ethyl acetate (90 mL). The solid was collected and dried at 50 °C in a vacuum oven for 17 hours to yield **C97-(2R,3S) enantiomer** as a solid (mixed with diatomaceous earth). Yield: 17.3 g, 23.9 mmol, 43.6%, 97.6% ee. ¹H NMR (400 MHz, DMSO-*d*₆) δ 7.89 - 7.91 (m, 4H), 7.59 - 7.65 (m, 3H), 7.44 - 7.49 (m, 4H), 7.09 (d, *J*=8.3 Hz, 1 H), 6.53 (d, *J*=2.3 Hz, 1 H), 6.49 (dd, *J*=8.3, 2.3 Hz, 1 H), 5.65 (s, 2H), 4.85 (dd, *J*=9.3, 4.9 Hz, 1 H), 4.30 (d, *J*=15.3 Hz, 1 H), 4.10 (d, *J*=15.3 Hz, 1 H), 3.74 (s, 3H), 3.72 (s, 3H), 3.68 - 3.70 (m, 1 H), 2.92 - 2.96 (dd, *J*=13.6, 5.4 Hz, 1 H), 2.85 - 2.90 (dd, *J*=13.6, 6.3 Hz, 1 H), 1.36 (s, 9H). HPLC retention time 5.1 minutes; column: Agilent Extended C-18 column (75 mm x 3 mm, 3.5 µm); column temperature 45 °C; flow rate 1.0 mL / minute; detection UV 230 nm; mobile phase: solvent A = acetonitrile (100%), solvent B = acetonitrile (5%) in 10 mM ammonium acetate; gradient elusion: 0-1.5 minutes solvent B (100%), 1.5-10.0 minutes solvent B (5%), 10.0-13.0 minutes solvent B (100%); total run time 13.0 minutes. Chiral HPLC retention time 9.1 minutes; column: Chiralcel OD-H column (250 mm x 4.6 mm); column temperature 40 °C; flow rate 1.0 mL / minute; detection UV 208 nm; mobile phase: solvent A = ethanol (18%), solvent B = heptane (85%); isocratic elusion; total run time 20.0 minutes.

**Step 5: Preparation of C98-(2R,3S) enantiomer.** A solution of **C97-(2R,3S) enantiomer.** (16.7 g, 23.1 mmol) in ethyl acetate (301 mL) was treated with diatomaceous earth (18.3 g) and 5% aqueous potassium phosphate tribasic (182 mL). The slurry was stirred for 30 minutes at room temperature, then filtered under vacuum and the filter cake washed with ethyl acetate (2 x 67 mL). The filtrate was washed with 5% aqueous potassium phosphate tribasic (18 mL) and the organic layer dried over magnesium sulfate. The solid was filtered and the filter cake washed with ethyl acetate (33 mL). The filtrate was concentrated to a volume of 42 mL and slowly added to heptane (251 mL) and the resulting solid was collected by filtration. The solid was washed with heptane and dried at 50 °C in a vacuum oven for 19 hours to yield **C98-(2R,3S) enantiomer** as a solid. Yield: 6.4 g, 17.5 mmol, 76%, 98.8% ee. ¹H NMR (400 MHz, DMSO-*d*₆) δ 7.64 (d, *J*=9.4 Hz, 1H), 7.14 (d, *J*=8.2 Hz, 1H), 6.56 (s, 1H), 6.49 (dd, *J*=8.20, 2.3 Hz, 1 H), 4.78 (dd, *J*=9.37, 5.1 Hz, 1H), 4.30 (d, *J*=14.8 Hz, 1H), 4.14 (d, *J*=14.8 Hz, 1H), 3.77 (s, 3H), 3.75 (s, 3H), 3.45 - 3.53 (m, 1H), 2.65 - 2.75 (m, 1H), 2.56 - 2.64 (m, 1 H), 1.38 (s, 9H), 1.30 - 1.35 (m, 2H). HPLC retention time 5.2 minutes; column: Agilent Extended C-18 column (75 mm x 3 mm, 3.5 µm); column temperature 45 °C; flow rate 1.0 mL / minute; detection UV 230 nm; mobile phase: solvent A = acetonitrile (100%), solvent B = acetonitrile (5%) in 10 mM ammonium acetate; gradient elusion: 0-1.5 minutes solvent B (100%), 1.5-10.0 minutes solvent B (5%), 10.0-13.0 minutes solvent B (100%); total run time 13.0 minutes. Chiral HPLC retention time 8.7 minutes; column: Chiralcel OD-H column (250 mm x 4.6 mm); column temperature 40 °C; flow rate 1.0 mL / minute; detection UV 208 nm; mobile phase: solvent A = ethanol (18%), solvent B = heptane (85%); isocratic elusion; total run time 20.0 minutes.

**Step 6: Preparation of C99.** A solution of potassium phosphate tribasic N-hydrate (8.71 g, 41.05 mmol) in water (32.0 mL) at 22 °C was treated with a slurry of **C26-mesylate salt** (12.1 g, 27.4 mmol, q-NMR potency 98%) in dichloromethane (100.00 mL). The slurry was stirred for 1 hour at 22 °C. The reaction mixture was transferred to a separatory funnel and the layers separated. The aqueous layer was back extracted with dichloromethane (50.0 mL). The organic layers were combined, dried over magnesium sulfate, filtered under vacuum and the filter cake washed with dichloromethane (2 x 16 mL). The filtrate (-190 mL, amine solution) was used directly in the next step.

A solution of 1,1'-carbonyldiimidazole (6.66 g, 41.0 mmol) in dichloromethane (100 mL) at 22 °C under nitrogen was treated with the previously prepared amine solution (∼190 mL) added dropwise using an addition funnel over 3 hour at 22 °C with stirring. After the addition, the mixture was stirred for 1 hour at 22 °C, then treated with **C98-(2R,3S) enantiomer.** (10.0 g, 27.4 mmol) followed by *N,N-*dimethylformamide (23.00 mL). The reaction mixture was stirred at 22 °C for 3 hours and then heated at 40 °C for 12 hours. The solution was cooled to room temperature and the dichloromethane was removed using the rotary evaporator. The reaction mixture was diluted with ethyl acetate (216.0 mL) and washed with 10% aqueous citric acid (216.0 mL), 5% aqueous sodium chloride (2 x 216.0 mL), dried over magnesium sulfate and filtered under vacuum. The filter cake was washed with ethyl acetate (3 x 13 mL) and the ethyl acetate solution was concentrated on the rotary evaporator to a volume of (∼110.00 mL) providing a suspension.

The suspension (∼110.00 mL) was warmed to 40 °C and transferred into a stirred solution of heptane (22 °C) over 1 hour, to give a slurry. The slurry was stirred for 1 hour and filtered under vacuum. The filter cake was washed with heptane (3 x 30 mL) and dried under vacuum at 50 °C for 12 hours to afford **C99** as a solid. Yield: 18.1 g, 24.9 mmol, 92%. LCMS *m*/*z* 728.4 (M+1). ¹H NMR (400 MHz, DMSO-*d*₆) δ 8.09 (s, 1 H), 7.62 (d, *J*=9.4 Hz, 1 H), 7.33-7.52 (m, 10H), 7.07 (d, *J*=8.3 Hz, 1 H), 6.51 (d, *J*=2.3 Hz, 1 H), 6.50 (m, 1 H), 6.44 (dd, *J*=8.3, 2.3 Hz, 1 H), 6.12 (m, 1 H), 6.07 (s, 1 H), 5.27 (s, 2H), 5.00 (s, 2H), 4.73 (dd, *J*=9.4, 5.2 Hz, 1 H), 4.38 (d, *J*=15.0 Hz, 1 H), 4.19 (m, 2H), 3.99 (d, *J*=15.0 Hz, 1H), 3.72 (s, 3H), 3.71 (s, 3H), 3.48 (m, 1H), 3.28 (m, 1H), 3.12 (m, 1H), 1.37 (s, 9H).

**Step 7: Preparation of C100.** A solution of **C99** (46.5 g, 63.9 mmol) in acetonitrile (697 mL and water (372 mL) was treated with potassium persulfate (69.1 g, 255.6 mmol) and potassium phosphate dibasic (50.1 g, 287.5 mmol). The biphasic mixture was heated to 75 °C and vigorously stirred for 1.5 hours. The pH was maintained between 6.0-6.5 by potassium phosphate dibasic addition (-12 g). The mixture was cooled to 20 °C, the suspension was filtered and washed with acetonitrile (50 mL). The filtrate was concentrated using the rotary evaporator and treated with water (50 mL) followed by ethyl acetate (200 mL). The slurry was stirred for 2 hours at room temperature, filtered and the solid dried under vacuum at 40 °C overnight. The solid was slurried in a mixture of ethyl acetate and water (6 : 1, 390.7 mL) at 20 °C for 1 hour then collected by filtration. The solid was dried in a vacuum oven to yield **C100.** Yield: 22.1 g, 38.3 mmol, 60%. ¹H NMR (400 MHz, DMSO-*d*₆) δ 8.17 (br s, 1 H), 7.96 (s, 1 H), 7.58 (d, *J*=9.6 Hz, 1 H), 7.29-7.50 (m, 10H), 6.49 (dd, *J*=8.0, 6.0 Hz, 1H), 6.08 (dd, *J*=5.6, 5.2 Hz, 1H), 5.93 (s, 1H), 5.22 (s, 2H), 4.96 (s, 2H), 4.77 (dd, *J*=9.6, 5.0 Hz, 1 H), 4.16 (m, 2H), 3.61 (m, 1 H), 3.11 (m, 2H), 1.36 (s, 9H). HPLC retention time 6.17 minutes; XBridge C8 column (4.6 x 75 mm, 3.5 µm); column temperature 45 °C; flow rate 2.0 mL/minute; detection UV 210 nm, 230 nm, and 254 nm; mobile phase: solvent A = methanesulfonic acid (5%) in 10 mmol sodium octylsulfonate, solvent B = acetonitrile (100%); gradient elusion: 0-1.5 minutes solvent A (95%) and solvent B (5%), 1.5-8.5 minutes solvent A (5%) and solvent B (95%), 8.5-10.0 minutes solvent A (5%) and solvent B (95%), 10.01-12.0 minutes solvent A (95%) and solvent B (5%); total run time 12.0 minutes.

**Step 8: Preparation of C101.** A solution of trifluoroacetic acid (120 mL, 1550 mmol) under nitrogen was treated with methoxybenzene (30 mL, 269 mmol) and cooled to -5 °C. Solid **C100** (17.9 g, 31.0 mmol) was charged in one portion at -5 °C and the resulting mixture stirred for 3 hours. The reaction mixture was cannulated with nitrogen pressure over 15 minutes to a stirred mixture of Celite (40.98 g) and methyl *tert*-butyl ether (550 mL) at 10 °C. The slurry was stirred at 16 °C for 30 minutes, then filtered under vacuum. The filter cake was rinsed with methyl *tert*-butyl ether (2 x 100 mL). The solid was collected and slurried in methyl tert-butyl ether (550 mL) with vigorous stirring for 25 minutes. The slurry was filtered by vacuum filtration and washed with methyl *tert*-butyl ether (2 x 250 mL). The solid was collected and dried in a vacuum oven at 60 °C for 18 hours to afford **C101** on Celite. Yield: 57.6 g total = **C101** + Celite; 16.61 g **C101,** 28.1 mmol, 91%. ¹H NMR (400 MHz, DMSO-*d*₆) δ 8.75-8.95 (br s, 2H), 8.65 (s, 1H), 8.21 (s, 1 H), 7.30-7.58 (m, 10H), 6.83 (brs, 1H), 6.65 (brs, 1H), 6.17 (s, 1 H), 5.30 (s, 2H), 5.03 (s, 2H), 4.45 (br s, 1 H), 4.22 (br s, 2H), 3.77 (m, 1 H), 3.36 (m, 1 H), 3.22 (m, 1 H). ¹⁹F NMR (376 MHz, DMSO-*d*₆) δ -76.0 (s, 3F). HPLC retention time 5.81 minutes; XBridge C8 column (4.6 x 75 mm, 3.5 µm); column temperature 45 °C; flow rate 2.0 mL/minute; detection UV 210 nm, 230 nm, and 254 nm; mobile phase: solvent A = methanesulfonic acid (5%) in 10 mmol sodium octylsulfonate, solvent B = acetonitrile (100%); gradient elusion: 0-1.5 minutes solvent A (95%) and solvent B (5%), 1.5-8.5 minutes solvent A (5%) and solvent B (95%), 8.5-10.0 minutes solvent A (5%) and solvent B (95%), 10.01-12.0 minutes solvent A (95%) and solvent B (5%); total run time 12.0 minutes.

**Step 9: Preparation of C90.** A suspension of **C101** (67.0 g, 30% activity on Celite = 33.9 mmol) in acetonitrile (281.4 mL) was treated with molecular sieves 4AE (40.2 g), **C5** (17.9 g, 33.9 mmol), 4-dimethylaminopyridine (10.4 g, 84.9 mmol) and the mixture was stirred at 40°C for 16 hours. The reaction mixture was cooled to 20 °C, filtered under vacuum and the filter cake washed with acetonitrile (2 x 100 mL). The filtrate was concentrated under vacuum to a volume of -50 mL. The solution was diluted with ethyl acetate (268.0 mL) and washed with 10% aqueous citric acid (3 x 134 mL) followed by 5% aqueous sodium chloride (67.0 mL). The organic layer was dried over magnesium sulfate and filtered under vacuum. The filter cake was washed with ethyl acetate (2 x 50 mL) and the filtrate was concentrated to a volume of -60 mL. The filtrate was added slowly to heptane (268 mL) with stirring and the slurry was stirred at 20 °C for 1 hour. The slurry was filtered under vacuum and the filter cake washed with a mixture of heptane and ethyl acetate (4: 1, 2 x 27 mL). The solid was collected and dried under vacuum for 12 hours at 50 °C to afford a solid. The crude product was purified via chromatography on silica gel (ethyl acetate / 2-propanol), product bearing fractions were combined and the volume was reduced to -60 mL. The solution was added dropwise to heptane (268 mL) with stirring. The slurry was stirred at room temperature for 3 hours, filtered and washed with heptane and ethyl acetate (4: 1, 2 x 27 mL). The solid was collected and dried under vacuum for 12 hours at 50 °C to afford **C90** as a solid. Yield: 16.8 g, 18.9 mmol, 58%. LCMS *m*/*z* 889.4 (M+1). ¹H NMR (400 MHz, DMSO-*d*₆) 11.90 (br s, 1 H), 9.25 (d, *J*=8.7 Hz, 1 H), 8.40 (br s, 1 H), 7.98 (s, 1 H), 7.50-7.54 (m, 2H), 7.32-7.47 (m, 8H), 7.28 (s, 1 H), 6.65 (br s, 1 H), 6.28 (br s, 1 H), 5.97 (s, 1 H), 5.25 (s, 2H), 5.18 (dd, *J*=8.8, 5 Hz, 1H), 4.99 (s, 2H), 4.16-4.28 (m, 2H), 3.74-3.80 (m, 1H), 3.29-3.41 (m, 1 H), 3.13-3.23 (m, 1 H), 1.42 (s, 9H), 1.41 (s, 3H), 1.39 (br s, 12H).

**Step 10: Preparation of C91.** A solution of **C90** (14.5 g, 16.3 mmol) in anhydrous *N,N-*dimethylformamide (145.0 mL) was treated with sulfur trioxide *N,N*-dimethylformamide complex (25.0 g, 163.0 mmol). The reaction mixture was stirred at room temperature for 45 minutes, then transferred to a stirred mixture of 5% aqueous sodium chloride (290 mL) and ethyl acetate (435 mL) at 0 °C. The mixture was warmed to 18 °C and the layers separated. The aqueous layer was extracted with ethyl acetate (145 mL) and the combined organic layers washed with 5% aqueous sodium chloride (3 x 290 mL) followed by saturated aqueous sodium chloride (145 mL). The organic layer was dried over magnesium sulfate, filtered through diatomaceous earth and the filter cake washed with ethyl acetate (72 mL). The filtrate was concentrated to a volume of 36 mL and treated with methyl *tert*-butyl ether (290 mL), the resulting slurry was stirred at room temperature for 1 hour. The solid was collected by filtration, washed with methyl *tert-*butyl ether (58 mL) and dried at 50 °C for 2 hours followed by 20 °C for 65 hours in a vacuum oven to yield **C91** as a solid. Yield: 15.0 g, 15.4 mmol, 95%. LCMS *m*/*z* 967.6 (M-1). ¹H NMR (400 MHz, DMSO-*d*₆) δ 11.62 (brs, 1 H), 9.29 (d, *J*=8.8 Hz, 1H), 9.02 (s, 1 H), 7.58-7.61 (m, 2H), 7.38-7.53 (m, 9H), 7.27 (s, 1 H), 7.07 (s, 1 H), 6.40 (br d, *J*=8.0 Hz, 1 H), 5.55 (s, 2H), 5.25 (s, 2H), 5.20 (dd, *J*=8.8, 5.6 Hz, 1 H), 4.46 (br dd, half of ABX pattern, *J*=17.0, 5.0 Hz, 1 H), 4.38 (br dd, half of ABX pattern, *J*=17.0, 6.0 Hz, 1 H), 3.92-3.98 (m, 1H), 3.79-3.87 (m, 1H), 3.07-3.17 (m, 1H), 1.40 (s, 9H), 1.39 (s, 3H), 1.38 (s, 12H).

**Step 11: Preparation of C92.** A solution of **C91** (20.0 g, 20.6 mmol) in dichloromethane (400 mL) was concentrated under reduced pressure (420 mmHg) at 45 °C to a volume of 200 mL. The solution was cooled to -5 °C and treated with 1 M boron trichloride in dichloromethane (206.0 mL, 206.0 mmol) added dropwise over 40 minutes. The reaction mixture was warmed to 15 °C over 1 hour with stirring. The slurry was cooled to -15 °C and treated with a mixture of 2,2,2-trifluoroethanol (69.2 mL) and methyl *tert*-butyl ether (400 mL), maintaining the temperature at -15 °C. The reaction mixture was warmed to 0 °C over 1 hour. The suspension was filtered using nitrogen pressure and the solid washed with methyl *tert*-butyl ether (2 x 200 mL). Nitrogen was passed over the solid for 2 hours. The solid was collected and suspended in methyl *tert*-butyl ether (400 mL) for 1 hour with stirring at 18 °C. The suspension was filtered using nitrogen pressure and the solid washed with methyl *tert*-butyl ether (2 x 200 mL). Nitrogen was passed over the resulting solid for 12 hours. A portion of the crude product was neutralized with 1 M aqueous ammonium formate to pH 5.5 with minimal addition of *N,N*-dimethylformamide to prevent foaming. The feed solution was filtered and purified via reverse phase chromatography (C-18 column; acetonitrile / water gradient with 0.2% formic acid modifier). The product bearing fractions were combined and concentrated to remove acetonitrile. The solution was captured on a GC-161M column, washed with deionized water and blown dry with nitrogen pressure. The product was released using a mixture of methanol / water (10: 1) and the product bearing fractions were added to a solution of ethyl acetate (6 volumes). The solid was collected by filtration to afford **C92** as a solid. Yield: 5.87 g, 9.28 mmol. LCMS m/z 633.3 (M+1). ¹H NMR (400 MHz, DMSO-*d*₆) δ 9.22 (d, *J*=8.7 Hz, 1H), 8.15 (s, 1H), 7.26-7.42 (br s, 2H), 7.18-7.25 (m, 1 H), 6.99 (s, 1 H), 6.74 (s, 1 H), 6.32-6.37 (m, 1 H), 5.18 (dd, *J*=8.7, 5.7 Hz, 1 H), 4.33 (br d, *J*=4.6 Hz, 2H), 3.94-4.00 (m, 1 H), 3.60-3.68 (m, 1H), 3.19-3.27 (m, 1 H), 1.40 (s, 3H), 1.39 (s, 3H).

### Example 5

### disodium 2-({[(1Z)-1-(2-amino-1,3-thiazol-4-yl)-2-({(2R,3S)-2-[({[(1,5-dihydroxy-4-oxo-1,4-dihydropyridin-2-yl)methoxy]carbonyl}amino)methyl]-4-oxo-1-sulfonatoazetidin-3-yl}amino)-2-oxoethylidene]amino}oxy)-2-methylpropanoate (C104-Bis Na salt).

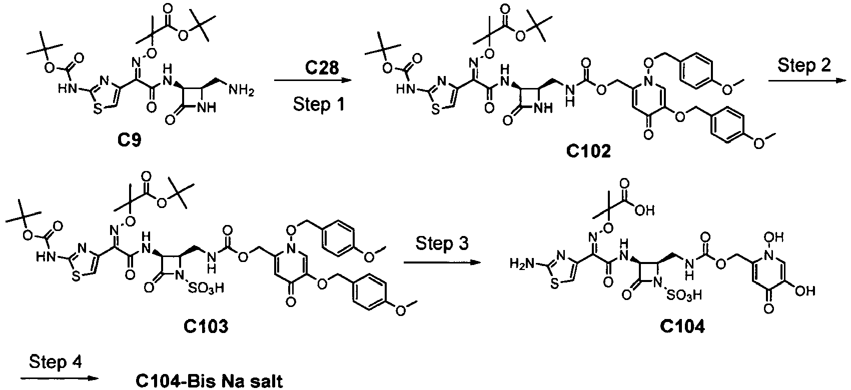

**Step 1: Preparation of C102.** A solution of **C28** (300 mg, 0.755 mmol) in tetrahydrofuran (10 mL) was treated with 1,1'-carbonyldiimidazole (379 mg, 2.26 mmol) at room temperature and stirred for 20 hours. The yellow reaction mixture was treated with a solution of **C9** (286 mg, 0.543 mmol) in tetrahydrofuran (25 mL). The mixture was stirred for 6 hours at room temperature, then treated with water (20 mL) and extracted with ethyl acetate (3 x 25 mL). The combined organic layers were dried over sodium sulfate, filtered and concentrated in vacuo. The crude material was purified via chromatography on silica gel (heptane / ethyl acetate / 2-propanol) to afford **C102** as a light yellow solid. Yield: 362 mg, 0.381 mmol, 62%. LCMS *m*/*z* 950.4 (M+1). ¹H NMR (400 MHz, DMSO-*d*₆), characteristic peaks: δ 9.31 (d, *J*=8.4 Hz, 1 H), 8.38 (s, 1 H), 8.00 (s, 1H), 7.41 (brd, *J*=8.2 Hz, 2H), 7.36 (brd, *J*=8.8 Hz, 2H), 7.26 (s, 1H), 6.10 (s, 1H), 5.20 (s, 2H), 4.92 (br s, 4H), 3.77 (s, 3H), 3.76 (s, 3H), 1.45 (s, 9H), 1.38 (s, 9H).

**Step 2: Preparation of C103.** A solution of **C102** (181 mg, 0.191 mmol) in anhydrous *N,N*-dimethylformamide (2.0 mL) was treated with sulfur trioxide pyridine complex (302 mg, 1.91 mmol). The reaction mixture was allowed to stir at room temperature for 6 hours, then cooled to 0 °C and quenched with water. The resulting solid was collected by filtration and dried in vacuo to yield **C103** as a white solid. Yield: 145 mg, 0.14 mmol, 74%. APCI m/z 1028.5 (M-1). ¹H NMR (400 MHz, DMSO-*d*₆), characteristic peaks: δ 11.65 (brs, 1H), 9.37 (d, *J*=8.6 Hz, 1H), 8.87 (s, 1 H), 7.49 (brd, *J*=8.6 Hz, 2H), 7.43 (br d, *J*=8.6 Hz, 2H), 7.26 (s, 1 H), 7.01 (br d, *J*=8.9 Hz, 2H), 7.00 (br d, *J*=8.8 Hz, 2H), 5.43 (s, 2H), 5.20 (dd, *J*=8.4, 6 Hz, 1 H), 4.01-4.07 (m, 1 H), 3.78 (s, 3H), 3.77 (s, 3H), 3.50-3.58 (m, 1 H), 3.29-3.37 (m, 1 H), 1.44 (s, 9H), 1.37 (s, 9H).

**Step 3: Preparation of C104.** A solution of **C103** (136 mg, 0.132 mmol) in anhydrous dichloromethane (5 mL) was treated with 1 M boron trichloride in *p*-xylenes (0.92 mL, 0.92 mmol) and allowed to stir at room temperature for 40 minutes. The reaction mixture was cooled in an ice bath, quenched with water (0.4 mL), and transferred into a solution of methyl *tert*-butyl ether: heptane (1:2, 12 mL). The solvent was removed in vacuo and the crude product was purified via reverse phase chromatography (C-18 column; acetonitrile / water gradient with 0.1% formic acid modifier) to yield **C104** as a light yellow solid. Yield: 43 mg, 0.068 mmol, 51%. LCMS *m*/*z* 634.4 (M+1). ¹H NMR (400 MHz, DMSO-*d*₆), characteristic peaks: δ 9.29 (d, *J*=8.5 Hz, 1 H), 8.10 (s, 1 H), 7.04-7.10 (m, 1H), 7.00 (s, 1H), 6.75 (s, 1H), 5.05-5.30 (m, 3H), 4.00-4.07 (m, 1H), 1.42 (s, 3H), 1.41 (s, 3H).

**Step 4: Preparation of C104-Bis Na salt.** A suspension of **C104** (212 mg, 0.33 mmol) in water (10 mL) was cooled to 0 °C and treated with a solution of sodium bicarbonate (56.4 mg, 0.67 mmol) in water (2 mL), added dropwise. The reaction mixture was cooled to -70 °C (frozen) and lyophilized to afford **C104-Bis Na salt** as a white solid. Yield: 210 mg, 0.31 mmol, 93%. LCMS *m*/*z* 632.5 (M-1). ¹H NMR (400 MHz, D₂O) δ 7.87 (s, 1 H), 6.94 (s, 1 H), 6.92 (s, 1 H), 5.35 (d, *J*=5 Hz, 1 H), 5.16 (s, 2H), 4.46-4.52 (m, 1 H), 3.71 (dd, half of ABX pattern, *J*=14.5, 6 Hz, 1 H), 3.55 (dd, half of ABX pattern, *J*=14.5, 6 Hz, 1 H), 1.43 (s, 3H), 1.42 (s, 3H).

### Example 6

### 2-({[(1Z)-1-(2-amino-1,3-thiazol-4-yl)-2-({(2S,3S)-2-[({N-[(4,5-dihydroxypyridin-2-yl)carbonyl]glycyl}oxy)methyl]-4-oxo-1-sulfoazetidin-3-yl}amino)-2-oxoethylidene]amino}oxy)-2-methylpropanoic acid (C106).

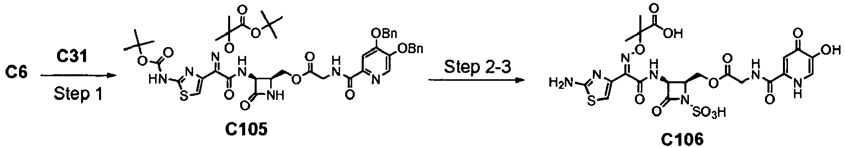

**Step 1: Preparation of C105.** A solution of **C31** (0.89 g, 2.27 mmol) in anhydrous dichloromethane (20 mL) was treated with **C6** (1.00 g, 1.90 mmol), 1,3-dicyclohexylcarbodiimide (0.47 g, 2.27 mmol), and 4-dimethylaminopyridine (0.035 g, 0.28 mmol). The reaction mixture was allowed to stir at room temperature for 1 hour, filtered, and the filtrate was concentrated in vacuo. The crude product was purified by chromatography on silica gel (heptane / ethyl acetate 0 to 100%) to yield **C105** as a white solid. Yield: 1.32 g, 1.46 mmol, 77%. LCMS *m*/*z* 903.1 (M+1). ¹H NMR (400 MHz, DMSO-*d*₆) δ 11.76 (br s, 1 H), 9.27 (d, *J*=9.0 Hz, 1 H), 8.90 (dd, *J*=6, 6 Hz, 1 H), 8.61 (br s, 1 H), 8.30 (s, 1H), 7.70 (s, 1 H), 7.31-7.48 (m, 10H), 7.26 (br s, 1 H), 5.31-5.34 (m, 4H), 5.27-5.31 (m, 1 H), 4.30 (dd, *J*=11.6, 3.2 Hz, 1 H), 4.15 (dd, *J*=11.5, 8.9 Hz, 1 H), 4.04-4.08 (m, 2H), 3.97-4.02 (m, 1 H), 1.44 (s, 9H), 1.42 (s, 3H), 1.39 (s, 3H), 1.38 (s, 9H).

**Steps 2-3: Preparation of C106. C105** was converted to **C106** by methods analogous to those described in Example 4, Route 1, Steps 2 and 3. The crude product was purified via reverse phase chromatography (C-18 column; acetonitrile / water gradient with 0.1% formic acid modifier) to yield **C106** as a white solid. LCMS *m*/*z* 644.7 (M-1). ¹H NMR (400 MHz, DMSO-*d*₆) δ 9.23 (d, *J*=8.6 Hz, 1H), 9.13 (br s, 1H), 8.13 (s, 1H), 7.98 (s, 1 H), 7.60 (s, 1 H), 6.82 (s, 1 H), 5.26 (dd, *J*=8.6, 5.3 Hz, 1 H), 4.56 (dd, *J*=10.8, 4.6 Hz, 1 H), 3.94-4.26 (m, 4H), 1.45 (s, 3H), 1.43 (s, 3H).

### Example 7

### 2-({[(1Z)-1-(2-amino-1,3-thiazol-4-yl)-2-({(2S,3S)-2-[({N-[(1,5-dihydroxy-4-oxo-1,4-dihydropyridin-2-yl)carbonyl]glycyl}oxy)methyl]-4-oxo-1-sulfoazetidin-3-yl}amino)-2-oxoethylidene]amino}oxy)-2-methylpropanoic acid (C108).

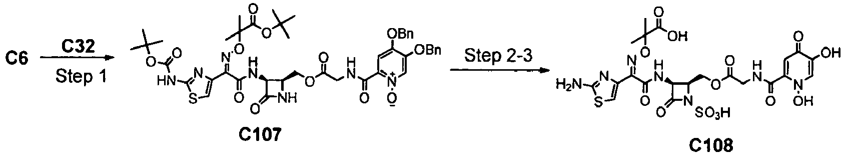

**Step 1: Preparation of C107.** A solution of **C32** (2.30 g, 3.40 mmol) in anhydrous *N,N-*dimethylformamide (30 mL) was treated with **C6** (1.73 g, 3.28 mmol), *O*-(7-azabenzotriazol-1-yl)-1,1,3,3-tetramethyluronium hexafluorophosphate (4.25 g, 11.2 mmol) and sodium bicarbonate (2.70 g, 32.0 mmol). The reaction was allowed to stir at room temperature, under nitrogen, for 5 hours. The mixture was quenched with water (100 mL) and the organic layer was extracted with ethyl acetate (3 x 150 mL), dried over sodium sulfate, filtered and concentrated in vacuo to yield a crude oil that was purified via chromatography on silica gel (ethyl acetate / 2-propanol 0 to 10%) to yield **C107** as a red solid. Yield: 1.4 g, 1.52 mmol, 75%. LCMS *m*/*z* 919.3 (M+1).

**Steps 2-3: Preparation of C108. C107** was converted to **C108** by methods analogous to those described in Example 4, Route 1, Steps 2 and 3. The crude product was purified via reverse phase chromatography (C-18 column; acetonitrile / water gradient with 0.1% formic acid modifier) to yield **C108** as a white solid. LCMS *m*/*z* 660.8 (M-1). ¹H NMR (400 MHz, DMSO-*d*₆) δ 11.80-11.85 (m, 1 H), 10.7-11.0 (v br s, 1 H), 9.24 (d, *J*=8.6 Hz, 1 H), 8.13 (s, 1H), 7.83 (s, 1 H), 7.55 (s, 1 H), 6.83 (brs, 1H), 5.26 (dd, *J*=8.8, 5.5 Hz, 1 H), 4.53 (dd, *J*=11.5, 4.7 Hz, 1 H), 4.14-4.27 (m, 3H), 4.07 (dd, half of ABX pattern, *J*=18.0, 5.7 Hz, 1H), 1.44 (s, 3H), 1.43 (s, 3H).

### Example 8

### 2-({[(1Z)-1-(2-amino-1,3-thiazol-4-yl)-2-{[(2R,3S)-2-{[({1-[(5-hydroxy-4-oxo-1,4-dihydropyridin-2-yl)carbonyl]azetidin-3-yl}carbonyl)amino]methyl}-4-oxo-1-sulfoazetidin-3-yl]amino}-2-oxoethylidene]amino}oxy)-2-methylpropanoic acid (C110).

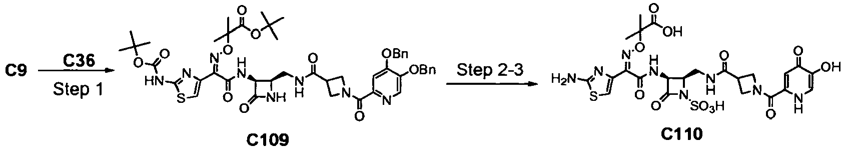

**Step 1: Preparation of C109.** A solution of **C36** (120 mg, 0.287 mmol) in *N,N-*dimethylformamide (3 mL) was treated with **C9** (160 mg, 0.30 mmol), *O*-(7-azabenzotriazol-1-yl)-*N,N,N,'N'*-tetramethyluronium hexafluorophosphate (225 mg, 0.58 mmol) and sodium bicarbonate (86 mg, 1.0 mmol). The reaction mixture was stirred for 3 hours. The mixture was diluted with dichloromethane, washed with water and the organic layer was separated. The aqueous layer was back extracted with dichloromethane (2x). The combined organic layers were dried over sodium sulfate, filtered and the solvent was removed in vacuo. The crude product was purified by chromatography on silica gel (heptane / ethyl acetate 20 to 100% then ethyl acetate / methanol 0 to 10%) to provide **C109** as a white solid. Yield: 141 mg, 0.152 mmol, 53%. LCMS *m*/*z* 927.4 (M+1). ¹H NMR (400 MHz, DMSO-*d*₆) δ 11.73 (br s, 1 H), 9.32 (d, *J*=8.6 Hz, 1 H), 8.36 (br s, 1H), 8.25 (s, 1 H), 7.95 (br dd, *J*=5.3, 5.1 Hz, 1 H), 7.65 (s, 1 H), 7.31-7.47 (m, 10H), 7.28 (s, 1 H), 5.29 (s, 4H), 5.14-5.19 (m, 1 H), 4.62-4.69 (m, 1 H), 4.52-4.59 (m, 1 H), 4.01-4.15 (m, 2H), 3.81-3.87 (m, 1 H), 3.21-3.41 (m, 3H, assumed; partially obscured by water peak), 1.44 (s, 9H), 1.42 (s, 3H), 1.39 (s, 9H), 1.37 (s, 3H). **Steps 2-3: Preparation of C110. C109** was converted to **C110** by methods analogous to those described in Example 4, Route 1, Steps 2 and 3. The crude product was purified by reverse phase chromatography (C-18 column; acetonitrile / water gradient with 0.1% formic acid modifier), the fractions were combined and the solvent was removed. The material was suspended in acetonitrile, sonicated and filtered to provide **C110** as a pink solid. LCMS *m*/*z* 671.5 (M+1). ¹H NMR (400 MHz, DMSO-*d*₆) δ 9.30 (d, *J*=8.0 Hz, 1H), 7.95 (s, 1H), 7.73-7.80 (m, 1 H), 7.34 (s, 1H), 6.79 (s, 1H), 5.11-5.19 (m, 1 H), 4.47-4.66 (m, 2H), 3.99-4.19 (m, 3H), 3.50-3.60 (m, 1 H), 3.29-3.42 (m, 2H), 1.43 (s, 3H), 1.41 (s, 3H).

### Example 9

### 2-{[(1Z)-1-(2-amino-1,3-thiazol-4-yl)-2-{[(2R,3S)-2-{[({1-[(5-hydroxy-4-oxo-1,4-dihydropyridin-2-yl)methyl]azetidin-3-yl}carbonyl)amino]methyl}-4-oxo-1-sulfoazetidin-3-yl]amino}-2-oxoethylidene]amino}oxy)-2-methylpropanoic acid (C112).

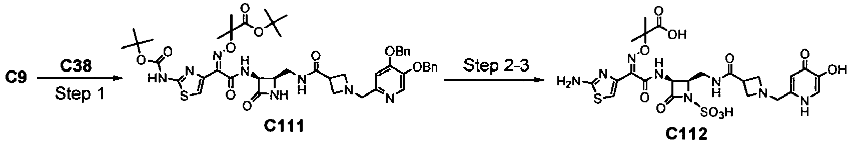

**Step 1: Preparation of C111.** A solution of **C38** (123 mg, 0.304 mmol) in *N,N-*dimethylformamide (3 mL) was treated with **C9** (192 mg, 0.365 mmol), O-(7-azabenzotriazol-1-yl)-1,1,3,3-tetramethyluronium hexafluorophosphate (238 mg, 0.61 mmol) and sodium bicarbonate (91 mg, 1.1 mmol). The reaction was stirred for 1 hour at room temperature. The mixture was diluted with dichloromethane and washed with water. The organic layer was separated and the aqueous layer was re-extracted with dichloromethane (2x). The combined organic layers were dried over sodium sulfate, filtered and the solvent was removed in vacuo. The crude product was purified by chromatography on silica gel (heptane / ethyl acetate 20 to 100% followed by ethyl acetate / methanol 0 to 10%) to provide **C111** as a yellow solid. Yield: 167 mg, 0.18 mmol, 60%. LCMS *m*/*z* 913.5 (M+1). ¹H NMR (400 MHz, DMSO-*d*₆) δ 11.76 (br s, 1H), 9.33 (d, *J*=8.4 Hz, 1H), 8.33 (s, 1H), 8.21 (s, 1H), 8.05-8.12 (bs, 1H), 7.30-7.49 (m, 10H), 7.28 (s, 1H), 7.24 (br s, 1H), 5.23 (s, 2H), 5.22 (s, 2H), 5.16-5.21 (m, 1H), 4.28-4.44 (br m, 2H), 3.95-4.21 (br m, 4H), 3.82-3.88 (m, 1H), 3.41-3.51 (br m, 1H), 2.81-2.96 (br m, 2H), 1.35-1.48 (m, 24H).

**Steps 2-3: Preparation of C112. C111** was converted to **C112** by methods analogous to those described in Example 4, Route 1, Steps 2 and 3. The crude material was purified via reverse phase chromatography (C-18 column; acetonitrile / water with 0.1% formic acid modifier) to provide **C112** as a yellow solid. LCMS *m*/*z* 655.5 (M-1). ¹H NMR (400 MHz, DMSO-*d*₆), characteristic peaks: δ 9.27 (d, *J*=8.4 Hz, 1H), 7.86-7.91 (m, 1H), 7.23 (br s, 2H), 6.71 (s, 1H), 5.10 (dd, *J*=8.2, 5.9 Hz, 1H), 4.19 (br s, 2H), 1.38 (s, 3H), 1.35 (s, 3H).

### Example 10

### 2-({[(1Z)-1-(2-amino-1,3-thiazol-4-yl)-2-{[(2R,3S)-2-({[(5-hydroxy-4-oxo-1,4-dihydropyridin-2-yl)carbonyl]amino}methyl)-4-oxo-1-sulfoazetidin-3-yl]amino}-2-oxoethylidene]amino}oxy)-2-methylpropanoic acid (C114).

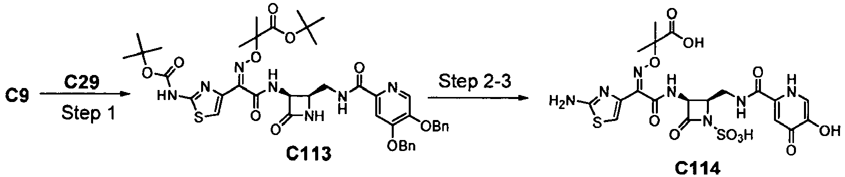

**Step 1: Preparation of C113.** To a solution of **C29** (100 mg, 0.298 mmol) in tetrahydrofuran (3 mL) was added **C9** (188 mg, 0.358 mmol), *O*-(7-azabenzotriazol-1-yl)-*N,N,N',N'*-tetramethyluronium hexafluorophosphate (234 mg, 0.60 mmol) and sodium bicarbonate (90 mg, 1.1 mmol). The reaction was stirred at room temperature for 18 hours. The mixture was diluted with dichloromethane and washed with water. The aqueous layer was back-extracted with dichloromethane (2 x). The combined organic layers were dried over sodium sulfate, filtered and the solvent was removed in vacuo. The crude product was purified by chromatography on silica gel (heptane / ethyl acetate 50 to 100%) to provide **C113** as a yellow solid. Yield: 277 mg, 0.33 mmol, 74%. LCMS *m*/*z* 844.4 (M+1). ¹H NMR (400 MHz, DMSO-*d*₆) δ 11.80 (br s, 1 H), 9.37 (d, *J*=8.6 Hz, 1 H), 8.59 (br dd, *J*=6, 6 Hz, 1 H), 8.40 (br s, 1 H), 8.24 (s, 1 H), 7.72 (s, 1 H), 7.31-7.49 (m, 10H), 7.27 (s, 1H), 5.33 (s, 2H), 5.31 (s, 2H), 5.15-5.19 (m, 1H), 3.89-3.95 (m, 1 H), 3.42-3.58 (m, 2H), 1.36-1.47 (m, 24H).

**Steps 2-3: Preparation of C114. C113** was converted to **C114** by methods analogous to those described in Example 4, Route 1, Steps 2 and 3. The crude material was purified by reverse phase chromatography (C-18 column; acetonitrile / water with 0.5% formic acid modifier). The fractions were combined and the solvent was removed. The material was suspended in acetonitrile, sonicated and filtered to provide **C114** as a yellow solid. ¹H NMR (400 MHz, DMSO-*d*₆) δ 9.35 (d, *J*=8.4 Hz, 1 H), 8.85-8.92 (m, 1 H), 8.13 (s, 1H), 7.93 (s, 1 H), 7.51 (s, 1H), 6.78 (s, 1H), 5.23 (dd, *J*=8.4, 5.7 Hz, 1H), 4.10-4.17 (m, 1 H), 3.76-3.84 (m, 1H), 3.47-3.56 (m, 1H), 1.44 (s, 3H), 1.43 (s, 3H).

### Example 11

### 2-({[(1Z)-1-(2-amino-1,3-thiazol-4-yl)-2-{[(2R,3S)-2-({[(1,5-dihydroxy-4-oxo-1,4-dihydropyridin-2-yl)carbonyl]amino}methyl)-4-oxo-1-sulfoazetidin-3-yl]amino}-2-oxoethylidene]amino}oxy)-2-methylpropanoic acid, disodium salt (C116).

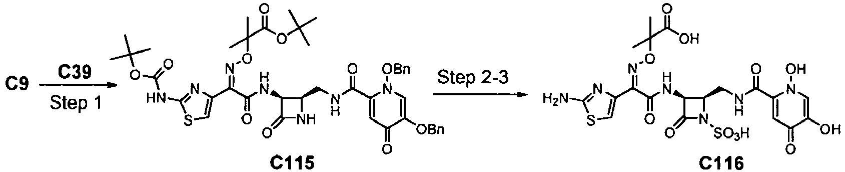

**Step 1: Preparation of C115.** A suspension of **C39** (3.99 g, 10.2 mmol) in dimethyl sulfoxide (50 mL) was treated with *N*-hydroxysuccinimide (1.30 g, 11.3 mmol), 1-ethyl-3-(3-dimethylaminopropyl)carbodiimide hydrochloride (3.14 g, 16.4 mmol) and pyridine hydrochloride (1.30 g, 11.3 mmol) and the mixture was stirred for 2 hours at room temperature. The reaction mixture was treated with **C9** (3.6 g, 6.84 mmol) and the solution was stirred at room temperature for 2 hours. To the mixture was added water and stirring was continued for 5 minutes. The resulting slurry was filtered under vacuum. The collected solids were washed with water (3x). The solid was dissolved in ethyl acetate and washed with 1 N aqueous hydrochloric acid, sodium bicarbonate (saturated aqueous) and water, respectively. The organic layer was dried over sodium sulfate, filtered and concentrated to a minimal volume. The solution was added to heptane and the mixture was concentrated under reduced pressure to afford a solid. The solid was slurried in diethyl ether (75 mL) and stirred at room temperature for 30 minutes. To the mixture was added heptane and the thin slurry was stirred at room temperature for 2 hours. The mixture was filtered under vacuum and the solids were washed with diethyl ether / heptane (1:1, 100 mL). The resulting solids were dried in vacuo to afford **C115** as a light yellow solid. Yield: 4.32 g, 5.02 mmol, 74%. LCMS *m*/*z* 860.6 (M+1). ¹H NMR (400 MHz, CD₃OD) δ 7.80 (s, 1 H), 7.31-7.45 (m, 11 H), 6.49 (s, 1 H), 5.34 (AB quartet, *J*_{AB}=9.4 Hz, Δν_{AB}=13.3 Hz, 2H), 5.25 (d, *J*=4.9 Hz, 1 H), 5.01 (br s, 2H), 4.00 (ddd, *J*=8.5, 4.8, 4.7 Hz, 1H), 3.71 (dd, *J*=14.2, 4.5 Hz, 1H), 3.58 (dd, *J*=14.2, 8.3 Hz, 1 H), 1.49 (s, 3H), 1.49 (s, 9H), 1.48 (s, 3H), 1.46 (s, 9H).

**Steps 2-3: Preparation of C116-Bis Na salt. C115** was converted to **C116** by methods analogous to those described in Example 4, Route 1, Steps 2 and 3. Crude product was purified via reverse phase chromatography (C18 column in acetonitrile : water solvent system with 0.1% formic acid modifier) to afford an off-white solid (240.8 mg). The solid was slurried in deionized water (10 mL) and cooled to 0 °C. To this mixture was added sodium bicarbonate (2 equivalents) and the resulting solution was freeze dried to afford **C116-Bis Na salt.** LCMS *m*/*z* 602.4 (M-1). ¹H NMR (400 MHz, D₂O) δ 7.66 (s, 1 H), 7.37 (s, 1 H), 6.84 (s, 1 H), 5.45 (d, *J*=5.7 Hz, 1 H), 4.58 (ddd, *J*=6.0, 5.8, 5.8 Hz, 1 H), 3.96 (dd, half of ABX pattern, *J*=14.6, 5.7 Hz, 1 H), 3.89 (dd, half of ABX pattern, *J*=14.5, 6.2 Hz, 1 H), 1.44 (s, 3H), 1.44 (s, 3H).

### Example 12

### 2-({[(1Z)-1-(2-amino-1,3-thiazol-4-yl)-2-({(2S,3S)-2-[({[3-(5-hydroxy-4-oxo-1,4-dihydropyridin-2-yl)isoxazol-5-yl]carbonyl}oxy)methyl]-4-oxo-1-sulfoazetidin-3-yl}amino)-2-oxoethylidene]amino}oxy)-2-methylpropanoic acid (C118).

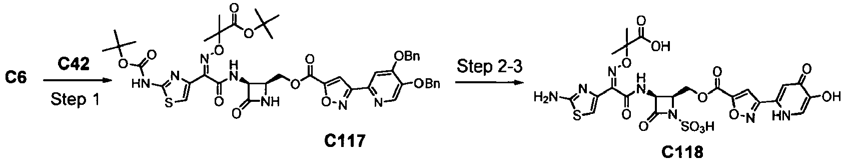

**Step 1: Preparation of C117.** A solution of **C42** (240 mg, 0.596 mmol) in *N,N-*dimethylformamide (2.5 mL) was treated with **C6** (314 mg, 0.596 mmol). The reaction mixture was treated with *O*-(7-azabenzotriazol-1-yl)-1,1,3,3-tetramethyluronium hexafluorophosphate (680 mg, 1.79 mmol) followed by sodium bicarbonate (451 mg, 5.36 mmol). The solution was stirred at 25 °C under nitrogen for 6 hours. The reaction mixture was treated with water (16 mL) and extracted with ethyl acetate (3 x 25 mL). The combined organic layers were dried over sodium sulfate, filtered and concentrated in vacuo. Crude material was purified via chromatography on silica gel (heptane / ethyl acetate 30 to 80%) to yield **C117** as a white solid. Yield: 240 mg, 0.26 mmol, 44%. LCMS *m*/*z* 913.3 (M+1).

**Steps 2-3: Preparation of C118. C117** was converted to **C118** by methods analogous to those described in Example 4, Route 1, Steps 2 and 3. The solid was collected and purified via reverse phase chromatography (C-18 column; acetonitrile / water gradient with 0.1% formic acid modifier) to yield **C118** as a light yellow solid. LCMS *m*/*z* 654.8 (M-1). ¹H NMR δ (400 MHz, DMSO-*d*₆), characteristic peaks: δ 9.27-9.38 (m, 1H), 8.07 (s, 1 H), 7.69 (s, 1 H), 7.49 (s, 1 H), 6.74 (s, 1 H), 5.26-5.38 (m, 1 H), 4.65-4.76 (m, 1 H), 4.47-4.58 (m, 1H), 4.27-4.36 (m, 1H), 1.34-1.46 (brs, 6H).

### Example 13

### 2-({[(1Z)-1-(2-amino-1,3-thiazol-4-yl)-2-({(2S,3S)-2-[({[3-(1,5-dihydroxy-4-oxo-1,4-dihydropyridin-2-yl)isoxazol-5-yl]carbonyl}oxy)methyl]-4-oxo-1-sulfoazetidin-3-yl}amino)-2-xoethylidene]amino}oxy)-2-methylpropanoic acid (C120).

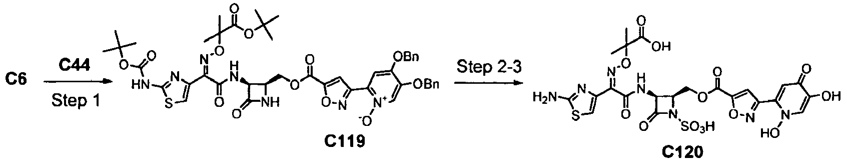

**Step 1: Preparation of C119.** A solution of **C44** (300 mg, 0.716 mmol) in *N,N-*dimethylformamide (8 mL) was treated with **C6** (378 mg, 0.716 mmol). To the reaction mixture was added *O*-(7-azabenzotriazol-1-yl)-*N,N,N',N'*-tetramethyluronium hexafluorophosphate (817 mg, 2.15 mmol) followed by sodium bicarbonate (541 mg, 6.44 mmol). The reaction mixture was stirred at room temperature under nitrogen for 6 hours. The solution was treated with water (16 mL) and extracted with ethyl acetate (3 x 25 mL). The combined organic layers were dried over sodium sulfate, filtered and concentrated, then cooled to 0 °C and treated with water dropwise to give a precipitate. The solid was collected by filtration to afford **C119** as a white solid. Yield: 345 mg, 0.371 mmol, 51%. LCMS *m*/*z* 929.4 (M+1). ¹H NMR (400 MHz, DMSO-*d*₆) δ 11.77 (br s, 1H), 9.35 (d, *J*=8.8 Hz, 1H), 8.74 (brs, 1H), 8.35 (s, 1H), 8.06 (s, 1H), 7.65 (s, 1H), 7.33-7.48 (m, 10H), 7.27 (s, 1 H), 5.33-5.37 (m, 1 H), 5.33 (s, 2H), 5.29 (s, 2H), 4.57 (dd, *J*=11.5, 3.5 Hz, 1 H), 4.44 (dd, *J*=11.3, 9.5 Hz, 1 H), 4.13-4.19 (m, 1 H), 1.46 (s, 9H), 1.45 (s, 3H), 1.42 (s, 3H), 1.38 (s, 9H).

**Steps 2-3: Preparation of C120. C119** was converted to **C120** by methods analogous to those described in Example 4, Route 1, Steps 2 and 3. Crude product was purified via reverse phase chromatography (C-18 column; acetonitrile / water gradient with 0.1% formic acid modifier) to yield **120** as a light yellow solid. LCMS *m*/*z* 672.1 (M+1). ¹H NMR (400 MHz, DMSO-*d*₆) δ 9.25 (d, *J*=9 Hz, 1 H), 8.00 (s, 1H), 7.98 (s, 1 H), 7.41 (s, 1H), 6.74 (s, 1H), 5.34 (dd, *J*=8.7, 5.6 Hz, 1 H), 4.75 (dd, *J*=11.5, 5.3 Hz, 1H), 4.52 (dd, *J*=11.5, 6.3 Hz, 1 H), 4.29-4.34 (m, 1 H), 1.43 (s, 3H), 1.40 (s, 3H).

### Example 14

### 2-({[(1Z)-1-(2-amino-1,3-thiazol-4-yl)-2-({(2R,3S)-2-[({[3-(5-hydroxy-4-oxo-1,4-dihydropyridin-2-yl)isoxazol-5-yl]carbonyl}amino)methyl]-4-oxo-1-sulfoazetidin-3-yl}amino)-2-oxoethylidene]amino}oxy)-2- methylpropanoic acid (C122).

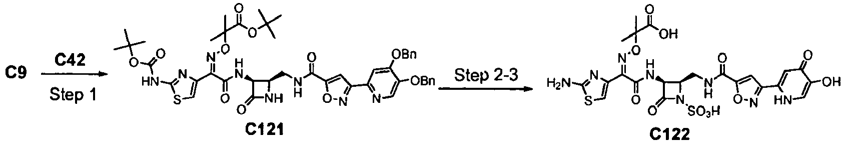

**Step 1: Preparation of C121.** A solution of **C42** (237 mg, 0.590 mmol) in *N,N-*dimethylformamide (5 mL) was treated with **C9** (311 mg, 0.590 mmol). The reaction mixture was treated with *O*-(7-azabenzotriazol-1-yl)-1,1,3,3-tetramethyluronium hexafluorophosphate (673 mg, 1.77 mmol) followed by sodium bicarbonate (446 mg, 5.31 mmol). The solution was stirred at 25 °C under nitrogen overnight. The reaction mixture was quenched with water (15 mL) and extracted with ethyl acetate (3 x 20 mL). The combined organic layers were dried over sodium sulfate, filtered and concentrated in vacuo. Crude material was purified via chromatography on silica gel (heptane / ethyl acetate 30 to 80%) to yield **C121** as a white solid. Yield: 300 mg, 0.33 mmol, 55%. LCMS *m*/*z* 912.3 (M+1). ¹H NMR (400 MHz, DMSO-*d*₆) δ 11.78 (br s, 1 H), 9.43 (d, *J*=8.5 Hz, 1H), 8.94 (br dd, *J*=6, 5 Hz, 1H), 8.45 (brs, 1 H), 8.40 (s, 1H), 7.75 (s, 1H), 7.52 (s, 1H), 7.32-7.51 (m, 10H), 7.29 (s, 1H), 5.36 (s, 2H), 5.31 (s, 2H), 5.19-5.25 (m, 1 H), 3.96-4.02 (m, 1 H), 3.43-3.57 (m, 2H), 1.46 (s, 9H), 1.44 (s, 3H), 1.38 (s, 12H).

**Steps 2-3: Preparation of C122. C121** was converted to **C122** by methods analogous to those described in Example 4, Route 1, Steps 2 and 3. The solid was collected and purified via reverse phase chromatography (C-18 column; acetonitrile / water gradient with 0.1% formic acid modifier) to yield **C122** as a light yellow solid. LCMS *m*/*z* 655.1 (M+1). ¹H NMR (400 MHz, DMSO-*d*₆), characteristic peaks: δ 9.39 (d, *J*=8.2 Hz, 1H), 8.82 (br s, 1 H), 8.08 (s, 1 H), 7.53 (s, 1 H), 7.48 (s, 1 H), 6.81 (s, 1 H), 5.25 (dd, *J*=8.3, 5.8 Hz, 1H), 4.15-4.22 (m, 1H), 3.77-3.85 (m, 1H), 1.46 (s, 3H), 1.44 (s, 3H).

### Example 15

### 2-({[(1Z)-1-(2-amino-1,3-thiazol-4-yl)-2-({(2R,3S)-2-[({[3-(1,5-dihydroxy-4-oxo-1,4-dihydropyridin-2-yl)isoxazol-5-yl]carbonyl}amino)methyl]-4-oxo-1-sulfoazetidin-3-yl}amino)-2-oxoethylidene]amino}oxy)-2-methylpropanoic acid, bis sodium salt (C125-Bis Na salt).

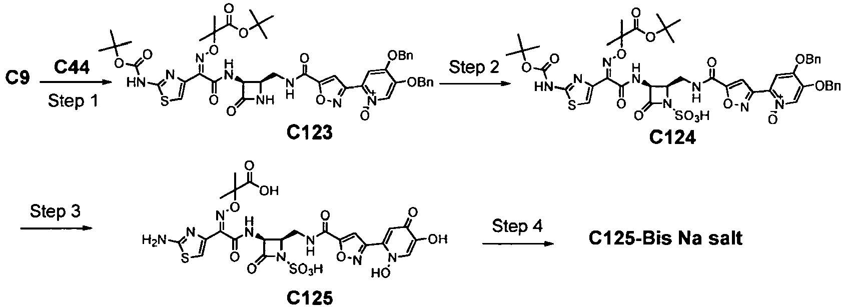

**Step 1: Preparation of C123.** A solution of **C9** (11 g, 20.89 mmol) in *N*,*N-*dimethylformamide (75 mL) was treated with **C44** (7.5 g, 17.92 mmol). *O*-(7-azabenzotriazol-1-yl)-1,1,3,3-tetramethyluronium hexafluorophosphate (21.1 g, 53.8 mmol) was added, followed by sodium bicarbonate (13.6 g, 161 mmol). The heterogeneous reaction mixture was stirred overnight at room temperature. The reaction was cooled to 0 °C, and diluted with water (115 mL). The reaction turned to a thick slurry upon water addition. The mixture was warmed to room temperature, and further diluted with water and ethyl acetate. The layers were separated and the aqueous layer was extracted three times with ethyl acetate. The combined organic extracts were washed once with brine solution, then twice with water, dried over sodium sulfate, filtered and concentrated to afford -19 g of crude solid. The residue was dissolved in a minimal amount of ethyl acetate and passed through a 500 g silica gel plug eluting with ethyl acetate (4-5 L) and then the desired product was eluted (5 L, 10% 2-propanol in ethyl acetate) to afford **C123** as a reddish brown solid. Yield: 12.03 g, 12.97 mmol, 72%. LCMS *m*/*z* 927.3 (M+1). ¹H NMR (400 MHz, DMSO-*d*₆) δ 11.78 (br s, 1 H), 9.42 (d, *J*=8.6 Hz, 1 H), 9.06 (br dd, *J*=6, 5 Hz, 1 H), 8.47 (br s, 1 H), 8.35 (s, 1 H), 7.99 (s, 1H), 7.64 (s, 1H), 7.33-7.48 (m, 10H), 7.28 (s, 1H), 5.32 (s, 2H), 5.28 (s, 2H), 5.23 (ddd, *J*=8.6, 5.3, 1 Hz, 1 H), 3.97-4.02 (m, 1 H), 3.43-3.57 (m, 2H), 1.46 (s, 9H), 1.44 (s, 3H), 1.39 (s, 12H).

**Step 2: Preparation of C124.** A solution of **C123** (11.1 g, 11.97 mmol) in N,N-dimethylformamide (65 mL) was treated with sulfur trioxide *N,N*-dimethylformamide complex (18.3 g, 120 mmol). The reaction mixture was stirred at room temperature for 1 hour, then cooled to 0 °C in an ice-water bath. The reaction was diluted with a large excess of water, and the aqueous layer was extracted (2X) with ethyl acetate. The combined organic extracts were washed three times with water, and concentrated to afford 11.32 g of crude material. The residue was suspended in diethyl ether (350 mL) and stirred for 35 minutes. The mixture was filtered to isolate the solids. The filter cake was again suspended in diethyl ether, solids were ground into a fine powder and filtered to isolate the solids. The filter cake was washed with diethyl ether, and the solid dried under vacuum to afford **C124** as a tan solid. Yield: 10.31 g, 10.2 mmol, 85%. LCMS *m*/*z* 1005.6 (M-1). ¹H NMR (400 MHz, DMSO-*d*₆) δ 11.68 (br s, 1 H), 9.41 (d, *J*=8.6 Hz, 1 H), 8.79-8.83 (m, 1 H), 8.36 (s, 1 H), 7.84 (s, 1 H), 7.66 (s, 1 H), 7.32-7.49 (m, 10H), 7.28 (s, 1 H), 5.33 (s, 2H), 5.28 (s, 2H), 5.26 (dd, *J*=8.7, 5.6 Hz, assumed, 1 H; partially obscured by adjacent signal), 4.16-4.21 (m, 1 H), 3.83-3.90 (m, 1 H), 3.42-3.50 (m, 1 H), 1.46 (s, 9H), 1.44 (s, 3H), 1.41 (s, 3H), 1.38 (s, 9H).

**Step 3: Preparation of C125.** A solution of **C124** (10.64 g, 10.565 mmol) in dichloromethane (250 mL) was cooled to 0 °C and treated with 1 M boron trichloride in dichloromethane (74.0 mL, 74.0 mmol) added dropwise over 30 minutes. The reaction was stirred at 0 °C for 30 minutes and then treated with 2,2,2-trifluoroethanol (77 mL, 1060 mmol). A mixture of methyl *tert*-butyl ether: heptane (1:2, 450 mL) was added. The suspension was stirred at 0 °C for 10 minutes, and then filtered to isolate the solids. The filter cake was washed with additional heptane. The solid filter cake was triturated with diethyl ether, and the mixture filtered to isolate the solids. The filter cake was dried to afford crude product (8.45 g). The crude product was purified via reverse phase chromatography (C-18 column; acetonitrile / water gradient with 0.1% formic acid modifier). The desired product was lyophilized to produce a light tan solid. The solid was slurried in acetonitrile and collected by filtration to afford **C125** as a tan solid. Yield: 3.05 g, 4.55 mmol, 43%. LCMS *m*/*z* 669.4 (M-1). ¹H NMR (400 MHz, DMSO-*d*₆) δ 9.35 (d, *J*=8.5 Hz, 1H), 8.81-8.85 (m, 1H), 8.00 (s, 1H), 7.77 (s, 1H), 7.41 (s, 1H), 6.78 (s, 1 H), 5.25 (dd, *J*=8.6, 5.7 Hz, 1 H), 4.15-4.21 (m, 1 H), 3.76-3.84 (m, 1 H), 3.46-3.54 (m, 1 H), 1.45 (s, 3H), 1.43 (s, 3H).

**Step 4: Preparation of C125-Bis Na salt.** A suspension of **C125** (2.0 g, 2.98 mmol) in water (40 mL) was sonicated, then cooled to 0 °C. A solution of sodium bicarbonate (504 mg, 5.96 mmol) in water (10 mL) was slowly added dropwise over 2 minutes. The reaction mixture was lyophilized to afford **C125-Bis Na salt** as a tan solid. Yield: 2.07 g, 2.90 mmol, 97%. LCMS *m*/*z* 669.6 (M-1). ¹H NMR (400 MHz, D₂O) δ 7.90 (s, 1 H), 7.57 (s, 1 H), 7.29 (s, 1 H), 6.96 (s, 1 H), 5.42 (d, *J*=5.8 Hz, 1 H), 4.65 (ddd, *J*=6, 6, 6 Hz, 1 H), 3.93 (dd, half of ABX pattern, *J*=14.2, 7.1 Hz, 1 H), 3.83 (dd, half of ABX pattern, *J*=14.4, 5.9 Hz, 1 H), 1.45 (s, 6H).

### Example 16

### 2-({[(1Z)-1-(2-amino-1,3-thiazol-4-yl)-2-({(2R,3S)-2-[({[5-(1,5-dihydroxy-4-oxo-1,4-dihydropyridin-2-yl)isoxazol-3-yl]carbonyl}amino)methyl]-4-oxo-1-sulfoazetidin-3-yl}amino)-2-oxoethylidene]amino}oxy)-2-methylpropanoic acid (C127).

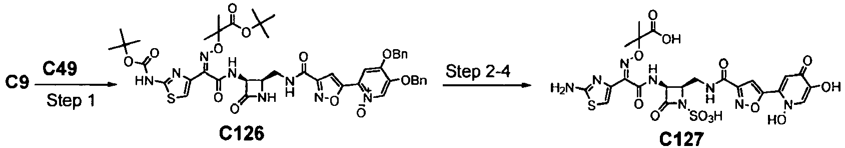

**Step 1: Preparation of C126.** *O*-(7-Azabenzotriazol-1-yl)-1,1,3,3-tetramethyluronium hexafluorophosphate (0.221 g, 0.58 mmol) and sodium bicarbonate (0.122 g, 1.45 mmol) were added sequentially to a solution of **C9** (0.153 g, 0.29 mmol) and **C49** (0.125 g, 0.299 mmol) in *N,N*-dimethylformamide (4.0 mL). The resulting mixture was allowed to stir at room temperature overnight and then diluted with ethyl acetate and water, then the layers were separated. The aqueous layer was back extracted three times with ethyl acetate. The combined organic layer were washed twice with water and once with brine solution, dried over magnesium sulfate, filtered and concentrated in vacuo. The residue was purified by chromatography on silica gel (ethyl acetate / 2-propanol) to yield **C126** as a solid. Yield: 0.093 g, 0.100 mmol, 35%. LCMS *m*/*z* 927.2 (M+1). ¹H NMR (400 MHz, DMSO-*d*₆) δ 11.77 (br s, 1 H), 9.41 (d, *J*=8.4 Hz, 1 H), 8.87 (br dd, *J*=6, 5 Hz, 1 H), 8.45 (br s, 1 H), 8.40 (s, 1H), 7.88 (s, 1H), 7.69 (s, 1 H), 7.34-7.50 (m, 10H), 7.28 (s, 1 H), 5.38 (s, 2H), 5.29 (s, 2H), 5.20-5.25 (m, 1 H), 3.97-4.02 (m, 1 H), 3.44-3.61 (m, 2H), 1.46 (s, 9H), 1.44 (s, 3H), 1.39 (s, 12H).

**Steps 2-4: Preparation of C127. C126** was converted to **C127** by methods analogous to those described in Example 4, Route 1, Step 2 and Example 2, Steps 2 and 3. The crude **C127** was purified by reverse phase chromatography (C-18 column; acetonitrile / water with 0.1% formic acid modifier) to give **C127** as a solid. LCMS *m*/*z* 671.0 (M+1). ¹H NMR (400 MHz, DMSO-*d*₆) δ 9.35 (d, *J*=8.5 Hz, 1 H), 8.63-8.67 (m, 1 H), 7.96 (s, 1 H), 7.71 (s, 1H), 7.42 (s, 1H), 6.82 (s, 1H), 5.24 (dd, *J*=8.4, 5.9 Hz, 1 H), 4.12-4.18 (m, 1 H), 3.82-3.91 (m, 1 H), 3.44-3.52 (m, 1 H), 1.46 (s, 3H), 1.45 (s, 3H).

### Example 17

### 2-({[(1Z)-1-(2-amino-1,3-thiazol-4-yl)-2-({(2R,3S)-2-[({[2-(1,5-dihydroxy-4-oxo-1,4-dihydropyridin-2-yl)-1,3-oxazol-4-yl]carbonyl}amino)methyl]-4-oxo-1-sulfoazetidin-3-yl}amino)-2-oxoethylidene]amino}oxy)-2-methylpropanoic acid, bis sodium salt (C129-Bis Na salt).

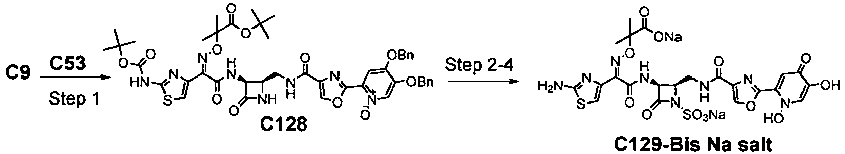

**Step 1: Preparation of C128.** To a suspension of **C53** (1.21 g, 2.89 mmol) in dichloromethane (20 mL) was added **C9** (1.52 g, 2.89 mmol) followed by N-(3-dimethylaminopropyl)-*N*'-ethylcarbodiimide hydrochloride (0.55 g, 2.89 mmol). The reaction was stirred overnight at room temperature. The reaction was diluted with ethyl acetate (50 mL) and washed with 10% aqueous sodium bicarbonate (20 mL), 10% aqueous hydrochloric acid (20 mL), and brine solution (20 mL). The organic layer was then dried with sodium sulfate, filtered, and concentrated to give crude material. The crude material was purified by chromatography on silica gel (dichloromethane / 2-propanol 2 to 10%) to yield **C128** as a solid. Yield: 1.23 g, 1.33 mmol, 46%. LCMS *m*/*z* 927.3 (M+1). ¹H NMR (400 MHz, DMSO-*d*₆) δ 11.74 (br s, 1 H), 9.45 (d, *J*=8.4 Hz, 1 H), 8.83 (s, 1 H), 8.44 (brs, 1 H), 8.33 (s, 1H), 8.30 (br dd, *J*=6, 5 Hz, 1H), 7.67 (s, 1H), 7.31-7.46 (m, 10H), 7.26 (s, 1H), 5.27 (s, 2H), 5.14-5.22 (m, 3H), 3.90-3.96 (m, 1H), 3.57-3.66 (m, 1H), 3.38-3.45 (m, 1H), 1.45 (s, 9H), 1.42 (s, 3H), 1.36 (s, 9H), 1.36 (s, 3H).

**Steps 2-4: Preparation of C129-Bis Na salt. C128** was converted to **C129-Bis Na** salt by methods analogous to those described in Example 4, Route 1, Steps 2-4. The crude **C129** was purified via reverse phase chromatography (C-18 column; acetonitrile / water gradient). Lyophilization provided **C129-Bis Na salt** as a solid. ¹H NMR (400 MHz, D₂O) δ 8.48 (s, 1 H), 7.83 (s, 1 H), 7.32 (s, 1 H), 6.94 (s, 1 H), 5.44 (d, *J*=5.3 Hz, 1 H), 4.59-4.66 (m, 1 H, assumed; partially obscured by solvent peak), 4.01 (dd, *J*=14, 6 Hz, 1 H), 3.76 (dd, *J*=14, 7 Hz, 1H), 1.45 (s, 3H), 1.44 (s, 3H).

### Example 18

### 2-({[(1Z)-1-(2-amino-1,3-thiazol-4-yl)-2-({(2R,3S)-2-[({[2-(5-hydroxy-4-oxo-1,4-dihydropyridin-2-yl)-1,3-oxazol-4-yl]carbonyl}amino)methyl]-4-oxo-1-sulfoazetidin-3-yl}amino)-2-oxoethylidene]amino}oxy)-2-methylpropanoic acid, bis sodium salt (C131- Bis Na salt).

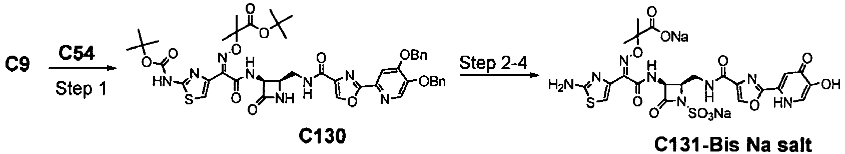

**Step 1: Preparation of C130.** A solution of **C54** (78.9 mg, 0.196 mmol) in *N*,*N-*dimethylformamide (1 mL) was treated with sodium bicarbonate (82.3 mg, 0.980 mmol), *O*-(7-azabenzotriazol-1-yl)-*N,N,N',N*'-tetramethyluronium hexafluorophosphate (149 mg, 0.392 mmol), followed by **C9** (103 mg, 0.196 mmol). The mixture was stirred at room temperature for 2 hours. The reaction mixture was quenched with water (5 mL) and extracted with ethyl acetate (3 x 5 mL). The organic layer was washed with brine solution, dried over sodium sulfate, filtered, and concentrated in vacuo to give crude material. The crude material was purified via chromatography on silica gel (dichloromethane / 2-propanol 2 to 10%) to provide **C130** as a solid. Yield: 0.11 g, 0.12 mmol, 62%. LCMS *m*/*z* 911.4 (M+1). ¹H NMR (400 MHz, DMSO-*d*₆) δ 11.73 (br s, 1 H), 9.44 (d, *J*=8.4 Hz, 1 H), 8.75 (s, 1 H), 8.44 (br s, 1 H), 8.40 (s, 1 H), 8.32 (br dd, *J*=6, 6 Hz, 1H), 7.84 (s, 1 H), 7.31-7.48 (m, 10H), 7.28 (s, 1H), 5.31 (s, 2H), 5.26 (s, 2H), 5.20 (br ddd, *J*=8, 5, 1 Hz, 1 H), 3.92-3.97 (m, 1 H), 3.56-3.65 (m, 1 H), 3.42 (ddd, *J*=14, 5, 4 Hz, 1 H), 1.43 (s, 12H), 1.37 (s, 12H).

**Steps 2-4: Preparation of C131- Bis Na salt. C130** was converted to **C131-Bis Na salt** by methods analogous to those described in Example 4, Route 1, Steps 2-4. The crude **C131** was purified via reverse phase chromatography (C-18 column; acetonitrile / water gradient with 0.1% formic acid modifier). Lyophilization provided **C131-Bis Na salt** as a solid. ¹H NMR (400 MHz, D₂O) δ 8.47 (s, 1 H), 7.73 (s, 1 H), 7.21 (s, 1 H), 6.94 (s, 1H), 5.41 (d, *J*=5.6 Hz, 1H), 4.61 (ddd, *J*=6, 6, 6 Hz, 1H), 3.94 (dd, half of ABX pattern, *J*=14.4, 6.7 Hz, 1H), 3.84 (dd, half of ABX pattern, *J*=14.4, 6.0 Hz, 1H), 1.45 (s, 3H), 1.44 (s, 3H).

### Example 19

### 2-({[(1Z)-1-(2-amino-1,3-thiazol-4-yl)-2-({(2S,3S)-2-[({[2-(5-hydroxy-4-oxo-1,4-dihydropyridin-2-yl)-1,3-thiazol-4-yl]carbonyl}oxy)methyl]-4-oxo-1-sulfoazetidin-3-yl}amino)-2-oxoethylidene]amino}oxy)-2-methylpropanoic acid (C133).

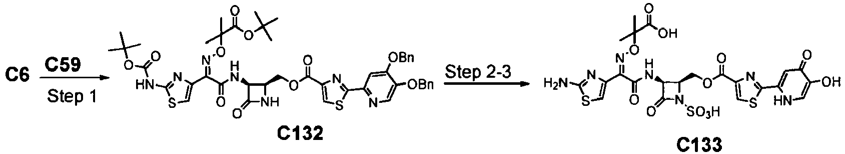

**Step 1: Preparation of C132.** A solution of **C59** (150 mg, 0.34 mmol) in dichloromethane (7 mL) at 0 °C was treated with **C6** (181 mg, 0.34 mmol), followed by triethylamine (0.14 mL, 1.0 mmol). The reaction mixture was stirred overnight with gradual warming to room temperature. The solution was concentrated under vacuum, the residue taken up in ethyl acetate and washed with water (3 x 10 mL), aqueous sodium bicarbonate solution and brine solution (2 x 10 mL). The organic layer was dried over sodium sulfate, filtered and concentrated to provide crude material that was purified via chromatography on silica gel (heptane / ethyl acetate 0 to 100%) to afford **C132** as a light yellow foam. Yield: 125 mg, 0.13 mmol, 39%. LCMS *m*/*z* 928.4 (M+1). ¹H NMR (400 MHz, DMSO-*d*₆) δ 11.79 (br s, 1 H), 9.34 (d, *J*=8.9 Hz, 1 H), 8.66 (br s, 1H), 8.64 (s, 1H), 8.34 (s, 1H), 7.79 (s, 1H), 7.31-7.56 (m, 10H), 7.28 (d, *J*=0.8 Hz, 1 H), 5.35-5.39 (m, 1 H), 5.35 (s, 2H), 5.30 (s, 2H), 4.50 (dd, half of ABX pattern, *J*=11.6, 4.2 Hz, 1H), 4.40 (dd, half of ABX pattern, *J*=11.6, 8.0 Hz, 1 H), 4.11-4.16 (m, 1 H), 1.45 (s, 9H), 1.43 (s, 3H), 1.40 (s, 3H), 1.37 (s, 9H).

**Steps 2-3: Preparation of C133**. **C132** was converted to **C133** by methods analogous to those described in Example 4, Route 1, Steps 2 and 3. Crude material was purified via reverse phase chromatography (C-18 column; acetonitrile / water gradient with 0.1% formic acid modifier) to yield **C133** as a light yellow solid. LCMS *m*/*z* 672.0 (M+1). ¹H NMR (400 MHz, DMSO-*d*₆) δ 9.41 (d, *J*=8.4 Hz, 1 H), 8.57 (s, 1 H), 8.01 (s, 1 H), 7.57 (s, 1H), 6.85 (s, 1H), 5.36 (dd, *J*=8.6, 5.5 Hz, 1 H), 4.74 (dd, *J*=11.1, 4.5 Hz, 1H), 4.37 (dd, *J*=11.1, 7.5 Hz, 1 H), 4.25-4.31 (m, 1 H), 1.44 (s, 3H), 1.39 (s, 3H).

### Example 20

### 2-({[(1Z)-1-(2-amino-1,3-thiazol-4-yl)-2-({(2R,3S)-2-[({[2-(5-hydroxy-4-oxo-1,4-dihydropyridin-2-yl)-1,3-thiazol-4-yl]carbonyl}amino)methyl]-4-oxo-1-sulfoazetidin-3-yl}amino)-2-oxoethylidene]amino}oxy)-2-methylpropanoic acid (C135).

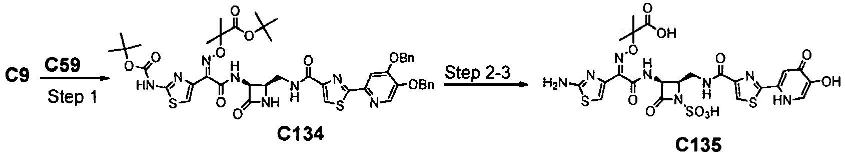

**Step 1: Preparation of C134.** A solution of **C59** (185 mg, 0.423 mmol) in dichloromethane (7 mL) at 0 °C was treated with **C9** (223 mg, 0.423 mmol), followed by triethylamine (0.177 mL, 1.27 mmol). The reaction mixture was stirred overnight with gradual warming to room temperature. The mixture was concentrated under vacuum and the residue taken up in ethyl acetate and washed with water (3 x 10 mL), aqueous sodium bicarbonate solution and brine solution (2 x 10 mL). The organic layer was dried over sodium sulfate, filtered and concentrated in vacuo to give crude product. The crude material was purified via chromatography on silica gel to give **C134** as a light yellow foam. Yield: 123 mg, 0.132 mmol, 31%. LCMS *m*/*z* 927.5 (M+1). ¹H NMR (400 MHz, DMSO-*d*₆) δ 11.66 (br s, 1 H), 9.63 (d, *J*=7.8 Hz, 1 H), 8.47 (br s, 1 H), 8.32 (s, 1 H), 8.30 (s, 1H), 8.24-8.29 (m, 1H), 8.09 (s, 1H), 7.26-7.44 (m, 10H), 7.24 (s, 1H), 5.26 (s, 2H), 5.15-5.19 (m, 1H), 5.13 (AB quartet, *J*_{AB}=11.4, Δν_{AB}=40.9 Hz, 2H), 3.81-3.91 (m, 2H), 3.24-3.31 (m, 1 H), 1.45 (s, 9H), 1.34 (s, 3H), 1.30 (s, 9H), 1.26 (s, 3H).

**Steps 2-3: Preparation of C135. C134** was converted to **C135** by methods analogous to those described in Example 4, Route 1, Steps 2 and 3. The crude material was purified by reverse phase chromatography (C-18 column; acetonitrile / water with 0.1% formic acid modifier) to give **C135** as a light yellow solid. LCMS *m*/*z* 672 (M+1). ¹H NMR (400 MHz, DMSO-*d*₆) δ 9.36 (d, *J*=8.8 Hz, 1 H), 8.70-8.74 (m, 1 H), 8.23 (s, 1 H), 8.00 (s, 1 H), 7.60 (s, 1 H), 6.82 (s, 1 H), 5.26 (dd, J=8.6, 5.7 Hz, 1 H), 4.07-4.12 (m, 1 H), 3.97-4.05 (m, 1 H), 3.34-3.43 (m, 1 H), 1.46 (s, 3H), 1.45 (s, 3H).

### Example 21

### 2-({[(1Z)-1-(2-amino-1,3-thiazol-4-yl)-2-({(2S,3S)-2-[({[2-(5-hydroxy-4-oxo-1,4-dihydropyridin-2-yl)pyrimidin-4-yl]carbonyl}oxy)methyl]-4-oxo-1-sulfoazetidin-3-yl}amino)-2-oxoethylidene]amino}oxy)-2-methylpropanoic acid (C137).

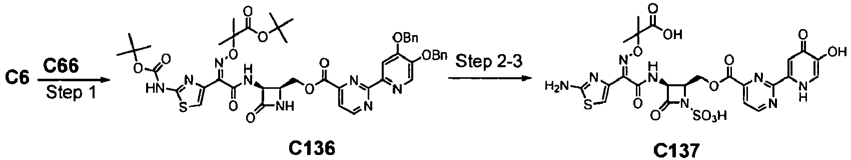

**Step 1: Preparation of C136.** A solution of **C66** (140 mg, 0.34 mmol) in *N,N-*dimethylformamide (2.5 mL) was treated with *O*-(7-azabenzotriazol-1-yl)-1,1,3,3-tetramethyluronium hexafluorophosphate (248 mg, 0.63 mmol), **C6** (169 mg, 0.32 mmol), followed by *N,N*-diisopropylethylamine (0.17 mL, 0.92 mmol). The reaction mixture was stirred at room temperature for 18 hours. The mixture was treated with dichloromethane (10 mL) and 50% saturated aqueous sodium bicarbonate (5 mL), and the layers were separated. The aqueous layer was washed with dichloromethane (2 x 10 mL). The combined organic layers were washed with water (2 x 5 mL), dried over sodium sulfate, filtered and concentrated to afford crude product. The crude material was purified via chromatography on silica gel (heptane / dichloromethane: ethyl acetate (90:10)) to afford **C136.** Yield: 132 mg, 0.14 mmol, 45%. LCMS *m*/*z* 923.8 (M+1). ¹H NMR (500 MHz, DMSO-*d*₆) δ 11.78 (br s, 1 H), 9.35 (d, *J*=8.9 Hz, 1 H), 9.21 (d, *J*=5.0 Hz, 1H), 8.67 (br s, 1H), 8.39 (s, 1H), 8.14 (s, 1 H), 8.04 (d, *J*=4.9 Hz, 1 H), 7.47-7.52 (m, 4H), 7.38-7.43 (m, 4H), 7.32-7.37 (m, 2H), 7.25 (s, 1H), 5.41 (br ddd, *J*=9, 5, 1 Hz, 1 H), 5.35 (s, 2H), 5.31 (s, 2H), 4.60 (dd, *J*=11.7, 3.6 Hz, 1 H), 4.49 (dd, *J*=11.6, 7.9 Hz, 1 H), 4.18-4.22 (m, 1H), 1.44 (s, 9H), 1.39 (s, 6H), 1.36 (s, 9H).

**Steps 2-3: Preparation of C137. C136** was converted to **C137** by methods analogous to those described in Example 4, Route 1, Steps 2 and 3. The crude material was purified via reverse phase chromatography (C-18 column; acetonitrile / water gradient with 0.1% formic acid modifier) to yield **C137** as a solid. LCMS *m*/*z* 667.4 (M+1). ¹H NMR (500 MHz, DMSO-*d*₆) δ 9.29-9.34 (m, 2H), 8.29 (d, *J*=4.9 Hz, 1 H), 7.93 (br s, 2H), 7.14-7.41 (br s, 2H), 6.70 (s, 1H), 5.34 (dd, *J*=8.3, 5.7 Hz, 1 H), 4.75 (dd, *J*=11.7, 6.4 Hz, 1 H), 4.55 (dd, *J*=11.5, 5.0 Hz, 1 H), 4.35-4.40 (m, 1 H), 1.40 (s, 3H), 1.35 (s, 3H).

### Example 22

### 2-({[(1Z)-1-(2-amino-1,3-thiazol-4-yl)-2-({(2R,3S)-2-[({[2-(5-hydroxy-4-oxo-1,4-dihydropyridin-2-yl)pyrimidin-4-yl]carbonyl}amino)methyl]-4-oxo-1-sulfoazetidin-3-yl}amino)-2-oxoethylidene]amino}oxy)-2-methylpropanoic acid (C139).

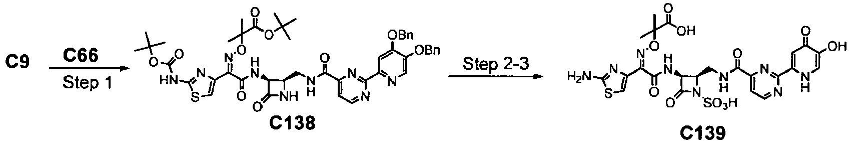

**Step 1: Preparation of C138.** A solution of **C66** (210 mg, 0.508 mmol) in *N*,*N-*dimethylformamide (3 mL) was treated with *O*-(7-azabenzotriazol-1-yl)-1,1,3,3-tetramethyluronium hexafluorophosphate (399 mg, 1.05 mmol) and **C9** (283 mg, 0.537 mmol), followed by solid sodium bicarbonate (153 mg, 1.82 mmol). The reaction mixture was stirred at room temperature for 15 hours. The mixture was treated with dichloromethane (20 mL) and water (5 mL), and the layers were separated. The aqueous layer was washed with dichloromethane (2 x 5 mL). The combined organic layers were washed with water (2 x 5 mL), dried over sodium sulfate, filtered and concentrated to afford crude product. The crude material was purified via chromatography on silica gel (heptane / dichloromethane / ethyl acetate, followed by ethyl acetate / isopropanol) to afford **C138.** Yield: 170 mg, 0.18 mmol, 36%. LCMS *m*/*z* 922.8 (M+1). ¹H NMR (500 MHz, DMSO-*d*₆) δ 11.69 (brs, 1H), 9.56 (d, *J*=7.8 Hz, 1H), 9.16 (d, *J*=5.1 Hz, 1H), 8.87 (dd, *J*=7.3, 4.9 Hz, 1H), 8.49 (br s, 1 H), 8.40 (s, 1H), 8.32 (s, 1 H), 7.99 (d, *J*=5.1 Hz, 1H), 7.29-7.47 (m, 10H), 7.25 (s, 1 H), 5.34 (AB quartet, *J*_{AB}=12.1 Hz, Δ _{AB}=20.8 Hz, 2H), 5.32 (s, 2H), 5.19 (ddd, *J*=7.7, 4.9, 1.6 Hz, 1H), 4.00 (ddd, *J*=7.8, 5.1, 4.9 Hz, 1H), 3.76-3.83 (m, 1H), 3.48 (ddd, *J*=13.9, 4.6, 4.6 Hz, 1H), 1.45 (s, 9H), 1.38 (s, 3H), 1.34 (s, 9H), 1.32 (s, 3H).

**Steps 2-3: Preparation of C139. C138** was converted to **C139** by methods analogous to those described in Example 4, Route 1, Steps 2 and 3. The crude material was purified via reverse phase chromatography (C-18 column; acetonitrile / water with 0.1% formic acid modifier) to yield **C139** as a solid. LCMS *m*/*z* 666.4 (M+1). ¹H NMR (500 MHz, DMSO-*d*₆) δ 9.61-9.69 (m, 1H), 9.52-9.60 (m, 1H), 9.24-9.31 (m, 1H), 8.06-8.16 (m, 1H), 7.93 (br s, 1H), 7.78 (brs, 1H), 7.24-7.42 (br s, 2H), 6.79 (brs, 1H), 5.26-5.34 (m, 1 H), 4.16-4.24 (m, 1 H), 3.84-3.93 (m, 1 H), 3.57-3.67 (m, 1 H), 1.44 (s, 3H), 1.39 (s, 3H).

### Example 23

### 2-({[(1Z)-1-(2-amino-1,3-thiazol-4-yl)-2-({(2R,3S)-2-[({[2-(1,5-dihydroxy-4-oxo-1,4-dihydropyridin-2-yl)pyrimidin-4-yl]carbonyl}amino)methyl]-4-oxo-1-sulfoazetidin-3-yl}amino)-2-oxoethylidene]amino}oxy)-2-methylpropanoic acid (C141).

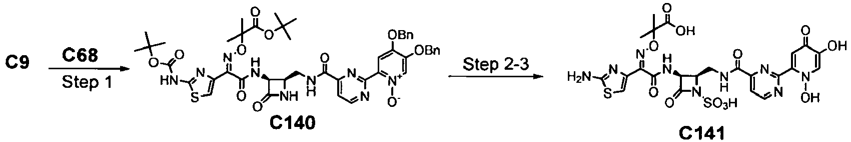

**Step 1: Preparation of C140.** A suspension of **C68** (115 mg, 0.268 mmol), *O*-(7-azabenzotriazol-1-yl)-1,1,3,3-tetramethyluronium hexafluorophosphate (218 mg, 0.56 mmol) in *N,N-*dimethylformamide (2 mL) was treated with **C9** (151 mg, 0.287 mmol), followed by solid sodium bicarbonate (89 mg, 1.06 mmol). The reaction mixture was stirred at room temperature for 15 hours and then treated with chloroform (3 mL) and water (2 mL). The layers were separated and the aqueous layer was back extracted with chloroform (2 x 3 mL). The combined organic layers were washed with water (2 x 3 mL), dried over sodium sulfate, filtered and concentrated to give crude product. The crude material was purified by chromatography on silica gel (ethyl acetate / 2-propanol) to afford **C140.** Yield: 59 mg, 0.063 mmol, 24%. LCMS *m*/*z* 938.7 (M+H).

**Steps 2-3: Preparation of C141. C140** was converted to **C141** by methods analogous to those described in Example 4, Route 1, Steps 2 and 3. The solid was triturated with dichloromethane (4 x 1 mL), then washed with water (2 x 1 mL) to afford **C141** as a solid. LCMS *m*/*z* 682.1 (M+H). ¹H NMR (400 MHz, DMSO-*d*₆), characteristic peaks: δ 9.28 (d, *J*=5.1 Hz, 1H), 8.38 (s, 1H), 8.19 (d, *J*=5.1 Hz, 1H), 7.85 (s, 1H), 6.77 (s, 1 H), 5.26 (dd, *J*=8.6, 5.7 Hz, 1H), 4.13-4.18 (m, 1H), 1.45 (s, 3H), 1.43 (s, 3H).

### Example 24

### 2-({[(1Z)-1-(2-amino-1,3-thiazol-4-yl)-2-({(2R,3S)-2-[({[2-(5-hydroxy-4-oxo-1,4-dihydropyridin-2-yl)pyrimidin-5-yl]carbonyl}amino)methyl]-4-oxo-1-sulfoazetidin-3-yl}amino)-2-oxoethylidene]amino}oxy)-2-methylpropanoic acid (C143).

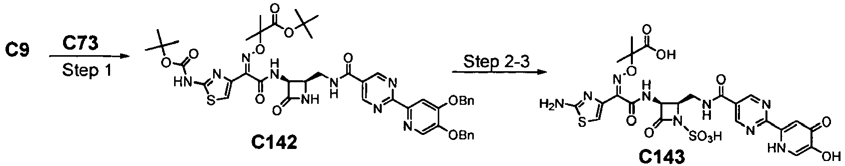

**Step 1: Preparation of C142.** A mixture of **C73** (180 mg, 0.435 mmol) and O-(7-azabenzotriazol-1-yl)-*N,N,N',N*'-tetramethyluronium hexafluorophosphate (341 mg, 0.87 mmol) in *N,N-*dimethylformamide (3 mL) was treated with **C9** (267 mg, 0.456 mmol), followed by solid sodium bicarbonate (145 mg, 1.73 mmol). The reaction mixture was stirred at room temperature for 16 hours, then treated with dichloromethane (15 mL) and water (5 mL). The layers were separated and the aqueous layer was back-extracted with dichloromethane (2 x 5 mL). The combined organic layers were washed with water (2 x 5 mL), dried over sodium sulfate, filtered and concentrated to afford crude product. Crude material was purified via chromatography on silica gel (n-heptane / dichloromethane / ethyl acetate, followed by ethyl acetate / isopropanol) to afford **C142.** Yield: 252 mg, 0.27 mmol, 63%. LCMS *m*/*z* 922.9 (M+H). ¹H NMR (400 MHz, DMSO-*d*₆), characteristic peaks: δ 11.75 (br s, 1 H), 9.45 (br d, *J*=8 Hz, 1 H), 9.24 (s, 2H), 8.79-8.89 (brs, 1H), 8.48 (brs, 1 H), 8.44 (s, 1H), 8.17 (s, 1 H), 7.46-7.52 (m, 4H), 7.32-7.44 (m, 6H), 5.36 (s, 2H), 5.34 (s, 2H), 5.19-5.25 (br m, 1 H), 1.44 (br s, 9H), 1.40 (s, 3H), 1.39 (s, 12H).

**Steps 2-3: Preparation of C143. C142** was converted to **C143** by methods analogous to those described in Example 4, Route 1, Steps 2 and 3. The crude product was purified by reverse phase chromatography (C-18 column; acetonitrile / water with 0.05% formic acid modifier) to give **C143** as a solid. LCMS *m*/*z* 666.2 (M+H). ¹H NMR (400 MHz, DMSO-*d*₆), characteristic peaks: δ 9.47 (d, *J*=8.2 Hz, 1H), 9.27 (s, 2H), 8.79-8.85 (m, 1H), 7.81 (s, 1H), 7.78 (s, 1H), 6.79 (s, 1H), 5.17-5.27 (m, 1H), 4.18-4.24 (m, 1H), 1.45 (s, 3H), 1.43 (s, 3H).

### Example 25

### 2-({[(1Z)-1-(2-amino-1,3-thiazol-4-yl)-2-({(2S,3R)-2-[({[(1,5-dihydroxy-4-oxo-1,4-dihydropyridin-2-yl)methyl]carbamoyl}amino)methyl]-4-oxo-1-sulfoazetidin-3-yl}amino)-2-oxoethylidene]amino}oxy)-2-methylpropanoic acid (C92').

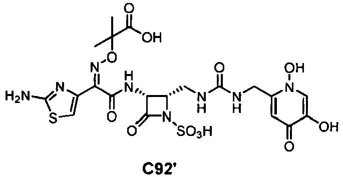

**C92'** was prepared in a manner analogous to that described for Example 4, Route 1. Chromatography Method B provided **C92'.** LCMS *m*/*z* 633.5 (M+1). ¹H NMR (400 MHz, DMSO-*d*₆) δ 9.21 (d, *J*=8.7 Hz, 1 H), 8.14 (s, 1 H), 7.25-7.41 (br s, 2H), 7.17-7.24 (m, 1 H), 6.98 (s, 1 H), 6.74 (s, 1 H), 6.31-6.36 (m, 1 H), 5.18 (dd, *J*=8.7, 5.7 Hz, 1 H), 4.33 (br d, *J*=4.7 Hz, 2H), 3.93-4.00 (m, 1 H), 3.60-3.68 (m, 1 H), 3.19-3.28 (m, 1 H), 1.40 (s, 3H), 1.39 (s, 3H).

### Example 26

### 1-({[(1Z)-1-(2-amino-1,3-thiazol-4-yl)-2-({(2R,3S)-2-[({[(1,5-dihydroxy-4-oxo-1,4-dihydropyridin-2-yl)methyl]carbamoyl}amino)methyl]-4-oxo-1-sulfoazetidin-3-yl}amino)-2-oxoethylidene]amino}oxy)cyclopentanecarboxylic acid (C150).

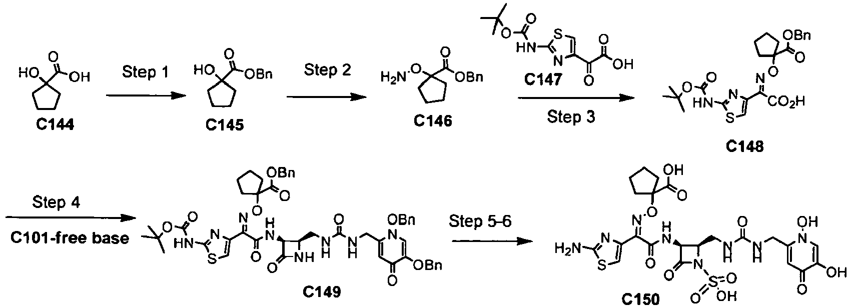

**Step 1: Preparation of C145.** A solution of **C144** (1.50g, 11.5mmol) (see Roussis, V., et al., Journal of Organic Chemistry 1988, 53, 2011-2015) in dimethylformamide (20 mL) at room temperature was treated with potassium carbonate (2.07 g, 15.0 mmol) followed by benzyl bromide (1.50 mL, 12.7 mmol) and the mixture was stirred overnight at room temperature. The reaction mixture was treated with water and then extracted with diethyl ether. The aqueous layer was back extracted with diethyl ether. The combined organic layers were washed with water, brine and then dried over magnesium sulfate. The suspension was filtered, and concentrated in vacuo to give a colorless oil. Chromatography on silica gel with n-heptane-ethyl acetate (20% ethyl acetate) afforded **C145** as a colorless oil. Yield: 2.36g, 10.7 mmol, 93%. ¹H NMR (400 MHz, CDCl₃) δ 7.39-7.30 (m, 5H), 5.20 (s, 2H), 3.04 (s, 1H), 2.12-2.03 (m, 2H), 1.90-1.70 (m, 6H).

**Step 2: Preparation of C146.** A solution of **C145** (2.36 g, 10.7 mmol), phenyl diphenylphosphinite (4.47 g, 16.1 mmol) and 2-hydroxy-1H-isoindole-1,3(2H)-dione (2.62 g, 16.1 mmol) in tetrahydrofuran (40 mL) at 0 °C was treated with 40% diethyl diazene-1,2-dicarboxylate in toluene (7.3 mL, 16 mmol). The reaction mixture was allowed to warm to room temperature and stirred for 48 hours. The reaction mixture was evaporated in vacuo onto silica gel. Chromatography on silica gel using an n-heptane-ethyl acetate gradient (10%-40% ethyl acetate) gave an impure colorless oil (4.07 g). The crude material was dissolved in dichloromethane (25 mL), cooled to 0 °C and treated with methyl hydrazine (667 uL, 12.3 mmol). The reaction mixture was stirred for 2 hours at room temperature resulting in the formation of a white precipitate. The reaction mixture was diluted with n-heptane and filtered. The filtrate was evaporated in vacuo to give **C146** as a colorless oil. Yield: 2.41 g, 10.2 mmol, 96%. ¹H NMR (400 MHz, CDCl₃) δ 7.38-7.29 (m, 5H), 5.39 (br s, 2H), 5.19 (s, 2H), 2.07-1.97 (m, 4H), 1.78-1.66 (m, 4H).

**Step 3: Preparation of C148.** A solution of **C146** (2.41 g, 10.2 mmol) in methanol (20 mL) was treated with **C147** (2.54 g, 9.31 mmol) and was stirred overnight at ambient temperature. The reaction mixture was evaporated in vacuo onto silica gel. Chromatography on silica gel using a dichloromethane-methanol gradient (1%-5% methanol) afforded **C148** as a tan foam. Yield: 4.62 g, 9.43 mmol, 92%. LCMS *m*/*z* 490.1 (M+H)⁺. ¹H NMR (400 MHz, CDCl₃) δ 7.28-7.24 (m, 5H), 7.22 (s, 1H), 5.11 (s, 2H), 2.32-2.24 (m, 2H), 2.11-2.03 (m, 2H), 1.83-1.65 (m, 4H), 1.52 (s, 9H).

**Steps 4: Preparation of C149.** A solution of **C148** (260 mg, 0.53 mmol) in anhydrous dimethylformamide (10 mL) at ambient temperature was treated with N-[(dimethylamino)(3H-[1,2,3]triazolo[4,5-b]pyridin-3-yloxy)methylene]-N-methylmethanaminium hexafluorophosphate (398 mg, 1.01 mmol), followed by sodium bicarbonate (171 mg, 2.03 mmol). The resulting mixture was stirred at ambient temperature for 30 minutes. To this mixture was added **C101-free base** (300 mg, 0.51 mmol) and the resulting light brown mixture was stirred overnight at ambient temperature. The reaction mixture was diluted with water and the resulting precipitate was collected by filtration, rinsed with water and dried in vacuo. Chromatography on silica gel with an ethyl acetate-methanol gradient afforded **C149** as a solid. Yield: 95.3 mg, 0.10 mmol, 19.8%. LCMS *m*/*z* 949.2 (M+H)⁺.

**Steps 5-6: Preparation of C150. C149** was converted to **C150** by methods analogous to those described in Example 4, Route 1, Steps 2-3. Chromatography Method A afforded of **C150** as a light yellow solid. ¹H NMR (400 MHz, DMSO-d₆) δ 9.15 (d, 1H, J=8.8 Hz), 8.10 (s, 1 H), 7.36-7.171 (br s, 2H), 7.15-7.09 (m, 1H), 6.94 (s, 1 H), 6.70 (s, 1 H), 6.33-6.26 (m, 1 H), 5.14 (dd, J=8.6, 5.6 Hz, 1 H), 4.25-4.34 (m, 2H), 3.95-3.90 (m, 1 H), 3.65-3.58 (m, 1 H), 2.05-1.88 (m, 4H), 1.68-1.45 (m, 4H). MS *m*/*z* 658.8 (M)⁺.

### Example 27

### 4-({[(1Z)-1-(2-amino-1,3-thiazol-4-yl)-2-({(2R,3S)-2-[({[(1,5-dihydroxy-4-oxo-1,4-dihydropyridin-2-yl)methyl]carbamoyl}amino)methyl]-4-oxo-1-sulfoazetidin-3-yl}amino)-2-oxoethylidene]amino}oxy)tetrahydro-2H-pyran-4-carboxylic acid (C158).

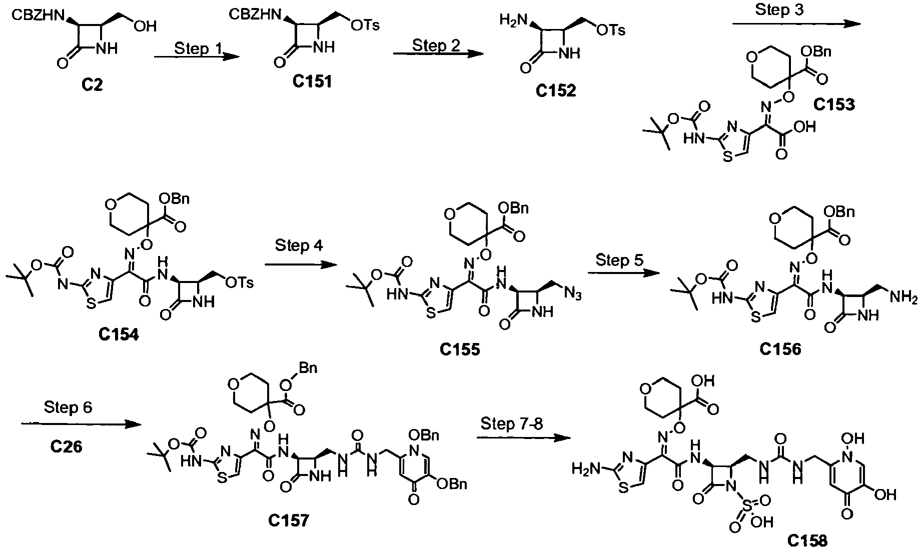

**Step 1: Preparation of C151.** A solution of C2 (33.0 g, 132 mmol) in anhydrous pyridine (200 mL), cooled to 0 °C, was treated with p-toluene sulfonyl chloride (35.3 g, 185 mmol). The reaction mixture was stirred at 0 °C for 4 hours, and then treated with 85% aqueous lactic acid (130 mL), added slowly to maintain the temperature below 5 °C. The resulting mixture was stirred at 0 °C for 30 min, then diluted with ethyl acetate (1 L). The organic layer was washed with 2N HCl (300 mL), 1 N HCl (300 mL), brine solution (200 mL), dried over sodium sulfate, filtered, and concentrated in vacuo to give **C151** as a solid. Yield 44 g, 109 mmol, 83%. LCMS *m*/*z* 405.1 (M+H)⁺. ¹H NMR (400 MHz, DMSO-*d*₆) δ ppm 8.47 (s, 1 H) 7.98 (d, *J*=9.8 Hz, 1 H) 7.73 (d, *J*=8.4 Hz, 2H) 7.44 (d, *J*=8.0 Hz, 2H) 7.37-7.27 (m, 4H) 5.00 (d, *J*=5.6 Hz, 2H) 4.93 (ddd, *J*=9.6, 5.2, 1.2 Hz, 1 H) 4.11-4.00 (m, 2H) 3.87 (dt, *J*=8.0, 5.0 Hz, 1 H) 2.39 (s, 3H).

**Step 2: Preparation of C152.** A solution of **C151** (527.6 mg, 1.30 mmol) in ethanol (17 mL) was treated with 10% palladium on carbon (99.7 mg). The reaction mixture was agitated under 40 psi hydrogen for 2.5 hours. The catalyst was removed by filtration and the filtrate concentrated in vacuo to afford the crude **C152** which was directly used in the following step. LCMS *m*/*z* 271.0 (M+H)⁺.

**Step 3: Preparation of C154.** A solution of **C153,** prepared in a fashion analogous to that described for **C148** in Example 26 (495 mg, 0.98 mmol) in DMF (3.5 mL) was treated with N,N-diisopropylethylamine (0.372 mL, 2.15 mmol), a solution of **C152** (1.30 mmol) in DMF (3.5 mL) and N-[(dimethylamino)(3H-[1,2,3]triazolo[4,5-b]pyridin-3-yloxy)methylene]-N-methylmethanaminium hexafluorophosphate (469 mg, 1.23 mmol). The resulting reaction mixture was stirred at room temperature for about 15 hours, then treated with ethyl acetate and water. The organic layer was separated and concentrated in vacuo to afford crude material. Chromatography on silica gel using an n-heptane/ethyl acetate gradient (30%-70% ethyl acetate) afforded **C154.** Yield: 431.5 mg, 0.57 mmol, 58%. LCMS *m*/*z* 758.2 (M+H)⁺. ¹H NMR (400 MHz, CDCl₃) δ 8.89 (br s, 1H), 7.80 (m, *J*=8.0 Hz, 2H), 7.36 (m, *J*=8.4 Hz, 2H), 7.32-7.29 (m, 5H), 7.23 (s, 1 H), 6.17 (s, 1H), 5.22-5.16 (m, 1H), 5.19 (ABq, *J*_{AB}= 12.1 Hz, Δν_{AB}=62.0 Hz, 2H), 4.53 (dd, *J*=10.9, 2.3 Hz, 1 H), 4.23-4.16 (m, 1 H), 4.10-4.04 (m, 1 H), 3.86-3.64 (m, 4H), 2.46 (s, 3 H), 2.34-2.12 (m, 4H), 1.54 (s, 9H).

**Step 4: Preparation of C155.** A solution of **C154** (427.6 mg, 0.56 mmol) in DMF (6 mL) was treated with sodium iodide (14.8 mg, 0.098 mmol) and sodium azide (117.3 mg, 1.80 mmol). The reaction mixture was stirred at 65 °C for 3 hours. The reaction was treated with ethyl acetate and water. The organic layer was separated and evaporated in vacuo to afford crude material. Chromatography on silica gel using an n-heptane/ethyl acetate gradient (30%-80% ethyl acetate) afforded **C155** as an off-white solid. Yield: 212 mg, 0.34 mmol, 60%. LCMS *m*/*z* 629.2 (M+H)⁺. ¹H NMR (400 MHz, CDCl₃) δ 8.40 (br s, 1 H), 7.67 (d, *J*=8.2 Hz, 1 H), 7.31 (s, 5H), 7.25 (s, 1 H), 6.25 (s, 1 H), 5.46-5.39 (m, 1 H), 5.19 (ABq, *J*=12.3 Hz, Δν_{AB}=16.0 Hz, 2H), 4.08-4.00 (m, 1 H), 3.88-3.67 (m, 4H), 3.64 (dd, *J*=12.9, 3.9 Hz, 1H), 3.34 (dd, *J*=13.2, 7.1 Hz, 1H), 2.35-2.12 (m, 4H), 1.55 (s, 9H).

**Step 5: Preparation of C156.** A solution of **C155** (187.4 mg, 0.30 mmol) in THF (4.5 mL) was treated with water (0.054 mL, 3.0 mmol) and triphenylphosphine (240.8 mg, 0.91 mmol). The crude reaction mixture was stirred at 40 °C for 8 hours. The crude reaction mixture was directly loaded onto an Analogix SF15-12g silica column, eluting first with dichloromethane/ethyl acetate to remove the triphenylphosphine oxide followed by a gradient of ethyl acetate/ ethyl acetate:2-propanol (1:1) to afford **C156.** Yield: 94 mg, 0.16 mmol, 52%. LCMS *m*/*z* 603.1 (M+H)⁺. ¹H NMR (400 MHz, CDCl₃) δ 8.00 (br s, 1H), 7.30 (s, 5H), 7.22 (s, 1H), 6.20 (s, 1H), 5.48 (d, *J*=5.5 Hz, 1H), 5.19 (ABq, *J*_{AB}=12.3 Hz, Δν_{AB}=24.4 Hz, 2H), 3.92 (q, *J*=4.6 Hz, 1H), 3.85-3.70 (m, 4H), 3.01(dd, *J*=13.9, 5.9 Hz, 1 H), 2.83 (dd, *J*=14.1, 2.6 Hz, 1 H), 2.30-2.10 (m, 4H), 1.54 (s, 9H).

**Step 6: Preparation of C157.** A solution of **C26** (92.3 mg, 0.27 mmol) in dichloromethane (0.5 mL) was added to a solution of 1,1'-carbonyl diimidazole (49.3 mg, 0.30 mmol) in THF (0.5 mL) at 0 °C. Triethylamine (42 uL, 0.30 mmol) was added and the stirring continued at 0 °C for 50 minutes providing a suspension. A portion of this suspension (0.6 mL, 0.16 mmol) was then transferred to a suspension of **C156** (70 mg, 0.12 mmol) in THF (0.5 mL) at room temperature. The resulting reaction mixture was stirred at room temperature for 19 hours. The reaction mixture was diluted with ethyl acetate (5 mL) and washed with 10% citric acid (2 mL). The aqueous layer was re-extracted with ethyl acetate (2 x 3 mL). The combined organic layer was washed with brine (2 mL) and concentrated to afford the crude product. The second portion of the initial suspension (0.15 ml, 0.043 mmol) was treated with **C156** (19.9 mg, 0.033 mmol) similarly. The two batches of the crude products were combined and purified with silica gel chromatography using a SF10-4 g silica column to afford **C157.** Yield: 110 mg, 0.110 mmol, 76%. LCMS *m*/*z* 965.2 (M+H)⁺. ¹H NMR (400 MHz, CD₃OD) δ 7.74 (s, 1 H), 7.48-7.26 (m, 15H), 7.23 (s, 1H), 6.31 (s, 1H), 5.27 (d, *J*=4.7 Hz, 1H), 5.24 (s, 2H), 5.22 (s, 2H), 5.02 (s, 2H), 4.28 (ABq, *J*_{AB}=17.1 Hz, Δν_{AB}=33.5 Hz, 2H), 3.97 (q, *J*=6.4 Hz, 1 H), 3.83-3.67 (m, 4H), 3.44 (d, *J*=6.6 Hz, 2H), 2.18-2.03 (m, 4H), 1.48 (s, 9H).

**Steps 7-8: Preparation of C158. C157** was converted to **C158** by methods analogous to those described in Example 4, Route 1, Steps 2-3. Chromatography Method A afforded **C158.** Yield: 7 mg, 0.010 mmol, 10 %. LCMS *m*/*z* 675.3 (M+H)⁺.

### Example 28

### 1-({[(1Z)-1-(2-amino-1,3-thiazol-4-yl)-2-({(2R,3S)-2-[({[(1,5-dihydroxy-4-oxo-1,4-dihydropyridin-2-yl)methyl]carbamoyl}amino)methyl]-4-oxo-1-sulfoazetidin-3-yl}amino)-2-oxoethylidene]amino}oxy)cyclohexanecarboxylic acid (C160).

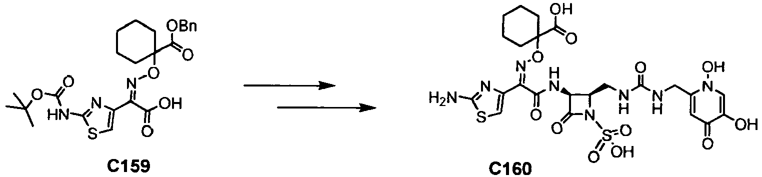

Benzyl 1-hydroxycyclohexanecarboxylate (*Journal of Organic Chemistry* **1954***, 19*, 490-492) was converted to **C159** by the method described in Example 26, Steps 1-3. LCMS m/z 504.0 (M-H)⁻. ¹H NMR (400 MHz, CDCl₃) δ 7.27 (br s, 5H), 7.22 (s, 1 H), 5.11 (s, 2H), 2.26-2.17 (m, 2H), 1.83-1.72 (m, 2H), 1.65-1.46 (m, 6H) overlapping with 1.52 (s, 9H).

**C159** was converted to **C160** by methods analogous to those described in Example 27, Steps 3-8. Chromatography was performed on an Analogix SF25-100 g reversed phase column with an acetonitrile/water gradient to provide **C160.** LCMS *m*/*z* 671.4 (M-1)⁺. ¹H NMR (400 MHz, DMSO-*d*₆) δ ppm 9.17 (d, *J*=9.2 Hz, 1 H), 8.10 (s, 1 H), 7.31-7.21 (bs, 2H), 7.03-7.12 (m, 1H), 6.86 (s, 1H), 6.70 (s, 1 H), 6.37-6.30 (m, 1 H), 5.17 (dd, *J*=8.9, 6.05 Hz, 1 H), 4.29-4.20 (m, 2H), 3.99-3.91 (m, 1 H), 3.67-3.56 (m, 1 H), 3.30-3.20 (m, 1H), 1.96-1.84 (m, 2H), 1.73-1.60 (m, 2H), 1.58-1.42 (m, 4 H), 1.42-1.31 (m, 2H).

### Example 29

### 2-({[(1Z)-1-(2-amino-5-chloro-1,3-thiazol-4-yl)-2-({(2R,3S)-2-[({[(1,5-dihydroxy-4-oxo-1,4-dihydropyridin-2-yl)methyl]carbamoyl}amino)methyl]-4-oxo-1-sulfoazetidin-3-yl}amino)-2-oxoethylidene]amino}oxy)-2-methylpropanoic acid (C166).

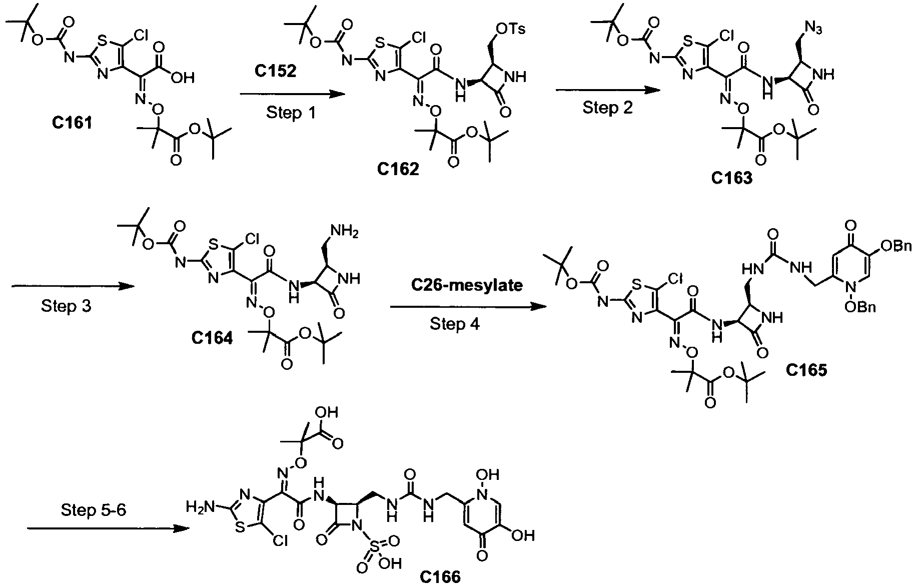

**Step 1: Preparation of C162.** A solution of **C161** (prepared according to Yamawaki, K., et al., Bioorganic & Medicinal Chemistry 2007, 15, 6716-6732) (650 mg, 2.40 mmol) and **C152** (1120 mg, 2.40 mmol) in anhydrous dimethylformamide (12 mL) at ambient temperature was treated with N-[(dimethylamino)(3H-[1,2,3]triazolo[4,5-b]pyridin-3-yloxy)methylene]-N-methylmethanaminium hexafluorophosphate (1010 mg, 2.65 mmol), followed by diisopropylethyl amine (486 uL, 2.81 mmol). The resulting mixture was stirred at ambient temperature overnight. The reaction mixture was diluted with ethyl acetate and water. The phases were separated and the aqueous layer was extracted (2X) with ethyl acetate. The combined organic extracts were washed with water and brine. The resulting solution was dried over magnesium sulfate, filtered and concentrated *in vacuo.* Chromatography on silica gel with an n-heptane-ethyl acetate gradient afforded **C162** as a yellow solid. Yield: 1023 mg, 1.43 mmol, 59.4%. LCMS m/z 716.1 (M+H)⁺.

**Step 2: Preparation of C163.** A solution of **C162** (1023 mg, 1.42 mmol) in anhydrous dimethylformamide (15 mL) at ambient temperature was treated with sodium iodide (21.4 mg, 0.14 mmol) and sodium azide (278 mg, 4.28 mmol). The resulting mixture was stirred at 60 °C for 2.5 hours. The mixture was cooled to ambient temperature and diluted with ethyl acetate and water. The phases were separated and the aqueous layer was extracted twice with ethyl acetate. The combined organic extracts were washed three times with water and once with brine. The resulting solution was dried over magnesium sulfate, filtered and concentrated to dryness. Chromatography on silica gel with an n-heptane-ethyl acetate gradient afforded **C163** as an off-white solid. Yield: 524.7 mg, 0.89 mmol, 62.6%. LCMS *m*/*z* 587.0 (M+H)⁺.

**Step 3: Preparation of C164.** A solution of **C163** (524 mg, 0.89 mmol) in tetrahydrofuran (8 mL) and methanol (1 mL) at ambient temperature was treated with triphenylphosphine (258 mg, 0.98 mmol). The resulting mixture was stirred at ambient temperature overnight. The mixture was concentrated in vacuo and crude **C164** was used directly without purification presuming quantitative conversion.

**Step 4: Preparation of C165.** A suspension of **C26-mesylate** (503 mg, 1.16 mmol) in anhydrous tetrahydrofuran/dichloromethane (1:1, 3 mL) at ambient temperature was treated dropwise with triethylamine (181 mg, 1.79 mmol). The resulting solution was added dropwise to a solution of N,N'-carbonyldiimidazole (202 mg, 1.21 mmol) in anhydrous tetrahydrofuran (3 mL) over 20 minutes. The resulting mixture was stirred at ambient temperature for 2 hours. The reaction mixture was treated with **C164** (534 mg, 0.89 mmol) and the mixture was stirred at ambient temperature overnight. The reaction mixture was concentrated in vacuo to afford a viscous residue. The residue was partitioned between ethyl acetate and water and the layers were separated. The aqueous layer was extracted with ethyl acetate. The combined organic layers were washed with water and concentrated in vacuo onto silica gel. Chromatography on silica gel with a methylene chloride-methanol gradient afforded impure title compound. Chromatography on silica gel with an n-heptane-ethyl acetate gradient afford impure title compound. HPLC chromatography on Sepax 2-Ethyl Pyridine 250 x 4.6 5µ with an n-heptane-ethanol gradient afforded **C165** as an off-white solid. Yield: 171.8 mg, 0.19 mmol, 20.8%. MS *m*/*z* 921.5 (M-H)⁻.

**Steps 5-6: Preparation of C166. C165** was converted to **C166** by methods analogous to those described in Example 4, Route 1, Steps 2-3. Chromatography Method A provided **C166.** ¹H NMR (400 MHz, DMSO-d₆) δ 9.23 (d, J=8.8 Hz, 1H), 7.97 (s, 1H), 7.44-7.36 (brs, 2H), 7.19-7.13 (m, 1H), 6.83 (s, 1H), 6.32-6.23 (m, 1H), 5.13 (dd, J=8.8, 5.8 Hz, 1 H), 4.24 (d, J=4.9 Hz, 2H), 3.93-3.89 (m, 1 H), 3.75-3.69 (m, 1 H), 3.19-3.14 (m, 1 H), 1.40 (s, 3H), 1.39 (s, 3H). MS *m*/*z* 665.3 (M-H)⁻.

### Example 30 Route 1

### 1-({[(1Z)-1-(2-amino-1,3-thiazol-4-yl)-2-({(2R,3S)-2-[({[(1,5-dihydroxy-4-oxo-1,4-dihydropyridin-2-yl)methyl]carbamoyl}amino)methyl]-4-oxo-1-sulfoazetidin-3-yl}amino)-2-oxoethylidene]amino}oxy)cyclobutanecarboxylic acid (C178).

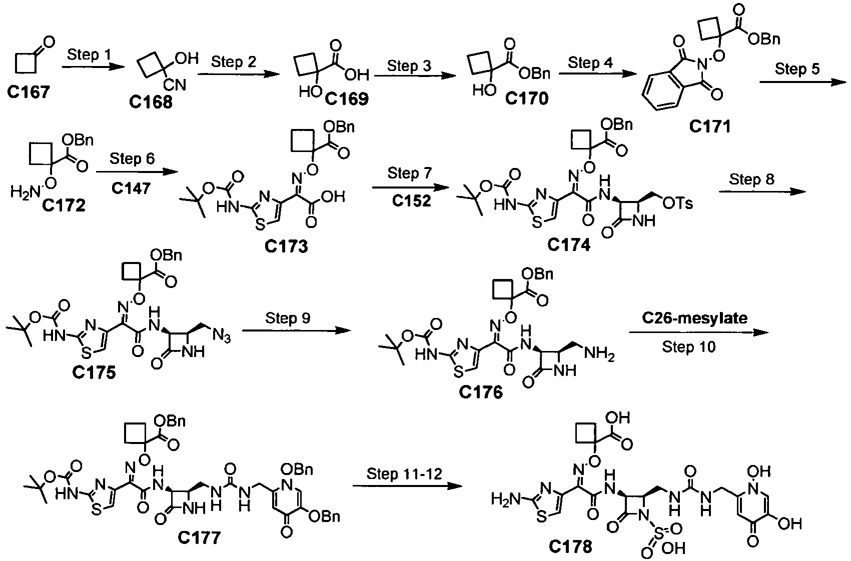

**Step 1: Preparation of C168.** A solution of **C167** (15.75, 225 mmol) and sodium cyanide (14.3 g, 292 mmol) in water (50 mL) was cooled to 2 °C and treated dropwise with a solution of sodium bisulfite (30.3 g) in 30 mL of water. The reaction was warmed to room temperature and allowed to stir for 1.5 hours. The organic and water layers were separated and the water layer was back extracted with ether (2x50 mL). The combined ether extracts were combined with the original reaction organic layer, dried over magnesium sulfate, filtered, and concentrated in vacuo to afford **C168** as a clear oil. Yield: 17.8 g, 183.3 mmol, 82%. ¹H NMR (400 MHz, CHCl₃-*d*) δ 3.35-3.22 (m, 1H), 2.68-2.58 (m, 2H), 2.38-2.25 (m, 2H), 2.00-1.88 (m, 2H).

**Step 2: Preparation of C169.** A solution of **C168** (17.8 g, 160 mmol) in concentrated aqueous hydrochloric acid was refluxed for 2.5 hours. The solvent was removed to give crude material which was purified by trituration with dichloromethane (4 x 40 mL) to afford **C169** as a colored oil which solidified upon standing. Yield: 18.75 g, 161.5 mmol, 99%. ¹H NMR (400 MHz, CHCl₃-*d*) δ 2.63-2.53 (m, 2H), 2.38-2.28 (m, 2H), 2.05-1.87 (m, 2H).

**Step 3: Preparation of C170.** A solution of **C169** (18.75 g, 161.5 mmol), potassium carbonate (29.0 g, 210 mmol), and benzyl bromide (30.4 g, 178 mmol) in N,N-dimethylformamide (300 mL) was allowed to stir at room temperature for 25 hours. The reaction mixture was diluted with diethyl ether (500 mL) and water (750 mL). The layers were separated and the aqueous layer was back extracted with ether (3x 200 mL) and the combined organic layers were washed with water (3x 500 mL) followed by brine (500 mL). The organic layer was dried over magnesium sulfate, filtered, and concentrated in vacuo to afford **C170** as a light yellow oil. (33.82 g, 161.5 mmol, 100%). ¹H NMR (400 MHz, CHCl₃-*d*) δ 7.41-7.28 (m, 5H), 5.26 (s, 2H), 3.40 (bs, 1H), 2.55-2.47 (m, 2H), 2.35-2.26 (m, 2H), 1.98-1.81 (m, 2H).

**Step 4: Preparation of C171.** A solution of **C170** (19.5 g, 83 mmol), phenoxydiphenylphosphine (25.2 g, 90.6 mmol), and *N*-hydroxyphthalimide (16.0 g, 98.1 mmol) in anhydrous tetrahydrofuran was cooled to 2 °C and treated dropwise with a solution of diethyl azodicarboxylate (40.0 g, 92 mmol.) in toluene (42 mL). The reaction mixture was warmed to room temperature and allowed to stir for 16 hours. Solvent was removed in vacuo to give crude material (89 g) which was combined with a previous batch prepared in the same manner using 26.0 g, 110 mmol of C170. The combined crude material was purified by silica gel chromatography using a toluene/ethyl acetate gradient to afford **C171.** Yield: 29.86 g, 84.99 mmol, 43%. LCMS m/z 352.2 (M+H)⁺. ¹H NMR (400 MHz, CHCl₃-*d*) δ 7.83-7.80 (m, 2H), 7.76-7.72 (m, 2H), 7.42-7.29 (m, 5H), 5.26 (s, 2H), 2.60-2.54 (m, 2H), 2.08-1.97 (m, 2H), 1.79-1.69 (m, 2H).

**Step 5: Preparation of C172.** A solution of **C171** (31.7 g, 90.1 mmol) in dichloromethane (200 mL) was cooled to 2 °C with an ice bath. To this was added hydrazine monohydrate (5.2 mL) dropwise. The reaction was allowed to warm to ambient temperature and stirred 4 hours. A precipitate was removed by filtration and the solid was rinsed with dichloromethane (3 X 33 mL). It was determined that the solid contained a mixture of **C171** and **C172,** so the mixture was taken up in dichloromethane (200 mL) and treated with additional hydrazine monohydrate (3.5 mL). After stirring for 1 hour at ambient temperature, the solids were removed by filtration, rinsed with dichloromethane and the filtrate was concentrated in vacuo to afford **C172** Yield: 20.7g, 104%.

**Step 6: Preparation of C173.** A solution of **C172** (19.94 g, 90.1 mmol) and **C147** (25.80 g, 94.8 mmol) in anhydrous methanol (300 mL) was stirred at room temperature for 12 hours. The methanol was removed in vacuo (17 torr, 40 °C) to give crude material (45.75 g) as an off-white solid. The crude material was purified by silica gel chromatography using a methanol and dichloromethane gradient to afford **C173** as a white solid. Yield: 19.50 g, 41.0 mmol, 46%. LCMS *m*/*z* 476.1 (M+H)⁺. ¹H NMR (400 MHz, DMSO-*d*₆) δ 11.80 (s, 1 H), 7.37-7.24 (m, 6H), 5.18 (s, 2H), 2.54-2.45 (m, 2H), 2.31-2.19 (m, 2H), 1.97-1.76 (m, 2H), 1.44 (s, 9H).

**Step 7: Preparation of C174**. A solution of **C173** (19.60 g, 41.2 mmol) and **C152** (12.00 g, 44.4 mmol) in anhydrous N,N-dimethylformamide (200 mL) was treated with *O*-(7-azabenzotriazol-1-yl)*N,N,N'N*'-tetramethyluronium hexafluorophosphate (17.20 g, 45.2 mmol) followed by *N,N*'-diisopropylethylamine (8.3 mL, 48.2 mmol). The reaction mixture was stirred at room temperature for 13 hours. The mixture was then diluted with water (1700 mL) and stirred vigorously for 30 minutes. The resulting precipitate was filtered, washed with water, n-heptane, and dried under vacuum to give crude material (30.10 g) as a light yellow solid. The crude material was purified by silica gel chromatography using an ethyl acetate and n-heptane gradient to afford **C174** as a white solid. Yield: 23.11 g, 31.8 mmol, 77%. LCMS *m*/*z* 728.2 (M+H)⁺. ¹H NMR (400 MHz, DMSO-*d*₆) δ 11.81 (s, 1H), 9.28 (d, *J*=9.0 Hz, 1H), 8.73 (s, 1H), 7.73 (dt, *J*=8.5, 2.0 Hz, 2H), 7.42 (dd, *J*=8.5, 0.6 Hz, 2H), 7.35-7.25 (m, 5H), 7.18 (d, *J*=0.8 Hz, 1H), 5.29 (ddd, *J*=9.0, 5.3, 1.5 Hz, 1 H), 5.16 (ABq, *J*_{AB}=12.8 Hz, Δ _{AB}=7.3 Hz, 2H), 4.19-4.09 (m, 2H), 4.04-3.96 (m, 1 H), 2.50-2.32 (m, 2H), 2.37 (s, 3H), 2.27-2.21 (m, 2H), 1.91-1.70 (m, 2H), 1.43 (s, 9H).

**Step 8: Preparation of C175**. A solution of **C174** (23.11 g, 31.8 mmol), tetrabutylammonium iodide (6.01 g, 16.0 mmol), and tetrabutylammonium azide (23.57 g, 80.0 mmol) in anhydrous tetrahydrofuran (230 mL) was allowed to stir at room temperature for 13 hours. The reaction was concentrated in vacuo to give a crude gum which was dissolved in a water/ methyl tert-butyl ether (1:2) solution. The organic layer was separated, washed with water, filtered, and concentrated in vacuo to give a crude white solid which was purified with silica gel chromatography using an ethyl acetate and n-heptane gradient to afford **C175** as a colorless solid. Yield: 15.84 g, 26.5 mmol, 84%. LCMS *m*/*z* 599.2 (M+H)⁺. ¹H NMR (400 MHz, DMSO-*d*₆) δ11.83 (s, 1 H), 9.27 (d, *J*=8.9 Hz, 1 H), 8.64 (s, 1 H), 7.37-7.26 (m, 5H), 7.25 (d, *J*=0.8 Hz, 1 H), 5.25 (ddd, *J*=8.9, 5.2, 1.6 Hz, 1 H), 5.18 (ABq, *J*_{AB}=12.7 Hz, Δ _{AB}=14.3 Hz, 2H), 3.90 (dt, *J*=9.1, 4.3 Hz, 1 H), 3.62 (dd, half of ABX pattern, *J*=12.0, 4.3 Hz, 1 H), 3.40 (dd, half of ABX pattern, *J*=12.0, 9.1 Hz, 1 H), 2.53-2.42 (m, 2H), 2.38-2.25(m, 2H), 1.95-1.75(m, 2H), 1.44 (s, 9H).

**Step 9: Preparation of C176.** Under nitrogen, a solution of **C175** (15.84 g, 26.46 mmol) in ethanol (280 mL) was charged with platinum (IV) oxide (3.00 g, 13.20 mmol). The mixture was purged with hydrogen and pressurized to 30 psi hydrogen. The mixture was agitated at room temperature for 3 hours. The mixture was filtered through Celite and the filter cake was rinsed with ethanol. The ethanolic filtrate was concentrated in vacuo to afford **C176** as a light gray solid. Yield: 14.97 g, 26.2 mmol, 99%. LCMS *m*/*z* 573.2 (M+H)⁺. ¹H NMR (400 MHz, DMSO-*d*₆) δ 8.27 (s, 1H), 7.37-7.24 (m, 5H), 7.23 (s, 1 H), 5.22-5.12 (m, 3H), 3.64 (q, J=5.8 Hz, 1 H), 2.78 (dd, half of ABX pattern, *J*=13.3, 5.8 Hz, 1H), 2.63 (dd, half of ABX pattern, *J*=13.3, 6.6 Hz, 1H), 2.55-2.27 (m, 4H), 1.99-1.73(m, 2H), 1.43 (s, 9H).

**Step 10: Preparation of C177. C176** and **C26-mesylate** were converted to **C177** by a method analogous to that described in Example 29, Step 4. Chromatography on silica gel with a dichloromethane-methanol gradient afforded **C177** as a grey solid. Yield: 3279 mg, 3.51 mmol, 80.3%. MS *m*/*z* 933.8 (M-H)⁻.

**Steps 11-12: Preparation of C178**. **C177** was converted to **C178** by methods analogous to those described in Example 4, Route 1, Steps 2-3. Chromatography Method A provided **C178.** MS *m*/*z* 644.8 (M)⁺. ¹H NMR (400 MHz, DMSO-d₆) δ 9.25 (d, *J*=8.8 Hz, 1H), 8.12 (s, 1H), 7.42-7.22 (br s, 2H), 7.22-7.14 (m, 1 H), 6.95 (s, 1H), 6.74 (s, 1 H), 6.36-6.30 (m, 1 H), 5.20 (dd, *J*=9.0, 5.8 Hz, 1 H), 4.31 (d, *J*=4.7 Hz, 2H), 4.00-3.93 (m, 1H), 3.70-3.60 (m, 1H), 3.30-3.21 (m, 1H), 2.45-2.20 (m, 4H), 1.91-1.68 (m, 2H).

### Example 30 Route 2

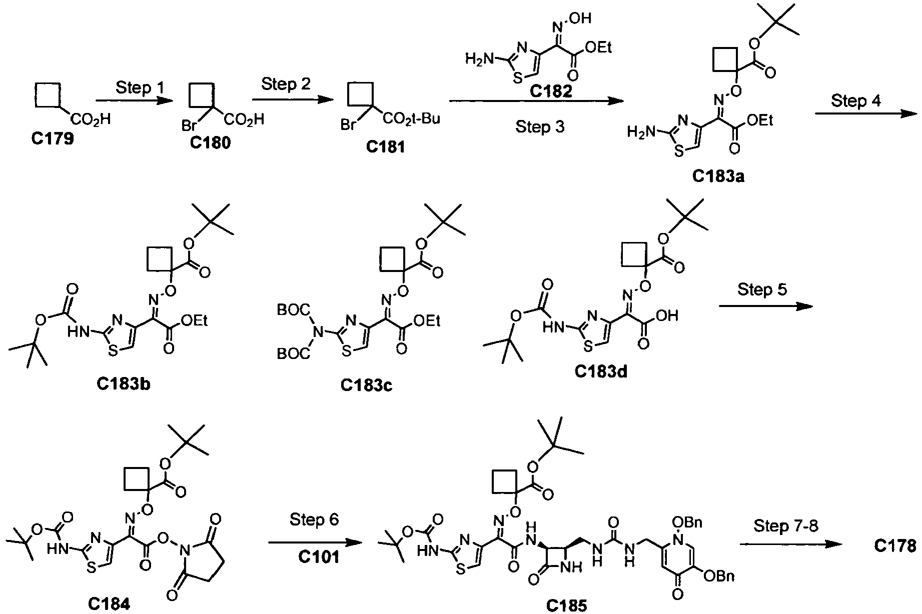

**Step 1: Preparation of C180**. A 50 L flask was evacuated to ≤-0.08 MPa, and then filled with nitrogen to normal pressure. This was repeated 3 times. Maintaining the temperature at 20-30 °C, phosphorus tribromide (4.7 kg, 17.4 mol) and C179 (18.7 kg, 104.5 mol) were added into the flask. The mixture was heated to 100-105 °C. Maintaining the temperature at 98-107 °C, bromine (42.0 kg, 262.8 mol) which was dried with concentrated sulfuric acid (5.1 kg), was added into the mixture. After addition, the mixture was stirred at 100-105 °C. After 1hr, the reaction was monitored by GC every 1-2 hours. The reaction was considered complete when the content of cyclobutanecarboxylic acid was ≤ 5%. (Sampling method for GC analysis: take 5 ml of the reaction mixture into 10% sodium bisulfite solution, and then extract with dichloromethane. The organic phase was analyzed by GC.) The mixture was cooled to 0-15 °C, then dichloromethane (8.1 kg) was added into the mixture. Maintaining the temperature at ≤ 20 °C, the mixture was quenched with 10% sodium bisulfite solution (8.5 kg). The mixture was transferred into a 300 L glass-lined reactor at ≤ 30 °C. Maintaining the temperature at ≤ 30 °C, dichloromethane (94.5 kg) and 10% sodium bisulfite solution (48.1 kg) were added into the 300 L glass-lined reactor and stirred it for 0.5 hours and held for 0.5 hours before separation. The aqueous phase was extracted with dichloromethane (18.9 kg) at ≤ 30 °C. It was stirred for 0.5hourand held for 0.5hour before separation. The organic phases were combined and washed with saturated brine (50.5 kg×2) at ≤ 30 °C. Each time it was stirred for 0.5hourand held for 0.5hourbefore separation. The organic phase was dried with magnesium sulfate (6.0 kg) for 2-3 hours. The mixture was filtered with a 50 L vacuum filter which was pre-loaded with silica gel (5.0 kg) while maintaining the temperature at ≤ 30 °C. The filtrate was concentrated at ≤ 35 °C under reduced pressure (≤-0.08 MPa) until no more distillate was observed. Dichloromethane (30.1 kg) was added and concentration continued until KF (water content) was ≤0.5%, giving **C180** as a brownish red liquid. Residue Weight: 35.2 kg solution (25.8 kg corrected by wt%) Wt% by GC: 73.3%. Purity by GC: 84.1% Wt% yield: 77.2%

**Step 2: Preparation of C181.** A 300 L glass-lined reactor was evacuated to ≤-0.08 MPa and then was filled with nitrogen to normal pressure. This was repeated 3 times. The solution of **C180** was charged into the 300 L glass-lined reactor, followed by the addition of tert-butanol (14.9 kg, 201.0 mol) and 4-dimethylaminopyridine (1.8 kg, 14.7 mol). Maintaining the temperature at 25-40 °C, triethylamine (31.9 kg, 315.2 mol) was added dropwise into the mixture. The mixture was cooled to 0-5 °C. Maintaining the temperature at 0-10 °C, di-tert-butyl dicarbonate (40.7 kg, 186.5 mol) was added into the mixture. After addition, the mixture was stirred at 0-10 °C for 1-2 hours. The mixture was heated to 20-30 °C and then stirred at this temperature. After 1 hour, the reaction was monitored by GC every 1∼2 hours. The reaction was considered complete when the content of **C180** was ≤ 3%. (Sampling method: Take 5 ml of the reaction mixture into dichloromethane, adjust pH to 3-4 with 3M hydrochloric acid. After separating, the organic phase was analyzed by GC). The mixture was cooled to 0-15 °C. Maintaining the temperature at ≤ 15 °C, 4M hydrochloric acid solution (77.2 kg) was added into the mixture to adjust the pH to 3-4, then the mixture was stirred at ≤ 15°C for 1∼2 hours. Maintaining the temperature at ≤ 20 °C, the mixture was extracted with dichloromethane (67.0 kg×2). For each extraction the mixture was stirred for 0.5 hour and held for 0.5 hour before separation. Maintaining the temperature at ≤ 25 °C, the organic phase was washed with saturated sodium bicarbonate solution (51.0 kg×2). Maintaining the temperature at ≤ 25 °C, the organic phase was washed with saturated brine (67.6 kg+67.7 kg). For each wash the mixture was stirred for 0.5 hour and held for 0.5 hour before separation. Active carbon (1.3 kg) was added into the organic phase and stirred for 2∼3 hours at 20-30 °C. The mixture was filtered with a vacuum filter which was pre-loaded with silica gel (5.1 kg). The filter cake was rinsed with dichloromethane (12.8 kg). The filtrate was concentrated at 40 °C under reduced pressure (≤-0.08 MPa) until no more distillate was observed. Dichloromethane (30.2 kg) was added and concentration continued until KF (water content) was ≤ 0.05% to give **C181** as a brownish red liquid. Weight: 33.6 kg solution (19.5 kg corrected by wt%) Wt% by GC: 57.9% Purity by GC: 68.2% Wt% yield: 58.2%

**Step 3: Preparation of C183a.** A 500 L glass-lined reactor was evacuated to ≤-0.08 MPa and then filled with nitrogen to normal pressure. This was repeated 3 times. Maintaining the temperature < 40 °C, dimethyl sulfoxide (72.0 kg) and **C182** (13.1 kg, 60.9 mol) were added. After the reaction was stirred for 10 minutes, potassium carbonate (16.8 kg, 121.5 mol) was added into the mixture. The mixture was heated to 42∼50 °C. Maintaining the temperature at 42-50 °C, **C181** (18.5 kg) was added dropwise into the mixture at the rate of 6-10 kg/hr. After addition, the mixture was stirred at 42-50 °C and monitored by HPLC. After the mixture reacted for 17 hours, additional **C181** (0.3 kg+0.7 kg) and potassium carbonate (16.9 kg, 122.3 mol) were added. Then the mixture was maintained at 42-50 °C until the content of **C181** was < 1% and the change of **C182** content between consecutive samples was <1%. (Sampling method: Take 2 ml mixture into methanol and hold for a minute. The upper layer was analyzed by HPLC). After reaction completion, the mixture was cooled to 25-30 °C. Then the mixture was transferred into a 1000 L glass-lined reactor containing purified water (261.7 kg) which was pre-cooled to 10-20 °C. The wall of the 500 L glass-lined reactor was rinsed with purified water (65.5 kg) and the wash liquor was transferred into the 1000 L glass-lined reactor. The mixture was cooled to -5-5 °C. The mixture was stirred at this temperature for crystallization; 10 hours later, the mixture was sampled every 1∼3 hours until the wt% **C183a** in the filtrate was ≤ 0.5%. The mixture was filtered. The filter cake was washed with purified water (52.4 kg×3). Then the filter cake was washed with methanol (10.3 kg×2), which was cooled to 0-10 °C in advance, until the purity of the filter cake was >90%. The filter cake was dried at 40-45 °C until KF (water content) was ≤0.5% to give **C183a** as an off-white solid. Weight: 12.5 kg (corrected by wt%) Wt% by HPLC: 95.1% Purity by HPLC: 93.4% Wt% yield: 55.6%.

**Step 4: Preparation of C183d.** A 500L glass-lined reactor was evacuated to ≤-0.08 MPa and then filled with nitrogen to normal pressure. This was repeated 3 times. THF (92.4 kg), N,N,N',N'-tetramethylethylenediamine (0.2 kg, 1.72 mol)) and C183a (12.9 kg) were added into the 500 L glass-lined reactor. Then the mixture was stirred for 30 minutes. The mixture was cooled to 0-10 °C. Maintaining the temperature at 0-10 °C, a solution of di-tert-butyl dicarbonate (11.4 kg, 52.2 mol) in THF (45.9 kg) was added dropwise into the 500 L reactor at a rate of 15-20 kg/hr. The mixture was heated to 10-20 °C and maintained at this temperature for 2 hours. Heating was continued at the rate of 5-10 °C/hr until it reached 25-30 °C. Starting 5 hours later, the mixture was sampled and detected by HPLC every 1-2 hours. The reaction was considered complete when the content of **C183a** was ≤ 1%. (Sampling method: Take 2 ml mixture and analyze by HPLC). The mixture was concentrated at ≤ 40 °C under reduced pressure (≤-0.08 MPa) until 30-40 L remained and then the residue was diluted with methanol (41.5 kg). Mixture concentration continued at ≤ 40 °C under reduced pressure (≤-0.08 MPa) until 30-40 L remained and then the residue was diluted with methanol (30.7 kg) until the content of THF was ≤ 5% giving a mixture of **C183b** and **C183c.** Methanol (81.5 kg) was added into the mixture and the mixture was cooled to < 25 °C. Maintaining the temperature ≤ 25 °C, a 2% aqueous lithium hydroxide solution (88.3 kg, 3686 mol) was added dropwise into the 500 L reactor at the rate of 15-20 kg/hr. The mixture was heated to 52∼60 °C and was stirred at 52-60 °C for 4 hours. The mixture was sampled and detected by HPLC every 1-2 hours. The reaction was considered complete when the contents of **C183b** and **C183c** were ≤ 3% and the change of **C183b** and **C183c** contents between consecutive samples were ≤ 0.5%. The mixture was cooled to 15-25 °C. Maintaining the temperature at 10-20 °C, the mixture pH was adjusted to 7∼8 with 1M hydrochloric acid solution (33.3 kg). The mixture was concentrated at ≤ 45 °C under reduced pressure (≤-0.08 MPa) until the content of methanol was < 20%. The mixture was transferred into a 1000 L glass-lined reactor via an in-line fluid filter. Maintaining the temperature at 10-20 °C, the mixture pH was adjust to 3.5-4.5 with 1 M hydrochloric acid solution (38.3 kg). The mixture was cooled to 0-5 °C and maintained at this temperature for crystallization. Starting 8 hours later, the mixture was sampled every 1-2 hours until the wt% of **C183d** in the mother liquor was ≤ 0.1 % or the change of wt% of **C183d** in mother liquor between two consecutive samples was < 0.05%. The mixture was filtered. The filter cake was washed with purified water (19.4 kg×2) and petroleum ether (19.4 kgx2). The filter cake was added into anhydrous ethanol (8.1 kg), then the mixture was heated to 65±5 °C and maintained for 0.5 hour. Purified water (10.1 kg) was added into the mixture at 65±5 °C. After addition, the mixture was cooled to 10-20 °C and stirred at this temperature for 1 hour. Filtration followed by rinsing with the mixed solvent of anhydrous ethanol (0.7 kg) and purified water (1.3 kg) afforded a filter cake that was dried in a drying room at 40-45 °C until KF (water content) was ≤ 0.5% to give **C183d** as a light yellow solid. Weight: 8.8 kg Yield: 57.1% Purity: 98.1%. ¹H NMR (400 MHz, DMSO-*d*₆) δ ppm 13.83 (br. s., 1 H) 11.44 - 12.00 (br. s, 1 H) 7.36 (s, 1 H) 2.40 - 2.46 (m, 2 H) 2.15 - 2.26 (m, 2 H) 1.71 - 1.95 (m, 2 H) 1.45 (s, 9 H) 1.38 (s, 9 H). Mass Spec *m*/*z* 442.6/386.6/330.3 (M+1).

**Step 5: Preparation of C184.** A solution of **C183** (100 g, 226.5 mmol) and N-hydroxysuccinimide (31.1 g, 269.8 mmol) in methylene chloride (1000 mL) at 5 °C was treated with diisopropylcarbodiimide (41 mL, 261.7 mmol) over 5 minutes. The mixture was stirred at 5 °C for 15 minutes and then warmed to ambient temperature with stirring for 1.5 hours. The mixture was filtered through a pad of Celite and rinsed twice with methylene chloride (400 mL). The resulting solution was concentrated via rotary evaporation to a volume of -250 mL. The solution was diluted with methanol (700 mL) and then concentrated to a volume of -700 mL. The solution was diluted with methanol (150 mL) and concentrated to a volume of -250 mL. The resulting slurry was treated with n-heptane (250 mL) at 32 °C and stirred at this temperature for 30 minutes. The slurry was cooled to 18 °C and stirred for 1 hour. The precipitate was filtered, rinsed twice with n-heptane (150 mL) and dried in vacuo to afford **C184** as a white solid. Yield: 119.5 g, 98%. ¹H NMR (400 MHz, CDCl3) δ 8.06 (bs, 1H), 7.52 (s, 1 H), 2.90 (s, 4H), 2.67-2.58 (m, 2H), 2.52-2.42 (m, 2H), 2.11-1.89 (m, 2H), 1.53 (s, 9H), 1.44 (s, 9H).

**Step 6: Preparation of C185.** A mixture of **C101** (213.7 g, 101.9 mmol, TFA salt, 28% activity, calculated, on Celite), **C184** (54.9 g, 101.9 mmol) and molecular sieves (120.5 g, Type 3A) in acetonitrile (843.7 mL) was treated with N,N-dimethyl-4-pyridinamine (31.1 g, 254.7 mmol) at ambient temperature. The mixture was heated to 38 °C and stirred for 3 hours. The mixture was cooled to ambient temperature and treated with citric acid (aq. 10% wt, 120.5 mL) followed with water (397.7 mL). The mixture was concentrated to a volume of -250 mL. To the mixture was added ethyl acetate (854.8 mL) and the resulting slurry was stirred for 15 minutes at ambient temperature. The mixture was filtered and the wet cake was washed with ethyl acetate (397.7 mL) and water (150.7 mL); the washes were repeated one time. The phases were separated and the organic layer was washed with citric acid (aq. 10% wt., 602.6 mL) and then twice with sodium chloride (aq., 10% wt., 371.2 mL). The organic layer was dried over magnesium sulfate and filtered. The filter cake was rinsed twice with ethyl acetate (198.9 mL). The filtrates were combined and concentrated to a volume of -300 mL. The solution was added via addition funnel to a stirring solution of heptane (1390 mL) over 30 minutes. The mixture was stirred for 30 minutes at ambient temperature. The mixture was filtered and the wet cake washed twice with heptane/ethyl acetate (4:1, 391.7 mL). The wet cake was dried in vacuo. Chromatography using dichloromethane and methanol on silica gel afforded **C185** as a solid. Yield: 27.93 g, 31.0 mmol, 30.4%. LCMS *m*/*z* 901.5 (M+H)⁺.

**Steps 7-8: Preparation of C178**. **C185** was converted to **C178** by methods analogous to those described in Example 4, Route 1, Steps 2-3. **C178** was purified by reverse phase chromatography on a C-18 column with a water-acetonitrile gradient containing 0.1 % formic acid.

### Example 31

### (2S)-2-({[(1Z)-1-(2-amino-1,3-thiazol-4-yl)-2-({(2R,3S)-2-[({[(1,5-dihydroxy-4-oxo-1,4-dihydropyridin-2-yl)methyl]carbamoyl}amino)methyl]-4-oxo-1-sulfoazetidin-3-yl}amino)-2-oxoethylidene]amino}oxy)-4-methylpentanoic acid (C190).

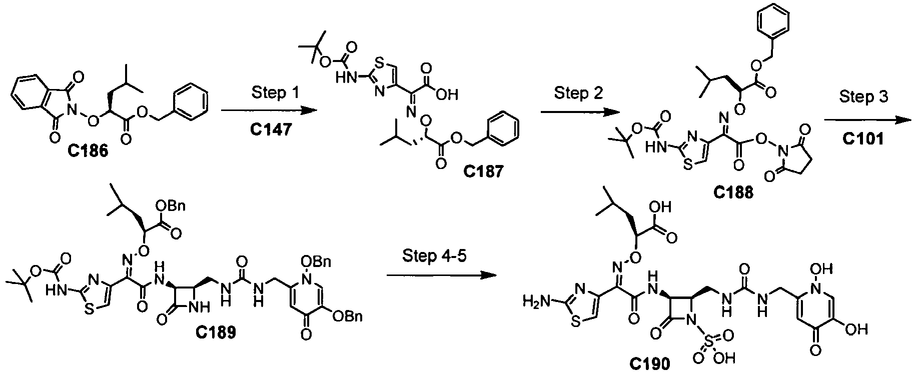

**Step 1: Preparation of C187**. A solution of **C186** (Prepared as described in: Shin, I., *et al., Journal of Organic Chemistry* **2000,** *65*, 7667-7675) (1.59 g, 4.33 mmol) in dichloromethane (10 mL) at 0 °C was treated with hydrazine monohydrate (210 µL, 4.33 mmol). The reaction mixture was stirred at ambient temperature for 2 hours which resulted in the formation of a white precipitate. The reaction mixture was diluted with dichloromethane, filtered, and the filtrate concentrated in vacuo to afford benzyl (2S)-2-(aminooxy)-4-methylpentanoate as a white solid. This solid was dissolved in methanol and treated with **C147** (1.24 g, 4.54 mmol) and the reaction mixture was stirred overnight at ambient temperature. The reaction mixture was evaporated in vacuo onto silica gel. Chromatography on silica gel using n-heptane-ethyl acetate (75% ethyl acetate) followed by a dichloromethane-methanol gradient (1%-15% methanol) afforded **C187** as a tan foam. Yield: 801 mg, 1.62 mmol, 36%. LCMS *m*/*z* 492.1 (M+H)⁺.

**Step 2: Preparation of C188.** A solution of **C187** (800 mg, 1.62 mmol) in dichloromethane (5.0 mL) was treated with 1-hydroxy-succinimide (217 mg, 1.88 mmol) and N,N'-dicyclohexylcarbodiimide (364 mmol, 1.76 mmol). A white precipitate formed and the reaction mixture was stirred at ambient temperature overnight. The reaction mixture was filtered and the solids were rinsed with dichloromethane. The filtrate was concentrated in vacuo and the resulting residue was purified by chromatography on silica gel using n-heptane-ethyl acetate (75% ethyl acetate) to afford **C188** as a yellow foam. Yield: 458 mg, 0.778 mmol, 48%. ¹H NMR (400 MHz, CDCl₃) δ 7.53 (s, 1 H), 7.33-7.25 (m, 5H), 5.15 (ABq, *J*_{AB}=12.3 Hz, Δ _{AB} = 34.15 Hz, 2H), 4.99 (dd, J=9.5, 3.9 Hz, 1 H), 2.87 (br s, 4H), 1.97-1.83 (m, 2H), 1.72-1.63 (m, 1 H), 1.53 (s, 9H), 0.95-0.91 (dd, J=6.2, 3.9 Hz, 6H).

**Step 3: Preparation of C189**. A mixture of **C101** (461 mg, 0.78 mmol), **C188** (458 mg, 0.78 mmol) and Celite (2 g) in anhydrous acetonitrile (5.8 mL) was treated with triethylamine (224 µL, 1.56 mmol) at ambient temperature. The mixture was heated to 40 °C and stirred overnight. The mixture was cooled to ambient temperature and filtered into a stirring flask of water. The resulting precipitate was filtered and washed with water followed by n-heptane. The solid was dried in vacuo to a constant weight. Chromatography on silica gel using a methylene chloride-methanol gradient afforded **C189** as a solid. Yield: 384.7 mg, 0.40 mmol, 51.9%. LCMS *m*/*z* 951.5 (M+H)⁺.

**Steps 4-5: Preparation of C190. C189** was converted to **C190** by methods analogous to those described in Example 4, Route 1, Steps 2-3. Chromatography Method A provided C190. MS *m*/*z* 660.8 M)⁺, ¹H NMR (400 MHz, DMSO-d₆) δ 9.25 (d, J=8.8 Hz, 1 H), 7.92 (s, 1 H), 7.30-7.20 (br s, 2H), 7.03-6.95 (m, 1 H), 6.81 (s, 1 H), 6.74 (s, 1 H), 6.22-6.16 (m, 1 H), 5.12 (dd, J=8.6, 5.6 Hz, 1 H), 4.48 (dd, J=9.0, 5.1 Hz, 1 H), 4.22 (d, J=4.7 Hz, 2H), 3.89-3.96 (m, 1 H), 3.56-3.52 (m, 1 H), 3.27-3.22 (m, 1 H), 1.76-1.64 (m, 2H), 1.49-1.42 (m, 1 H), 0.79-0.84 (m, 6H).

### Example 32

### (2S)-2-({[(1Z)-1-(2-amino-1,3-thiazol-4-yl)-2-({(2R,3S)-2-[({[(1,5-dihydroxy-4-oxo-1,4-dihydropyridin-2-yl)methyl]carbamoyl}amino)methyl]-4-oxo-1-sulfoazetidin-3-yl}amino)-2-oxoethylidene]amino}oxy)-3-phenylpropanoic acid (C191).

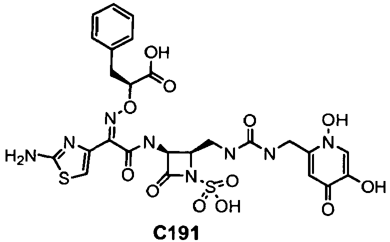

**C191** was prepared by methods analogous to those described in Example 31 only employing D-phenylalanine as starting material. Chromatography Method A provided **C191.** MS *m*/*z* 694.8 (M)⁺. ¹H NMR (400 MHz, DMSO-d₆) δ 9.25 (d, J=8.8 Hz, 1H), 7.99 (s, 1 H), 7.30-7.12 (m, 6H), 7.10-7.04 (m, 1 H), 6.88 (s, 1 H), 6.76 (s, 1 H), 6.31-6.24 (m, 1H), 5.17 (dd, J=8.8, 5.8 Hz, 1 H), 4.73-4.61 (m, 1H), 4.20-4.29 (m, 2H), 3.91-3.99 (m, 1 H), 3.63-3.57 (m, 1 H), 3.30-3.24 (m, 1 H), 3.10-2.98 (m, 2H).

### Example 33

### 2-({[(1Z)-1-(5-amino-1,2,4-thiadiazol-3-yl)-2-({(2R,3S)-2-[({[(1,5-dihydroxy-4-oxo-1,4-dihydropyridin-2-yl)methyl]carbamoyl}amino)methyl]-4-oxo-1-sulfoazetidin-3-yl}amino)-2-oxoethylidene]amino}oxy)-2-methylpropanoic acid (C199).

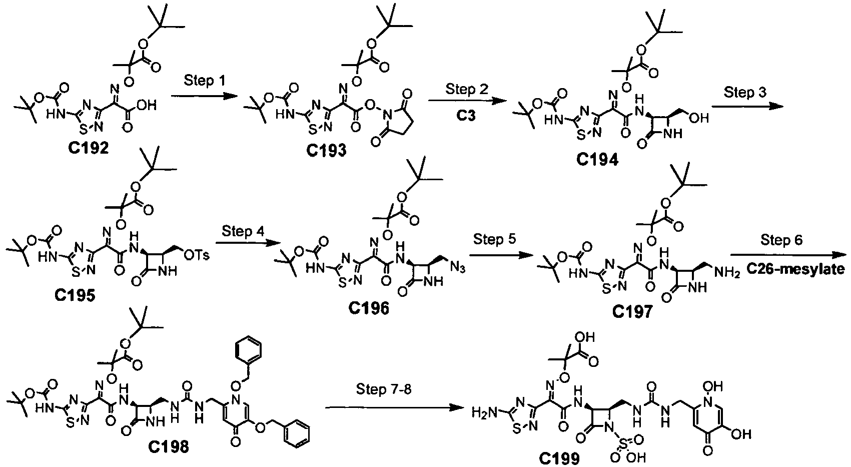

**Step 1: Preparation of C193.** A solution of **C192** (Prepared as described in: Biorg. Med. Chem. 2007, 15, 6716-6732) (2.65 g, 6.15 mmol) in dichloromethane (48 mL) was treated with N-hydroxysuccinimide (0.82 g, 6.76 mmol). The reaction mixture was cooled to 0 °C, N,N'-dicyclohexylcarbodiimide (1.37 g, 6.46 mmol) was added, and the resulting mixture was allowed to stir at 0 °C for 30 minutes. The reaction mixture was warmed to room temperature and stirred for an additional 3 hours. The reaction mixture was filtered over Celite and the filtrate was concentrated in vacuo to afford **C193** as a colorless solid. Yield: 3.24 g, 6.15 mmol, 100%. ¹H NMR (400 MHz, DMSO-*d*₆) δ 12.75 (s, 1 H), 2.88-2.77 (m, 4H), 1.49 (s, 6H), 1.48 (s, 9H), 1.34 (s, 9H).

**Step 2: Preparation of C194.** A solution of **C193** (3.24 g, 6. 14 mmol) and **C3** (0.86 g, 7.37 mmol) in ethanol/toluene (25:3, 90 mL) was concentrated down to 10 mL at 40 °C. After 6 hours, the solvent was removed, the crude material was held under vacuum for 10 hours, the desired product was treated with ethyl acetate/tetrahydrofuran (1:1, 80 mL), and the solution was poured onto saturated sodium bicarbonate (50 mL). The aqueous layer was separated and back extracted with additional ethyl acetate/tetrahydrofuran (1:1, 50 mL). The organic layers were combined, dried over sodium sulfate, filtered and concentrated in vacuo to give crude material (3.00 g) which was purified by silica gel chromatography using an ethyl acetate/heptanes gradient to afford **C194** as a colorless solid. Yield: 1.34 g, 2.5 mmol, 45%. LCMS *m*/*z* 529.1 (M+H)⁺.

**Step 3: Preparation of C195**. Under nitrogen, a solution of **C194** (1.34 g, 2.5 mmol) in anhydrous pyridine (4.4 mL) was cooled to 2 °C and treated with 4-methylbenzenesulfonyl chloride (1.23 g, 6.3 mmol). The reaction vessel was packed in ice and placed in an 8 °C refrigerator, without stirring, for 19 hours. The reaction was cooled to 0 °C, quenched with 10% aqueous citric acid (0.7 mL), and diluted with ethyl acetate (25 mL). The reaction mixture was washed with 2 N HCl (2 x 25 mL), 1N HCl (2 x 25 mL), water (1 x 25 mL), and brine (1 x 25 mL). The organic layer was dried over sodium sulfate, filtered, and concentrated in vacuo to give crude material which was purified by silica gel chromatography using an ethyl acetate/heptanes gradient to afford **C195** as a colorless solid. Yield: 1.51 g, 2.2 mmol, 87%. LCMS m/z 683.2 (M+H)⁺. ¹H NMR (400 MHz, DMSO-*d*₆) δ 12.62 (s, 1 H), 9.24 (d, J=8.8 Hz, 1 H), 8.77 (s, 1 H), 7.76 (dt, *J*=8.4, 2.0 Hz, 2H), 7.46 (dd, *J*=8.4, 0.6 Hz, 2H), 5.28-5.23 (m, 1 H), 4.20- 3.97 (m, 2H), 2.41 (s, 3H), 1.47 (s, 9H), 1.36 (s, 3H), 1.34 (s, 9H), 1.33 (s, 3H).

**Step 4: Preparation of C196.** Under nitrogen, a solution of **C195** (1.50 g, 2.0 mmol) in anhydrous N,N-dimethylformamide (9.2 mL) was treated with sodium azide (0.41 g, 6.3 mmol). The reaction mixture was stirred at 60 °C for 5 hours before being cooled to room temperature and diluted with ethyl acetate/water (2:1, 150 mL). The aqueous layer was back extracted with ethyl acetate (2 x 25 mL). The combined organic layers were washed with water (3 x 25 mL), dried over sodium sulfate, filtered, and concentrated in vacuo to give crude material (1.12 g) which was purified by silica gel chromatography using an ethyl acetate/heptanes gradient to afford of **C196** as a colorless solid. Yield: 0.78 g, 1.4 mmol, 71%. LCMS m/z 554.2 (M+H)⁺.

**Step 5: Preparation of C197.** Under nitrogen, a solution of **C196** (0.78 g, 1.41 mmol) in ethanol (80 mL) was charged with platinum (IV) oxide (0.16 g, 0.71 mmol). The mixture was purged with hydrogen and pressurized to 30 psi hydrogen. The mixture was agitated at room temperature for 3 hours. The mixture was filtered through Celite and the filter cake was rinsed with ethanol. The filtrate was concentrated to afford **C197** as a light gray solid. Yield: 0.74 g, 1.4 mmol, 100%. LCMS m/z 528.2 (M+H)⁺. HPLC retention time 3.199 minutes; Zorbax SB-CN (StableBond Analytical) column (4.6 x 150 mm, 5.0 µm); flow rate 2.8 mL/minute; detection UV 210 nm, 230 nm, and 254 nm; mobile phase: solvent A = phosphoric acid (0.2%) in water, solvent B = acetonitrile (100%); gradient elusion: 0-8.00 minutes solvent A (90%) and solvent B (10%),.8.00-9.00 minutes solvent A (10%) and solvent B (90%), 9.00-10.00 minutes solvent A (95%) and solvent B (5%) total run time 10 minutes.

**Step 6: Preparation of C198**. A solution of **C26-meslylate** (1.0 g, 2.3 mmol) and N,N'-carbonyldiimidazole (0.43 g, 2.6 mmol) in anhydrous THF (20 mL) was cooled to 0 °C, treated dropwise with triethylamine (0.7 mL, 4.7 mmol), and allowed to stir at 0 °C for 5 minutes. The reaction mixture was warmed to room temperature, treated with a solution of **C197** (0.74 g, 1.3 mmol) in anhydrous tetrahydrofuran (10 mL), and allowed to stir for 16.5 hours before being diluted with ethyl acetate (100 mL). The reaction mixture was quenched with water (50 mL) and the organic layer was separated, dried over sodium sulfate, filtered, and concentrated in vacuo to give crude material which was purified by silica gel chromatography using a 2-propanol/ethyl acetate gradient to afford **C198** as a colorless solid. Yield: 0.51 g, 0.57 mmol, 46%. LCMS m/z 890.4 (M+H)⁺.

**Steps 7-8: Preparation of C199**. **C198** was converted to **C199** by methods analogous to those described in Example 4, Route 1, Steps 2-3. Chromatography Method B provided **C199.** LCMS *m*/*z* 634.0 (M+H)⁺. ¹H NMR (400 MHz, DMSO-*d*₆) δ 9.30 (d, *J*=8.4 Hz, 1H), 8.17 (bs, 2H), 8.14 (s, 1 H), 7.22-7.14 (m, 1H), 6.98 (s, 1H), 6.32-6.25 (m, 1 H), 5.14 (dd, *J*=8.4, 5.7 Hz, 1 H), 4.31 (d, *J*=4.9 Hz, 2H), 3.95-3.89 (m, 1 H), 3.61-3.52 (m, 1H), 3.28-3.18 (m, 1H), 1.39 (s, 3H), 1.38 (s, 3H).

### Example 34

### (2S)-2-({[(1Z)-1-(2-amino-5-chloro-1,3-thiazol-4-yl)-2-({(2R,3S)-2-[({[(1,5-dihydroxy-4-oxo-1,4-dihydropyridin-2-yl)methyl]carbamoyl}amino)methyl]-4-oxo-1-sulfoazetidin-3-yl}amino)-2-oxoethylidene]amino}oxy)propanoic acid (C200).

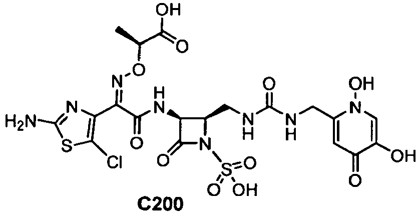

**C200** was prepared by methods analogous to those described in Example 33. Chromatography Method B provided **C200.** LCMS *m*/*z* 651.3 (M-H)⁻. ¹H NMR (400 MHz, DMSO-*d*₆) δ 9.31 (d, *J*=8.6 Hz, 1H), 8.13 (s, 1H), 7.39 (bs, 2H), 7.26-7.18 (m, 1 H), 6.96 (s, 1 H), 6.37-6.33 (m, 1 H), 5.14 (dd, *J*=8.6, 5.1 Hz, 1 H), 4.60 (q, *J*=7.0 Hz, 1 H), 4.30 (d, *J*=4.9 Hz, 2H), 3.95-3.90 (m, 1 H), 3.75-3.67 (m, 1 H), 3.17-3.08 (m, 1 H), 1.35 (d, *J*=7.0 Hz, 3H).

### Example 35

### 2-({[(1Z)-1-(2-amino-1,3-thiazol-4-yl)-2-{[(2R,3S)-2-({[(5-hydroxy-1-methyl-4-oxo-1,4-dihydropyridin-2-yl)carbonyl]amino}methyl)-4-oxo-1-sulfoazetidin-3-yl]amino}-2-oxoethylidene]amino}oxy)-2-methylpropanoic acid (C204).

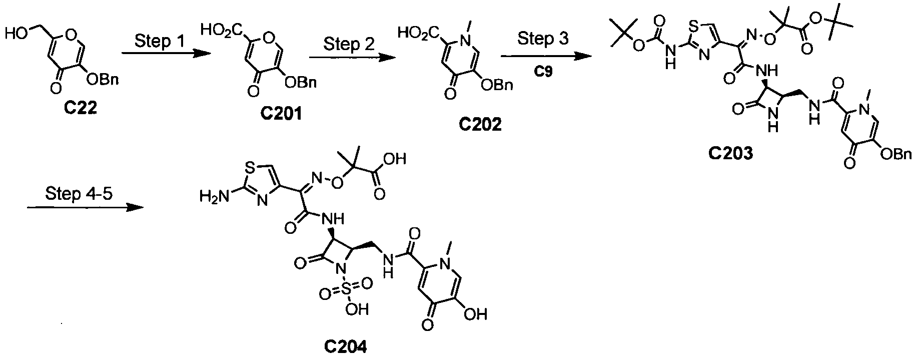

**Step 1: Preparation of C201.** A solution of **C22** (50.0 g, 215 mmol) and 2,2,6,6-tetramethyl-piperidin-1-oxyl (2.40 mL, 15.1 mmol) in acetonitrile (850 mL) at 20 °C in a three neck round bottom was treated with a 0.67M sodium phosphate buffer (600 mL of 1:1 mixture of 0.67M sodium dihydrogen phosphate and 0.67M sodium hydrogen phosphate = pH 6.7). A solution of sodium chlorite was prepared by dissolving 80% sodium chlorite (48.7 g) in water (180 mL) and a dilute solution of sodium hypochlorite was prepared by diluting bleach (5.25% sodium hypochlorite, 4.31 mmol, 6.10 mL) in water (100 mL). The reaction mixture was heated to 35 °C and the dilute sodium chlorite and dilute bleach solutions were added via addition funnels 10% at a time over 20 minutes. After complete addition, the reaction was cooled to ambient temperature and diluted with water (150 mL). The pH was adjusted to 8.0 with addition of 2.0N sodium hydroxide (approximately 100 mL). The mixture was poured into a sodium sulfite solution (54.0 g in 800 mL water) while maintaining a temperature of < 20 °C. After 30 minutes the mixture was extracted with methyl tert-butyl ether. The organic layer was discarded and the aqueous layer was acidified with 2.0N hydrochloric acid (approximately 200 mL) to pH = 2 resulting in a white precipitate. The precipitate was collected via filtration to afford **C201** as a white solid. Yield: 39.8 g, 161 mmol, 75%. LCMS *m*/*z* 247.3 (M+1). ¹H NMR (400 MHz, DMSO-*d*₆) δ 8.31 (s, 1 H), 7.42-7.31 (m, 5H), 6.87 (s, 1H), 4.95 (s, 2H).

**Step 2: Preparation of C202**. **C202** was prepared as in WO 2008/116301, example 22, page 66. Yield: 1.22 g, 4.69 mmol, 57.7%. ¹H NMR (400 MHz, CD₃OD) δ 8.02 (s, 1H), 7.48-7.44 (m, 2H), 7.39-7.31 (m, 3H), 7.18 (s, 1H), 4.86 (s, 2H), 4.07 (s, 3H).

**Step 3: Preparation of C203.** A solution of **C9** (750 mg, 1.42 mmol) and **C202** (425 mg, 1.64 mmol) in anhydrous N,N-dimethylformamide (10 mL) was treated with N-[(dimethylamino)(3H-[1,2,3]triazolo[4,5-b]pyridin-3-yloxy)methylene]-N-methylmethanaminium hexafluorophosphate (1.12 g, 2.85 mmol) and sodium bicarbonate (301 mg, 2.85 mmol). The resulting mixture was stirred at ambient temperature overnight. The mixture was diluted with water (40 mL) and stirred at ambient temperature for 15 minutes. The resulting precipitate was filtered, washed twice with water and once with heptane. The wet cake was dried in vacuo to afford a crude solid. Chromatography on silica gel using a dichloromethane-methanol gradient afforded **C203** as a solid. Yield: 817.5 mg, 1.06 mmol, 74.8%. LCMS *m*/*z* 768.3 (M+H)⁺.

**Steps 4-5: Preparation of C204**. **C203** was converted to **C204** by methods analogous to those described in Example 4, Route 1, Steps 2-3. Chromatography Method A provided **C204.** ¹H NMR (400 MHz, DMSO-d₆) δ 9.33 (d, J=8.2 Hz, 1 H), 8.88-8.80 (m, 1H), 8.04 (s, 1H), 7.32 (br s, 2H), 7.15 (s, 1H), 6.73 (s, 1 H), 5.18 (dd, J=8.2, 5.6 Hz, 1H), 4.24-4.19 (m, 1H), 3.90 (s, 3H), 3.63-3.48 (m, 2H), 1.41 (s, 3H), 1.38 (s, 3H). LCMS m/z 600.3 (M-H)⁻.

### Example 36

### 2-({[(1Z)-1-(2-amino-1,3-thiazol-4-yl)-2-({(2R,3S)-2-[({N-[(1,5-dihydroxy-4-oxo-1,4-dihydropyridin-2-yl)carbonyl]glycyl}amino)methyl]-4-oxo-1-sulfoazetidin-3-yl}amino)-2-oxoethylidene]amino}oxy)-2-methylpropanoic acid (C208).

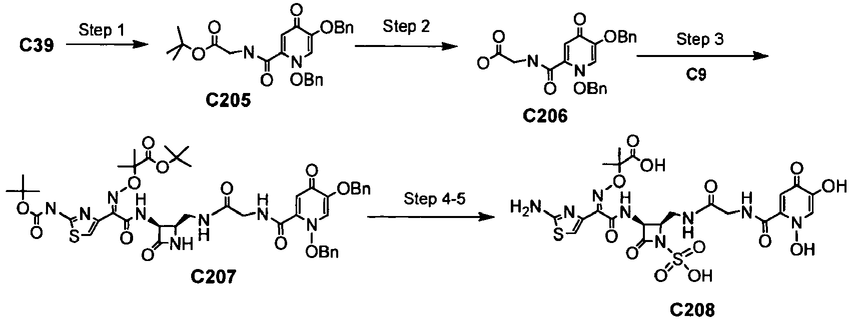

**Step 1: Preparation of C205.** A solution of **C39** (315 mg, 0.90 mmol) and triethylamine (318 mg, 3.14 mmol) in dichloromethane (7 mL) was treated with tert-butyl glycinate (141 mg, 1.08 mmol) and N-[(dimethylamino)(3H-[1,2,3]triazolo[4,5-b]pyridin-3-yloxy)methylene]-N-methylmethanaminium hexafluorophosphate (375 mg, 0.99 mmol). The reaction mixture was allowed to stir at ambient temperature for 72 hours. The reaction mixture was diluted with dichloromethane and washed with water. The organic layer was separated and concentrated in vacuo to give an orange oil. Chromatography on silica gel using a dichloromethane-methanol gradient (0-10% methanol) afforded **C205** as an orange oil. Yield was presumed to be quantitative and product was used without further purification. LCMS *m*/*z* 465.1 (M+1)⁺.

**Step 2: Preparation of C206.** Crude **C205** (417 mg, 0.89 mmol) was dissolved in 96% formic acid (9 mL). The reaction mixture was stirred at ambient temperature for 3 hours, 50 °C for 2.5 hours and then ambient temperature overnight. The reaction mixture was concentrated in vacuo and then dissolved in water and ethyl acetate. Saturated aqueous sodium bicarbonate was added until the pH reach about 9. The aqueous layer was separated and acidified with hydrochloric acid and then extracted with dichloromethane. The organic layer was concentrated in vacuo to afford **C206** as a white solid. Yield: 390 mg, 0.95 mmol, 106%. LCMS *m*/*z* 409.0 (M+1)⁺.

**Step 3: Preparation of C207.** A solution of **C206** (300 mg, 0.735 mmol) and triethylamine (260 mg, 2.57 mmol) in dichloromethane (5.6 mL) was treated with **C9** (464 mg, 0.882 mmol) and then N-[(dimethylamino)(3H-[1,2,3]triazolo[4,5-b]pyridin-3-yloxy)methylene]-N-methylmethanaminium hexafluorophosphate (364 mg, 0.956 mmol). The reaction mixture was stirred at ambient temperature for 3 hours and then was quenched with saturated aqueous sodium bicarbonate. The organic layer was separated and the aqueous layer was extracted twice with dichloromethane. The combined organic layers were dried over sodium sulfate, filtered and concentrated in vacuo to afford a dark foam. Reverse phase chromatography using a Phenomenex Max-RP 150 x 21.2mm 5µ column with a water, methanol, 0.1% formic acid gradient afforded **C207** as a white solid. Yield: 200 mg, 0.22 mmol, 30%. LCMS *m*/*z* 917.3 (M+1)⁺. ¹H NMR (400 MHz, DMSO-*d*₆) δ 11.80 (br s, 1 H), 9.33 (d, *J*=8.6 Hz, 1 H), 9.09 (t, *J*=5.6 Hz, 1H), 8.34 (s, 1H), 8.04 (s, 1H), 7.97 (t, *J*=5.3 Hz, 1 H), 7.31-7.51 (m, 10H), 7.27 (s, 1H), 6.27 (s, 1H), 5.32 (s, 2H), 5.19 (dd, *J*=7.9, 4.8 Hz, 1H), 5.01 (s, 2H), 3.75-3.97 (m, 3H), 3.36 - 3.44 (m, 1 H), 3.17 - 3.28 (m, 1 H), 1.46 (s, 9H), 1.42 (s, 3H), 1.38 (s, 9H), 1.37 (s, 3H).

**Steps 4-5: Preparation of C208**. **C207** was converted to **C208** by methods analogous to those described in Example 4, Route 1, Steps 2-3. Chromatography Method A provided **C208.** LCMS m/z 660.7 (M+1)⁺. ¹H NMR (400 MHz, DMSO-*d*₆) δ 11.64 (t, *J*=5.3 Hz, 1H), 9.28 (d, J=8.8 Hz, 1H), 7.81 (s, 1H), 7.80-7.75 (m, 1H), 7.51 (s, 1H), 6.87 (s, 1 H), 5.15 (dd, J=8.6, 5.6 Hz, 1 H), 4.02-3.95 (m, 1 H), 3.95-3.83 (m, 2H), 3.59-3.50 (m, 1H), 3.35-3.26 (m, 1 H), 1.44 (s, 3H), 1.42 (s, 3H).

### Example 37

### 2-({[(1Z)-1-(2-amino-1,3-thiazol-4-yl)-2-{[(2R,3S)-2-{[({[(1,5-dihydroxy-4-oxo-1,4-dihydropyridin-2-yl)methyl]amino}sulfonyl)amino]methyl}-4-oxo-1-sulfoazetidin-3-yl]amino}-2-oxoethylidene]amino}oxy)-2-methylpropanoic acid (C211).

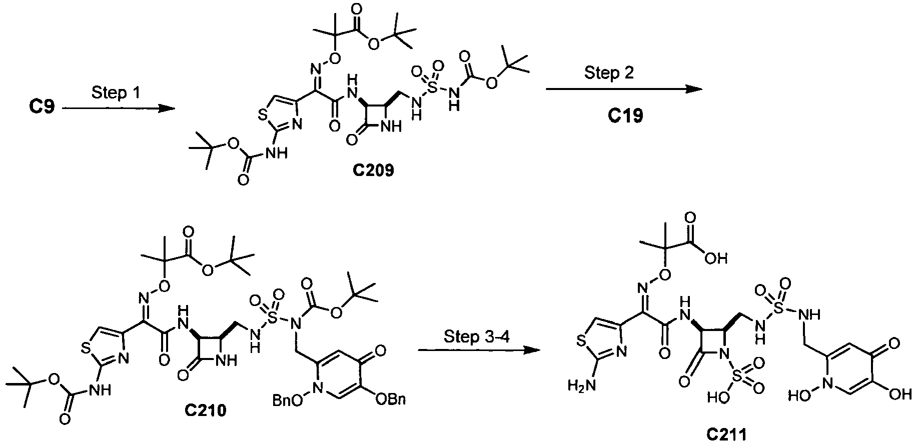

**Step 1: Preparation** of **C209.** A solution of chlorosulfonyl isocyanate (0.41 g, 2.9 mmol) in dry dichloromethane (4 mL) was cooled with an ice bath, and treated with a slow addition of tert-butanol (0.48 mL, 5.0 mmol). In a separate flask, **C9** (1.28 g, 2.43 mmol), and triethylamine (0.30 g, 0.42 mL, 2.9 mmol) were dissolved in dry dichloromethane (6 mL) and cooled in an ice bath, then treated with a slow addition of the first solution, keeping the temperature below 5 °C. The reaction mixture was allowed to warm to ambient temperature and stirred for 4 hours. The reaction mixture was washed with water (2x5 mL) and brine (2x5 mL). The organic layer was dried over sodium sulfate, filtered, concentrated in vacuo and purified by chromatography on silica gel eluting with 5% methanol in dichloromethane to afford **C209** as colorless foam. Yield: 1.28 g, 2.06 mmol, 75%. LCMS *m*/*z* 706.2 (M+H)⁺. ¹H NMR (400 MHz, DMSO-*d*₆) δ ppm 11.69 (br s, 1 H), 10.85 (s, 1H), 9.22 (d, *J*=8.8 Hz, 1H), 8.26 (s, 1H), 7.63 (t, *J*=5.8 Hz, 1 H), 7.20 (d, *J*=0.6 Hz, 1 H), 5.17 (ddd, *J*=9.2, 5.5, 1.2 Hz, 1H), 3.88-3.81 (m, 1H), 3.15-2.98 (m, 2H), 1.43 (s, 9 H), 1.40 (br s, 3 H), 1.39 (s, 9 H), 1.37 (br s, 3 H), 1.36 (s, 9 H).

**Step 2: Preparation of C210.** A mixture of **C209** (0.706 g, 1.0 mmol), **C19** (0.34 g, 1.0 mmol) and triphenylphosphine (265 mg, 1.01 mmol) in dry THF (6 mL) was cooled to 0 °C and treated with diisopropylazodicarboxylate (0.215 g, 0.211 mL, 1.06 mmol), maintaining the temperature below 5 °C. The reaction mixture was stirred at 0-5 °C for 1 hour, and then ambient temperature for 2 hours. The reaction was concentrated in vacuo and the residue was purified by chromatography on silica gel eluting with a gradient of 50% heptanes in ethyl acetate to 100% ethyl acetate. The product was eluted with 10% methanol in ethyl acetate) to afford **C210** as colorless foam. Yield: 0.55 g, 0.53 mmol, 53%. LCMS *m*/*z* 1025.3 (M+H)⁺. ¹H NMR (400 MHz, CD₂Cl₂) δ 9.27-9.19 (m, 1H), 8.42 (d, *J*=5.7 Hz, 1H), 7.52-7.29 (m, 12H), 7.12 (s, 1H), 7.02-6.94 (m, 1H), 6.16 (s, 1H), 5.23 (t, *J*=5.5 Hz, 1 H), 5.10 (d, *J*=4.5 Hz, 2H), 4.95 (s, 2H), 4.72 (s, 2H), 4.00-3.93 (m, 1H), 3.40-3.32 (m, 1H), 3.28-3.19 (m, 1H), 1.55 (s, 6H), 1.54-1.51 (m, 9H), 1.50 (s, 9H), 1.43 (s, 9H).

**Steps 3-4: Preparation of C211. C210** was converted to **C211** by methods analogous to those described in Example 4, Route 1, Steps 2-3. Chromatography Method A provided **C211.** LCMS *m*/*z* 667.3 (M-1)⁻. ¹H NMR (400 MHz, DMSO-*d*₆) δ ppm 9.25 (d, J=8.8 Hz, 1H), 8.14 (s, 1 H), 7.90-7.84 (m, 1H), 7.20 (m, 1H), 6.85-6.79 (m, 1 H), 6.77 (s, 1H), 5.21 (dd, J=8.8, 5.6 Hz, 1 H), 4.29 (d, *J*=5.1 Hz, 2H), 4.20-4.12 (m, 1H), 3.43-3.34 (m, 1H), 3.27-3.17 (m, 1H), 1.41 (s, 6H).

### Example 38

### 2-(5-{[({[(2R,3S)-3-{[(2Z)-2-(2-amino-1,3-thiazol-4-yl)-2-{[(2-carboxypropan-2-yl)oxy]imino}acetyl]amino}-4-oxo-1-sulfoazetidin-2-yl]methyl}carbamoyl)oxy]methyl}isoxazol-3-yl)-1,5-dihydroxy-4-oxo-1,4-dihydropyridinium (C214).

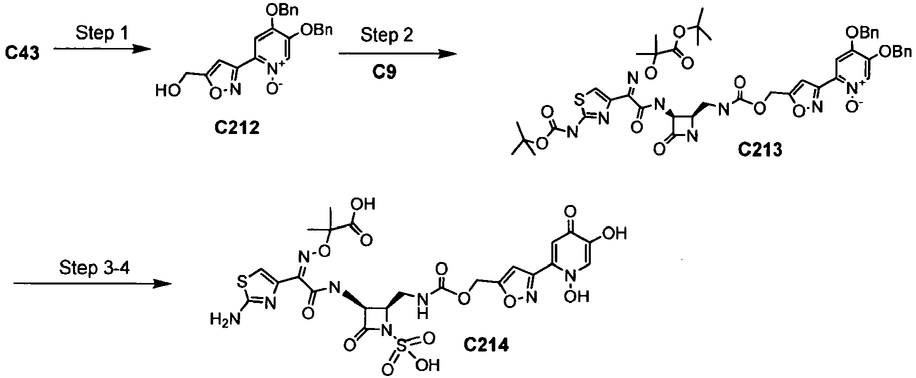

**Step 1: Preparation of C212.** A suspension of **C43** (500 mg, 1.16 mmol) in tetrahydrofuran (5 mL) and water (1 mL) at 0 °C was treated with sodium borohydride (67 mg, 1.73 mmol). The reaction mixture was stirred at room temperature for 2 hours. The reaction was diluted with ethyl acetate (100 mL) and the organic layer was washed with saturated ammonium chloride (10 mL), water (10 mL) and brine (10 mL). The organic layer was dried over sodium sulfate, filtered and the filtrate concentrated in vacuo to afford **C212** as a white solid. Yield: 392 mg, 0.969 mmol, 84%. LCMS *m*/*z* 405.1 (M+H)⁺. ¹H NMR (400 MHz, DMSO-*d*₆) δ ppm 8.25 (s, 1H), 7.55 (s, 1H), 7.28 - 7.46 (m, 10H), 7.23 (s, 1H), 5.68 (t, J=6.1 Hz, 1H), 5.27 (s, 2H), 5.24 (s, 2H), 4.61 (d, J=6.1 Hz, 2H).

**Step 2: Preparation of C213.** A suspension of **C212** (387 mg, 0.957 mmol) in tetrahydrofuran (5 mL) was treated with (diimidazol-1-yl)ketone (155 mg, 0.957 mmol). The reaction mixture was stirred at room temperature for 3 hours. To the reaction was added **C9** (504 mg, 0.957 mmol). The reaction was stirred for 56 hours. The reaction was diluted with ethyl acetate (100 mL) and the organic layer was washed with water (10 mL) and brine (10 mL). The organic layer was dried over sodium sulfate, filtered and the filtrate concentrated in vacuo to give crude material as a solid. The crude material was purified on silica gel (heptanes, ethyl acetate, 2-propanol) to afford **C213** as a white solid. Yield: 370 mg, 0.387 mmol, 40%. LCMS *m*/*z* 957.5 (M+H)⁺. ¹H NMR (400 MHz, DMSO-*d*₆) δ ppm 9.29 (d, J=8.6 Hz, 1 H), 8.36 (s, 1 H), 8.27 (s, 1 H), 7.60 (s, 1 H), 7.55 (s, 1H), 7.29 - 7.45 (m, 10H), 7.23 (s, 1 H), 6.98 (br s, 1H), 5.27 (s, 2H), 5.24 (s, 2H), 5.21 (s, 2H), 5.10 - 5.17 (m, 1 H), 3.75 - 3.83 (m, 1 H), 3.22 - 3.27 (m, 1 H), 3.09-3.21 (m, 1 H), 1.43 (s, 9H), 1.39 (s, 6H), 1.35 (s, 9H).

**Steps 3-4: Preparation of C214**. **C213** was converted to **C214** by methods analogous to those described in Example 4, Route 1, Steps 2-3. Chromatography Method A provided **C213.** LCMS *m*/*z* 701.3 (M+H)⁺. ¹H NMR (400 MHz, DMSO-*d*₆) δ ppm 9.27 (d, *J*=8.6 Hz, 1 H), 8.04 (s, 1 H), 7.37 (s, 1 H), 7.23 (s, 1 H), 6.89-6.85 (m, 1 H), 6.75 (s, 1 H), 5.20 (s, 2H), 5.14 (dd, *J*=8.5, 5.6 Hz, 1 H), 3.94 - 4.02 (m, 1 H), 3.35 - 3.45 (m, 2H), 1.40(s, 3H), 1.39 (s, 3H).

### Example 39

### 2-({[(1Z)-1-(2-amino-1,3-thiazol-4-yl)-2-{[(2R,3S)-2-({[4-({[(1,5-dihydroxy-4-oxo-1,4-dihydropyridin-2-yl)methyl]amino}sulfonyl)benzoyl]amino}methyl)-4-oxo-1-sulfoazetidin-3-yl]amino}-2-oxoethylidene]amino}oxy)-2-methylpropanoic acid (C217).

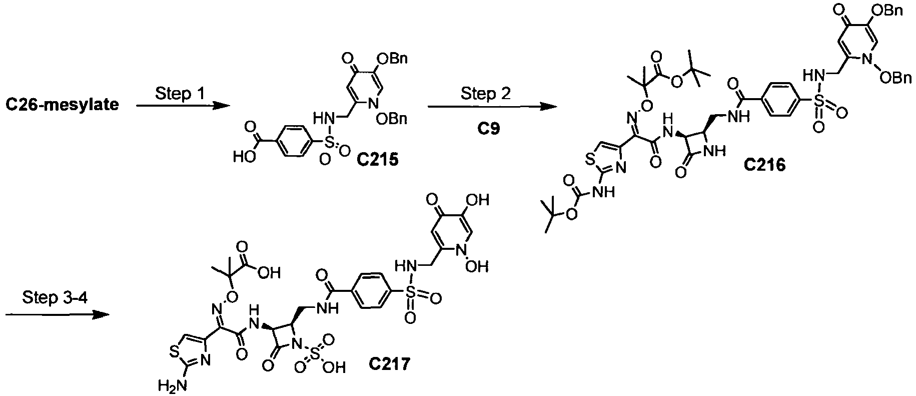

**Step 1: Preparation of C215.** C26-mesylate (5.77 g, 12 mmol presuming 1.5 eq. of the methanesulfonic acid) was slurried in dichloromethane (50 ml) for 20 min, then to the slurry was added 20% potassium phosphate tribasic (35.7 g in 150 ml water, 15 ml of the solution). The mixture was stirred for 30 min, the layers were separated and the organic layer was concentrated in vacuo. The remaining material was dissolved in acetonitrile (20 ml). To the solution were added water (20 ml), sodium bicarbonate (3.4 g, 40 mmol), and 4-(chlorosulfonyl)-benzoic acid (2.3 g, 10 mmol). The reaction mixture was stirred at ambient temperature for 10 hours, then decanted from the oily residue on the bottom of the reaction flask. The acetonitrile was removed using the rotary evaporator and the residual aqueous solution acidified to pH 1-2. The resulting precipitate was filtered, washed sequentially with water, acetonitrile, and dried under high vacuum to provided **C215.** Yield 3.7 g, 7.1 mmol, 71.1%. LCMS *m*/*z* 521.1 (M+1) ⁺. ¹H NMR (400 MHz, DMSO-*d*₆) δ13.45 (brs, 1H) 8.46 (t, *J*=6.2 Hz, 1H) 8.04 - 8.10 (m, 2H) 7.92 (s, 1 H) 7.81 - 7.85 (m, 2H) 7.30 - 7.42 (m, 10H) 5.95 (s, 1 H) 5.15 (s, 2H) 4.89 - 4.94 (m, 2H) 3.87 (d, *J*=6.2 Hz, 2H).

**Step 2: Preparation of C216**. A solution of **C215** (1.00 g, 1.9 mmol) and **C9** (1.00 g, 1.9 mmol) in anhydrous N,N-dimethylformamide (7.7 mL) was treated with *O*-(7-azabenzotriazol-1-yl)-*N,N,N'N*'-tetramethyluronium hexafluorophosphate (0.80 g, 2.1 mmol) and *N,N'-*diisopropylethylamine (0.4 mL, 2.3 mmol). The reaction mixture was allowed to stir at room temperature for 7.5 hours. The reaction mixture was diluted with ethyl acetate (200 mL) and water (200 mL). The aqueous layer was back extracted with ethyl acetate (3 x 100 mL). The organic layers were combined, washed with water (100 mL), washed with brine (100 mL), dried over sodium sulfate, filtered, and concentrated in vacuo to give crude material which was purified via silica gel chromatography using a 2-propanol/ethyl acetate gradient to afford **C216** as a colorless solid. Yield: 1.34 g, 1.30 mmol, 67%. LCMS *m*/*z* 1029.6 (M+H)⁺. ¹H NMR (400 MHz, DMSO-*d*₆) δ 11.74 (s, 1H), 9.41 (d, *J*=8.2 Hz, 1 H), 8.57 (t, *J*=5.2 Hz, 1 H), 8.45 (d, *J*=1.0, 1 H), 8.39 (t, *J*=6.4 Hz, 1H), 7.99 (d, *J*=8.6 Hz, 2H), 7.94 (s, 1H), 7.81 (d, *J*=8.6 Hz, 2H), 7.42-7.29 (m, 10H), 7.26 (d, *J*=0.8 Hz, 1H), 5.95 (s, 1H), 5.19-5.12 (m, 1H), 5.16 (s, 2H), 4.94 (s, 2H), 3.97-3.90 (m, 1 H), 3.86 (d, *J*=6.4 Hz, 2H), 3.56-3.38 (m, 2H), 1.43 (s, 9H), 1.40 (s, 3H), 1.36 (s, 9H), 1.35 (s, 3H).

**Steps 3-4: Preparation of C217**. **C216** was converted to **C217** by methods analogous to those described in Example 4, Route 1, Steps 2-3. Chromatography Method B provided **C217.** LCMS *m*/*z* 773.0 (M+H)⁺. ¹H NMR (400 MHz, DMSO-*d*₆) δ 9.36 (d, *J*=8.8 Hz, 1H), 8.64-8.57 (m, 1H), 8.50-8.44 (m, 1H), 8.03 (s, 1H), 7.96 (d, *J*=8.4 Hz, 2H), 7.86 (d, *J*=8.4 Hz, 2H), 7.08 (s, 1 H), 6.73 (s, 1 H), 5.20 (dd, *J*=8.5, 5.9 Hz, 1 H), 4.19-4.09 (m, 3H), 3.85-3.76 (m, 1 H), 3.52-3.42 (m, 1 H), 1.42 (s, 3H), 1.39 (s, 3H).

### Example 40

### 2-({[(1Z)-1-(2-amino-1,3-thiazol-4-yl)-2-{[(2R,3S)-2-({[3-({[(1,5-dihydroxy-4-oxo-1,4-dihydropyridin-2-yl)methyl]amino}sulfonyl)benzoyl]amino}methyl)-4-oxo-1-sulfoazetidin-3-yl]amino}-2-oxoethylidene]amino}oxy)-2-methylpropanoic acid (C218).

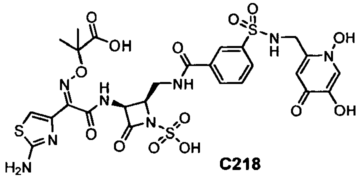

**C218** was prepared by methods analogous to those described in Example 39. Chromatography Method B provided **C218.** LCMS *m*/*z* 773.1 (M+H)⁺. ¹H NMR (400 MHz, DMSO-*d*₆) δ 9.36 (d, *J*=8.6 Hz, 1 H), 8.65-8.58 (m, 1 H), 8.57-8.51 (m, 1 H), 8.25 (s, 1 H), 8.04 (s, 1 H), 8.02 (d, *J*=7.8 Hz, 1 H), 7.92 (d, *J*=7.8 Hz, 1 H), 7.68 (t, *J*=7.8 Hz, 1 H), 7.10 (s, 1H), 6.72 (s, 1H), 5.21 (dd, *J*=8.6, 5.8 Hz, 1H), 4.19-4.11 (m, 3H), 3.87-3.78 (m, 1H), 3.51-3.41 (m, 1H), 1.41 (s, 3H), 1.39 (s, 3H).

### Example 41

### 2-({[(1Z)-1-(2-amino-1,3-thiazol-4-yl)-2-{[(2R,3S)-2-({[{[(4,6-dihydroxy-3-oxocyclohexa-1,4-dien-1-yl)methyl]amino}(oxo)acetyl]amino}methyl)-4-oxo-1-sulfoazetidin-3-yl]amino}-2-oxoethylidene]amino}oxy)-2-methylpropanoic acid (C221).

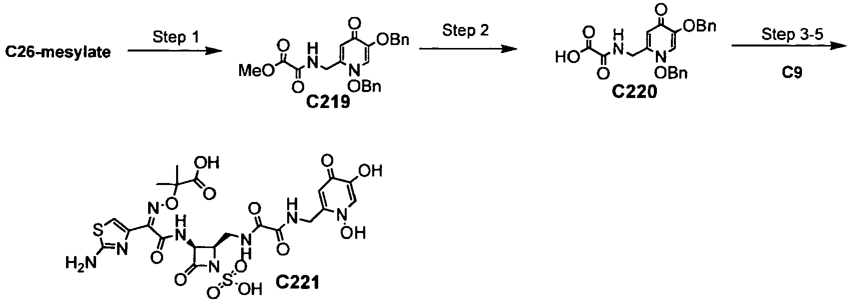

**Step 1: Preparation of C219**. A solution of **C26-mesylate** (7.00 g, 14.6 mmol) and triethylamine (4.10 mL, 29.1 mmol) in dichloromethane (50 mL) at 0 °C was treated with methyl chloro(oxo)acetate (1.78 g, 14.6 mmol). A white precipitate formed after addition. The reaction mixture was stirred at ambient temperature overnight. The reaction mixture was treated with water and extracted with dichloromethane. The aqueous layer was back extracted with dichloromethane. The combined organic layers were washed with brine and dried over magnesium sulfate. The suspension was filtered and concentrated in vacuo to afford **C219** as a tan foam. Yield: 5.96 g, 14.1 mmol, 97%. LCMS *m*/*z* 423.1 (M+H)⁺. ¹H NMR (400 MHz, CDCl₃) δ 8.74 (t, J=6.2 Hz, 1 H), 7.45-7.34 (m, 3H), 7.33-7.24 (m, 7H), 7.08 (s, 1 H), 6.37 (s, 1 H), 5.14 (s, 2H), 4.92 (s, 2H), 4.41 (d, J=6.2 Hz, 2H), 3.82 (s, 3H).

**Step 2: Preparation of C220.** A solution of **C219** (5.92 g, 14.0 mmol) in tetrahydrofuran-methanol-water (2:2:1, 50 mL) was treated with lithium hydroxide monohydrate (764 mg, 18.2 mmol) and stirred at ambient temperature overnight. The reaction mixture was acidified to pH 3 with 1 N aqueous hydrochloric acid and the resulting white solid suspension was stirred for 30 minutes at ambient temperature. The solid was filtered and washed with water. To remove residual water, the solid was frozen in a dry ice-acetone bath and lyophilized to afford **C220** as a white solid. Yield: 4.29 g, 10.5 mmol, 75%. LCMS *m*/*z* 409.1 (M+H)⁺.

**Steps 3-5: Preparation of C221**. **C220** was converted **C221** by methods analogous to those described in Example 35 Step 3 and Example 4, Route 1, Steps 2-3. Chromatography Method A provided **C221.** MS *mlz* 660.7 (M)⁺. ¹H NMR (400 MHz, DMSO-d₆) δ 9.44 (t, J=6.2 Hz, 1 H), 9.28 (d, J=8.8 Hz, 1H), 8.79-8.76 (m, 1H), 8.13 (s, 1H), 7.47-7.19 (br s, 2H), 6.88 (s, 1H), 6.72 (s, 1H), 5.18 (dd, J=8.8, 5.8 Hz, 1H), 4.45 (d, J=6.0 Hz, 2H), 4.03-3.98 (m, 1H), 3.76-3.70 (m, 1H), 3.38-3.22 (m, 1H), 1.41 (s, 3H), 1.40 (s, 3H).

### Example 42

### 2-({[(1Z)-1-(2-amino-1,3-thiazol-4-yl)-2-{[(2R,3S)-2-{[(4-{[(1,5-dihydroxy-4-oxo-1,4-dihydropyridin-2-yl)methyl]amino}-4-oxobutanoyl)amino]methyl}-4-oxo-1-sulfoazetidin-3-yl]amino}-2-oxoethylidene]amino}oxy)-2-methylpropanoic acid (C223).

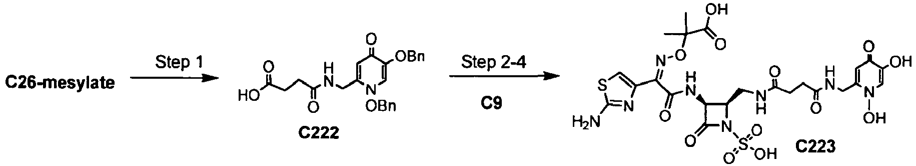

**Step 1: Preparation of C222.** A solution of **C26-mesylate** (7.10 g, 14.8 mmol) and triethylamine (4.16 mL, 29.6 mmol) in dichloromethane (50 mL) was treated with succinic anhydride (1.48 g, 14.8 mmol) and the reaction was stirred overnight at room temperature. The reaction mixture was treated with 1 N aqueous hydrochloric acid and extracted with dichloromethane. The aqueous layer was back extracted with dichloromethane. The combined organic layers were washed with brine and dried over magnesium sulfate. The suspension was filtered and the filtrate was concentrated in vacuo to give **C222** as a white solid. Yield: 5.80 g, 13.2 mmol, 90%. LCMS: *m*/*z* 437.1 (M+H)⁺. ¹H NMR (400 MHz, CD₃OD) δ 8.70 (s, 1 H), 7.51-7.35 (m, 10H), 7.14 (s, 1 H), 5.52 (s, 2H), 5.24 (s, 2H), 4.47 (s, 2H), 2.65-2.60 (m, 2H), 2.58-2.53 (m, 2H).

**Steps 2-4: Preparation of C223**. **C222** was converted to **C223** by methods analogous to those described in Example 35 Step 3 and Example 4, Route 1, Steps 2-3. Chromatography Method A provided **C223.** MS *m*/*z* 688.7 (M)⁺. ¹H NMR (400 MHz, DMSO-d₆) δ 9.25 (d, J=8.6 Hz, 1 H), 8.57 (t, J=5.8 Hz, 1 H), 8.13 (s, 1 H), 7.54-7.51 (m, 1 H), 7.45-7.20 (br s, 2H), 6.90 (s, 1 H), 6.73 (s, 1 H), 5.12 (dd, J=8.6, 5.8 Hz, 1 H), 4.38 (d, J=6.0, 2H), 3.98-3.94 (m, 1 H), 3.55-3.48 (m, 1 H), 3.31-3.25 (m, 1 H), 2.46-2.40 (m, 2H), 2.34-2.27 (m, 2H), 1.39 (s, 3H), 1.38 (s, 3H).

### Example 43

### 2-({[(1Z)-1-(2-amino-1,3-thiazol-4-yl)-2-{[(2R,3S)-2-{[(4-{[(1,5-dihydroxy-4-oxo-1,4-dihydropyridin-2-yl)methyl]carbamoyl}benzoyl)amino]methyl}-4-oxo-1-sulfoazetidin-3-yl]amino}-2-oxoethylidene]amino}oxy)-2-methylpropanoic acid (C226).

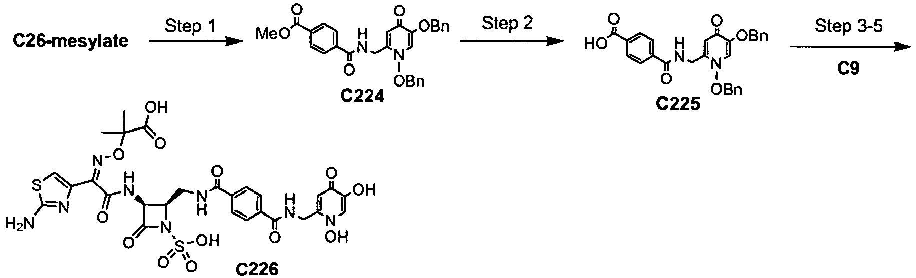

**Step 1: Preparation of C224**. To 4-(methoxycarbonyl) benzoic acid (2.69 g, 14.9 mmol) in dimethylacetamide (25 mL) was added 1,1'-carbonyldiimidazole (2.72 g, 16.7 mmol). The resulting reaction mixture was stirred at room temperature for 1.5 hours. A solution of **C26-mesylate** (acid equivalent 1.5) (7.21 g, 15.0 mmol) and triethylamine (3.14 mL, 22.5 mmol) in dimethylacetamide (25 mL) was then added dropwise and the stirring continued for 3.5 hours. The reaction mixture was diluted with water (50 mL) and extracted with dichloromethane (2 x 100 mL). The organic layer was washed with saturated ammonium chloride (2 x 50 mL), brine (100 mL) and concentrated to afford 26.6 g of crude product, which was re-dissolved in dichloromethane (50 mL) and further washed water (3 x 50 mL) removing residual dimethylacetamide to provide **C224** as a solid. Yield: 7.3 g, 14.6 mmol, 98%. LCMS *m*/*z* 499.2 (M+H)⁺. ¹H NMR (400 MHz, CDCl₃) δ ppm 9.14 (t, *J*=5.7 Hz, 1 H), 8.01 (q, *J*=8.8 Hz, 4H), 7.21 - 7.46 (m, 10H), 6.94 (s, 1H), 6.23 (s, 1H), 5.12 (s, 2H), 4.83 (s, 2H), 4.53 (d, *J*=5.7 Hz, 2H), 3.92 (s, 3H).

**Step 2: Preparation of C225**. To **C224** (7.0 g, 14 mmol) in tetrahydrofuran (50 mL) was added aqueous lithium hydroxide (0.375 M, 52 mL). The reaction mixture was stirred at room temperature for 22 hours, then the THF was removed in vacuo. The aqueous phase was acidified with 6 M HCl (8 mL). The precipitate was filtered and stirred with ethyl acetate/n-heptane (1 : 2, 105 mL) at room temperature overnight. The desired product was collected by filtration and dried under high vacuum to provide **C225** as a solid. Yield: 6.44 g, 13.3 mmol, 95%. LCMS *m*/*z* 485.2 (M+1)⁺. ¹H NMR (400 MHz, methanol-*d*₄) δ ppm 8.13 (d, J=8.4 Hz, 2H), 8.11 (s, 1H), 7.95 (d, J=8.4 Hz, 2H), 7.33-7.52 (m, 11 H), 6.61 (s, 1 H), 5.42 (s, 2H), 5.10 (s, 2H), 4.56 (s, 2H).

**Steps 3-5: Preparation of C226**. **C225** was converted **C226** by methods analogous to those described in Example 35 Step 3 and Example 4, Route 1, Steps 2-3. Chromatography Method A provided **C226.** MS *m*/*z* 736.7 (M)⁺. ¹H NMR (400 MHz, DMSO-d₆) δ 9.36 (d, J=8.4 Hz), 1H), 9.30 (t, J=5.5 Hz, 1 H), 8.46-8.43 (m, 1 H), 8.14 (s, 1 H), 7.96 (d, J=8.4 Hz, 2H), 7.91 (d, J=8.4 Hz, 2H)7.40-7.22 (br s, 2H), 6.94 (s, 1 H), 6.73 (s, 1H), 5.20 (dd, J=8.6, 5.6 Hz, 1H), 4.59 (d, J=5.6 Hz, 2H), 4.15-4.10 (m, 1H), 3.86-3.80 (m, 1 H), 3.48-3.43 (m, 1 H), 1.42 (s, 3H), 1.40 (s, 3H).

### Example 44

### 2-({[(1Z)-1-(2-amino-1,3-thiazol-4-yl)-2-{[(2R,3S)-2-{[(3-{[(1,5-dihydroxy-4-oxo-1,4-dihydropyridin-2-yl)methyl]carbamoyl}benzoyl)amino]methyl}-4-oxo-1-sulfoazetidin-3-yl]amino}-2-oxoethylidene]amino}oxy)-2-methylpropanoic acid (C229).

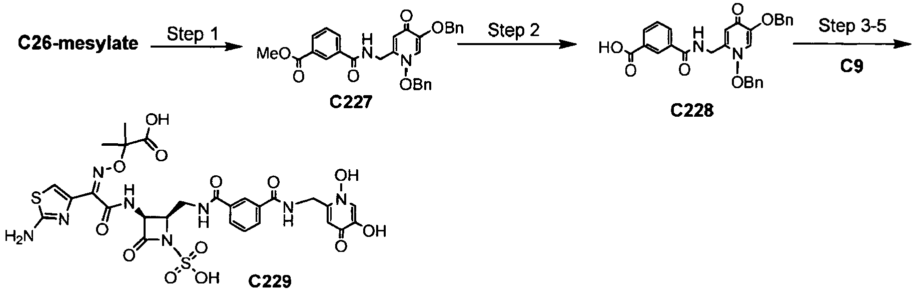

**Step 1: Preparation of C227**. A suspension of 3-(methoxycarbonyl)benzoic acid (2.91 g, 16.1 mmol) in dichloromethane (50 mL) at ambient temperature was treated with 1,1'-carbonyldiimidazole (2.62 g, 16.1 mmol). The reaction mixture became clear after stirring for 5 minutes. After 2 hours stirring the reaction mixture was treated with **C26-mesylate** (7.05 g, 14.7 mmol) and triethylamine (3.10 mL, 22.0 mmol) and stirred overnight at ambient temperature. The reaction mixture was diluted with ethyl acetate and was washed with 10% aqueous citric acid, saturated aqueous sodium bicarbonate, brine and then dried over magnesium sulfate. The suspension was filtered and the filtrate was concentrated in vacuoto afford **C227** as an off-white solid. Yield: 6.57 g, 13.1 mmol, 90%. LCMS *m*/*z* 499.2 (M+H)⁺. ¹H NMR (400 MHz, CD₃OH) δ 8.51 (t, J=1.4 Hz, 1H), 8.19 (dt, J=7.9, 1.4 Hz, 1H), 8.08 (dt, J=7.9, 1.4 Hz, 1H), 7.77 (s, 1H), 7.60 (t, J=7.9 Hz, 1H), 7.46-7.29 (m, 10H), 6.32 (s, 1H), 5.31 (s, 2H), 5.01 (s, 2H), 4.47 (s, 2H), 3.92 (s, 3H).

**Step 2: Preparation of C228**. A suspension of **C227** (4.24 g, 8.50 mmol) in tetrahydrofuran (80 mL) was treated with 1.0M aqueous lithium hydroxide (9.36 mL, 9.36 mmol) and the resulting slurry was stirred at ambient temperature for 4 hours. Additional portions of 1.0N lithium hydroxide (4 x 1.0 mL portions) were added until reaction was complete. The reaction mixture was acidified to pH 3 with 1 N aqueous hydrochloric acid and extracted with ethyl acetate. The organic layer was washed with brine, dried over magnesium sulfate, filtered and concentrated in vacuo to afford a tan solid (3.85 g). Purification on silica gel with a dichloromethane-methanol gradient (1-10% methanol) afforded **C228** as an off-white foam. Yield: 1.45 g, 2.99 mmol, 35%. LCMS *m*/*z* 485.2 (M+H)⁺. ¹H NMR (400 MHz, CD₃OD) δ 8.52 (t, J=1.7 Hz, 1H), 8.19 (dt, J=7.8, 1.2 Hz, 1H), 8.07 (ddd, J=7.8, 1.7, 1.2 Hz, 1H), 7.77 (s, 1 H), 7.59 (t, J=7.8 Hz, 1H), 7.48-7.26 (m, 10H), 6.33 (s, 1H), 5.31 (s, 2H), 5.00 (s, 2H), 4.47 (s, 2H). **Steps 3-5: Preparation of C229**. **C228** was converted to **C229** by methods analogous to those described in Example 35 Step 3 and Example 4, Route 1, Steps 2-3. Chromatography Method A provided **C229.** MS *m*/*z* 736.7 (M)⁺. ¹H NMR (400 MHz, DMSO-d₆) δ 9.40-9.29 (m, 2H), 8.46-8.41 (m, 1H), 8.38 (s, 1H), 8.17 (s, 1H), 8.10 (s, 1 H), 8.03 (d, J=7.4 Hz, 1 H), 7.96 (d, J=7.4 Hz, 1 H), 7.60 (t, J=7.7 Hz, 1 H), 7.44-7.21 (br s, 2H), 6.97 (s, 1 H), 6.73 (s, 1 H), 5.22 (dd, 8.2, 5.6 Hz, 1 H), 4.60 (d, J=5.6 Hz, 2H), 4.15-4.11 (m, 1 H), 3.87-3.86 (m, 1 H), 3.48-3.43 (m , 1 H), 1.42 (s, 3H), 1.40 (s, 3H).

### Example 45

### 2-({[(1Z)-1-(2-amino-1,3-thiazol-4-yl)-2-{[(2R,3S)-2-({[(4-{[(1,5-dihydroxy-4-oxo-1,4-dihydropyridin-2-yl)carbonyl]amino}phenyl)acetyl]amino}methyl)-4-oxo-1-sulfoazetidin-3-yl]amino}-2-oxoethylidene]amino}oxy)-2-methylpropanoic acid (C232).

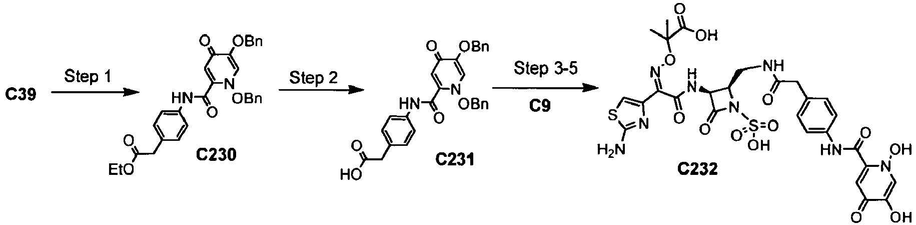

**Step 1: Preparation of C230**. A solution of **C39** (2.06 g, 5.86 mmol), ethyl (4-aminophenyl)acetate (1.08 g, 6.02 mmol) and diisopropylethyl amine (1.16 mL, 6.74 mmol) in dimethylformamide (10 mL) was treated with N-[(dimethylamino)(3H-[1,2,3]triazolo[4,5-b]pyridin-3-yloxy)methylene]-N-methylmethanaminium hexafluorophosphate (2.34 g, 6.16 mmol) and stirred overnight at room temperature. The reaction mixture was treated with water and extracted with ethyl acetate. The aqueous layer was back extracted with ethyl acetate. The combined organic layers were washed with brine and dried over magnesium sulfate. The suspension was filtered and the filtrate was concentrated in vacuo to afford a tan oily solid. Chromatography on silica gel with 10% methanol-dichloromethane afforded a colorless oil. The oil was dissolved in ethyl acetate and washed twice with 10% aqueous citric acid, twice with saturated aqueous sodium bicarbonate, brine and dried over magnesium sulfate. The suspension was filtered and the filtrate was concentrated in vacuo to afford **C230** as a tan solid. Yield: 700 mg, 1.37 mmol, 23%. LCMS *m*/*z* 513.3 (M+H)⁺. ¹H NMR (400 MHz, CDCl₃) δ 10.43 (brs, 1H), 7.72 (d, J=8.4 Hz, 2H), 7.39-7.29 (m, 5H), 7.20-7.08 (m, 7H), 6.89 (s, 1H), 6.36 (s, 1H), 5.27 (s, 2H), 4.65 (s, 2H), 4.13 (q, J=7.2 Hz, 2H), 3.55 (s, 2H), 1.24 (t, J=7.2 Hz, 3H).

**Step 2: Preparation of C231.** A solution of **C230** (700 mg, 1.37 mmol) in tetrahydrofuran-methanol-water (2:2:1, 8.0 mL) was treated with lithium hydroxide monohydrate (86.0 mg, 2.05 mmol) and stirred overnight at room temperature. The reaction mixture was acidified to pH 2 with 1 N aqueous hydrochloric acid and extracted with ethyl acetate and water. The aqueous layer was back extracted with ethyl acetate. The combined organic layers were dried over magnesium sulfate, filtered and concentrated in vacuo to afford **C231** as a golden yellow solid. Yield: 675 mg, 1.39 mmol, 102%. LCMS *m*/*z* 485.3 (M+H)⁺. ¹H NMR (400 MHz, DMSO-d₆) δ 12.27 (br s, 1 H), 10.81 (s, 1 H), 8.17 (s, 1 H), 7.59 (d, J=8.4 Hz, 2H), 7.47-7.30 (m, 10H), 7.24 (d, J=8.4 Hz, 2H), 6.35 (s, 1 H), 5.34 (s, 2H), 5.04 (s, 2H), 3.53 (s, 2H).

**Steps 3-5: Preparation of C232. C231** was converted to **C232** by methods analogous to those described in Example 35 Step 3 and Example 4, Route 1, Steps 2-3. Chromatography Method B provided **C232.** LCMS *m*/*z* 736.8 (M+H)⁺. ¹H NMR (400 MHz, DMSO-*d*₆) δ 9.28 (d, *J*=8.6 Hz, 1 H), 7.89 (s, 1 H), 7.68 (dd, *J*=6.6, 3.6 Hz, 1 H), 7.63 (s, 1 H), 7.54 (d, *J*=8.6 Hz, 2H), 7.20 (d, *J*=8.6 Hz, 2H), 6.85 (s, 1 H), 5.15 (dd, *J*=8.6, 5.8 Hz, 1 H), 3.99 (dt, *J*=7.8, 5.8 Hz, 1 H), 3.57-3.49 (m, 1 H), 3.33 (d, *J*=1.8 Hz, 2H), 3.31-3.22 (m, 1 H), 2.04 (s, 1 H), 1.42 (s, 3H), 1.40 (s, 3H).

### Example 46

### 2-({[(1Z)-1-(2-amino-1,3-thiazol-4-yl)-2-{[(2R,3S)-2-{[(5-{[(1,5-dihydroxy-4-oxo-1,4-dihydropyridin-2-yl)methyl]carbamoyl}pyridin-2-yl)amino]methyl}-4-oxo-1-sulfoazetidin-3-yl]amino}-2-oxoethylidene]amino}oxy)-2-methylpropanoic acid (C235).

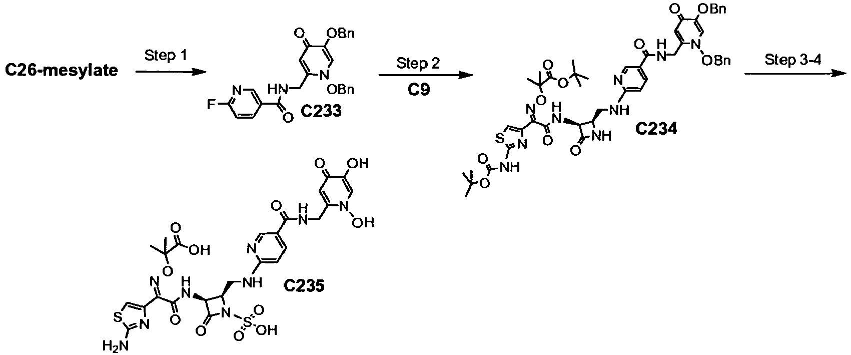

**Step 1: Preparation of C233**. A solution of 6-fluoronicotinic acid (0.50 g, 3.5 mmol) and 1,1'-carbonyldiimidazole (0.63 g, 3.9 mmol) in dimethylacetamide (6.0 mL) was stirred for 15 minutes at room temperature. The solution was treated dropwise with a solution of **C26-mesylate** (1.7 g, 3.5 mmol) and triethylamine (0.75 mL) in dimethylacetamide (6.0 mL). The reaction mixture was allowed to stir at room temperature for 5 hours before being quenched with water (10 mL). The desired product was extracted into dichloromethane (2x 10 mL). The combined organic extracts were washed with a 0.33 M aqueous solution of citric acid (2x10 mL), concentrated in vacuo to 10 mL, washed with water (3 x 5mL) and concentrated in vacuo to give crude material (1.05 g) which was purified using silica gel chromatography (2-propanol/ethyl acetate gradient) to afford C233 as a colorless solid. Yield: 0.53 g, 1.15 mmol, 33%. LCMS *m*/*z* 460.1 (M+H)⁺. ¹H NMR (400 MHz, DMSO-*d*₆) δ 9.29 (t, *J*=4.6 Hz, 1 H), 8.75 (d, *J*=1.8 Hz, 1 H), 8.43 (td, *J*=6.4, 1.8 Hz, 1 H), 8.05 (s, 1 H), 7.56-7.32 (m, 11 H), 6.02 (s, 1 H), 5.32 (s, 2H), 5.01 (s, 2H), 4.44 (d, *J*=4.6 Hz, 2H).

**Step 2: Preparation of C234**. A solution of **C233** (0.17 g, 0.37 mmol) and **C9** (0.23 g, 0.44 mmol) in deutrated dimethylsulfoxide (0.5 mL) was stirred at 60 °C for 27 hours, then 65 °C for 19 hours. Additional **C9** (0.08 g, 0.15 mmol) was added and the reaction was stirred at 65 °C for 10.5 hours. Additional deutrated dimethylsulfoxide (0.2 mL) was added and the reaction mixture was allowed to stir at 65 °C for 16.5 hours. Four other reactions piloted under similar conditions were then combined with the described reaction and concentrated under vacuum (2.3 torr, room temperature, 94 hours) to give crude material. The combined crude material was purified using silica gel chromatography and a 2-propanol/ethyl acetate gradient to afford **C234** as a colorless solid. Yield: 0.40 g, 0.41 mmol, 78%. LCMS *m*/*z* 966.5 (M+H)⁺.

**Steps 3-4: Preparation of C235**. **C234** was converted to **C235** by methods analogous to those described in Example 4, Route 1, Steps 2-3. Chromatography Method B provided **C235.** LCMS *m*/*z* 710.3 (M+H)⁺. ¹H NMR (400 MHz, DMSO-*d*₆) δ 9.34 (d, *J*=8.6 Hz, 1 H), 8.97 (t, *J*=5.8 Hz, 1 H), 8.53 (d, *J*= 2.4 Hz, 1 H), 8.13 (s, 1 H), 8.00-7.91 (m, 1 H), 7.38-7.21 (m, 1 H), 6.90 (s, 1 H), 6.76 (s, 1 H), 6.55 (d, *J*= 8.5 Hz, 1 H), 5.21 (dd, *J*=8.6, 5.5 Hz, 1 H), 4.54 (d, *J*=5.8 Hz, 2H), 4.09-4.03 (m, 1 H), 3.78-3.55 (m, 2H), 1.41 (s, 6H).

### Example 47

### 2-({[(1Z)-1-(2-amino-1,3-thiazol-4-yl)-2-{[(2R,3S)-2-{[({[3-(1,5-dihydroxy-4-oxo-1,4-dihydropyridin-2-yl)isoxazol-5-yl]methyl}carbamoyl)amino]methyl}-4-oxo-1-sulfoazetidin-3-yl]amino}-2-oxoethylidene]amino}oxy)-2-methylpropanoic acid (C239).

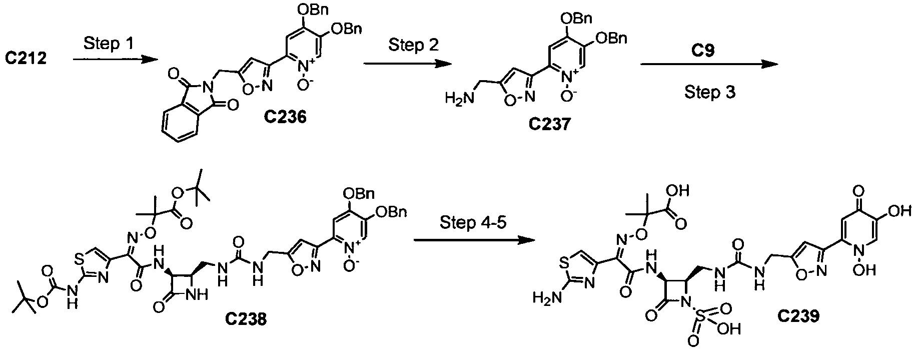

**Step 1: Preparation of C236**. To a solution of **C212** (1.0 g, 2.5 mmol) in tetrahydrofuran (40 mL) was added phthalimide (735 mg, 4.95 mmol) and triphenylphosphine (983 mg, 3.71 mmol). To the resulting suspension was added azodicarboxylic acid dibenzyl ester (872 mg, 3.71 mmol). The reaction was stirred at room temperature for 56 hours. The reaction was quenched with water (25 mL) and diluted with dichloromethane (150 mL). The organic layer was washed with water (25 mL) and brine (25 mL). The organic layer was dried over sodium sulfate. The suspension was filtered and the filtrate concentrated in vacuo to give crude material as a solid. The crude material was purified on silica gel (heptanes, ethyl acetate, 2-propanol) to afford **C236** as a white solid. Yield: 280 mg, 0.525 mmol, 21.0%. LCMS m/z 534.3 (M+H)⁺. ¹H NMR (400 MHz, DMSO-*d*₆) δ 8.24 (s, 1H), 7.83 - 7.93 (m, 4H), 7.52 (s, 1 H), 7.28 - 7.45 (m, 11 H), 5.25 (s, 2H), 5.22 (s, 2H), 5.00 (s, 2H).

**Step 2: Preparation of C237**. To a suspension of **C236** (434 mg, 0.813 mmol) in ethanol (10 mL) was added hydrazine hydrate (0.2 mL, 4.06 mmol). The reaction was stirred at reflux for 1 hour. The reaction was filtered hot and the solid was washed with dichloromethane. The filtrate was allowed to sit at room temperature for 30 minutes. The solids that precipitated were collected by filteration. The combined solids afforded **C237** as a white solid. Yield: 336 mg, 0.833 mmol, quantitative. LCMS *m*/*z* 404.1 (M+H)⁺. ¹H NMR (400 MHz, DMSO-*d*₆) δ 8.25 (s, 1H) 7.54 (s, 1H) 7.28 - 7.46 (m, 10H) 7.20 (s, 1 H) 5.27 (s, 2H) 5.24 (s, 2H) 3.83 (d, *J*=0.8 Hz, 2H).

**Step 3: Preparation of C238**. A suspension of **C237** (330 mg, 0.818 mmol) in tetrahydrofuran (5 mL) was treated with 1,1'-carbonyldiimidazole (133 mg, 0.818 mmol). The reaction mixture was stirred at room temperature for 18 hours. To the reaction was added **C9** (431 mg, 0.818 mmol). The reaction was stirred for 4 hours. The reaction was diluted with ethyl acetate (100 mL) and the organic layer was washed with water (10 mL) and brine (10 mL). The organic layer was dried over sodium sulfate. The suspension was filtered and the filtrate was concentrated in vacuo to give crude material as a solid. The crude material was purified on silica gel (heptanes, ethyl acetate, 2-propanol) to afford **C238** as a white solid. Yield: 334 mg, 0.349 mmol, 42.7%. LCMS *m*/*z* 956.4 (M+H)⁺. ¹H NMR (400 MHz, DMSO-*d*₆) δ 9.20 (br s, 1 H), 8.24 (s, 1H) 8.20 (s, 1H) 7.53 (s, 1H) 7.30 - 7.46 (m, 10H), 7.25 (s, 1H), 7.12 (s, 1H), 6.98 (br s, 1 H), 5.26 (s, 2H), 5.23 (s, 2H), 5.11 - 5.20 (m, 1 H), 4.34 - 4.48 (m, 2H), 3.65 - 3.77 (m, 1H), 2.82 (dd, *J*=13.3, 5.1 Hz, 1H), 2.67 (dd, *J*=12.9, 7.2 Hz, 1H), 1.43 (s, 6H) 1.36 (s, 18H).

**Steps 4-5: Preparation of C239. C238** was converted to **C239** by methods analogous to those described in Example 4, Route 1, Steps 2-3. Chromatography Method A provided **C238.** LCMS m/z 698.2 (M-H)⁺. ¹H NMR (400 MHz, DMSO-*d*₆) δ 9.19 (d, *J*=8.6 Hz, 1H), 8.06 (s, 1H), 7.36 (s, 1H), 7.10 (brs, 1H), 6.98 (s, 1 H), 6.75 (s, 1H), 6.06 (br s, 1 H), 5.14 (dd, J=8.9, 5.9 Hz, 1 H), 4.30 - 4.37 (m, 2H), 3.86 - 3.93 (m, 1 H), 3.54-3.65 (m, 1 H), 3.14 - 3.26 (m, 1 H), 1.39 (s, 3H), 1.38(s, 3H).

### Example 48

### 2-({[(1Z)-1-(2-amino-1,3-thiazol-4-yl)-2-{[(2R,3S)-2-({[(4-{[(1,5-dihydroxy-4-oxo-1,4-dihydropyridin-2-yl)methyl]carbamoyl}phenyl)sulfonyl]amino}methyl)-4-oxo-1-sulfoazetidin-3-yl]amino}-2-oxoethylidene]amino}oxy)-2-methylpropanoic acid (C242).

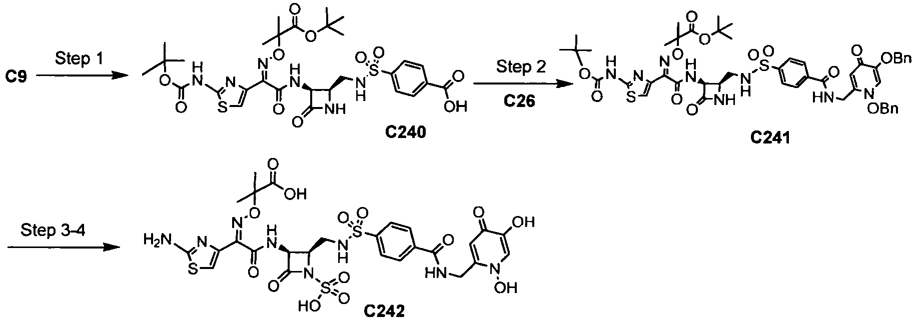

**Step 1: Preparation of C240**. A solution of **C9** (1.05 g, 2.0 mmol) in acetonitrile (5 mL) was treated with a solution of sodium bicarbonate (0.86 g, 10 mmol) in water (10 mL), followed by a suspension of 4-(chlorosulfonyl)-benzoic acid in acetonitrile (5 mL). The reaction mixture was stirred for 1 hour at ambient temperature, concentrated in vacuo, and acidified with 1% citric acid to form a precipitate. The precipitate was collected by filteration and purified via chromatography on silica gel using an ethyl acetate/heptane, gradient from 20 to 100% of ethyl acetate to afford **C240.** Yield: 0.92 g, 1.3 mmol, 65%. LCMS *m*/*z* 711.2 (M+H)⁺.

**Step 2: Preparation of C241.** A mixture of **C240** (0.48 g, 0.68 mmol), **C26** (0.23 g, 0.68 mmol) in dichloromethane (15 mL) was treated with N-[(dimethylamino)(3H-[1,2,3]triazolo[4,5-b]pyridin-3-yloxy)methylene]-N-methylmethanaminium hexafluorophosphate (0.27 g, 0.68 mmol), and triethylamine (0.07 g, 0.1 mL, 0.68 mmol) and was stirred at ambient temperature for 2 hours. The reaction mixture was washed with 1% citric acid (3 x 10 mL) and the organic layer was dried over magnesium sulfate, filtered and concentrated in vacuo. The resulting residue was dissolved in ethyl acetate, filtered through a pad of silica gel, which was rinsed with ethyl acetate. Concentration of the filtrates afforded **C241.** Yield 0.42 g, 0.41 mmol, 60%. LCMS m/z 1029.3 (M+H)⁺. ¹H NMR (400 MHz, CDCl₃) δ 9.94 (s, 1H), 9.02 (s, 1H), 8.59 (s, 1 H), 7.92 (d, J=7.2 Hz, 2 H) 7.84 (d, J=7.2 Hz, 2 H), 7.42-7.14 (m, 10H), 7.11-7.01 (m, 1H), 6.35-6.19 (m, 1 H), 5.10 (br s, 2H), 5.08-4.99 (m, 1 H) 4.84-4.78 (m, 1 H), 4.83 - 4.72 (m, 2 H), 4.53-4.38 (m, 2H), 3.58-3.40 (m, 2H), 3.37 (s, 2H), 3.23- 3.07 (m, 1H), 1.51-1.29 1.48 (br s, 3H), 1.44 (s, 9H,) 1.39 (br s, 3H), 1.35 (s, 9H).

**Steps 3-4: Preparation of C242. C241** was converted to **C242** by methods analogous to those described in Example 4, Route 1, Steps 2-3. Chromatography Method B provided **C242.** LCMS *m*/*z* 773.3 (M+H)⁺. ¹H NMR (400 MHz, DMSO-*d*₆) δ 12.76-12.40 (m, 1 H), 9.40 (t, *J*=5.3 Hz, 1 H), 9.16 (d, *J*=9.0 Hz, 1H), 8.11 (brs, 1H), 8.09 (dd, *J*=8.7, 2.0 Hz, 2H), 7.90 (d, *J*=8.6 Hz, 2H), 7.44-7.38 (m, 1H), 7.34- 7.19 (m, 2H), 6.90 (s, 1H), 6.73 (s, 1H), 5.11 (dd, *J*=9.0, 2.7 Hz, 1H), 4.60-4.54 (m, 2H), 3.87 (m, 1H), 3.23-3.17 (m, 2H), 1.38 (s, 3H), 1.37 (s, 3H).

### Example 49

### 2-({[(1Z)-1-(2-amino-1,3-thiazol-4-yl)-2-{[(2R,3S)-2-{[(N-{[(1,5-dihydroxy-4-oxo-1,4-dihydropyridin-2-yl)methyl]carbamoyl}-D-alanyl)amino]methyl}-4-oxo-1-sulfoazetidin-3-yl]amino}-2-oxoethylidene]amino}oxy)-2-methylpropanoic acid (C246).

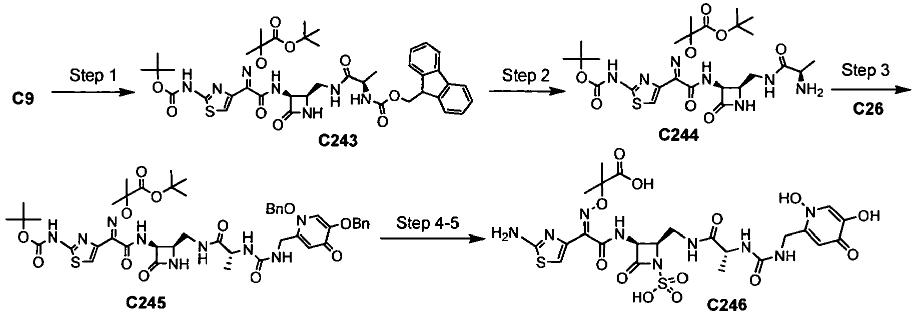

**Step 1: Preparation of C243**. A solution of **C9** (1.05 g, 2.0 mmol) in dichloromethane (15 mL) was treated with N-[(9H-fluoren-9-ylmethoxy)carbonyl]-D-alanine) (0.64 g, 2.0 mmol), N-[(dimethylamino)(3H-[1,2,3]triazolo[4,5-b]pyridin-3-yloxy)methylene]-N-methylmethanaminium hexafluorophosphate (0.80 g, 2.0 mmol), and triethylamine (0.29 mL, 2.0 mmol). The reaction mixture was stirred at ambient temperature for 1 hour and then washed with 1% citric acid (3 x 10 mL). The organic layer was dried over magnesium sulfate, filtered and concentrated in vacuo. Chromatography of the residue on silica gel with a gradient from 50% heptane in ethyl acetate to 100% ethyl acetate to 10% isopropanol in ethyl acetate afforded **C243.** Yield 0.5 g, 0.6 mmol, 30%. LCMS *m*/*z* 820.3 (M+H)⁺. ¹H NMR (400 MHz, CDCl₃) δ 8.87-8.76 (m, 1 H), 8.01 (d, *J*=5.3 Hz, 1H), 7.72 (d, *J*=7.6 Hz, 2H), 7.52 (dd, *J*=7.4, 3.3 Hz, 2H), 7.36 (t, *J*=7.8 Hz, 2H), 7.29-7.22 (m, 5H), 6.49-6.42 (m, 1 H), 5.65 (d, *J*=8.6 Hz, 1 H), 5.17-5.12 (m, 1 H), 4.29-4.17 (m, 3H), 4.11 (d, *J*=7.0 Hz, 2H), 3.65-3.58 (m, 1H), 1.55 (s, 6H), 1.46 (s, 9H), 1.42 (s, 9H), 1.38 (d, *J*=7.0 Hz, 3H).

**Step 2: Preparation** of **C244**. A solution of **C243** (1.0 g, 1.2 mmol) in dichloromethane (15 mL) was treated with morpholine (3.0 g, 34 mmol). The reaction mixture was stirred at ambient temperature for 1 hour, then washed with water (20 mL). The organic layer was washed with brine (20 mL), dried over sodium sulfate, filtered and concentrated in vacuo. Chromatography of the residue on silica gel with ethyl acetate and then eluting with 10% triethylamine in 1:1 isopropanol/ethyl acetate afforded **C244.** Yield 0.47 g, 0.79 mmol, 65%. LCMS *m*/*z* 598.3 (M+H)⁺. ¹H NMR (400 MHz, CDCl₃) δ 7.91-7.82 (m, 2H), 7.34 (s, 1 H), 5.97-5.93 (m, 1 H), 5.21-5.17 (m, 1H), 4.07-3.98 (m, 2H), 3.72-3.68 (m, 1 H), 3.64 (d, J=6.8 Hz, 2H), 3.32-3.22 (m, 2H), 1.60 (s, 6H), 1.51 (s, 9H), 1.43 (s, 9H), 1.41 (d, J=6.8 Hz, 3H).

**Step 3: Preparation of C245.** A solution of 1,1'-carbonyldiimidazole (0.16 g, 1.0 mmol) in dry dichloromethane (5 mL) was treated with a solution of **C26** (336 mg, 1.0 mmol) in dry dichloromethane (10 mL) via a slow addition over 20 min. To the resulting solution was added **C244** (0.47 g, 0.79 mmol). The reaction mixture was stirred at ambient temperature for 10 hours, then washed with aqueous sodium bicarbonate. The organic layer was dried over magnesium sulfate, filtered, and concentrated in vacuo to afford impure **C245.** Yield 0.84 g, 0.70 mmol, 89%. LCMS *m*/*z* 960.5 (M+H)⁺. The product was used without further purification.

**Steps 4-5: Preparation of C246. C245** was converted to **C246** by methods analogous to those described in Example 4, Route 1, Steps 2-3. Chromatography Method B provided **C246.** LCMS *m*/*z* 704.3 (M+H)⁺. ¹H NMR (400 MHz, DMSO-*d*₆) δ ppm 13.03-12.22 (m, 2H), 9.20 (d, *J*=8.6 Hz, 1 H), 8.09 (d, *J*=9.6 Hz, 1 H), 7.76 (dd, *J*=7.4, 2.9 Hz, 1H), 7.28 (brs, 2H), 6.97 (s, 1H), 6.73-6.65 (m, 3H), 5.16 (dd, *J*=8.6, 2.7 Hz, 1H), 4.32 (d, *J*=4.9 Hz, 2H), 4.00 (t, *J*=6.9 Hz, 1 H), 3.94 (ddd, *J*=4.1, 3.3, 1.9 Hz, 1 H), 3.66 (qd, *J*=3.9, 3.1 Hz, 1H), 3.22-3.13 (m, 1H), 1.39 (s, 3 H) 1.38 (s, 3 H) 1.18 (d, *J*=7.0 Hz, 3H).

### Example 50

### 2-({[(1Z)-1-(2-amino-1,3-thiazol-4-yl)-2-{[(2R,3S)-2-{[(4-{[(1,5-dihydroxy-4-oxo-1,4-dihydropyridin-2-yl)methyl]carbamoyl}pyridin-2-yl)amino]methyl}-4-oxo-1-sulfoazetidin-3-yl]amino}-2-oxoethylidene]amino}oxy)-2-methylpropanoic acid (C249).

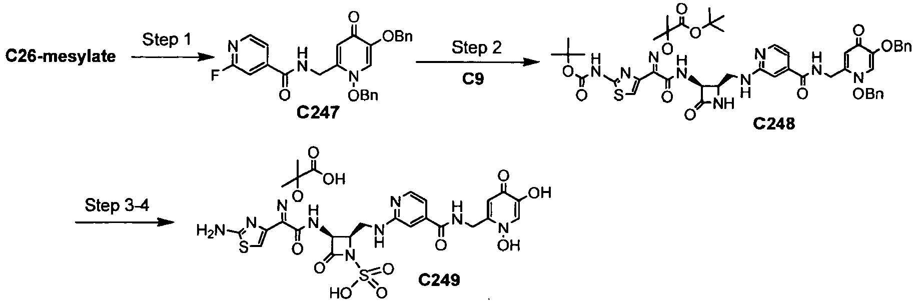

**Step 1: Preparation of C247.** To 2-fluoropyridine-4-carboxylic acid (1.48 g, 10.5 mmol) in dimethylacetamide (15 mL) was added 1,1'- carbonyldiimidazole (2.20 g, 13.6 mmol). The resulting reaction mixture was stirred at room temperature for 2.5 hours. A solution of **C26-mesylate** (acid equivalent 1.5) (5.05 g, 10.5 mmol) and triethylamine (2.20 mL, 15.8 mmol) in dimethylacetamide (20 mL) was then added dropwise and the stirring continued for 14 hours. The reaction mixture was diluted with water (50 mL) and extracted with dichloromethane (100 mL). The organic layer was washed successively with citric acid solution (21 g of citric acid monohydrate in 300 mL of water, 2 x 50 mL) and water (2 x 50 mL), dried over sodium sulfate, filtered and concentrated to afford a white solid, which was triturated with n-heptane (250 mL) to remove residue dimethylacetamide. **C247** was collected by filtration to provide a white solid. Yield: 3.10 g, 6.7 mmol, 64%. LCMS *m*/*z* 460.1 (M+H)⁺. ¹H NMR (400 MHz, Chloroform-*d*) δ ppm 9.53 (t, *J*=5.7 Hz, 1H), 8.20 (d, *J*=5.3 Hz, 1H), 7.83 (d, *J*=5.5 Hz, 1H), 7.60 (s, 1H), 7.39 - 7.48 (m, 3H), 7.18 - 7.36 (m, 7H), 6.93 (s, 1H), 6.20 (s, 1H), 5.15 (s, 2H), 4.70(s, 2H), 4.53 (d, *J*=5.5 Hz, 2H).

**Step 2: Preparation of C248**. A mixture of **C9** (0.53 g, 1 mmol) and **C247** (0.46 g, 1.0 mmol) in DMSO-d₆ (2 mL) was stirred in a nitrogen stream at 55 °C for 5 days. The resulting mixture was diluted with ethyl acetate (15 mL), washed with water (10 mL). The organic layer was concentrated in vacuo, and the residue was purified by chromatography on silica gel eluting with ethyl acetate/isopropanol, gradient from 0 to 50% isopropanol to afford **C248.** Yield: 0.2 g, 0.2 mmol, 21%. LCMS *m*/*z* 966.5 (M+H)⁺.

**Steps 3-4: Preparation of C249. C248** was converted to **C249** by methods analogous to those described in Example 4, Route 1, Steps 2-3. Chromatography Method B provided **C249.** LCMS *m*/*z* 710.3 (M+H)⁺. ¹H NMR (400 MHz, DMSO-*d*₆) δ 9.31 (d, *J*=8.6 Hz, 1 H), 9.28-9.22 (m, 1 H), 8.10 (s, 1 H), 8.06 (d, J=5.6 Hz, 1 H), 8.03 (s, 1 H), 7.51-7.27 (m, 2H), 6.99-6.92 (m, 2H), 6.83 (s, 1 H), 6.73 (s, 1 H), 5.21 (dd, J=8.8, 2.9 Hz, 1 H), 4.51 (d, *J*=5.6 Hz, 2H), 4.11 (q, *J*=6.0 Hz, 1 H), 3.75-3.57 (m, 2H), 1.40 (s, 3H), 1.38 (s, 3H).

### Example 5.

### 2-({[(1Z)-1-(2-amino-1,3-thiazol-4-yl)-2-{[(2R,3S)-2-({[4-({[(4,5-dihydroxypyridin-2-yl)carbonyl]amino}methyl)benzoyl]amino}methyl)-4-oxo-1-sulfoazetidin-3-yl]amino}-2-oxoethylidene]amino}oxy)-2-methylpropanoic acid (252).

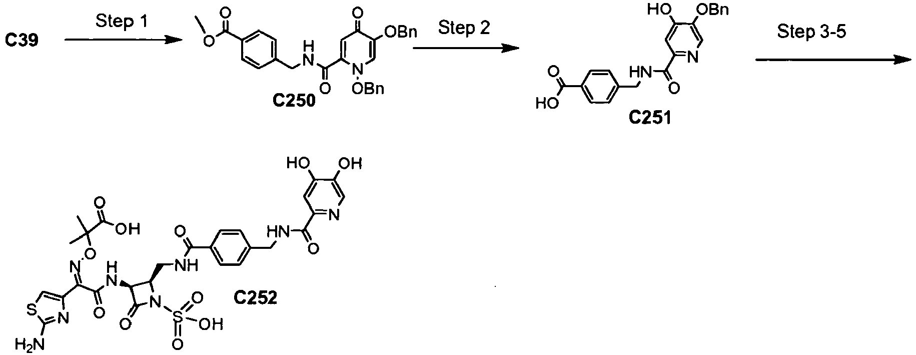

**Step 1: Preparation of C250**. To a suspension of **C39** (3.03 g, 8.6 mmol) in dimethylacetamide (10 mL) was added 1,1'- carbonyldiimidazole (1.40 g, 8.6 mmol). The resulting reaction mixture was stirred at room temperature for 4 hours. A suspension of methyl 4-(aminomethyl)benzoate hydrochloride salt (1.74 g, 8.6 mmol) and triethylamine (1.20 mL, 8.6 mmol) in dimethylacetamide (10 mL) was added dropwise. The resulting reaction mixture was stirred at room temperature for 17 hours. The reaction mixture was diluted with water (30 mL) and extracted with dichloromethane (2 x 60 mL). The organic layer was washed with 30% saturated sodium bicarbonate (30 mL). The white precipitate that formed in the aqueous phase was collected by filteration to afford 1.38 g of pure **C250.** The organic layer was then washed with citric acid solution (7.2 g of citric acid monohydrate in 100 mL of water, 2 x 50 mL) and water (2 x 30 mL), dried over sodium sulfate, filtered and concentrated to afford a white solid, which was triturated with n-heptane (150 mL) to remove residual dimethylacetamide. The desired product was collected by filtration to provide an additional 1.74 g of **C250** as a white solid. Yield: 3.1 g, 6.2 mmol, 72%. LCMS *m*/*z* 499.2 (M+H)⁺. ¹H NMR (400 MHz, DMSO-*d*₆) δ 9.51 (t, *J*=6.0 Hz, 1H), 8.13 (s, 1H), 7.84 (d, *J*=8.4 Hz, 2H), 7.33-7.48 (m, 12H), 6.24 (s, 1 H), 5.31 (s, 2H), 5.03 (s, 2H), 4.51 (d, *J*=6.0 Hz, 2H), 3.85 (s, 3H).

**Step 2: Preparation of C251.** To **C250** (1.38 g, 2.8 mmol) in THF (7 mL) was added aqueous lithium hydroxide (0.5 M, 7 mL). The reaction mixture was stirred at room temperature for 20 hours and then treated with 1 M hydrochloric acid (1 mL, 1 mmol), water (2 mL) and THF (3 mL). The mixture was stirred at room temperature for 31 hours and then treated with additional lithium hydroxide (0.375 M, 6.6 mL). After 21 hours more lithium hydroxide (1 M, 3 mL) and THF (10 mL) were added and the reaction mixture was stirred for 23 hours. A second batch of **C250** (1.74 g, 3.5 mmol) was treated in a similar fashion as above. The two crude reaction mixtures were combined and washed with dichloromethane (50 mL). The aqueous phase was acidified with 4 M hydrochloric acid (4.7 mL) with cooling in an ice bath. The precipitate was filtered, washed with ice-water (10 mL) and dried under high vacuum to afford 2.1 g of a crude **C251.** The crude **C251** was triturated with acetonitrile (30 mL) at room temperature overnight. The desired product was collected by filtration and dried under high vacuum to provide **C251** as a solid. Yield: 1.2 g, 3.2 mmol, 44%. LCMS *m*/*z* 379.1 (M+H)⁺. ¹H NMR (400 MHz, DMSO-*d*₆) δ 9.20 (t, J=6.6 Hz, 1 H), 8.16 (brs, 1 H), 7.88 (d, J=8.2 Hz, 2H), 7.31 - 7.54 (m, 8H), 5.26 (s, 2H), 4.50 (d, *J*=6.4 Hz, 2H).

**Steps 3-5: Preparation of C252. C251** was converted to **C252** by methods analogous to those described in Example 39, Step 2 and Example 4, Route 1, Steps 2-3. Chromatography Method B provided **C252.** LCMS *m*/*z* 721.8 (M+H)⁺. ¹H NMR (400 MHz, DMSO-*d*₆) δ 9.47 (bs, 1H), 9.35 (d, *J*=9.35, 1H), 8.28 (dd, *J*= 7.8, 2.8 Hz, 1H), 7.94 (s, 1 H), 7.74 (d, *J*=8.4 Hz, 2H), 7.61 (s, 1 H), 7.37 (d, *J*=8.4 Hz, 2H), 6.77 (s, 1 H), 5.20 (dd, *J*=8.6, 5.5 Hz, 1 H), 4.51 (d, *J*=5.8 Hz, 2H), 4.11-4.05 (m, 1 H), 3.87-3.79 (m, 1 H), 3.43-3.34 (m, 1 H), 1.42 (s, 3H), 1.40 (s, 3H).

### Example 52

### 2-({[(1Z)-1-(2-amino-1,3-thiazol-4-yl)-2-{[(2R,3S)-2-{[(4-{[(1,5-dihydroxy-4-oxo-1,4-dihydropyridin-2-yl)carbonyl]amino}benzoyl)amino]methyl}-4-oxo-1-sulfoazetidin-3-yl]amino}-2-oxoethylidene]amino}oxy)-2-methylpropanoic acid (C256).

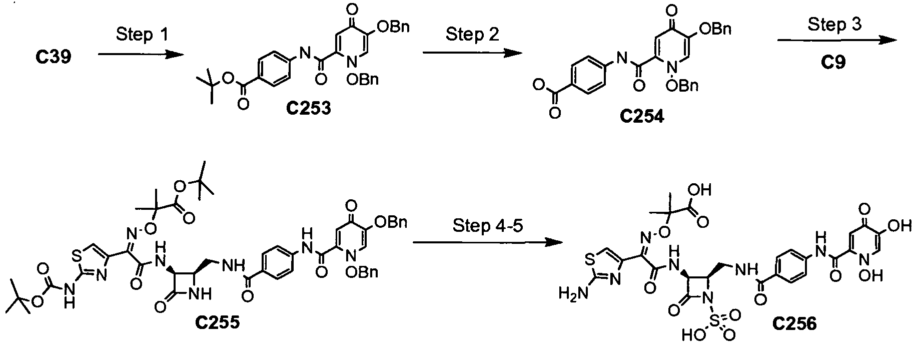

**Step 1: Preparation of C253.** A solution of **C39** (2.09 g, 5.96 mmol), tert-butyl 4-aminobenzoate (1.15 g, 5.96 mmol) and diisopropylethyl amine (1.19 mL, 6.86 mmol) in dimethylformamide (10 mL) was treated with N-[(dimethylamino)(3H-[1,2,3]triazolo[4,5-b]pyridin-3-yloxy)methylene]-N-methylmethanaminium hexafluorophosphate (2.38 g, 6.26 mmol) and stirred overnight at room temperature. The reaction mixture was treated with water and extracted with ethyl acetate. The organic layer was washed 3 times with water then brine and dried over magnesium sulfate. The suspension was filtered and the filtrate was concentrated in vacuo to afford a tan solid (3.65 g). The solid was triturated with 1:1 heptane-ethyl acetate and filtered to afford **C253** as an off-white solid. Yield: 2.85 g, 5.41 mmol, 91%. LCMS ***m*/*z*** 527.4 (M+H)⁺. ¹H NMR (400 MHz, DMSO-d₆) δ 11.12 (s, 1 H), 8.23 (s, 1 H), 7.90 (d, J=8.8 Hz, 2H), 7.77 (d, J=8.8 Hz, 2H), 7.47-7.29 (M, 10H), 6.43 (s, 1 H), 5.34 (s, 2H), 5.05 (s, 2H), 1.52 (s, 9H).

**Step 2: Preparation of C254.** A solution of **C253** (1.40 g, 2.65 mmol) in dichloromethane (15.0 mL) was treated with trifluoroacetic acid (1.5 mL) and stirred overnight at room temperature. The reaction mixture was concentrated in vacuo to afford a yellow oil. The oil was dissolved in minimal dichloromethane; diethyl ether was added to precipitate a tan solid. Concentration in vacuo afforded **C254** as a tan solid. Yield: 1.55 g, 3.29 mmol, 124%. LCMS *m*/*z* 471.3 (M+H)⁺. ¹H NMR (400 MHz, DMSO-d₆) δ 11.17 (s, 1 H), 8.39 (s, 1 H), 7.95 (d, J=8.8Hz, 2H), 7.77 (d, J=8.8 Hz, 2H), 7.88-7.29 (m, 10H), 6.61 (s, 1 H), 5.38 (s, 2H), 5.09 (s, 2H).

**Step 3: Reparation of C255.** A suspension of **C254** (0.61 g, 1.0 mmol) in dimethylformamide (15 mL) was treated with triethylamine (0.15 mL, 1.0 mmol) to form a clear solution. The reaction mixture was treated with **C9** (0.55 g, 1.04 mmol), and N-[(dimethylamino)(3H-[1,2,3]triazolo[4,5-b]pyridin-3-yloxy)methylene]-N-methylmethanaminium hexafluorophosphate (0.42 g, 1.0 mmol) and stirred at ambient temperature for 10 hours. The reaction mixture was treated with water (100 mL) and the resulting precipitate was filtered, washed with water, and then suspended in dichloromethane (40 mL). The suspension was washed with 20% potassium phosphate tribasic, until the organic layer became clear. The organic layers were washed with brine solution and concentrated in vacuo. Chromatography of the residue on silica gel eluting with a gradient from 2.5 to 5% of methanol in dichloromethane afforded **C255.** Yield 0.52 g, 0.53 mmol, 51%. LCMS *m*/*z* 980.2 (M+H)⁺.

**Steps 4-5: Preparation of C256. C255** was converted to **C256** by methods analogous to those described in Example 4, Route 1, Steps 2-3. Chromatography Method A provided **C256.** LCMS *m*/*z* 723.8 (M+H)⁺. ¹H NMR (400 MHz, DMSO-*d*₆) δ 11.31-11.09 (m, 1H), 11.09-10.91 (m, 1H), 9.34 (d, *J*=8.8 Hz, 1H), 8.24 (dd, *J*=7.0, 3.5 Hz, 1H), 7.91 (s, 1 H), 7.80 (d, *J*=9.0 Hz, 2H), 7.72 (d, *J*=9.0 Hz, 2H), 7.66 (s, 1 H), 6.74 (s, 1H), 5.21 (dd, *J*=8.6, 2.7 Hz, 1H), 4.14-4.07 (m, 1H), 3.86- 3.77 (m, 2H), 1.43 (s, 3H), 1.40 (s, 3H).

### Example 53

### 2-({[(1Z)-1-(2-amino-1,3-thiazol-4-yl)-2-{[(2R,3S)-2-{[(N-{[(1,5-dihydroxy-4-oxo-1,4-dihydropyridin-2-yl)methyl]carbamoyl}glycyl)amino]methyl}-4-oxo-1-sulfoazetidin-3-yl]amino}-2-oxoethylidene]amino}oxy)-2-methylpropanoic acid (C260).

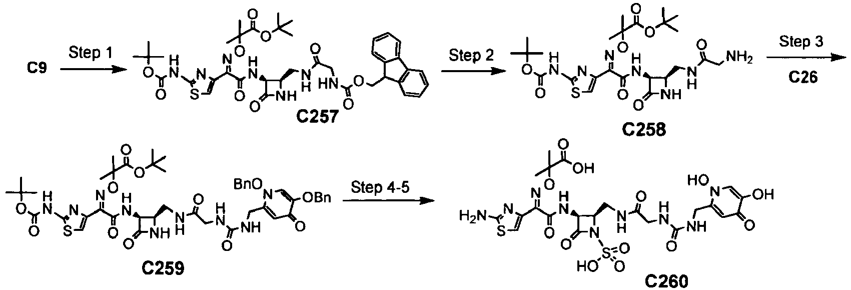

**Step 1: Preparation** of **C257.** A solution of **C9** (1.05 g, 2.0 mmol) in dichloromethane (15 mL) was treated with N-[(9H-fluoren-9-ylmethoxy)carbonyl]glycine (0.595 g, 2.0 mmol), N-[(dimethylamino)(3H-[1,2,3]triazolo[4,5-b]pyridin-3-yloxy)methylene]-N-methylmethanaminium hexafluorophosphate (0.80 g, 2.0 mmol), and triethylamine (0.21 g, 0.29 mL, 2.0 mmol). The reaction mixture was stirred at ambient temperature for 10 hours and washed with 1% citric acid. The organic layer was washed with brine, dried over magnesium sulfate, filtered, and concentrated in vacuo to afford **C257.** Yield 1.2 g, 1.5 mmol, 74%. LCMS *m*/*z* 806.5 (M+H)⁺. The product was used in the next step without further purification.

**Step 2: Preparation of C258.** A solution of **C257** (1.2 g, 1.5 mmol) in dimethylformamide (10 mL) was treated with morpholine (2 mL, 2 mmol). The reaction mixture was stirred at ambient temperature for 1 hour and a precipitate formed. The reaction mixture was treated with water (50 mL) and the precipitate was filtered off. The filtrate was extracted with ethyl acetate (150 mL) and the organic layer was washed with water (2 x 50 mL), brine (2 x 50 mL), dried over sodium sulfate, filtered and concentrated in vacuo to afford **C258** as a solid foam. Yield 0.79 g, 1.4 mmol, 91%. LCMS *m*/*z* 584.9 (M+H)⁺. ¹H NMR (400 MHz, CDCl₃) δ 8.04 (d, *J*=5.3 Hz, 1 H), 7.86 (t, *J*=5.6 Hz, 1H), 7.35 (s, 1H), 6.05-6.01 (m, 1H), 5.20 (td, *J*=6.2, 2.2 Hz, 1 H), 4.06- 4.01 (m, 1 H), 3.83-3.75 (m, 1 H), 3.51-3.39 (m, 2H), 3.20 (s, 2H), 1.61 (s, 3H), 1.52 (s, 3H), 1.51 (s, 9H), 1.43 (s, 9H), 1.18 (s, 2H).

**Step 3: Preparation of C259.** To a solution of 1,1'-carbonyldiimidazole (0.24 g, 1.5 mmol) in dry dichloromethane (5 mL) was added a solution of **C26** (0.504 g, 1.5 mmol) in dry dichloromethane (20 mL) drop wise over 40 min. To the resulting solution was added **C258** (0.78 g, 1.34 mmol), the reaction mixture was stirred at ambient temperature for 10 hours, washed with 10% citric acid (15 mL), aqueous sodium bicarbonate (15 mL), brine solution (15 mL), dried over sodium sulfate, filtered, and concentrated in vacuo. Purification by flash chromatography on silica gel (heptane/ethyl acetate, gradient from 50 to 100% of ethyl acetate, then ethyl acetate/isopropanol, gradient from 0 to 50% of isopropanol provided **C259.** Yield 0.77 g, 0.81 mmol, 60%. LCMS *m*/*z* 947.2 (M+H)⁺. ¹H NMR (400 MHz, CDCl₃) δ 10.32-9.99 (m, 1 H), 9.01-8.76 (m, 1 H), 8.30-8.14 (m, 1 H), 7.45-7.33 (m, 4H), 7.34-7.25 (m, 6H), 6.92 (s, 1 H), 6.67 (br s, 1 H), 5.04 (dd, *J*=22.0, 11.1 Hz, 2H), 4.99-4.90 (m, 1 H), 4.84 (d, *J*=4.7 Hz, 2H), 4.77-4.60 (m, 1 H), 4.32-4.18 (m, 1H), 4.12 (s, 1 H), 3.91 (br s, 2H), 3.71 (d, *J*=17.8 Hz, 1 H), 3.17 (d, *J*=12.1 Hz, 1 H), 1.84 (br s, 4H), 1.53-1.37 1.51 (s, 3H), 1.47 (s, 9H), 1.44 (s, 3H), 1.41 (s, 9H).

**Steps 4-5: Preparation of C260. C259** was converted to **C260** by methods analogous to those described in Example 4, Route 1, Steps 2-3. Chromatography Method B provided **C260.** LCMS *m*/*z* 690.7 (M+H)⁺. ¹H NMR (400 MHz, DMSO-*d*₆) δ 12.93-12.38 (m, 2H), 9.25 (d, *J*=8.2 Hz, 1 H), 8.11 (s, 1 H), 8.08 (s, 1 H), 7.80 (q, *J*=3.5 Hz, 1 H), 7.36-7.21 (m, 2H), 7.00 (s, 1H), 6.83 (dd, *J*=6.2, 5.4 Hz, 1H), 6.73 (s, 1H), 6.67 (t, *J*=5.8 Hz, 1 H), 5.16 (dd, *J*=8.6, 2.9 Hz, 1 H), 4.33 (d, *J*=5.3 Hz, 2H), 4.02-3.94 (m, 1 H), 3.61-3.56 (m, assumed 2H; partially obscured by water), 3.00-3.50 (assumed 2H; obscured by water), 1.40 (br s, 6H).

### Example 54

### 2-({[(1Z)-1-(2-amino-1,3-thiazol-4-yl)-2-({(2R,3S)-2-[({N-[(1,5-dihydroxy-4-oxo-1,4-dihydropyridin-2-yl)carbonyl]-L-alanyl}amino)methyl]-4-oxo-1-sulfoazetidin-3-yl}amino)-2-oxoethylidene]amino}oxy)-2-methylpropanoic acid (C264).

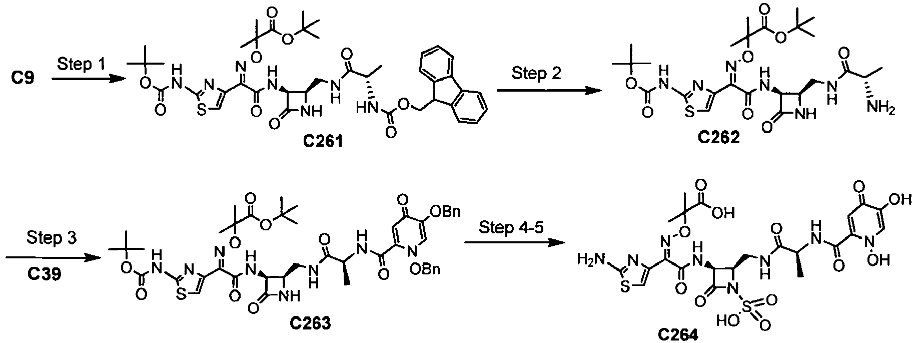

**Step 1: Preparation of C261. C261** was prepared by a method analogous to that described for Example 49, Step 1. Yield 1.27 g, 1.55 mmol, 77%. LCMS *m*/*z* 821.1 (M+H)⁺. ¹H NMR (400 MHz, CDCl₃) δ 9.22-9.10 (m, 1 H), 8.43-8.30 (m, 1 H), 7.71 (t, *J*=7.2 Hz, 1H), 7.56 (d, *J*=8.0 Hz, 1 H), 7.49 (d, *J*=8.0 Hz, 1H), 7.39-7.25 (m, 6H), 7.19 (t, *J*=8.0 Hz, 1H), 6.37-6.27 (m, 1H), 5.50 (d, *J*=8.0 Hz, 1H), 5.12 (t, *J*=5.3 Hz, 1H), 4.41 (dd, *J*=10.5, 3.7 Hz, 1H), 4.35-4.21 (m, 2H), 4.18-4.11 (m, 1H), 4.07-4.00 (m, 1H), 3.63-3.54 (m, 1H), 3.50-3.41 (m, 1H), 1.60 (s, 6H), 1.53 (d, *J*=3.5 Hz, 3 H), 1.44-1.39 (m, 18H).

**Step 2: Peparation of C262. C262** was prepared by a method analogous to that described for Example 53, Step 2. Yield 0.93 g, 1.6 mmol, 101%. LCMS *m*/*z* 598.9 (M+H)⁺. ¹H NMR (400 MHz, CDCl₃) δ 8.04 (d, *J*=5.5 Hz, 1 H), 8.02 (s, 1 H), 7.91 (t, *J*=5.8 Hz, 1 H), 7.38 (s, 1 H), 6.00-5.96 (m, 2H), 5.20 (td, *J*=4.9, 2.9 Hz, 1 H), 4.02 (qd, *J*=4.7, 2.5 Hz, 1 H), 3.84 (qd, *J*=7.4, 2.1 Hz, 1 H), 3.74-3.67 (m, 1 H), 3.13-3.03 (m, 1 H), 1.64 (s, 6H), 1.51 (s, 9H), 1.44 (s, 9H), 1.40 (d, *J*=6.8 Hz, 3 H).

**Step 3: Preparation of C263.** A solution of **C39** (0.304 g, 0.78 mmol) and **C262** (0,47 g, 0.78 mmol) in dry dichloromethane (10 mL) was treated with N-[(dimethylamino)(3H-[1,2,3]triazolo[4,5-b]pyridin-3-yloxy)methylene]-N-methylmethanaminium hexafluorophosphate (0.31 g, 0.78 mmol) and triethylamine (0.11 mL, 0.78 mmol). The reaction mixture was stirred at ambient temperature for 10 hours and treated with 10% citric acid (10 mL). The organic layer was washed with aqueous sodium bicarbonate (10 ml), brine (10 mL), dried over sodium sulfate, filtered and concentrated *in vacuo.* Chromatography on silica gel eluting with ethyl acetate/isopropanol gradient from 0 to 30% of isopropanol to afford **C263.** Yield 0.29 g, 0.31 mmol, 40%. LCMS *m*/*z* 931.5 (M+H)⁺.

**Steps 4-5: Preparation of C264. C263** was converted to **C264** by methods analogous to those described in Example 4, Route 1, Steps 2-3. Chromatography Method A provided **C264.** LCMS *m*/*z* 675.1 (M+H)⁺. ¹H NMR (400 MHz, DMSO-*d*₆) δ 11.79 (d, *J*=6.8 Hz, 1H), 11.12-10.48 (m, 1H), 9.30 (d, *J*=8.4 Hz, 1 H), 7.89 (t, *J*=5.3 Hz, 1H), 7.80 (s, 1H), 7.51 (s, 1H), 6.87 (s, 1H), 5.15 (dd, *J*=8.5, 5.7 Hz, 1 H), 4.32 (dt, *J*=13.8, 6.8 Hz, 1H), 3.99 (dd, *J*=12.6, 5.4 Hz, 1H), 3.52-3.42 (m, 1H), 3.41-3.31 (m, 1H), 1.44 (s, 3H), 1.43(s, 3H), 1.28 (d, *J*=6.8 Hz, 3H).

### Example 55

### 2-({[(1Z)-1-(2-amino-1,3-thiazol-4-yl)-2-{[(2R,3S)-2-{[(N-{[(1,5-dihydroxy-4-oxo-1,4-dihydropyridin-2-yl)methyl]carbamoyl}-L-alanyl)amino]methyl}-4-oxo-1-sulfoazetidin-3-yl]amino}-2-oxoethylidene]amino}oxy)-2-methylpropanoic acid (C266).

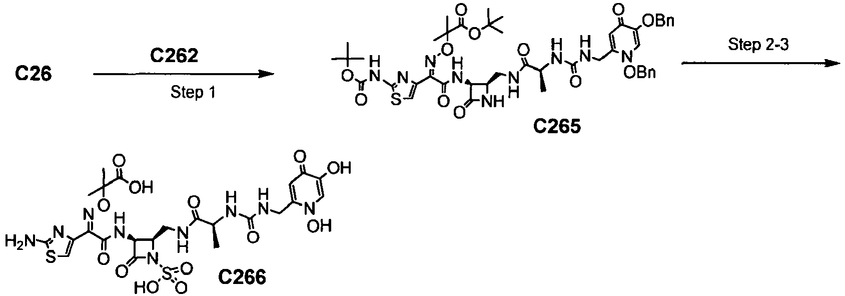

**Step 1: Preparation of C265.** To a solution of 1,1'-carbonyldiimidazole (1.26 g, 7.6 mmol) in dry dichloromethane (50 mL) was added a solution of **C26** (2.33 g, 6.93 mmol) in dry dichloromethane (50 mL) dropwise over 2 hours at ambient temperature. The reaction mixture was then placed in the refrigerator. After approximately 3 weeks, the flask was examined and a precipitate had formed. The solid was collected by filteration and used directly in the next step. The solid (0.43 g, 1 mmol) and **C262** (0.47 g, 0.78 mmol) in dichloromethane (5 mL) and THF (5 mL) was stirred at ambient temperature for 10 hours. The resulting solution was washed with water (2 x 10 mL), dried over sodium sulfate, filtered and concentrated in vacuo. The crude product was purified by flash chromatography on silica gel to afford **C265.** Yield 0.5 g, 0.52 mmol, 66.7%. LCMS *m*/*z* 961.2 (M+H)⁺.

**Steps 2-3: Preparation of C266. C265** was converted to **C266** by methods analogous to those described in Example 4, Route 1, Steps 2-3. Chromatography Method A provided **C266.** LCMS *m*/*z* 704.1 (M+H)⁺. ¹H NMR (400 MHz, DMSO-*d*₆) δ 9.26 (d, *J*=8.6 Hz, 1H), 8.16 (s, 1 H), 7.91 (q, *J*=3.5 Hz, 1H), 7.22 - 7.48 (m, 1H), 7.04 (s, 1H), 6.76 (m, 2H), 5.15 (dd, *J*=8.4, 2.9 Hz, 1 H), 4.37 (d, *J*=4.5 Hz, 2H), 3.98 (m, 2H), 3.15-3.68, (assumed, 2H; obscured by water), 1.41 (m, 3H), 1.40 (m, 3H), 1.19 (d, *J*=7.02 Hz, 3H).

### Example 56

### 2-({[(1Z)-1-(2-amino-1,3-thiazol-4-yl)-2-{[(2R,3S)-2-({[(3-{[(1,5-dihydroxy-4-oxo-1,4-dihydropyridin-2-yl)carbonyl]amino}phenyl)acetyl]amino}methyl)-4-oxo-1-sulfoazetidin-3-yl]amino}-2-oxoethylidene]amino}oxy)-2-methylpropanoic acid (C269).

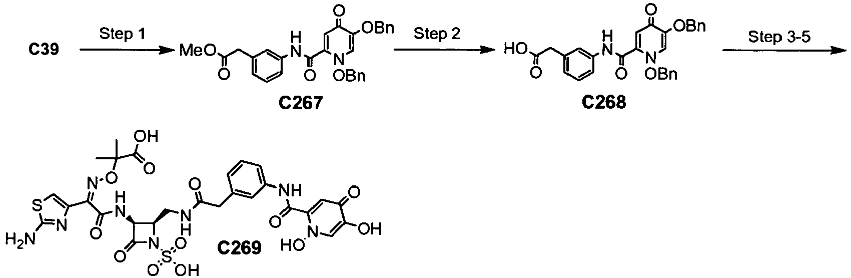

**Step 1: Preparation of C267.** A solution of **C39** (2.09 g, 5.96 mmol), methyl (3-aminophenyl)acetate (1.01 g, 5.94 mmol) and diisopropylethyl amine (1.20 mL, 6.94 mmol) in dimethylformamide (10 mL) was treated with N-[(dimethylamino)(3H-[1,2,3]triazolo[4,5-b]pyridin-3-yloxy)methylene]-N-methylmethanaminium hexafluorophosphate (2.37 g, 6.24 mmol) and stirred overnight at ambient temperature. The reaction mixture was treated with water and a tan precipitate formed. The precipitate was filtered, rinsed with water and dried in vacuo to afford **C267** as a tan solid. Yield: 3.13 g, 6.27 mmol, 105%. LCMS *m*/*z* 499.8 (M+H)⁺. ¹H NMR (400 MHz, CDCl₃) δ 10.67 (br s, 1 H), 7.75 (s, 1 H), 7.69 (d, J=7.4 Hz, 1 H), 7.41-7.16 (m, 12H), 7.11 (s, 1 H), 7.06 (d, J=7.4 Hz, 1 H), 5.37 (s, 2H), 4.78 (s, 2H), 3.65 (s, 3H), 3.59 (s, 2H).

**Step 2: Preparation of C268.** A solution of **C267** (1.58 g, 3.17 mmol) in tetrahydrofuran (15 mL) was treated with 1.0 M aqueous lithium hydroxide (3.8 mL, 3.8 mmol) and was stirred at ambient temperature for 4 hours. The reaction mixture was acidified to pH 2 with 1 N aqueous hydrochloric acid and extracted with ethyl acetate. The organic layer was separated and concentrated in vacuo to afford **C268** as a yellow-tan solid. Yield: 1.58 g, 3.26 mmol, 105%. LCMS *m*/*z* 485.7 (M+H)⁺. ¹H NMR (400 MHz, DMSO-d₆) δ 12.34 (s, 1 H), 10.84 (s, 1H), 8.13 (s, 1H), 7.61 (s, 1H), 7.53 (d, J=7.4 Hz, 1 H), 7.47-7.26 (m, 11 H), 7.03 (d, J=7.4 Hz, 1 H), 6.31 (s, 1 H), 5.33 (s, 2H), 5.02 (s, 2H), 3.30 (s, 2H).

**Steps 3-5: Preparation of C269. C268** was converted to **C269** by methods analogous to those described for Example 35 Step 3 and Example 4, Route 1, Steps 2-3. Chromatography Method A provided **C269.** MS *m*/*z* 736.8 (M)⁺. ¹H NMR (400 MHz, DMSO-d₆) δ 9.29 (d, J=8.6 Hz, 1H), 7.91 (s, 1 H), 7.77-7.71 (m, 1H), 7.65 (s, 1 H), 7.59-7.55 (m, 1H), 7.46 (s, 1 H), 7.25 (t, J=7.8 Hz, 1H), 7.00 (d, J=7.8 Hz, 1 H), 6.85 (s, 1H), 5.17 (dd, J=8.6, 5.6 Hz, 1H), 4.04-3.98 (m, 1 H), 3.58-3.49 (m, 1H), 3.37 (d, J=4.3 Hz, 2H), 3.34-3.27 (m, 1 H), 1.43 (s, 3H), 1.41 (s, 3H).

### Example 57

### 2-({[(1Z)-1-(2-amino-1,3-thiazol-4-yl)-2-{[(2R,3S)-2-({[(5-hydroxy-4-oxo-1,4-dihydropyridin-2-yl)carbamoyl]amino}methyl)-4-oxo-1-sulfoazetidin-3-yl]amino}-2-oxoethylidene]amino}oxy)-2-methylpropanoic acid (C272).

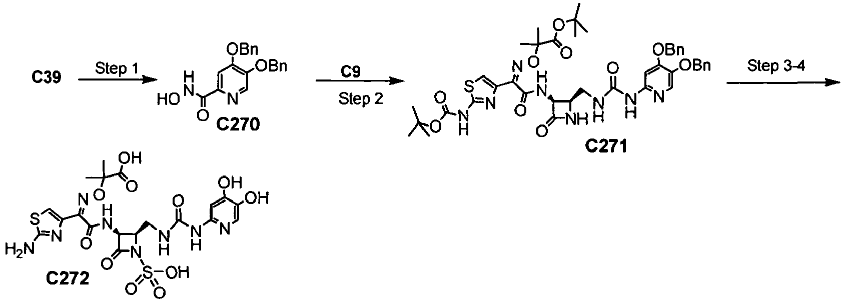

**Step 1: Preparation of C270.** *O*-(7-Azabenzotriazol-1-yl)-N,N,N',N'-tetramethyluronium hexafluorophosphate (10.8 g, 28.3 mmol) was added to a suspension of C39 (7.6 g, 23 mmol), O-(tetrahydro-2H-pyran-2-yl)hydroxylamine (3.32 g, 28.3 mmol), and diisopropylethylamine (8.79 g, 68.0 mmol) in N,N-dimethylformamide (20 mL). After 2 hours the reaction was poured into water (250 mL) and the resulting suspension was stirred for 10 minutes. Methyl *tert*-butyl ether (250 mL) was added and the biphasic suspension was stirred for 2 hours then filtered to collect a white solid. The solid was washed with methyl *tert*-butyl ether (3 x 100 mL). The solid was dissolved in tetrahydrofuran (100 mL), 1 N hydrochloric acid (112 mL) was added and the solution was stirred at room temperature for 2 hours. The reaction was concentrated and the wet residue was partitioned between methyl *tert*-butyl ether (150 mL) and saturated aqueous sodium bicarbonate (150 mL). The resulting suspension was stirred overnight. A white precipitate was collected by filtration and washed with water (3 x 100 mL) then methyl *tert*-butyl ether (3 x 100 mL) to afford **C270** as a white solid. Yield: 7.12 g, 20.32 mmol, 90%. LCMS *m*/*z* 351.4 (M+1). ¹H NMR (400 MHz, DMSO-d₆) δ 8.19 (s, 1 H) 7.64 (s, 1 H) 7.28 - 7.48 (m, 10 H) 5.29 (d, *J*=9.76 Hz, 4 H).

**Step 2: Preparation of C271**. 1,1'-Carbonyldiimidazole (386 mg, 2.31 mmol) was added to a suspension of **C270** (754 mg, 2.15 mmol) in acetonitrile (50 mL). After 75 minutes **C9** (810 mg, 1.54 mmol) was added and the reaction was heated to 60 °C. After 4 hours the reaction was cooled to room temperature and stirred overnight. The reaction was filtered to remove a white solid. The filtrate was concentrated and purified by flash column chromatography on a Biotage SNAP 50 g KP-SIL column (DCM/MeOH = 99:1 - 97:3) to afford **C271** as a white solid. Yield: 0.7 g, 0.815 mmol, 53 %. LCMS *m*/*z* 859.4 (M+1).

**Steps 3-4: Preparation of C272. C271** was converted to **C272** by methods analogous to those described for Example 4, Route 1, Steps 2-3. Reverse phase purification on a C-18 modified column using a 10-40% (0.1% TFA-ACN/ 0.1% TFA-water gradient) afforded **C272** as white solid. LCMS *m*/*z* 603.3 (M+H)⁺. ¹H NMR (400 MHz, DMSO-d₆ + D₂O) δ ppm 7.58 (s, 1 H), 6.73 (s, 1 H), 6.54 (s, 1 H), 5.14 (d, *J*=5.7 Hz, 1 H), 3.95 - 4.18 (m, 1 H), 3.65 (d, *J*=3.1 Hz, 1H), 3.30 (dd, *J*=14.5, 8.5 Hz, 1 H), 1.39 (s, 3H), 1.38 (s, 3H).

### Example 58

### 1-({[(1Z)-1-(2-amino-1,3-thiazol-4-yl)-2-{[(2R,3S)-2-({[(1,5-dihydroxy-4-oxo-1,4-dihydropyridin-2-yl)carbonyl]amino}methyl)-4-oxo-1-sulfoazetidin-3-yl]amino}-2-oxoethylidene]amino}oxy)cyclobutanecarboxylic acid (C274).

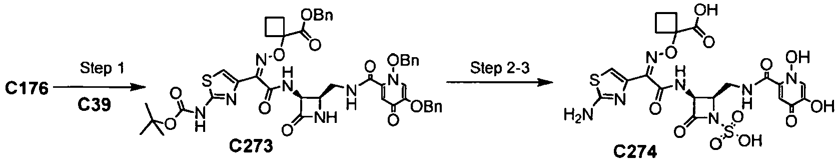

**Step 1: Preparation of C273. C176** and **C39** were converted to **C273** by a method analogous to that described in Example 35, Step 3. Yield: 673.8 mg, 0.74 mmol, 56.8%. LCMS *m*/*z* 907.2 (M+H)⁺.

**Steps 2-3: Preparation of C274. C273** was converted to **C274** by methods analogous to those described for Example 4, Route 1, Steps 2-3. Chromatography Method A provided **C274.** MS *m*/*z* 614.5 (M-1)⁻. ¹H NMR (400 MHz, DMSO-d₆) δ 11.08 (t, J=5.5 Hz, 1 H), 9.36 (d, J=9.0 Hz, 1 H), 7.80 (s, 1 H), 7.56 (s, 1 H), 6.94 (s, 1 H), 5.25 (dd, J=9.2, 5.3 Hz, 1H), 4.12-4.01 (m, 2H), 3.50-3.42 (m, 1H), 2.52-2.41 (m, 2H), 2.35-2.25 (m, 2H), 1.92-1.83 (m, 1H), 1.78-1.71 (m, 1H).

### Example 59

### 1-({[(1Z)-1-(2-amino-1,3-thiazol-4-yl)-2-{[(2R,3S)-2-({[3-({[(1,5-dihydroxy-4-oxo-1,4-dihydropyridin-2-yl)methyl]amino}sulfonyl)benzoyl]amino}methyl)-4-oxo-1-sulfoazetidin-3-yl]amino}-2-oxoethylidene]amino}oxy)cyclobutanecarboxylic acid (C276).

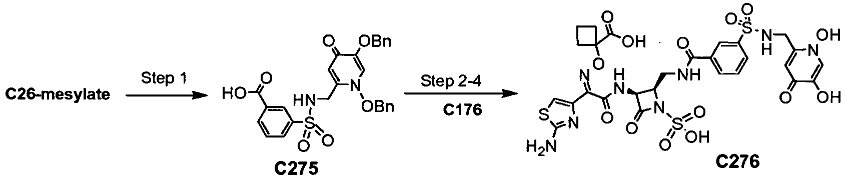

**Step 1: Preparation of C275.** To **C26-mesylate** (equivalent of acid 1.5) (5.23 g, 10.9 mmol) and 3-(chlorosulfonyl)benzoic acid (2.40 g, 10.9 mmol) in acetone (100 ml) and water (20 mL) was added a solution of sodium bicarbonate ((2.31 g, 27.2 mmol) in water (20 mL) at room temperature. The resulting reaction mixture was stirred at room temperature for 7.5 hours. The acetone was removed in vacuo. Dichloromethane (70 mL) and methanol (4 mL) were added and the resulting mixture was stirred at room temperature for 21 hours. 1 M NaOH (15 mL) was slowly added and the mixture was stirred for 1 hour. The organic phase was separated and re-extracted by stirring with 1 M NaOH (15 mL). The combined aqueous phase was washed with dichloromethane (45 mL), and then acidified by the addition of 4 M HCl (5 mL). The suspension was stirred at room temperature for 1 hour. The resulting precipitate was collected by filteration and washed with n-pentane to afford **C275** as a solid. Yield: 2.49 g, 4.78 mmol, 44%. LCMS *m*/*z* 521.1 (M+H)⁺. ¹H NMR (400 MHz, DMSO-*d*₆) δ 8.48 (t, *J*=6.2 Hz, 1H), 8.27 (s, 1H), 8.15 (d, *J*=7.8 Hz, 1 H), 7.99 (s, 1 H), 7.96 (d, *J*=8.0 Hz, 1 H), 7.69 (t, *J*=7.8 Hz, 1 H), 7.26 - 7.48 (m, 10H), 6.03 (s, 1H), 5.17 (s, 2H), 4.96 (s, 2H), 3.86 (d, *J*=6.2 Hz, 2H).

**Steps 2-4: Preparation of C276. C275** was converted to **C276** by methods analogous to those described in Example 39, Step 2 and Example 4, Steps 2-3. Chromatography Method B provided **C276.** LCMS *m*/*z* 785.8 (M+1). ¹H NMR (400 MHz, DMSO-*d*₆) δ 9.42 (d, *J*=8.6 Hz, 1 H), 8.64 (t, *J=* 5.1 Hz, 1 H), 8.54 (dd, *J*= 6.8, 3.6 Hz, 1 H), 8.25 (s, 1 H), 8.07 (s, 1 H), 8.02 (d, *J*= 7.8 Hz, 1 H), 7.93 (d, *J*=7.8 Hz, 1 H), 7.68 (t, *J*=7.8 Hz, 1 H), 7.35 (bs, 2H), 7.12 (s, 1 H), 6.75 (s, 1 H), 5.24 (dd, *J*= 8.6, 5.7 Hz, 1 H), 4.18 (d, *J*=5.1 Hz, 2H), 4.24-4.13 (m, 1H), 3.90-3.81 (m, 1H), 3.56-3.45 (m, 1H), 2.50-2.22 (m, 4H), 1.93-1.69 (m, 2H).

### Example 60

### 1-({[(1Z)-1-(2-amino-1,3-thiazol-4-yl)-2-{[(2R,3S)-2-{[(4-{[(1,5-dihydroxy-4-oxo-1,4-dihydropyridin-2-yl)methyl]amino}-4-oxobutanoyl)amino]methyl}-4-oxo-1-sulfoazetidin-3-yl]amino}-2-oxoethylidene]amino}oxy)cyclobutanecarboxylic acid (C277).

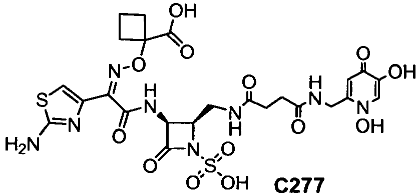

**C277** was prepared by methods analogous to those described in Example 42. Chromatography Method B provided **C277.** LCMS *m*/*z* 701.8 (M+H)⁺. ¹H NMR (400 MHz, DMSO-*d*₆) δ 9.31 (d, *J*=8.6 Hz, 1 H), 8.56 (t, *J*=5.9 Hz, 1 H), 8.12 (s, 1 H), 7.47 (dd, *J*= 6.3, 4.5 Hz, 1H), 7.30 (bs, 2H), 6.89 (s, 1H), 6.75 (s, 1H), 5.14 (dd, *J*=8.6, 5.6 Hz, 1 H), 4.37 (d, *J*=6.3 Hz, 2H), 3.97 (dt, *J*=7.8, 5.6 Hz, 1 H), 3.56-3.47 (m, 1 H), 3.41-3.32 (m, 1 H), 2.54-2.21 (m, 4H), 2.42 (t, *J*=7.7 Hz, 2H), 2.30 (t, *J*=7.7 Hz, 2H), 1.93-1.70 (m, 2H).

### Example 61

### 1-({[(1Z)-1-(2-amino-1,3-thiazol-4-yl)-2-{[(2R,3S)-2-({[4-({[(4,5-dihydroxypyridin-2-yl)carbonyl]amino}methyl)benzoyl]amino}methyl)-4-oxo-1-sulfoazetidin-3-yl]amino}-2-oxoethylidene]amino}oxy)cyclobutanecarboxylic acid (C278).

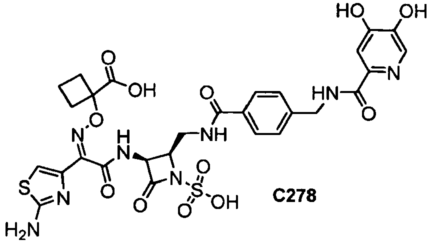

**C278** was prepared by methods analogous to those described in Example 51. Chromatography Method B provided **C278.** LCMS *m*/*z* 733.8 (M+H)⁺. ¹H NMR (400 MHz, DMSO-*d*₆) δ 9.38 (d, *J*=8.6 Hz, 1H), 8.29-8.24 (m, 1 H), 7.93 (s, 1 H), 7.73 (d, *J*=8.4 Hz, 2H), 7.57 (s, 1 H), 7.36 (d, *J*=8.4 Hz, 2H), 6.76 (s, 1 H), 5.22 (dd, *J*=8.6, 5.3 Hz, 1 H), 4.49 (d, *J*=6.2 Hz, 2H), 4.10 (dt, *J*=9.2, 4.2 Hz, 1 H), 3.90-3.81 (m, 1 H), 3.47-3.39 (m, 1 H), 2.52-2.22 (m, 4H), 1.92-1.69 (m, 2H).

### Example 62

### 1-({[(1Z)-1-(2-amino-1,3-thiazol-4-yl)-2-{[(2R,3S)-2-({[4-({[(1,5-dihydroxy-4-oxo-1,4-dihydropyridin-2-yl)carbonyl]amino}methyl)benzoyl]amino}methyl)-4-oxo-1-sulfoazetidin-3-yl]amino}-2-oxoethylidene]amino}oxy)cyclobutanecarboxylic acid (C280).

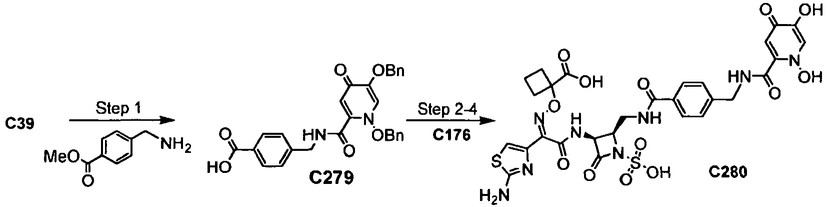

**Step 1: Preparation of C279.** To a suspension of **C39** (1.47 g, 4.2 mmol) in dimethylacetamide (6 mL) was added 1,1'- carbonyldiimidazole (0.68 g, 4.2 mmol). The resulting reaction mixture was stirred at room temperature for 4 hours. A suspension of 4-(aminomethyl)benzoic acid (650 mg, 4.2 mmol) in dimethylacetamide (4 mL) was added. The resulting reaction mixture was stirred at room temperature for 44 hours. The reaction mixture was diluted with citric acid solution (a solution of 7.5 g of citric acid monohydrate in 100 mL of water, 15 mL). A precipitate formed in the aqueous phase, which was collected by filteration and washed with citric acid solution (5 mL) to afford 1.5 g of crude product. The crude product was triturated with dichloromethane / methanol (9.5/0.5, 10 mL), collected by filteration and washed with n-pentane to provide **C279** as a white solid. Yield: 1.3 g, 2.7 mmol, 64%. LCMS *m*/*z* 485.1 (M+H)⁺. ¹H NMR (400 MHz, DMSO-d₆) δ 9.50 (t, *J*=6.0 Hz, 1 H), 8.12 (s, 1H), 7.83 (d, *J*=8.2 Hz, 2H), 7.31 - 7.48 (m, 12H), 6.24 (s, 1H), 5.31 (s, 2H), 5.03 (s, 2H), 4.50 (d, *J*=6.0 Hz, 2H).

**Steps 2-4: Preparation of C280. C279** was converted to **C280** by methods analogous to those described in Example 39, Step 2 and Example 4, Steps 2-3. Chromatography Method B provided **C280.** LCMS *m*/*z* 749.8 (M+H)⁺. ¹H NMR (400 MHz, DMSO-*d*₆) δ 12.05 (bs, 1 H), 11.94 (bs, 1H), 10.84 (bs, 2H), 9.40 (d, *J*=8.6 Hz, 1H), 8.28-8.23 (m, 1 H), 7.88 (d, *J*=8.4 Hz, 1 H), 7.81 (m, 1H), 7.74 (d, *J*=8.4 Hz, 1H), 7.55 (d, *J=* 1.6 Hz, 1H), 7.39 (d, *J*=8.4 Hz, 1H), 7.36 (d, *J*=8.4 Hz, 1H), 6.82-6.78 (m, 1H), 5.23 (dd, *J*=8.6, 5.7 Hz, 1 H), 4.60-4.53 (m, 2H), 4.14-4.08 (m, 1 H), 3.87 (m, 1 H), 3.51-3.42 (m, 1 H), 2.52-2.23 (m, 4H), 1.92-1.72 (m, 2H).

### Example 63

### 1-({[(1Z)-1-(2-amino-1,3-thiazol-4-yl)-2-{[(2R,3S)-2-({[(Z)-(cyanoimino){[(1,5-dihydroxy-4-oxo-1,4-dihydropyridin-2-yl)methyl]amino}methyl]amino}methyl)-4-oxo-1-sulfoazetidin-3-yl]amino}-2-oxoethylidene]amino}oxy)cyclobutanecarboxylic acid (C283).

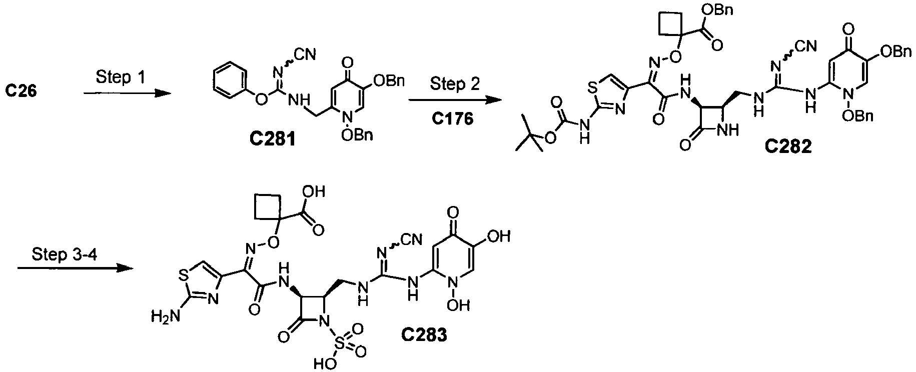

**Step 1: Preparation of C281.** A solution of **C26** (1.68 g, 5 mmol) and diphenylcyano carbonimidate (1.19 g, 5 mmol) in dry dichloromethane (25 mL) was stirred at ambient temperature for 1 hour. The resulting product **(C281)** was collected by filteration, washed with a minimal amount of dichloromethane and air-dried. Concentration of the filtrate and trituration with methyl tert-butyl ether produced additional **C281** which was combined with the previously isolated product. Yield 2.03 g, 4.23 mmol, 84.7%. LCMS *m*/*z* 481.8 (M+H)⁺. ¹H NMR (400 MHz, DMSO-d₆) δ 9.30 (br s, 0.5 H), 8.77 (br s, 0.5 H), 8.00 - 8.07 (m, 1 H), 6.95 - 7.52 (m, 15 H), 6.01 (br s, 0.5 H), 5.93 (br s, 0.5 H), 5.24 (s, 2 H), 4.99 (d, *J*=6.63 Hz, 2 H), 4.34 (d, *J*=18.73 Hz, 2 H).

**Step 2: Preparation of C282.** A mixture of **C281** (0.48 g, 1 mmol), **C176** (0.57 g, 1 mmol), N,N-diisopropylethylamine (131 mg, 0.175 mL, 1 mmol) and DMAP (0.12 g, 1 mmol) in acetonitrile (15 mL) was stirred at 40 °C for 72 hours. An additional amount of **C176** (0.57 g, 1 mmol) was added and heating was continued for 72 hours at 44 °C. The solvent was removed in vacuo and **C282** was purified by flash chromatography on silica gel. Yield 0.57 g, 0.59 mmol, 59.4%. LCMS *m*/*z* 960.4 (M+H)⁺.

**Steps 3-4: Preparation of C283. C282** was converted to **C283** by methods analogous to those described in Example 4, Route 1, Steps 2-3. Chromatography Method A provided **C283.** LCMS *m*/*z* 667.3 (M-H)⁻. ¹H NMR (400 MHz, DMSO-*d*₆) δ 12.34-12.99 (m, 1 H), 10.33-10.98 (m, 1 H), 9.27 (d, *J*=8.8 Hz, 1 H), 8.03 (s, 1 H), 7.67 (t, *J*=6.2 Hz, 1 H), 7.44-7.52 (m, 1 H), 7.22-7.37 (m, 2H), 6.88 (s, 1 H), 6.70 (s, 1 H), 5.19 (dd, *J*=8.2, 2.2 Hz, 1 H), 4.06-4.13 (m, 1 H), 3.44-3.62 (assumed, 2H; obscured by water), 2.33-2.46 (m, 2H), 2.19-2.33 (m, 2H), 1.71-1.93 (m, 2H).

### Example 64

### 1-({[(1Z)-1-(2-amino-1,3-thiazol-4-yl)-2-{[(2R,3S)-2-{[(3-{[(1,5-dihydroxy-4-oxo-1,4-dihydropyridin-2-yl)methyl]carbamoyl}benzoyl)amino]methyl}-4-oxo-1-sulfoazetidin-3-yl]amino}-2-oxoethylidene]amino}oxy)cyclobutanecarboxylic acid (C284).

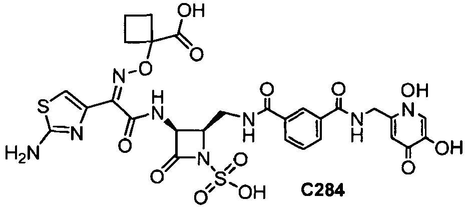

**C284** was prepared by methods analogous to those described in Example 44 providing an off white solid. Chromatography Method A provided **C284.** MS *m*/*z* 748.6 (M)⁺. ¹H NMR (400 MHz, DMSO-d₆) δ 9.41 (d, *J*=8.6 Hz, 1 H), 9.32 (t, *J*=5.5 Hz, 1 H), 8.46-8.41 (m, 1H), 8.39 (s, 1H), 8.14 (s, 1H), 8.04 (d, *J*=7.2 Hz, 1H), 7.97 (d, *J*=7.2 Hz, 1 H), 7.60 (t, *J*=7.7 Hz, 1H), 7.40-7.17 (br s, 2H), 6.93 (s, 1H), 6.75 (s, 1 H), 5.25 (dd, *J*=8.6, 5.6 Hz, 1 H), 4.59 (d, *J*= 5.8 Hz, 2H), 4.18-4.13 (m, 1 H), 3.92-3.86 (m, 1 H), 3.53-3.48 (m, 1H), 2.50-2.45 (m, 1H), 2.40-2.25 (m, 3H), 1.94-1-84 (m, 1H), 1.82-1.73 (m, 1H).

### Example 65

### 1-({[(1Z)-1-{2-amino-1,3-thiazol-4-yl)-2-{[(2R,3S)-2-({[{[(1,5-dihydroxy-4-oxo-1,4-dihydropyridin-2-yl)methyl]amino}(oxo)acetyl]amino}methyl)-4-oxo-1-sulfoazetidin-3-yl]amino}-2-oxoethylidene]amino}oxy)cyclobutanecarboxylic acid (C286).

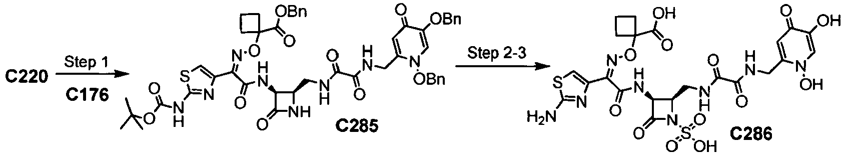

**Step 1: Preparation of C285.** A solution of **C176** (750 mg, 1.31 mmol) and **C220** (535 mg, 1.31 mmol) in anhydrous dimethylformamide (5 mL) was treated with N-[(dimethylamino)(3H-[1,2,3]triazolo[4,5-b]pyridin-3-yloxy)methylene]-N-methylmethanaminium hexafluorophosphate (548 mg, 1.44 mmol) and pyridine hydrochloride (159 mg, 1.38 mmol). The resulting mixture was stirred at ambient temperature overnight. The mixture was diluted with ethyl acetate and washed three times with water and once with brine. The organic layer was concentrated in vacuo onto silica gel. Chromatography on silica gel using a methylene chloride-methanol gradient afforded **C285** as an orange foam. Yield: 717.7 mg, 0.75 mmol, 56.9%. LCMS *m*/*z* 963.4 (M+H)⁺.

**Steps 2-3: Preparation of C286. C285** was converted to **C286** by methods analogous to those described in Example 4, Route 1, Steps 2-3 to provide an off white solid. Chromatography Method A provided **C286.** MS m/z 672.7 (M)⁺. ¹H NMR (400 MHz, DMSO-d₆) δ 9.43 (t, J=5.8 Hz, 1 H), 9.33 (d, J=8.8 Hz, 1 H), 8.82-8.79 (m, 1 H), 8.14 (s, 1 H), 7.49-7.19 (br s, 2H), 6.89 (s, 1 H), 6.74 (s, 1 H), 5.23 (dd, J=8.6, 5.5 Hz, 1 H), 4.46 (d, J=6.0 Hz, 2H), 4.06-4.01 (m , 1 H), 3.81-3.75 (m, 1 H), 3.43-3.35 (m, 1 H), 2.47-2.24 (m, 4H), 1.93 (m, 1 H), 1.83-1.73 (m, 1 H).

### Example 66

### 1-({[(1Z)-1-(2-amino-1,3-thiazol-4-yl)-2-{[(2R,3S)-2-({[(1,5-dihydroxy-4-oxo-1,4-dihydropyridin-2-yl)carbonyl]amino}methyl)-4-oxo-1-sulfoazetidin-3-yl]amino}-2-oxoethylidene]amino}oxy)cyclopentanecarboxylic acid (C289).

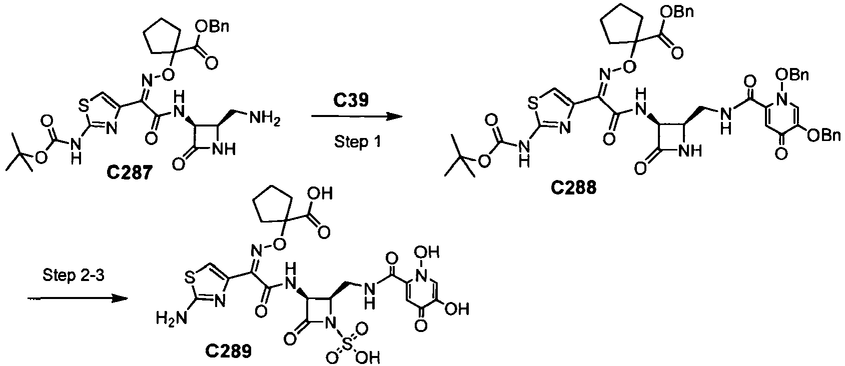

**Step 1: Preparation of C288. C287** was prepared by methods analogous to those described for Example 30, Route 1, Steps 1-9. **C287** and **C39** were converted to **C288** by a method analogous to that described for Example 35, Step 3. Yield: 525.1 mg, 0.57 mmol, 44.7%. LCMS *m*/*z* 920.5 (M+H)⁺.

**Steps 2-3: Preparation of C289. C288** was converted to **C289** by methods analogous to those described in Example 4, Route 1, Steps 2-3 to provide an off-white solid. Chromatography Method A provided **C289.** MS *m*/*z* 628.5 (M-H)⁻. ¹H NMR (400 MHz, DMSO-d₆) δ 11.18 (t, J=5.2 Hz, 1H), 9.23 (d, J=9.0 Hz, 1H), 7.81 (s, 1H), 7.55 (s, 1H), 6.92 (s, 1 H), 5.22 (dd, J=9.0, 5.5 Hz, 1 H), 4.05-3.93 (m, 2H), 3.49-3.42 (m, 1 H), 2.06-1.89 (m, 4H), 1.76-1.52 (m, 4H).

### Example 67

### 2-({[(1Z)-1-(2-amino-1,3-thiazol-4-yl)-2-{[(2R,3S)-2-{[(3-{[(1,5-dihydroxy-4-oxo-1,4-dihydropyridin-2-yl)carbonyl]amino}benzoyl)amino]methyl}-4-oxo-1-sulfoazetidin-3-yl]amino}-2-oxoethylidene]amino}oxy)-2-methylpropanoic acid (C290).

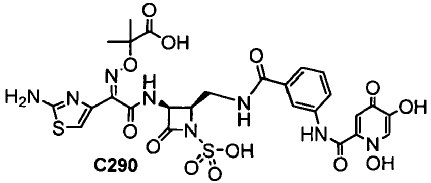

**C290** was prepared by methods analogous to those described in Example 36. Chromatography Method A provided **C290.** LCMS *m*/*z* 723.3 (M+1)⁺. ¹H NMR (400 MHz, DMSO-*d*₆) δ 9.38 (d, *J*=8.6 Hz, 1 H), 8.40-8.35 (m, 1 H), 8.05-8.03 (m, 1 H), 7.92 (s, 1 H), 7.88-7.86 (m, 1 H), 7.68 (s, 1 H), 7.55 - 7.51 (m, 1 H), 7.44 (t, *J*=7.8 Hz, 1 H), 6.81 (s, 1 H), 5.24 (dd, *J*=8.6, 5.8 Hz, 1 H), 4.18-4.08 (m, 1 H), 3.91-3.79 (m, 1 H), 3.50-3.35 (m, 1 H), 1.45 (s, 3H), 1.43 (s, 3H).

### Example 68

### 2-({[(1Z)-1-(2-amino-1,3-thiazol-4-yl)-2-{[(2R,3S)-2-({[(3-{[(1,5-dihydroxy-4-oxo-1,4-dihydropyridin-2-yl)methyl]carbamoyl}phenyl)sulfonyl]amino}methyl)-4-oxo-1-sulfoazetidin-3-yl]amino}-2-oxoethylidene]amino}oxy)-2-methylpropanoic acid (C293).

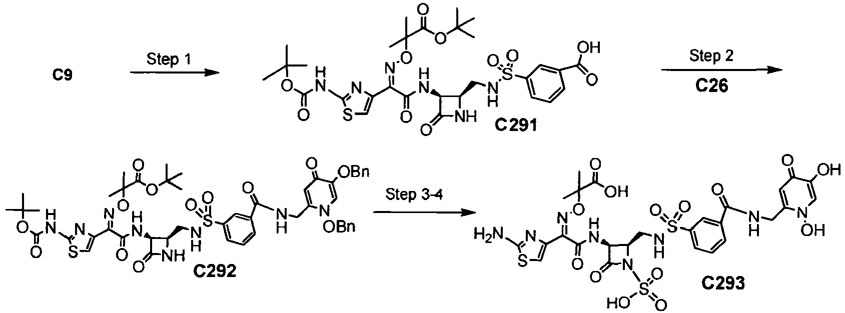

**Step 1: Preparation of C291.** A solution of **C9** (500 mg, 0.95 mmol) in acetonitrile (5 mL) was treated with a solution of sodium bicarbonate (410 mg, 4.87 mmol) in water (5 mL). To the resulting suspension was added 3-(chlorosulfonyl)benzoic acid (220 mg, 0.95 mmol). The resulting mixture stirred at ambient temperature for 30 minutes. The reaction mixture was concentrated in vacuo to afford **C291** as an impure solid. Yield: 674 mg, 0.95 mmol, quantitative. LCMS *m*/*z* 711.3 (M+1)⁺.

**Step 2: Preparation of C292.** A solution of crude **C291** (674mg, 0.948mmol), **C26** (319mg, 0.948mmol), N-[(dimethylamino)(3H-[1,2,3]triazolo[4,5-b]pyridin-3-yloxy)methylene]-N-methylmethanaminium hexafluorophosphate (379 mg, 0.948 mmol), and triethylamine (99 mg, 0.948 mmol) in dichloromethane (15 mL) was stirred at ambient temperature for 2 hours. The reaction mixture was quenched with saturated aqueous sodium bicarbonate and extracted (3x) with dichloromethane. The combined organic layers were concentrated in vacuo to afford a red foam. The crude product was purified using a Phenomenex HILIC (Diol) 250 x 21.2mm 5µ column with 5% ethanol in heptanes for 1.5 minutes; 5-100% ethanol in heptanes for 8.5 minutes; holding at 100% ethanol for 1 minute; and then decreasing from 100% to 5% ethanol over an additional 1.5 minutes, for a total of 12.5 minutes. The solvents were modified with 0.1% formic acid and run at a rate of 28.0 mL/minute) to afford **C292** as a light peach-colored solid. Yield: 120 mg, 0.10 mmol, 10%. LCMS *m*/*z* 1029.8 (M+1)⁺.

**Steps 3-4: Preparation of C293. C292** was converted to **C293** by methods analogous to those described in Example 4, Route 1, Steps 2-3 to provide the product as a light yellow solid. Chromatography Method A provided **C293.** LCMS *m*/*z* 773.2 (M+1)⁺. ¹H NMR (400 MHz, DMSO-*d*₆) δ 9.40 (t, J=5.7 Hz, 1 H), 9.19 (d, *J*=8.8 Hz, 1 H), 8.33 (br s, 1H), 8.19 (s, 1H), 8.17 (d, *J*=8.2 Hz, 1H), 7.99 (d, *J*=8.2 Hz, 1H), 7.76 (t, *J*=7.8 Hz, 1 H), 7.43-7.21 (br s, 2H), 7.40 (dd, *J*=7.4, 3.5 Hz, 1 H), 7.00 (s, 1 H), 6.75 (s, 1 H), 5.17 (dd, *J*=8.8, 5.6 Hz, 1 H), 4.62 (d, *J*=5.46 Hz, 2H), 3.94-3.87 (m, 1 H), 3.32 - 3.16 (m, 2H), 1.36 (s, 6H).

### Example 69

### ({[(1Z)-1-(2-amino-1,3-thiazol-4-yl)-2-({(2R,3S)-2-[({[(1,5-dihydroxy-4-oxo-1,4-dihydropyridin-2-yl)methyl]carbamoyl}amino)methyl]-4-oxo-1-sulfoazetidin-3-yl}amino)-2-oxoethylidene]amino}oxy)acetic acid (C296).

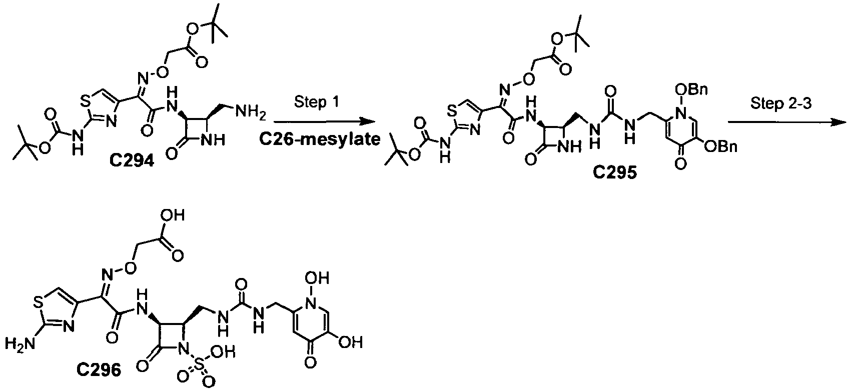

**Step 1: Preparation of C295. C294** was prepared by methods analogous to those described for C176 in Example 30, Route 1 was subsequently converted to **C295** by a method analogous to that described in Example 33, Step 6. Yield: 0.80 g, 0.93 mmol, 58%. LCMS *m*/*z* 861.5 (M+H)⁺.

**Steps 2-3: Preparation of C296. C295** was converted to **C296** by methods analogous to those described in Example 4, Route 1, Steps 2-3. Chromatography Method B provided **C296.** LCMS *m*/*z* 605.2 (M+H)⁺. ¹H NMR (400 MHz, DMSO-*d*₆) δ 9.32 (d, *J*= 8.6 Hz, 1H), 8.14 (s, 1 H), 7.28 (br s, 2H), 7.19 (t, *J*=5.1 Hz, 1H), 6.97 (s, 1H), 6.81 (s, 1H), 6.40-6.32 (m, 1H), 5.20 (dd, *J*=8.6, 5.5 Hz, 1H), 4.59 (AB quartet, *J*_{AB}=16.6 Hz, Δν_{AB}=21.2 Hz, 2H), 4.32 (d, *J*=5.1 Hz, 2H), 3.98-3.92 (m, 1 H), 3.67-3.58 (m, 1 H), 3.26-3.16 (m, 1H).

### Example 70

### 2-(((1-(2-aminothiazol-4-yl)-2-(((2S,3R)-2-((3-((1,5-dihydroxy-4-oxo-1,4-dihydropyridin-2-yl)methyl)ureido)methyl)-4-oxo-1-sulfoazetidin-3-yl)amino)-2-oxoethylidene)amino)oxy)-2-methylpropanoic acid

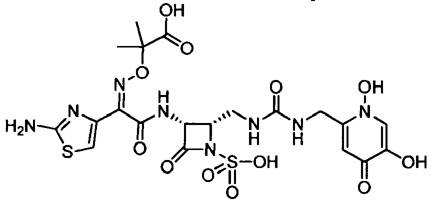

The Title compound was prepared using methods described herein.

### BIOLOGICAL EXAMPLES

In order to assess the compounds biological activity, the *in vitro* antibacterial activity of selected compounds described in the Examples 1-69 was evaluated by minimum inhibitory concentration (MIC) testing according to Clinical and Laboratory Standards Institute (CLSI, formerly NCCLS) guidelines. See: Clinical and Laboratory Standards Institute. Methods for Dilution Antimicrobial Susceptibility Tests for Bacteria that Grow Aerobically; Approved Standard-Eighth Edition. CLSI document M7-A8 [ISBN 1-56238-689-1]. Clinical and Laboratory Standards Institute, 940 West Valley Road, Suite 1400, Wayne, Pennsylvania 19087-1898 USA, 2006; also Clinical and Laboratory Standards Institute. Performance Standards for Antimicrobial Susceptibility Testing; Twentieth Informational Supplement. CLSI document M100-S20 [ISBN1-56238-716-2].Clinical and Laboratory Standards Institute.

The MIC determination is a standard laboratory method for evaluating the antibacterial activity of a compound. The MIC represents the lowest drug concentration that inhibits visible growth of bacteria following overnight incubation. In order to determine the MIC value, a range of drug concentrations (e.g. 0.06 µg/mL to 64 µg/mL) are incubated with a defined strain of bacteria. Typically, the drug concentration range is broken down into 2-fold increments (e.g. 0.06 µg/mL, 0.12 µg/mL. 0.25 µg/mL, 0.50 µg/mL, 1.0 µg/mL, etc.) and the various drug concentrations are all individually incubated overnight with approximately the same number of bacteria. The MIC is then determined by visually inspecting the drug effect at each concentration, and identifying the lowest drug concentration that has inhibited bacterial growth as compared to the drug free control. Typically, bacteria continue to grow at drug concentrations lower than the MIC and don't grow at concentrations at and above the MIC.

The MIC values described in Table 1 below were derived from assays wherein each test compound was evaluated in duplicate. In cases where the duplicate values varied by 0 - 2-fold, the lower of the two values was reported below. Generally speaking, if the duplicate values varied by more than 2-fold, the assay was considered non-valid and was repeated until the variation between duplicate runs was ≤ 2-fold. In line with the CLSI guidelines referred to above, both control organisms and reference compounds were utilized in each MIC assay to provide proper quality control. MIC values generated with these control organisms and reference compounds were required to fall within a defined range for the assay to be considered valid and be included herein. Those skilled in the art will recognize that MIC values can and do vary from experiment to experiment. Generally speaking, it should be recognized that MIC values often vary +/- 2-fold from experiment to experiment. While a single MIC is reported for each compound and each microorganism, the reader should not conclude that each compound was only tested once. Several of the compounds were subjected to multiple tests. The data reported in Table 1 is reflective of the compounds relative activity and different MICs may have been generated on these occasions in light with the guidelines described above.

The following bacterial strains were used in these MIC determinations:
1) *Pseudomonas aeruginosa* UC-12120: Wild-type, labeled as 1091-05;
*2) Klebsiella pneumoniae*: Ciprofloxacin-resistant isolate, expresses extended-spectrum beta-lactamases (ESBL), clinical isolate, labeled as 1000-02;
*3) Acinetobacter baumannii*/*haemolyticus*: Multidrug-resistant clinical isolate, labeled as AB-3167.

The following results (Table 1) were obtained with the final products described in Examples 1-69.

**TABLE 1**

| **Example Number** | **AB-MBT:Pseudomonas aeruginosa 1091-05:MIC (MstRcnt)** | **AB-MBT:Klebsiella pneumoniae 1000-02:MIC (MstRcnt)** | **AB-MBT:Acinetobacter baumanii AB-3167:MIC (MstRcnt)** |
|---|---|---|---|
| 1 | 0.250 ug/mL | 0.250 ug/mL | 0.500 ug/mL |
| 2 | 2.00 ug/mL | 8.00 ug/mL | 8.00 ug/mL |
| 3 | 0.125 ug/mL | 0.250 ug/mL | 0.500 ug/mL |
| 4 | 0.125 ug/mL | 0.125 ug/mL | 0.500 ug/mL |
| 5 | 0.125 ug/mL | 4.00 ug/mL | 1.00 ug/mL |
| 6 | | 8.00 ug/mL | 1.00 ug/mL |
| 7 | 0.125 ug/mL | 8.00 ug/mL | 0.500 ug/mL |
| 8 | 1.00 ug/mL | 4.00 ug/mL | 1.00 ug/mL |
| 9 | 4.00 ug/mL | 4.00 ug/mL | 2.00 ug/mL |
| 10 | 0.125 ug/mL | 0.500 ug/mL | 0.250 ug/mL |
| 11 | 0.500 ug/mL | 0.0600 ug/mL | 1.00 ug/mL |
| 12 | 4.00 ug/mL | 0.250 ug/mL | 16.0 ug/mL |
| 13 | 0.250 ug/mL | 0.125 ug/mL | 0.500 ug/mL |
| 14 | 1.00 ug/mL | 0.500 ug/mL | 0.500 ug/mL |
| 15 | 0.250 ug/mL | 1.00 ug/mL | 0.250 ug/mL |
| 16 | 0.250 ug/mL | 0.500 ug/mL | 1.00 ug/mL |
| 17 | 0.125 ug/mL | 1.00 ug/mL | 1.00 ug/mL |
| 18 | 0.250 ug/mL | 0.500 ug/mL | 1.00 ug/mL |
| 19 | 0.500 ug/mL | 1.00 ug/mL | 1.00 ug/mL |
| 20 | 1.00 ug/mL | 0.500 ug/mL | 1.00 ug/mL |
| 21 | 8.00 ug/mL | 64.0 ug/mL | >64.0 ug/mL |
| 22 | 4.00 ug/mL | 32.0 ug/mL | 16.0 ug/mL |
| 23 | 1.00 ug/mL | 0.250 ug/mL | 0.250 ug/mL |
| 24 | 1.00 ug/mL | 16.0 ug/mL | 2.00 ug/mL |
| 26 | 0.125 ug/mL | 0.125 ug/mL | 0.250 ug/mL |
| 27 | 1.00 ug/mL | 1.00 ug/mL | 2.00 ug/mL |
| 28 | 1.00 ug/mL | 0.500 ug/mL | 1.00 ug/mL |
| 29 | 0.250 ug/mL | 1.00 ug/mL | 0.500 ug/mL |
| 30 | 0.125 ug/mL | 0.125 ug/mL | 0.250 ug/mL |
| 31 | 1.00 ug/mL | 0.500 ug/mL | 0.500 ug/mL |
| 32 | 1.00 ug/mL | 1.00 ug/mL | 1.00 ug/mL |
| 33 | 0.250 ug/mL | 2.00 ug/mL | 0.500 ug/mL |
| 34 | 0.125 ug/mL | 0.500 ug/mL | 0.250 ug/mL |
| 35 | 4.00 ug/mL | 0.250 ug/mL | 0.500 ug/mL |
| 36 | 0.125 ug/mL | 1.00 ug/mL | 0.250 ug/mL |
| 37 | 1.00 ug/mL | 4.00 ug/mL | 2.00 ug/mL |
| 38 | 2.0 ug/mL | 0.5 ug/mL | 0.5 ug/mL |
| 39 | 0.250 ug/mL | 1.00 ug/mL | 0.500 ug/mL |
| 40 | 0.250 ug/mL | 0.500 ug/mL | 0.250 ug/mL |
| 41 | 0.0600 ug/mL | 0.500 ug/mL | 0.250 ug/mL |
| 42 | 0.125 ug/mL | 0.500 ug/mL | 0.125 ug/mL |
| 43 | 0.125 ug/mL | 0.500 ug/mL | 0.250 ug/mL |
| 44 | 0.250 ug/mL | 0.250 ug/mL | 0.250 ug/mL |
| 45 | 0.500 ug/mL | 4.00 ug/mL | 0.500 ug/mL |
| 46 | 0.250 ug/mL | 2.00 ug/mL | 0.500 ug/mL |
| 47 | 0.125 ug/mL | 0.250 ug/mL | 0.250 ug/mL |
| 48 | 1.00 ug/mL | 1.00 ug/mL | 2.00 ug/mL |
| 49 | 0.500 ug/mL | 0.500 ug/mL | 0.500 ug/mL |
| 50 | 0.125 ug/mL | 0.500 ug/mL | 0.500 ug/mL |
| 51 | 0.125 ug/mL | 2.00 ug/mL | 1.00 ug/mL |
| 52 | 0.500 ug/mL | 1.00 ug/mL | 0.500 ug/mL |
| 53 | 0.250 ug/mL | 1.00 ug/mL | 0.250 ug/mL |
| 54 | 0.250 ug/mL | 1.00 ug/mL | 0.250 ug/mL |
| 55 | 0.250 ug/mL | 2.00 ug/mL | 0.500 ug/mL |
| 56 | 0.500 ug/mL | 1.000 ug/mL | 1.000 ug/mL |
| 57 | 2.00 ug/mL | 4.00 ug/mL | 1.00 ug/mL |
| 58 | 0.250 ug/mL | 0.0600 ug/mL | 0.250 ug/mL |
| 59 | 0.250 ug/mL | 0.250 ug/mL | 0.250 ug/mL |
| 60 | 0.125 ug/mL | 0.500 ug/mL | 0.250 ug/mL |
| 61 | 32.0 ug/mL | 2.00 ug/mL | 1.00 ug/mL |
| 62 | 2.00 ug/mL | 1.00 ug/mL | 0.500 ug/mL |
| 63 | 0.250 ug/mL | 1.00 ug/mL | 0.500 ug/mL |
| 64 | 1.00 ug/mL | 0.250 ug/mL | 0.500 ug/mL |
| 65 | 0.0600 ug/mL | 0.250 ug/mL | 0.125 ug/mL |
| 66 | 0.500 ug/mL | 0.125 ug/mL | 0.500 ug/mL |
| 67 | 4.00 ug/mL | 4.00 ug/mL | 2.00 ug/mL |
| 68 | 4.00 ug/mL | 1.00 ug/mL | 4.00 ug/mL |
| 69 | 0.25 ug/mL | 1.00 ug/mL | 0.25 ug/mL |

## Claims

1. A compound of Formula (I): 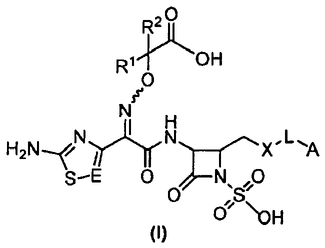 or a pharmaceutically acceptable salt thereof,
wherein
R¹ and R² are each independently hydrogen, optionally substituted (C₁-C₆)alkyl, or phenyl(C₁-C₆)alkyl wherein the phenyl and the (C₁-C₆)alkyl moieties of the phenyl(C₁-C₆)alkyl are optionally substituted; or
R¹ and R² together, with the carbon atom to which they are attached, form an optionally substituted (C₃-C₆)cycloalkyl or an optionally substituted 4-6-membered heterocycle;
E is C(H), C(F), C(CI), or N;
X is -O-C(=O)-, -NH-C(=O)-, -NH-SO₂-, -NH-C(=N-CN)-, -NH-T-, or triazole;
L is absent, -(CH₂)ₚ-, -(CH₂)ₚ-NH-(CH₂)_{q}-, -(CH₂)ₚ-O-(CH₂)_{q}-, -(CH₂)ₚ-C(=O)-NH-(CH₂)_{q}-, -(CH₂)ₚ-NH-C(=O)-(CH₂)_{q}-, -CH(CH₃)-NH-C(=O)-(CH₂)_{q}-, -(CH₂)ₚ-NH-C(=O)-NH-(CH₂)_{q}-, -CH(CH₃)-NH-C(=O)-NH-(CH₂)_{q}-, -(CH₂)ₚ-T-SO₂-NH-(CH₂)_{q}-, -(CH₂)ₚ-T-(CH₂)_{q}, -(CH₂)ₚ-T-C(=O)-NH-(CH₂)_{q}-, -(CH₂)ₚ-T-(CH₂)_{q}-NH-C(=O)-, -NH-(CH₂)ₚ-T-, -O-(CH₂)ₚ-T-, -(CH₂)ₚ-Y-C(=O)-(CH₂)_{q}-, or -(CH₂)ₚ-Y-(CH₂)_{q}-;
T is an optionally substituted phenyl or an optionally substituted 5- or 6-membered heteroaryl;
Y is an optionally substituted 4-6 membered heterocycle;
p and q are each independently 0, 1, 2, or 3;
A is 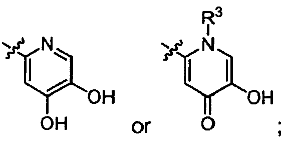 and
R³ is hydrogen, (C₁-C₃)alkyl, or OH;
provided that Formula (I) does not include 2-(((1-(2-aminothiazol-4-yl)-2-(((2S,3R)-2-((3-((1,5-dihydroxy-4-oxo-1,4-dihydropyridin-2-yl)methyl)ureido)methyl)-4-oxo-1-sulfoazetidin-3-yl)amino)-2-oxoethylidene)amino)oxy)-2-methylpropanoic acid.

2. A compound according to claim 1 of Formula (IA): 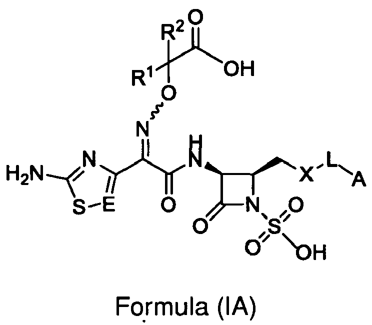 wherein
R¹ and R² are each independently hydrogen, optionally substituted (C₁-C₆)alkyl, or phenyl(C₁-C₆)alkyl wherein the phenyl and the (C₁-C₆)alkyl moieties of the phenyl(C₁-C₆)alkyl are optionally substituted; or
R¹ and R² together, with the carbon atom to which they are attached, form an optionally substituted (C₃-C₆)cycloalkyl or an optionally substituted 4-6-membered heterocycle;
E is C(H), C(F), C(CI), or N;
X is -O-C(=O)-, -NH-C(=O)-, -NH-SO₂-, -NH-C(=N-CN)-, -NH-T-, or triazole;
L is absent, -(CH₂)ₚ-, -(CH₂)ₚ-NH-(CH₂)_{q}-, -(CH₂)ₚ-O-(CH₂)_{q}-, -(CH₂)ₚ-C(=O)-NH-(CH₂)_{q}-, -(CH₂)ₚ-NH-C(=O)-(CH₂)_{q}-, -CH(CH₃)-NH-C(=O)-(CH₂)_{q}-, -(CH₂)ₚ-NH-C(=O)-NH-(CH₂)_{q}-, -CH(CH₃)-NH-C(=O)-NH-(CH₂)_{q}-, -(CH₂)ₚ-T-SO₂-NH-(CH₂)_{q}-, -(CH₂)ₚ-T-(CH₂)_{q}, -(CH₂)ₚ-T-C(=O)-NH-(CH₂)_{q}-, -(CH₂)ₚ-T-(CH₂)_{q}-NH-C(=O)-, -NH-(CH₂)ₚ-T-, -O-(CH₂)ₚ-T-, -(CH₂)ₚ-Y-C(=O)-(CH₂)_{q}-, or -(CH₂)ₚ-Y-(CH₂)_{q}-;
T is an optionally substituted phenyl or an optionally substituted 5- or 6-membered heteroaryl;
Y is an optionally substituted 4-6 membered heterocycle;
p and q are each independently 0, 1, 2, or 3;
A is 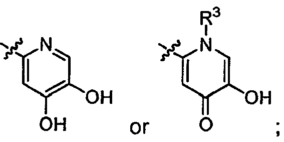 and
R³ is hydrogen, (C₁-C₃)alkyl, or OH.

3. The compound according to claim 1 or 2 wherein E is C(H).

4. The compound according to claim 1, 2, or 3 wherein
A is 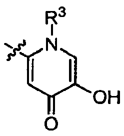 and R³ is OH.

5. The compound according to claim 1, 2, 3, or 4 wherein X is -NH-C(=O)-.

6. The compound according to claim 1, 2, 3, 4, or 5 wherein L is -(CH₂)ₚ-NH-(CH₂)_{q}-; p is 0 and q is 1.

7. The compound according to any of claims 1-6 wherein R¹ and R², together with the carbon atom to which they are attached, form a (C₃-C₆)cycloalkyl.

8. The compound according to any of claims 1-6 in which R¹ and R² are each methyl.

9. The compound according to any of claims 1, 2, 3, 4, or 6-8 wherein X is -O-C(=O)-.

10. The compound according to any of claims 1-5 and 7-9 wherein L is absent, -(CH₂)ₚ-NH-C(=O)-(CH₂)_{q}-, -(CH₂)ₚ-NH-(CH₂)_{q}-, -(CH₂)ₚ-C(=O)-NH-(CH₂)_{q}-, or -(CH₂)ₚ-T-(CH₂)_{q} wherein T is isoxazole, oxazole, thiazole, or pyrimidine;

11. The compound according to any of claims 1-5 and 7-9 wherein L is absent, -(CH₂)ᵣ-T-(CH₂)ₛ, -(CH₂)ₚ-NH-C(=O)-(CH₂)_{q}-, -CH(CH₃)-NH-C(=O)-NH-(CH₂)_{q}-, -CH(CH₃)-NH-C(=O)-(CH₂)_{q}-, -(CH₂)ₚ-C(=O)-NH-(CH₂)_{q}-, or -(CH₂)ₚ-T-C(=O)-NH-(CH₂)_{q}-.

12. A compound according to claim 1 of formula 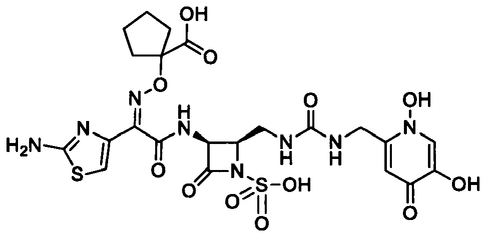 or a pharmaceutically acceptable salt thereof.

13. A compound according to claim 1 of formula 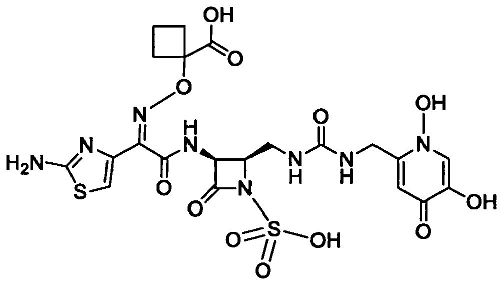 or a pharmaceutically acceptable salt thereof.

14. A compound according to claim 1 of formula 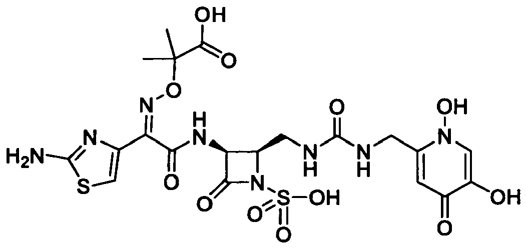 or a pharmaceutically acceptable salt thereof.

15. A pharmaceutical compostion comprising a compound according to any of claims 1-14 in admixture with at least one pharmaceutically acceptable carrier.

16. Use of a compound according to any of claims 1-14 in the manufacture of a medicament for treating bacterial infections.

17. A compound according to any are of claims 1-14 for treating bacterial infections.

## Patentansprüche

1. Verbindung der Formel (I): 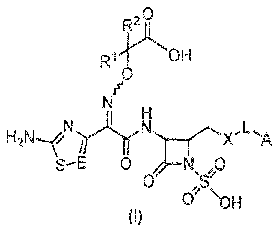 oder ein pharmazeutisch verträgliches Salz davon,
worin
R¹ und R² jeweils unabhängig Wasserstoff, gegebenenfalls substituiertes (C₁-C₆)-Alkyl oder Phenyl-(C₁-C₆)-alkyl sind, wobei die Phenyl- und die (C₁-C₆)-Alkylreste des Phenyl-(C₁-C₆)-alkyls gegebenenfalls substituiert sind; oder
R¹ und R² bilden zusammen mit dem Kohlenstoffatom, an das sie gebunden sind, einen gegebenenfalls substituierten (C₃-C₆)-Cycloalkylrest oder einen gegebenenfalls substituierten 4- bis 6-gliedrigen Heterocyclus;
E C(H), C(F), C(Cl) oder N ist;
X -O-C(=O)-, -NH-C(=O)-, -NH-SO₂-, -NH-C(=N-CN)-, -NH-T-oder Triazol ist;
L fehlt, -(CH₂)ₚ-, -(CH₂)ₚ-NH-(CH₂)_{q}-, -(CH₂)ₚ-O-(CH₂)_{q}-, -(CH₂)ₚ-C(=0) -NH-(CH₂)_{q}-, -(CH₂)ₚ-NH-C(=0) - (CH₂)q-, -CH(CH₃)-NH-C(=O)(CH₂)q-, - (CH₂)ₚ-NH-C(=O)-NH- (CH₂)q-, -CH(CH₃)-NH-C (=0) -NH- (CH₂)_{q}-, -(CH₂)ₚ-T-SO₂-NH- (CH₂)_{q}-, - (CH₂)ₚ-T-(CH₂)q, - (CH₂)ₚ-T-C(=0) -NH- (CH₂)q-, -(CH₂)ₚ-T-(CH₂)_{q}-NH-C(=O)-, -NH-(CH₂)ₚ-T-, -O-(CH₂)ₚ-T-, - (CH₂)ₚ-Y-C(=0) - (CH₂)q- oder - (CH₂)ₚ-Y-(CH₂)q- ist;
T ein gegebenenfalls substituierter Phenylrest oder ein gegebenenfalls substituierter 5- oder 6-gliedriger Hetero-arylrest ist;
Y ein gegebenenfalls substituierter 4- bis 6-gliedriger Heterocyclus ist;
p und q jeweils unabhängig 0, 1, 2 oder 3 sind;
A 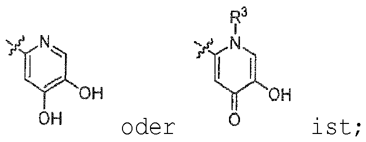 und
R³ Wasserstoff, (C₁-C₃)-Alkyl oder OH ist;
mit der Maßgabe, dass die Formel (I) nicht 2-(((1-(2-Aminothiazol-4-yl)-2-(((2S,3R)-2-((3-((1,5-dihydroxy-4-oxo-1,4-dihydropyridin-2-yl)methyl)ureido)methyl)-4-oxo-1-sulfoazetidin-3-yl)amino)-2-oxoethyliden)amino)oxy)-2-methylpropansäure beinhaltet.

2. Verbindung nach Anspruch 1 der Formel (IA): 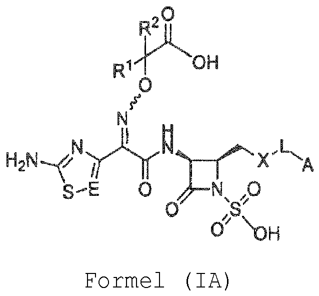 worin
R¹ und R² jeweils unabhängig Wasserstoff, gegebenenfalls substituiertes (C₁-C₆)-Alkyl oder Phenyl-(C₁-C₆)-alkyl sind, wobei die Phenyl- und die (C₁-C₆)-Alkylreste des Phenyl-(C₁-C₆)-alkyls gegebenenfalls substituiert sind; oder
R¹ und R² bilden zusammen mit dem Kohlenstoffatom, an das sie gebunden sind, einen gegebenenfalls substituierten (C₃-C₆)-Cycloalkylrest oder einen gegebenenfalls substituierten 4- bis 6-gliedrigen Heterocyclus;
E C(H), C(F), C(Cl) oder N ist;
X -O-C(=O)-, -NH-C(=O)-, -NH-SO₂-, -NH-C(=N-CN)-, -NH-T-oder Triazol ist;
L fehlt, -(CH₂)ₚ-, -(CH₂)ₚ-NH-(CH₂)q-, -(CH₂)ₚ-O-(CH₂)_{q}-, - (CH₂)ₚ-C(=0) -NH- (CH₂)q-, - (CH₂)ₚ-NH-C(=0) - (CH₂)q-, -CH(CH₃)-NH-C(=O)(CH₂)q-, - (CH₂)ₚ-NH-C(=O)-NH- (CH₂)q-, -CH(CH₃)-NH-C (=0) -NH- (CH₂)q-, - (CH₂)ₚ-T-SO₂-NH-(CH₂)q-, - (CH₂)ₚ-T-(CH₂)q, - (CH₂)ₚ-T-C(=0) -NH- (CH₂)_{q}-, -(CH₂)ₚ-T-(CH₂)_{q}-NH-C(=O)-, -NH-(CH₂)ₚ-T-, -O-(CH₂)ₚ-T-, -(CH₂)ₚ-Y-C(=O)-(CH₂)_{q}- oder --(CH₂)ₚ-Y-(CH₂)_{q}- ist;
T ein gegebenenfalls substituierter Phenylrest oder ein gegebenenfalls substituierter 5- oder 6-gliedriger Hetero-arylrest ist;
Y ein gegebenenfalls substituierter 4- bis 6-gliedriger Heterocyclus ist;
p und q jeweils unabhängig voneinander 0, 1, 2 oder 3 sind;
A 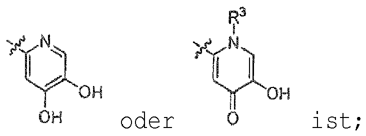 und
R³ Wasserstoff, (C₁-C₃)-Alkyl oder OH ist.

3. Verbindung nach Anspruch 1 oder 2, worin E C(H) ist.

4. Verbindung nach Anspruch 1, 2 oder 3, worin
A 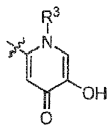 ist und R³ OH ist.

5. Verbindung nach Anspruch 1, 2, 3 oder 4, worin X -NH-C(=O)- ist.

6. Verbindung nach Anspruch 1, 2, 3, 4 oder 5, worin L -(CH₂)ₚ-NH-(CH₂)_{q}- ist; p 0 ist und q 1 ist.

7. Verbindung nach einem der Ansprüche 1-6, worin R¹ und R² zusammen mit dem Kohlenstoffatom, an das sie gebunden sind, einen (C₃-C₆)-Cycloalkylrest bilden.

8. Verbindung nach einem der Ansprüche 1-6, worin R¹ und R² jeweils Methyl sind.

9. Verbindung nach einem der Ansprüche 1, 2, 3, 4 oder 6-8, worin X -0-C(=0)- ist.

10. Verbindung nach einem der Ansprüche 1-5 und 7-9, worin L fehlt, -(CH₂)ₚ-NH-C(=O)-(CH₂)_{q}-, -(CH₂)ₚ-NH-(CH₂)_{q}-, -(CH₂)ₚ-C(=O)-NH-(CH₂)_{q}- oder -(CH₂)ₚ-T-(CH₂)_{q} ist, wobei T Is-oxazol, Oxazol, Thiazol oder Pyrimidin ist.

11. Verbindung nach einem der Ansprüche 1-5 und 7-9, worin L fehlt, (CH₂)ᵣ-T-(CH₂)ₛ, -(CH₂)ₚ-NH-C(=O)-(CH₂)_{q}-, -CH(CH₃)-NH-C(=O)-NH-(CH₂)_{q}-, -CH(CH₃)-NH-C(=O)-(CH₂)_{q}-, -(CH₂)ₚ-C(=O)-NH-(CH₂)_{q}- oder -(CH₂)ₚ-T-C(=O)-NH-(CH₂)_{q}- ist.

12. Verbindung nach Anspruch 1 der Formel 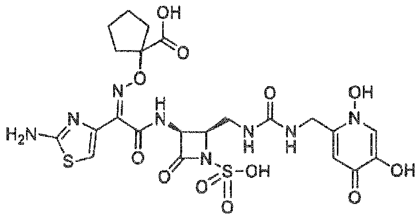 oder ein pharmazeutisch verträgliches Salz davon.

13. Verbindung nach Anspruch 1 der Formel 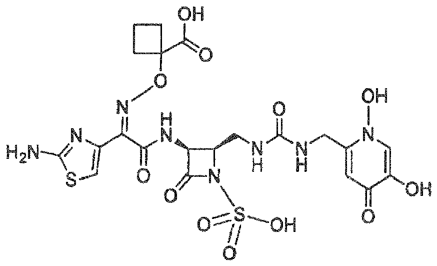 oder ein pharmazeutisch verträgliches Salz davon.

14. Verbindung nach Anspruch 1 der Formel 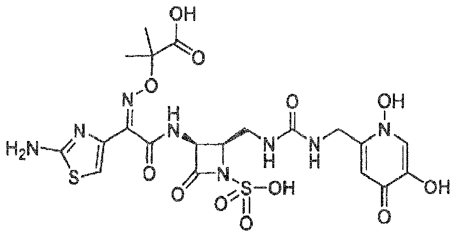 oder ein pharmazeutisch verträgliches Salz davon.

15. Pharmazeutische Zusammensetzung umfassend eine Verbindung nach einem der Ansprüche 1-14 im Gemisch mit mindestens einem pharmazeutisch verträglichen Trägerstoff.

16. Verwendung einer Verbindung nach einem der Ansprüche 1-14 zur Herstellung eines Medikaments zur Behandlung von bakteriellen Infektionen.

17. Verbindung nach einem der Ansprüche 1-14 zur Behandlung von bakteriellen Infektionen.

## Revendications

1. Composé de formule (I) : 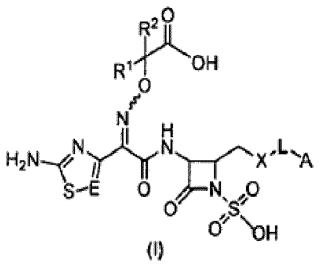 ou sel pharmaceutiquement acceptable de celui-ci,
dans lequel
R¹ et R² représentent chacun indépendamment un atome d'hydrogène, un groupe alkyle en C₁ à C₆ optionnellement substitué ou phénylalkyle en C₁ à C₆ dans lequel les groupements phényle et alkyle en C₁ à C₆ du groupe phénylalkyle en C₁ à C₆ sont optionnellement substitués ; ou
R¹ et R² conjointement, avec l'atome de carbone auquel ils sont fixés, forment un groupe cycloalkyle en C₃ à C₆ optionnellement substitué ou un hétérocycle de 4 à 6 chaînons optionnellement substitué ;
E représente C(H), C(F), C(Cl), ou N ;
X représente un groupe -O-C(=O)-, -NH-C(=O)-, -NH-SO₂-, -NH-C(=N-CN)-, -NH-T-, ou triazole ;
L est absent ou représente un groupe -(CH₂)ₚ-, -(CH₂)ₚ-NH- (CH₂)_{q}-, - (CH₂)ₚ-O- (CH₂)_{q}-, - (CH₂)ₚ-C(=O) -NH- (CH₂)_{q}-, - (CH₂)ₚ-NH-C(=0) - (CH₂)q-, -CH(CH₃)-NH-C(=O)-(CH₂)_{q}-, -(CH₂)ₚ-NH-C (=0) -NH- (CH₂)q-, -CH(CH₃)-NH-C(=O)-NH-(CH₂)_{q}-, -(CH₂)ₚ-T-SO₂-NH- (CH₂)_{q}-, - (CH₂)ₚ-T-(CH₂)_{q}-, - (CH₂)ₚ-T-C(=0) -NH- (CH₂)_{q}-, - (CH₂)ₚ-T-(CH₂)_{q}-NH-C(=O)-, -NH-(CH₂)ₚ-T-, -O-(CH₂)ₚ-T-, - (CH₂)ₚ-Y-C(=O)-(CH₂)q-, ou -(CH₂)ₚ-Y-(CH₂)_{q}- ;
T représente un groupe phényle optionnellement substitué ou hétéroaryle de 5 ou 6 chaînons optionnellement substitué ;
Y représente un hétérocycle de 4 à 6 chaînons optionnellement substitué ;
p et q valent chacun indépendamment 0, 1, 2, ou 3 ;
A représente 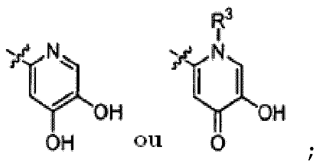 et
R³ représente un atome d'hydrogène, un groupe alkyle en C₁ à C₃, ou OH ;
à condition que la formule (I) ne comprenne pas l'acide 2-(((1-(2-aminothiazol-4-yl)-2-(((2S,3R)-2-((3-((1,5-dihydroxy-4-oxo-1,4-dihydropyridin-2-yl)méthyl)-uréido)méthyl)-4-oxo-1-sulfoazétidin-3-yl)amino)-2-oxo-éthylidène)amino)oxy)-2-méthylpropanoïque.

2. Composé selon la revendication 1 de formule (IA) : 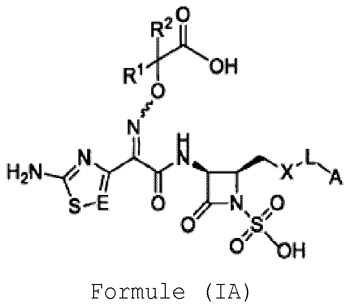 dans laquelle
R¹ et R² représentent chacun indépendamment un atome d'hydrogène, un groupe alkyle en C₁ à C₆ optionnellement substitué ou phénylalkyle en C₁ à C₆ dans lequel les groupements phényle et alkyle en C₁ à C₆ du groupe phénylalkyle en C₁ à C₆ sont optionnellement substitués ; ou
R¹ et R² conjointement, avec l'atome de carbone auquel ils sont fixés, forment un groupe cycloalkyle en C₃ à C₆ optionnellement substitué ou un hétérocycle de 4 à 6 chaînons optionnellement substitué ;
E représente C(H), C(F), C(Cl), ou N ;
X représente un groupe -O-C(=O)-, -NH-C(=O)-, -NH-SO₂-, -NH-C(=N-CN)-, -NH-T-, ou triazole ;
L est absent ou représente un groupe -(CH₂)ₚ-, -(CH₂)ₚ-NH-(CH₂)_{q}-, - (CH₂)ₚ-O-(CH₂)q-, - (CH₂)ₚ-C(=O)-NH-(CH₂)_{q}-, -(CH₂)ₚ-NH-C(=0) - (CH₂)_{q}-, -CH(CH₃)-NH-C(=O)-(CH₂)_{q}-, -(CH₂)ₚ-NH-C (=0) -NH- (CH₂)q-, -CH(CH₃)-NH-C(=O)-NH-(CH₂)_{q}-, -(CH₂)ₚ-T-SO₂-NH- (CH₂)q-, - (CH₂)ₚ-T-(CH₂)q-, - (CH₂)ₚ-T-C(=0) -NH- (CH₂)q-, - (CH₂)ₚ-T-(CH₂)_{q}-NH-C(=O)-, -NH-(CH₂)ₚ-T-, -O-(CH₂)ₚ-T-, - (CH₂)ₚ-Y-C(=O)-(CH₂)q-, ou -(CH₂)ₚ-Y-(CH₂)_{q}- ;
T représente un groupe phényle optionnellement substitué ou hétéroaryle de 5 ou 6 chaînons optionnellement substitué ;
Y représente un hétérocycle de 4 à 6 chaînons optionnellement substitué ;
p et q valent chacun indépendamment 0, 1, 2, ou 3 ;
A représente 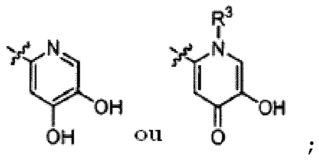 et
R³ représente un atome d'hydrogène, un groupe alkyle en C₁ à C₃, ou OH.

3. Composé selon la revendication 1 ou 2, dans lequel E représente C(H).

4. Composé selon la revendication 1, 2, ou 3, dans lequel
A représente 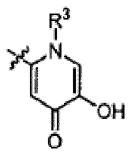 et R³ représente OH.

5. Composé selon la revendication 1, 2, 3, ou 4, dans lequel X représente un groupe -NH-C(=O)-.

6. Composé selon la revendication 1, 2, 3, 4, ou 5, dans lequel L représente un groupe -(CH₂)ₚ-NH-(CH₂)_{q}- ; p vaut 0 et q vaut 1.

7. Composé selon l'une quelconque des revendications 1 à 6, dans lequel R¹ et R² conjointement, avec l'atome de carbone auquel ils sont fixés, forment un groupe cycloalkyle en C₃ à C₆.

8. Composé selon l'une quelconque des revendications 1 à 6, dans lequel R¹ et R² représentent chacun un groupe méthyle.

9. Composé selon l'une quelconque des revendications 1, 2, 3, 4, ou 6 à 8, dans lequel X représente un groupe -O-C(=O)-.

10. Composé selon l'une quelconque des revendications 1 à 5 et 7 à 9, dans lequel L est absent ou représente -(CH₂)ₚ-NH-C(=0) - (CH₂)_{q}-, - (CH₂)ₚ-NH-(CH₂)_{q}-, -(CH₂)ₚ-C(=O)-NH-(CH₂)_{q}-, ou -(CH₂)ₚ-T-(CH₂)_{q}- où T représente un groupe isoxazole, oxazole, thiazole, ou pyrimidine.

11. Composé selon l'une quelconque des revendications 1 à 5 et 7 à 9, dans lequel L est absent ou représente un groupe -(CH₂)ᵣ-T-(CH₂)ₛ-, - (CH₂)ₚ-NH-C(=O)-(CH₂)_{q}-, -CH(CH₃)-NH-C(=O)-NH-(CH₂)_{q}-, -CH(CH₃)-NH-C(=O)-(CH₂)_{q}-, - (CH₂)ₚ-C(=0) -NH- (CH₂)_{q}-, ou -(CH₂)ₚ-T-C(=O)-NH-(CH₂)_{q}-.

12. Composé selon la revendication 1 de formule 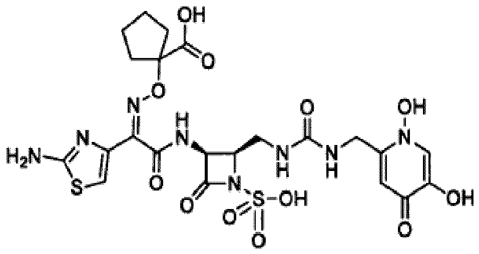 ou sel pharmaceutiquement acceptable de celui-ci.

13. Composé selon la revendication 1 de formule 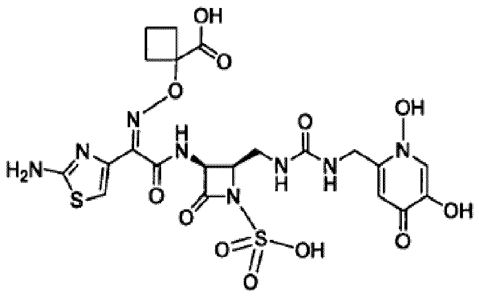 ou sel pharmaceutiquement acceptable de celui-ci.

14. Composé selon la revendication 1 de formule 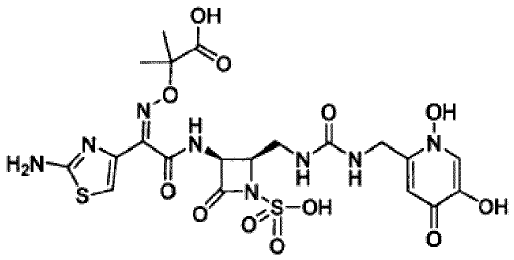 ou sel pharmaceutiquement acceptable de celui-ci.

15. Composition pharmaceutique comprenant un composé selon l'une quelconque des revendications 1 à 14 en mélange avec au moins un support pharmaceutiquement acceptable.

16. Utilisation d'un composé selon l'une quelconque des revendications 1 à 14 dans la fabrication d'un médicament pour le traitement d'infections bactériennes.

17. Composé selon l'une quelconque des revendications 1 à 14 pour le traitement d'infections bactériennes.
